# EUROPEAN PATENT APPLICATION

(11) **EP 2 270 498 A2**
(43) Date of publication of application: **05.01.2011**
(21) Application number: 10010312.6
(22) Date of filing: 22.09.2003
(51) Int. Cl.: G01N 33/50

(54) **Method for diagnosing non-small cell lung cancers**

(30) Priority: 30.09.2002 US 414673 P; 28.02.2003 US 451374 P; 28.04.2003 US 466100 P
(62) Divisional of application: 06022167.8
(71) Applicant: Oncotherapy Science, Inc., Kawasaki-shi Kanagawa 213-0012 (JP)
(72) Inventor: Nakamura, Yusuke, Yokohama-shi Kanagawa 225-0011 (JP); Daigo, Yataro, Yokohama-shi Kanagawa 222-0031 (JP); Nakatsuru, Shuichi, Saitama-shi Saitama 338-0002 (JP)
(74) Representative: Vossius & Partner

(57) **Abstract**

Disclosed are methods for detecting non-small cell lung cancer using differentially expressed genes. Furthermore, novel human genes whose expression is elevated in non-small cell lung cancer compared to no-cancerous tissues are provided. Also disclosed are methods of identifying compounds for treating and preventing non-small cell lung cancer.

SEQUENCE LISTING

<110> ONCOTHERAPY SCIENCE, INC.

<120> METHOD FOR DIAGNOSING NON-SMALL CELL LUNG CANCERS

<130> L1452 EP/8 S3

<150> US 60/414,673
<151> 2002-09-30

<150> US 60/451,374
<151> 2003-02-28

<150> US 60/466,100
<151> 2003-04-28

<160> 552

<170> PatentIn version 3.1

<210> 1
<211> 2235
<212> DNA
<213> Homo sapiens

<400> 1
ggcacgaggc ggcggaggcg gcggcggcgg cggcgatggc agcggaccct gagcgagctt 60

gagggctcgg acccagctcc ctcccgcgaa accttgggcg gatccggcgc tgcggcccca 120

gctcgctccg ctcctgctcc ctccccggcc gctgcctggg cggaggcaga ggcagaggcc 180

cgggctggcc gccctgctcg tgccccagct cggccccgga cggcccggct gctgtgcaga 240

gaggaggccg agtcggtagt gaaaagagaa tactgaagaa taggatctca agatgagtaa 300

aaagccccca aatcgccctg gaatcacttt tgagattggt gctcgtttgg aggcactgga 360

ctacttacaa aaatggtatc catcacgaat tgaaaaaatt gactatgagg agggcaagat 420

gttggtccat tttgagcgct ggagtcatcg ttatgatgag tggatttact gggatagcaa 480

tagattgcga ccccttgaga gaccagcact aagaaaagaa gggctaaaag atgaggaaga 540

tttctttgat tttaaagctg gagaagaagt tctggctcgt tggacagact gtcgctatta 600

ccctgccaag attgaagcaa ttaacaaaga aggaacattt acagttcagt tttatgatgg 660

agtaattcgt tgtttaaaaa gaatgcacat taaagccatg cccgaggatg ctaaggggca 720

ggtgaaatcc cagcatccac taagctggtg ttgtcctatc gacccagctg gatcgtgtaa 780

ccagtctatg ggaagtgagg attggatagc tttagtcaaa gcagctgctg cagctgcagc 840

caagaacaaa acagggagta aacctcgaac cagcgctaac agcaataaag ataaggataa 900

agatgagaga aagtggttta aagtaccttc aaagaaggag gaaacttcaa cttgtatagc 960

cacaccagac gtagagaaga aggaagatct gcctacatct agtgaaacat ttggacttca 1020

tgtagagaac gttccaaaga tggtctttcc acagccagag agcacattat caaacaagag 1080

gaaaaataat caaggcaact cgtttcaggc aaagagagct cgacttaaca agattactgg 1140

tttgttggca tccaaagctg ttggggttga tggtgctgaa aaaaaggaag actacaatga 1200

aacagctcca atgctggagc aggcgatttc acctaaacct caaagtcaga aaaaaaatga 1260

agctgacatt agcagttctg ccaacactca gaaacctgca ctgttatcct caactttgtc 1320

ttcagggaag gctcgcagca agaaatgcaa acatgaatct ggagattctt ctgggtgtat 1380

aaaaccccct aaatcaccac tttccccaga attaatacaa gtcgaggatt tgacgcttgt 1440

atctcagctt tcttcttcag tgataaataa aactagtcct ccacagcctg tgaatccccc 1500

tagacctttc aagcatagtg agcggagaag aagatctcag cgtttagcca ccttacccat 1560

gcctgatgat tctgtagaaa aggtttcttc tccctctcca gccactgatg ggaaagtatt 1620

ctccatcagt tctcaaaatc agcaagaatc ttcagtacca gaggtgcctg atgttgcaca 1680

tttgccactt gagaagctgg gaccctgtct ccctcttgac ttaagtcgtg gttcagaagt 1740

tacagcaccg gtagcctcag attcctctta ccgtaatgaa tgtcccaggg cagaaaaaga 1800

ggatacacag atgcttccaa atccttcttc caaagcaata gctgatggaa gaggagctcc 1860

agcagcagca ggaatatcga aaacagaaaa aaaagtgaaa ttggaagaca aaagctcaac 1920

agcatttggt atcaggagtt gggatttctc agcactgcta atgaagatcc cctcttatag 1980

tccaataagc ttatcaggac ttccagagtc atgacatgaa cagtttaatt gaacccatcc 2040

actctgggca ggtgactgga atagctgatt aaaacataaa tgctgctttt aggttaacca 2100

caaaggaaca actcaggatc agtcgtgatt gctaaagtat tcacttttat cttttaggta 2160

agagaaaaga aaaagataag gaaagaagag agaagagaga caaagatcac tacagaccaa 2220

aacagaagaa gaaga 2235

<210> 2
<211> 573
<212> PRT
<213> Homo sapiens

<400> 2
Met Ser Lys Lys Pro Pro Asn Arg Pro Gly Ile Thr Phe Glu Ile Gly
1               5                   10                  15

Ala Arg Leu Glu Ala Leu Asp Tyr Leu Gln Lys Trp Tyr Pro Ser Arg
            20                  25                  30

Ile Glu Lys Ile Asp Tyr Glu Glu Gly Lys Met Leu Val His Phe Glu
        35                  40                  45

Arg Trp Ser His Arg Tyr Asp Glu Trp Ile Tyr Trp Asp Ser Asn Arg
    50                  55                  60

Leu Arg Pro Leu Glu Arg Pro Ala Leu Arg Lys Glu Gly Leu Lys Asp
65                  70                  75                  80

Glu Glu Asp Phe Phe Asp Phe Lys Ala Gly Glu Glu Val Leu Ala Arg

85                  90                  95

Trp Thr Asp Cys Arg Tyr Tyr Pro Ala Lys Ile Glu Ala Ile Asn Lys
            100                 105                 110

Glu Gly Thr Phe Thr Val Gln Phe Tyr Asp Gly Val Ile Arg Cys Leu
        115                 120                 125

Lys Arg Met His Ile Lys Ala Met Pro Glu Asp Ala Lys Gly Gln Val
    130                 135                 140

Lys Ser Gln His Pro Leu Ser Trp Cys Cys Pro Ile Asp Pro Ala Gly
145                 150                 155                 160

Ser Cys Asn Gln Ser Met Gly Ser Glu Asp Trp Ile Ala Leu Val Lys
                165                 170                 175

Ala Ala Ala Ala Ala Ala Ala Lys Asn Lys Thr Gly Ser Lys Pro Arg
            180                 185                 190

Thr Ser Ala Asn Ser Asn Lys Asp Lys Asp Lys Asp Glu Arg Lys Trp
        195                 200                 205

Phe Lys Val Pro Ser Lys Lys Glu Glu Thr Ser Thr Cys Ile Ala Thr
    210                 215                 220

Pro Asp Val Glu Lys Lys Glu Asp Leu Pro Thr Ser Ser Glu Thr Phe
225                 230                 235                 240

Gly Leu His Val Glu Asn Val Pro Lys Met Val Phe Pro Gln Pro Glu
                245                 250                 255

Ser Thr Leu Ser Asn Lys Arg Lys Asn Asn Gln Gly Asn Ser Phe Gln
            260                 265                 270

Ala Lys Arg Ala Arg Leu Asn Lys Ile Thr Gly Leu Leu Ala Ser Lys
        275                 280                 285

Ala Val Gly Val Asp Gly Ala Glu Lys Lys Glu Asp Tyr Asn Glu Thr
    290                 295                 300

Ala Pro Met Leu Glu Gln Ala Ile Ser Pro Lys Pro Gln Ser Gln Lys
305                 310                 315                 320

Lys Asn Glu Ala Asp Ile Ser Ser Ser Ala Asn Thr Gln Lys Pro Ala
                325                 330                 335

Leu Leu Ser Ser Thr Leu Ser Ser Gly Lys Ala Arg Ser Lys Lys Cys
            340                 345                 350

Lys His Glu Ser Gly Asp Ser Ser Gly Cys Ile Lys Pro Pro Lys Ser
        355                 360                 365

Pro Leu Ser Pro Glu Leu Ile Gln Val Glu Asp Leu Thr Leu Val Ser
    370                 375                 380

Gln Leu Ser Ser Ser Val Ile Asn Lys Thr Ser Pro Pro Gln Pro Val
385                 390                 395                 400

Asn Pro Pro Arg Pro Phe Lys His Ser Glu Arg Arg Arg Arg Ser Gln
                405                 410                 415

Arg Leu Ala Thr Leu Pro Met Pro Asp Asp Ser Val Glu Lys Val Ser

            420                 425                 430

Ser Pro Ser Pro Ala Thr Asp Gly Lys Val Phe Ser Ile Ser Ser Gln
        435                 440                 445

Asn Gln Gln Glu Ser Ser Val Pro Glu Val Pro Asp Val Ala His Leu
    450                 455                 460

Pro Leu Glu Lys Leu Gly Pro Cys Leu Pro Leu Asp Leu Ser Arg Gly
465                 470                 475                 480

Ser Glu Val Thr Ala Pro Val Ala Ser Asp Ser Ser Tyr Arg Asn Glu
                485                 490                 495

Cys Pro Arg Ala Glu Lys Glu Asp Thr Gln Met Leu Pro Asn Pro Ser
            500                 505                 510

Ser Lys Ala Ile Ala Asp Gly Arg Gly Ala Pro Ala Ala Ala Gly Ile
        515                 520                 525

Ser Lys Thr Glu Lys Lys Val Lys Leu Glu Asp Lys Ser Ser Thr Ala
    530                 535                 540

Phe Gly Ile Arg Ser Trp Asp Phe Ser Ala Leu Leu Met Lys Ile Pro
545                 550                 555                 560

Ser Tyr Ser Pro Ile Ser Leu Ser Gly Leu Pro Glu Ser
                565                 570

<210> 3
<211> 23
<212> DNA
<213> Artificial

<220>
<223> Artificially synthesized primer sequence for RT-PCR

<400> 3
taaatggctt caggagactt cag 23

<210> 4
<211> 24
<212> DNA
<213> Artificial

<220>
<223> Artificially synthesized primer sequence for RT-PCR

<400> 4
ggttttaaat gcagctccta tgtg 24

<210> 5
<211> 34
<212> DNA
<213> Artificial

<220>
<223> Artificially synthesized primer sequence for RT-PCR

<400> 5

atggaatccg aggatttaag tggcagagaa ttga 34

<210> 6
<211> 37
<212> DNA
<213> Artificial

<220>
<223> Artificially synthesized primer sequence for RT-PCR

<400> 6
tttttttccc cttttttttt caaaagtctt ggaggat 37

<210> 7
<211> 20
<212> DNA
<213> Artificial

<220>
<223> Artificially synthesized primer sequence for RT-PCR

<400> 7
gcttcctcct ggaaattgac 20

<210> 8
<211> 23
<212> DNA
<213> Artificial

<220>
<223> Artificially synthesized primer sequence for RT-PCR

<400> 8
tctactgtac agggaaaaac cca 23

<210> 9
<211> 23
<212> DNA
<213> Artificial

<220>
<223> Artificially synthesized primer sequence for RT-PCR

<400> 9
agtcgtggtt cagaagttac agc 23

<210> 10
<211> 23
<212> DNA
<213> Artificial

<220>
<223> Artificially synthesized primer sequence for RT-PCR

<400> 10
tctcttacca aatgctgttg agc 23

<210>  11
<211>  23
<212>  DNA
<213>  Artificial

<220>
<223>  Artificially synthesized primer sequence for RT-PCR

<400>  11
catctggcat ttctgctctc tat                                              23


<210>  12
<211>  25
<212>  DNA
<213>  Artificial

<220>
<223>  Artificially synthesized primer sequence for RT-PCR

<400>  12
ctcagggaaa ggagaataaa agaac                                            25


<210>  13
<211>  23
<212>  DNA
<213>  Artificial

<220>
<223>  Artificially synthesized primer sequence for RT-PCR

<400>  13
gaagtatcaa aactccgctg tca                                              23


<210>  14
<211>  23
<212>  DNA
<213>  Artificial

<220>
<223>  Artificially synthesized primer sequence for RT-PCR

<400>  14
atgctgagta gacatgcaga tga                                              23


<210>  15
<211>  23
<212>  DNA
<213>  Artificial

<220>
<223>  Artificially synthesized primer sequence for RT-PCR

<400>  15
cggtatgcta atgaagatgg aga                                              23


<210>  16
<211>  23
<212>  DNA

<213>  Artificial

<220>
<223>  Artificially synthesized primer sequence for RT-PCR

<400>  16
cacagggtat cagcaactgt gta                                              23


<210>  17
<211>  22
<212>  DNA
<213>  Artificial

<220>
<223>  Artificially synthesized primer sequence for RT-PCR

<400>  17
agaagtatct gagcccctga tg                                               22


<210>  18
<211>  21
<212>  DNA
<213>  Artificial

<220>
<223>  Artificially synthesized primer sequence for RT-PCR

<400>  18
gtctaacctc ccagctgttc c                                                21


<210>  19
<211>  23
<212>  DNA
<213>  Artificial

<220>
<223>  Artificially synthesized primer sequence for RT-PCR

<400>  19
gctgcgtagc ttacagactt agc                                              23


<210>  20
<211>  23
<212>  DNA
<213>  Artificial

<220>
<223>  Artificially synthesized primer sequence for RT-PCR

<400>  20
aaggcgttta aaggtgatag gtg                                              23


<210>  21
<211>  23
<212>  DNA
<213>  Artificial

<220>

<223>  Artificially synthesized primer sequence for RT-PCR

<400>  21
gtttgcaacc aggagataca aag                                              23


<210>  22
<211>  23
<212>  DNA
<213>  Artificial

<220>
<223>  Artificially synthesized primer sequence for RT-PCR

<400>  22
gctgtgaggt acaacaaatc aca                                              23


<210>  23
<211>  23
<212>  DNA
<213>  Artificial

<220>
<223>  Artificially synthesized primer sequence for RT-PCR

<400>  23
cctcctttcc ctagagactc aat                                              23


<210>  24
<211>  23
<212>  DNA
<213>  Artificial

<220>
<223>  Artificially synthesized primer sequence for RT-PCR

<400>  24
agaagcaaca gcaagaccac tac                                              23


<210>  25
<211>  23
<212>  DNA
<213>  Artificial

<220>
<223>  Artificially synthesized primer sequence for RT-PCR

<400>  25
ttgcctatga aagataggtc ctg                                              23


<210>  26
<211>  23
<212>  DNA
<213>  Artificial

<220>
<223>  Artificially synthesized primer sequence for RT-PCR

<400>  26

gttttaatgc ccagatagca cag 23

<210> 27
<211> 21
<212> DNA
<213> Artificial

<220>
<223> Artificially synthesized primer sequence for RT-PCR

<400> 27
aggagaagtt ggaggtggaa a 21

<210> 28
<211> 23
<212> DNA
<213> Artificial

<220>
<223> Artificially synthesized primer sequence for RT-PCR

<400> 28
cagatgaaag atccaaattc caa 23

<210> 29
<211> 23
<212> DNA
<213> Artificial

<220>
<223> Artificially synthesized primer sequence for RT-PCR

<400> 29
tccacgactt cttattctcc ttg 23

<210> 30
<211> 23
<212> DNA
<213> Artificial

<220>
<223> Artificially synthesized primer sequence for RT-PCR

<400> 30
catttctttt agggactggg gta 23

<210> 31
<211> 23
<212> DNA
<213> Artificial

<220>
<223> Artificially synthesized primer sequence for RT-PCR

<400> 31
gagaaactga agtcccagga aat 23

<210>  32
<211>  23
<212>  DNA
<213>  Artificial

<220>
<223>  Artificially synthesized primer sequence for RT-PCR

<400>  32
ctgatacttc cattcgcttc aac                                              23


<210>  33
<211>  20
<212>  DNA
<213>  Artificial

<220>
<223>  Artificially synthesized primer sequence for RT-PCR

<400>  33
agctaagcca tgaggtaggg                                                  20


<210>  34
<211>  21
<212>  DNA
<213>  Artificial

<220>
<223>  Artificially synthesized primer sequence for RT-PCR

<400>  34
cgcatgtgtg ttcttctatg a                                                21


<210>  35
<211>  23
<212>  DNA
<213>  Artificial

<220>
<223>  Artificially synthesized primer sequence for RT-PCR

<400>  35
ccaagacagg cagagtaggt aaa                                              23


<210>  36
<211>  22
<212>  DNA
<213>  Artificial

<220>
<223>  Artificially synthesized primer sequence for RT-PCR

<400>  36
cattttcatt gtgatcagcc ag                                               22


<210>  37
<211>  23
<212>  DNA

<213>  Artificial

<220>
<223>  Artificially synthesized primer sequence for RT-PCR

<400>  37
tgtatggggg attacctaca cac                                              23


<210>  38
<211>  21
<212>  DNA
<213>  Artificial

<220>
<223>  Artificially synthesized primer sequence for RT-PCR

<400>  38
aaaggagcac aacaaacatg c                                                21


<210>  39
<211>  23
<212>  DNA
<213>  Artificial

<220>
<223>  Artificially synthesized primer sequence for RT-PCR

<400>  39
tgtccaagga gtctgaagtt ctc                                              23


<210>  40
<211>  23
<212>  DNA
<213>  Artificial

<220>
<223>  Artificially synthesized primer sequence for RT-PCR

<400>  40
cttgccacca tacctttatt ctg                                              23


<210>  41
<211>  23
<212>  DNA
<213>  Artificial

<220>
<223>  Artificially synthesized primer sequence for RT-PCR

<400>  41
cgagagagta ggagttgagg tga                                              23


<210>  42
<211>  23
<212>  DNA
<213>  Artificial

<220>

<223>  Artificially synthesized primer sequence for RT-PCR

<400>  42
cagaaatcca gcagatttca gac                                              23


<210>  43
<211>  20
<212>  DNA
<213>  Artificial

<220>
<223>  Artificially synthesized primer sequence for RT-PCR

<400>  43
gaacaggtgg ctgtgttcct                                                  20


<210>  44
<211>  23
<212>  DNA
<213>  Artificial

<220>
<223>  Artificially synthesized primer sequence for RT-PCR

<400>  44
atagaatcaa gtggtgtgct tcg                                              23


<210>  45
<211>  23
<212>  DNA
<213>  Artificial

<220>
<223>  Artificially synthesized primer sequence for RT-PCR

<400>  45
ctgagacttt gagtccttgg gag                                              23


<210>  46
<211>  23
<212>  DNA
<213>  Artificial

<220>
<223>  Artificially synthesized primer sequence for RT-PCR

<400>  46
ttcctcattt ctctcagtaa ccg                                              23


<210>  47
<211>  23
<212>  DNA
<213>  Artificial

<220>
<223>  Artificially synthesized primer sequence for RT-PCR

<400>  47

aacaatgcaa agtagtgctc ctc                                                23

<210>  48
<211>  23
<212>  DNA
<213>  Artificial

<220>
<223>  Artificially synthesized primer sequence for RT-PCR

<400>  48
gctgaacttc tttatgctct tcg                                                23

<210>  49
<211>  23
<212>  DNA
<213>  Artificial

<220>
<223>  Artificially synthesized primer sequence for RT-PCR

<400>  49
acctttgatt ttagactgag ggc                                                23

<210>  50
<211>  23
<212>  DNA
<213>  Artificial

<220>
<223>  Artificially synthesized primer sequence for RT-PCR

<400>  50
acactgggtt gtgtgttatt tcc                                                23

<210>  51
<211>  23
<212>  DNA
<213>  Artificial

<220>
<223>  Artificially synthesized primer sequence for RT-PCR

<400>  51
atgagcctct catccatgtc ttt                                                23

<210>  52
<211>  23
<212>  DNA
<213>  Artificial

<220>
<223>  Artificially synthesized primer sequence for RT-PCR

<400>  52
agtaagagtc tgcctgagac acg                                                23

<210>  53
<211>  21
<212>  DNA
<213>  Artificial

<220>
<223>  Artificially synthesized primer sequence for RT-PCR

<400>  53
agaaaatggg ggtgcaagta g                                                21


<210>  54
<211>  22
<212>  DNA
<213>  Artificial

<220>
<223>  Artificially synthesized primer sequence for RT-PCR

<400>  54
taaccaaatt aacacgtgct gg                                               22


<210>  55
<211>  23
<212>  DNA
<213>  Artificial

<220>
<223>  Artificially synthesized primer sequence for RT-PCR

<400>  55
agaaaagttg gagaagatga ggg                                              23


<210>  56
<211>  23
<212>  DNA
<213>  Artificial

<220>
<223>  Artificially synthesized primer sequence for RT-PCR

<400>  56
gccacctctg tgagagagtc taa                                              23


<210>  57
<211>  21
<212>  DNA
<213>  Artificial

<220>
<223>  Artificially synthesized primer sequence for RT-PCR

<400>  57
agaactagtg tgaccccacc c                                                21


<210>  58
<211>  22
<212>  DNA

<213>  Artificial

<220>
<223>  Artificially synthesized primer sequence for RT-PCR

<400>  58
gcttgccttt tcccttagta gg                                              22


<210>  59
<211>  23
<212>  DNA
<213>  Artificial

<220>
<223>  Artificially synthesized primer sequence for RT-PCR

<400>  59
agggaaatga agacaggaga act                                             23


<210>  60
<211>  23
<212>  DNA
<213>  Artificial

<220>
<223>  Artificially synthesized primer sequence for RT-PCR

<400>  60
gagacacggc ttaagaagtt ttg                                             23


<210>  61
<211>  23
<212>  DNA
<213>  Artificial

<220>
<223>  Artificially synthesized primer sequence for RT-PCR

<400>  61
gcttgtaaag tcctcggaaa gtt                                             23


<210>  62
<211>  23
<212>  DNA
<213>  Artificial

<220>
<223>  Artificially synthesized primer sequence for RT-PCR

<400>  62
atctcaactc tgcatcatct ggt                                             23


<210>  63
<211>  22
<212>  DNA
<213>  Artificial

<220>

<223>  Artificially synthesized primer sequence for RT-PCR

<400>  63
ataagagaaa tattggccat cg                                                22


<210>  64
<211>  23
<212>  DNA
<213>  Artificial

<220>
<223>  Artificially synthesized primer sequence for RT-PCR

<400>  64
gcaagcgtaa gagactggtt tta                                               23


<210>  65
<211>  23
<212>  DNA
<213>  Artificial

<220>
<223>  Artificially synthesized primer sequence for RT-PCR

<400>  65
caaatattag gtggagccaa cac                                               23


<210>  66
<211>  23
<212>  DNA
<213>  Artificial

<220>
<223>  Artificially synthesized primer sequence for RT-PCR

<400>  66
tagatcacct tggcaaagaa cac                                               23


<210>  67
<211>  22
<212>  DNA
<213>  Artificial

<220>
<223>  Artificially synthesized primer sequence for RT-PCR

<400>  67
acacacagag aggaggaagt ct                                                22


<210>  68
<211>  22
<212>  DNA
<213>  Artificial

<220>
<223>  Artificially synthesized primer sequence for RT-PCR

<400>  68

gagtctttat ggagctgtgt ca                                                22

<210>  69
<211>  21
<212>  DNA
<213>  Artificial

<220>
<223>  Artificially synthesized primer sequence for RT-PCR

<400>  69
caggccaagt gattttaatg g                                                 21

<210>  70
<211>  22
<212>  DNA
<213>  Artificial

<220>
<223>  Artificially synthesized primer sequence for RT-PCR

<400>  70
caatacagga tgcaagttcc aa                                                22

<210>  71
<211>  23
<212>  DNA
<213>  Artificial

<220>
<223>  Artificially synthesized primer sequence for RT-PCR

<400>  71
acagcccaga cacaaacaaa tac                                               23

<210>  72
<211>  20
<212>  DNA
<213>  Artificial

<220>
<223>  Artificially synthesized primer sequence for RT-PCR

<400>  72
accccattct ctccacagac                                                   20

<210>  73
<211>  23
<212>  DNA
<213>  Artificial

<220>
<223>  Artificially synthesized primer sequence for RT-PCR

<400>  73
tacaggccag gatagaaaca ctc                                               23

<210>  74
<211>  22
<212>  DNA
<213>  Artificial

<220>
<223>  Artificially synthesized primer sequence for RT-PCR

<400>  74
gttcaaatat tgaaagggcc ac                                                22


<210>  75
<211>  23
<212>  DNA
<213>  Artificial

<220>
<223>  Artificially synthesized primer sequence for RT-PCR

<400>  75
agttatgggt tcctgtgtgc tta                                               23


<210>  76
<211>  23
<212>  DNA
<213>  Artificial

<220>
<223>  Artificially synthesized primer sequence for RT-PCR

<400>  76
aaaggcctgt tcacaagcta agt                                               23


<210>  77
<211>  21
<212>  DNA
<213>  Artificial

<220>
<223>  Artificially synthesized primer sequence for RT-PCR

<400>  77
ctcgtgaagc ctcagatgtc c                                                 21


<210>  78
<211>  21
<212>  DNA
<213>  Artificial

<220>
<223>  Artificially synthesized primer sequence for RT-PCR

<400>  78
ctccaccgaa aagacccatt c                                                 21


<210>  79
<211>  21
<212>  DNA

<213>  Artificial

<220>
<223>  Artificially synthesized primer sequence for RT-PCR

<400>  79
agcgtacacc ctctgcactt g                                                21


<210>  80
<211>  25
<212>  DNA
<213>  Artificial

<220>
<223>  Artificially synthesized primer sequence for RT-PCR

<400>  80
tttgctgtat ggtatgtact caagg                                            25


<210>  81
<211>  23
<212>  DNA
<213>  Artificial

<220>
<223>  Artificially synthesized primer sequence for RT-PCR

<400>  81
cagaagagag aggagagaac acg                                              23


<210>  82
<211>  23
<212>  DNA
<213>  Artificial

<220>
<223>  Artificially synthesized primer sequence for RT-PCR

<400>  82
gaggttatct ctgatggaac caa                                              23


<210>  83
<211>  23
<212>  DNA
<213>  Artificial

<220>
<223>  Artificially synthesized primer sequence for RT-PCR

<400>  83
cttgaagaag aacttccaga cga                                              23


<210>  84
<211>  23
<212>  DNA
<213>  Artificial

<220>

<223>  Artificially synthesized primer sequence for RT-PCR

<400>  84
aatgttctaa agatgagagg ggg                                              23


<210>  85
<211>  23
<212>  DNA
<213>  Artificial

<220>
<223>  Artificially synthesized primer sequence for RT-PCR

<400>  85
aggaggctgc tggtacaaat act                                              23


<210>  86
<211>  23
<212>  DNA
<213>  Artificial

<220>
<223>  Artificially synthesized primer sequence for RT-PCR

<400>  86
gcaggaaata cagcaggaac ata                                              23


<210>  87
<211>  22
<212>  DNA
<213>  Artificial

<220>
<223>  Artificially synthesized primer sequence for RT-PCR

<400>  87
attcattctg gaccaaagat cc                                               22


<210>  88
<211>  23
<212>  DNA
<213>  Artificial

<220>
<223>  Artificially synthesized primer sequence for RT-PCR

<400>  88
tctactgtgg acaagaagcc tgt                                              23


<210>  89
<211>  23
<212>  DNA
<213>  Artificial

<220>
<223>  Artificially synthesized primer sequence for RT-PCR

<400>  89

gtgatctctt caaggtcaac tgc                                              23

<210>  90
<211>  23
<212>  DNA
<213>  Artificial

<220>
<223>  Artificially synthesized primer sequence for RT-PCR

<400>  90
ccagatgaga tgataaggca aag                                              23

<210>  91
<211>  22
<212>  DNA
<213>  Artificial

<220>
<223>  Artificially synthesized primer sequence for RT-PCR

<400>  91
attcgctact gcaatttaga gg                                               22

<210>  92
<211>  23
<212>  DNA
<213>  Artificial

<220>
<223>  Artificially synthesized primer sequence for RT-PCR

<400>  92
gtttaatgca acaggtgaca acg                                              23

<210>  93
<211>  23
<212>  DNA
<213>  Artificial

<220>
<223>  Artificially synthesized primer sequence for RT-PCR

<400>  93
cacttggatt ccttgcttgt tac                                              23

<210>  94
<211>  23
<212>  DNA
<213>  Artificial

<220>
<223>  Artificially synthesized primer sequence for RT-PCR

<400>  94
gggaaaaagt atgcaacact cag                                              23

<210>  95
<211>  22
<212>  DNA
<213>  Artificial

<220>
<223>  Artificially synthesized primer sequence for RT-PCR

<400>  95
aggcgatgac ctgaaggtac tg                                                22


<210>  96
<211>  22
<212>  DNA
<213>  Artificial

<220>
<223>  Artificially synthesized primer sequence for RT-PCR

<400>  96
caataggcca gcaatctcaa ta                                                22


<210>  97
<211>  23
<212>  DNA
<213>  Artificial

<220>
<223>  Artificially synthesized primer sequence for RT-PCR

<400>  97
aggttctgat ccgtttccat atc                                               23


<210>  98
<211>  23
<212>  DNA
<213>  Artificial

<220>
<223>  Artificially synthesized primer sequence for RT-PCR

<400>  98
atctttacat cctcagtgtt ggc                                               23


<210>  99
<211>  22
<212>  DNA
<213>  Artificial

<220>
<223>  Artificially synthesized primer sequence for RT-PCR

<400>  99
gaagacaaat ggtgtccaca aa                                                22


<210>  100
<211>  23
<212>  DNA

<213>  Artificial

<220>
<223>  Artificially synthesized primer sequence for RT-PCR

<400>  100
ccactggaag ttttcttcgt aca                                              23


<210>  101
<211>  23
<212>  DNA
<213>  Artificial

<220>
<223>  Artificially synthesized primer sequence for RT-PCR

<400>  101
ttcgttctct cctctcctct ctt                                              23


<210>  102
<211>  23
<212>  DNA
<213>  Artificial

<220>
<223>  Artificially synthesized primer sequence for RT-PCR

<400>  102
ggcagcagta caacaatcta agc                                              23


<210>  103
<211>  23
<212>  DNA
<213>  Artificial

<220>
<223>  Artificially synthesized primer sequence for RT-PCR

<400>  103
cagcacagag taggtgaaca cag                                              23


<210>  104
<211>  23
<212>  DNA
<213>  Artificial

<220>
<223>  Artificially synthesized primer sequence for RT-PCR

<400>  104
cctcagtaca ttttcaaccc atc                                              23


<210>  105
<211>  23
<212>  DNA
<213>  Artificial

<220>

<223>  Artificially synthesized primer sequence for RT-PCR

<400>  105
aggatgatga ggatgactga aga                                              23


<210>  106
<211>  23
<212>  DNA
<213>  Artificial

<220>
<223>  Artificially synthesized primer sequence for RT-PCR

<400>  106
gaatgggcct ctatctggta tct                                              23


<210>  107
<211>  23
<212>  DNA
<213>  Artificial

<220>
<223>  Artificially synthesized primer sequence for RT-PCR

<400>  107
tgtgtctcat ctgtgaactg ctt                                              23


<210>  108
<211>  23
<212>  DNA
<213>  Artificial

<220>
<223>  Artificially synthesized primer sequence for RT-PCR

<400>  108
ttcgtgttac ggtatatcct gct                                              23


<210>  109
<211>  23
<212>  DNA
<213>  Artificial

<220>
<223>  Artificially synthesized primer sequence for RT-PCR

<400>  109
cttctgttcc gtaaactcct tga                                              23


<210>  110
<211>  22
<212>  DNA
<213>  Artificial

<220>
<223>  Artificially synthesized primer sequence for RT-PCR

<400>  110

caattgtgta ctccaaaccc aa 22

<210> 111
<211> 22
<212> DNA
<213> Artificial

<220>
<223> Artificially synthesized primer sequence for RT-PCR

<400> 111
gcccttccaa cttgtcctta ac 22

<210> 112
<211> 23
<212> DNA
<213> Artificial

<220>
<223> Artificially synthesized primer sequence for RT-PCR

<400> 112
gcctctttat tcccatctcc tta 23

<210> 113
<211> 23
<212> DNA
<213> Artificial

<220>
<223> Artificially synthesized primer sequence for RT-PCR

<400> 113
gaacagatca ctggtttacc tcg 23

<210> 114
<211> 23
<212> DNA
<213> Artificial

<220>
<223> Artificially synthesized primer sequence for RT-PCR

<400> 114
atctttcagt aacagacctc ccc 23

<210> 115
<211> 23
<212> DNA
<213> Artificial

<220>
<223> Artificially synthesized primer sequence for RT-PCR

<400> 115
acaagatggc tagctcaaaa gtg 23

<210>  116
<211>  22
<212>  DNA
<213>  Artificial

<220>
<223>  Artificially synthesized primer sequence for RT-PCR

<400>  116
caacacgtgg tggttctaat tt                                                22


<210>  117
<211>  22
<212>  DNA
<213>  Artificial

<220>
<223>  Artificially synthesized primer sequence for RT-PCR

<400>  117
atgcaggacg gtaacttcct gc                                                22


<210>  118
<211>  21
<212>  DNA
<213>  Artificial

<220>
<223>  Artificially synthesized primer sequence for RT-PCR

<400>  118
tgggcccagg aagtcctcct t                                                 21


<210>  119
<211>  22
<212>  DNA
<213>  Artificial

<220>
<223>  Artificially synthesized primer sequence for RT-PCR

<400>  119
cccaacatgt gaagacagtg at                                                22


<210>  120
<211>  23
<212>  DNA
<213>  Artificial

<220>
<223>  Artificially synthesized primer sequence for RT-PCR

<400>  120
cctgtccacc tcatgtttta ttg                                               23


<210>  121
<211>  21
<212>  DNA

<213>  Artificial

<220>
<223>  Artificially synthesized primer sequence for RT-PCR

<400>  121
gctgaagtgt acgaccagga g                                                21


<210>  122
<211>  21
<212>  DNA
<213>  Artificial

<220>
<223>  Artificially synthesized primer sequence for RT-PCR

<400>  122
cacctttatc cgcactgtag g                                                21


<210>  123
<211>  22
<212>  DNA
<213>  Artificial

<220>
<223>  Artificially synthesized primer sequence for RT-PCR

<400>  123
aaagctgatg aggacagacc ag                                               22


<210>  124
<211>  22
<212>  DNA
<213>  Artificial

<220>
<223>  Artificially synthesized primer sequence for RT-PCR

<400>  124
ggcagaggca caatcatttt ag                                               22


<210>  125
<211>  23
<212>  DNA
<213>  Artificial

<220>
<223>  Artificially synthesized primer sequence for RT-PCR

<400>  125
gaagagaatg caggtgttga gtt                                              23


<210>  126
<211>  23
<212>  DNA
<213>  Artificial

<220>

<223>  Artificially synthesized primer sequence for RT-PCR

<400>  126
gtccacagca ttcataaaac agg                                              23


<210>  127
<211>  22
<212>  DNA
<213>  Artificial

<220>
<223>  Artificially synthesized primer sequence for RT-PCR

<400>  127
ctcctcagtg tccacacttc aa                                               22


<210>  128
<211>  21
<212>  DNA
<213>  Artificial

<220>
<223>  Artificially synthesized primer sequence for RT-PCR

<400>  128
gttacttgca gccaaaagca g                                                21


<210>  129
<211>  23
<212>  DNA
<213>  Artificial

<220>
<223>  Artificially synthesized primer sequence for RT-PCR

<400>  129
agtctctcct tttcagacat ccc                                              23


<210>  130
<211>  23
<212>  DNA
<213>  Artificial

<220>
<223>  Artificially synthesized primer sequence for RT-PCR

<400>  130
tccataaagt cagaccagca gtt                                              23


<210>  131
<211>  21
<212>  DNA
<213>  Artificial

<220>
<223>  Artificially synthesized primer sequence for RT-PCR

<400>  131

cctt ctggga ggacagactt t                                           21

<210>  132
<211>  25
<212>  DNA
<213>  Artificial

<220>
<223>  Artificially synthesized primer sequence for RT-PCR

<400>  132
tttctcttca ttagacttgg cctct                                           25

<210>  133
<211>  23
<212>  DNA
<213>  Artificial

<220>
<223>  Artificially synthesized primer sequence for RT-PCR

<400>  133
aacctagcct cccttcaaac tta                                             23

<210>  134
<211>  23
<212>  DNA
<213>  Artificial

<220>
<223>  Artificially synthesized primer sequence for RT-PCR

<400>  134
gagacaggat ggaaaaatct gtg                                             23

<210>  135
<211>  23
<212>  DNA
<213>  Artificial

<220>
<223>  Artificially synthesized primer sequence for RT-PCR

<400>  135
   cctttcctga cccttttagt ctt                                            23

<210>  136
<211>  23
<212>  DNA
<213>  Artificial

<220>
<223>  Artificially synthesized primer sequence for RT-PCR

<400>  136
caaatcctgt atttctcaca ggc                                             23

<210> 137
<211> 23
<212> DNA
<213> Artificial

<220>
<223> Artificially synthesized primer sequence for RT-PCR

<400> 137
gaaaaaggag agcatcttgg act 23


<210> 138
<211> 21
<212> DNA
<213> Artificial

<220>
<223> Artificially synthesized primer sequence for RT-PCR

<400> 138
aaaggaaaat gcttccgttc c 21


<210> 139
<211> 23
<212> DNA
<213> Artificial

<220>
<223> Artificially synthesized primer sequence for RT-PCR

<400> 139
taatgtagga tgacaggctc tcc 23


<210> 140
<211> 23
<212> DNA
<213> Artificial

<220>
<223> Artificially synthesized primer sequence for RT-PCR

<400> 140
ccaattgtat aaaggctctt ccc 23


<210> 141
<211> 21
<212> DNA
<213> Artificial

<220>
<223> Artificially synthesized primer sequence for RT-PCR

<400> 141
aggacaacgt cagctctcct g 21


<210> 142
<211> 23
<212> DNA

<213>  Artificial

<220>
<223>  Artificially synthesized primer sequence for RT-PCR

<400>  142
tccactattc cacccacagt aac                                              23


<210>  143
<211>  21
<212>  DNA
<213>  Artificial

<220>
<223>  Artificially synthesized primer sequence for RT-PCR

<400>  143
gaccgagagt ccagcatttt t                                                21


<210>  144
<211>  23
<212>  DNA
<213>  Artificial

<220>
<223>  Artificially synthesized primer sequence for RT-PCR

<400>  144
actgaacaga gcagacagaa acc                                              23


<210>  145
<211>  23
<212>  DNA
<213>  Artificial

<220>
<223>  Artificially synthesized primer sequence for RT-PCR

<400>  145
ctgctgttat taccccattc aag                                              23


<210>  146
<211>  23
<212>  DNA
<213>  Artificial

<220>
<223>  Artificially synthesized primer sequence for RT-PCR

<400>  146
gtgagtgaca gatggcaatt aca                                              23


<210>  147
<211>  23
<212>  DNA
<213>  Artificial

<220>

<223>  Artificially synthesized primer sequence for RT-PCR

<400>  147
ctcgggtaga atttgatgac aac                                              23


<210>  148
<211>  23
<212>  DNA
<213>  Artificial

<220>
<223>  Artificially synthesized primer sequence for RT-PCR

<400>  148
gctggtaaag caggtgtaaa aga                                              23


<210>  149
<211>  21
<212>  DNA
<213>  Artificial

<220>
<223>  Artificially synthesized primer sequence for RT-PCR

<400>  149
ccctgacaac atcaactggt c                                                21


<210>  150
<211>  23
<212>  DNA
<213>  Artificial

<220>
<223>  Artificially synthesized primer sequence for RT-PCR

<400>  150
gtccaccttc gcttttattg agt                                              23


<210>  151
<211>  22
<212>  DNA
<213>  Artificial

<220>
<223>  Artificially synthesized primer sequence for RT-PCR

<400>  151
ctctctgccc aatgataagg ag                                               22


<210>  152
<211>  23
<212>  DNA
<213>  Artificial

<220>
<223>  Artificially synthesized primer sequence for RT-PCR

<400>  152

gaaactttct ctcctcactg ctc                                                23

<210>  153
<211>  23
<212>  DNA
<213>  Artificial

<220>
<223>  Artificially synthesized primer sequence for RT-PCR

<400>  153
cagaagtttt gaggactgaa ctg                                                23

<210>  154
<211>  22
<212>  DNA
<213>  Artificial

<220>
<223>  Artificially synthesized primer sequence for RT-PCR

<400>  154
ccgacctacc ttccctagaa at                                                 22

<210>  155
<211>  23
<212>  DNA
<213>  Artificial

<220>
<223>  Artificially synthesized primer sequence for RT-PCR

<400>  155
ggggttttga aggatgtgta ctt                                                23

<210>  156
<211>  22
<212>  DNA
<213>  Artificial

<220>
<223>  Artificially synthesized primer sequence for RT-PCR

<400>  156
tatgaggcca ttctgcacat ta                                                 22

<210>  157
<211>  22
<212>  DNA
<213>  Artificial

<220>
<223>  Artificially synthesized primer sequence for RT-PCR

<400>  157
gggagtatga agtttccatc tg                                                 22

<210>  158
<211>  24
<212>  DNA
<213>  Artificial

<220>
<223>  Artificially synthesized primer sequence for RT-PCR

<400>  158
ggatgctggt ttatttactg tagg                                              24


<210>  159
<211>  23
<212>  DNA
<213>  Artificial

<220>
<223>  Artificially synthesized primer sequence for RT-PCR

<400>  159
aatatggaat ccctacccac agt                                               23


<210>  160
<211>  22
<212>  DNA
<213>  Artificial

<220>
<223>  Artificially synthesized primer sequence for RT-PCR

<400>  160
tttgacttca caacttcatg gg                                                22


<210>  161
<211>  23
<212>  DNA
<213>  Artificial

<220>
<223>  Artificially synthesized primer sequence for RT-PCR

<400>  161
gaggccattt tagttctgag gtt                                               23


<210>  162
<211>  23
<212>  DNA
<213>  Artificial

<220>
<223>  Artificially synthesized primer sequence for RT-PCR

<400>  162
ctttactgca tatggattct ggg                                               23


<210>  163
<211>  22
<212>  DNA

<213>  Artificial

<220>
<223>  Artificially synthesized primer sequence for RT-PCR

<400>  163
tcaacctcaa gttaaaggaa cg                                              22


<210>  164
<211>  23
<212>  DNA
<213>  Artificial

<220>
<223>  Artificially synthesized primer sequence for RT-PCR

<400>  164
agggaaaagt agagacaaat ggg                                             23


<210>  165
<211>  23
<212>  DNA
<213>  Artificial

<220>
<223>  Artificially synthesized primer sequence for RT-PCR

<400>  165
tctaggcaaa gtggaagtca aag                                             23


<210>  166
<211>  23
<212>  DNA
<213>  Artificial

<220>
<223>  Artificially synthesized primer sequence for RT-PCR

<400>  166
ctcctagaga aatgggttgg att                                             23


<210>  167
<211>  23
<212>  DNA
<213>  Artificial

<220>
<223>  Artificially synthesized primer sequence for RT-PCR

<400>  167
atacactgaa tgtggaagaa ccg                                             23


<210>  168
<211>  23
<212>  DNA
<213>  Artificial

<220>

<223>  Artificially synthesized primer sequence for RT-PCR

<400>  168
gggcacacaa tttcatgtag tct                                              23


<210>  169
<211>  23
<212>  DNA
<213>  Artificial

<220>
<223>  Artificially synthesized primer sequence for RT-PCR

<400>  169
agacattgca taccagctct cat                                              23


<210>  170
<211>  24
<212>  DNA
<213>  Artificial

<220>
<223>  Artificially synthesized primer sequence for RT-PCR

<400>  170
cctttacttc cttcacttta agcc                                             24


<210>  171
<211>  23
<212>  DNA
<213>  Artificial

<220>
<223>  Artificially synthesized primer sequence for RT-PCR

<400>  171
gtaacaaacg ccaccttaca ctc                                              23


<210>  172
<211>  23
<212>  DNA
<213>  Artificial

<220>
<223>  Artificially synthesized primer sequence for RT-PCR

<400>  172
ttctgttctt gcaactgagt cct                                              23


<210>  173
<211>  23
<212>  DNA
<213>  Artificial

<220>
<223>  Artificially synthesized primer sequence for RT-PCR

<400>  173

acctccagta aaagtttctt ccg                                                23

<210>  174
<211>  23
<212>  DNA
<213>  Artificial

<220>
<223>  Artificially synthesized primer sequence for RT-PCR

<400>  174
gtaaattcag cttcaaaccc tgg                                                23

<210>  175
<211>  23
<212>  DNA
<213>  Artificial

<220>
<223>  Artificially synthesized primer sequence for RT-PCR

<400>  175
cattgagcct tctctgatca ctc                                                23

<210>  176
<211>  23
<212>  DNA
<213>  Artificial

<220>
<223>  Artificially synthesized primer sequence for RT-PCR

<400>  176
gcactgttac agatagtctg ggg                                                23

<210>  177
<211>  23
<212>  DNA
<213>  Artificial

<220>
<223>  Artificially synthesized primer sequence for RT-PCR

<400>  177
tatcagtaac tgctccgtgt tca                                                23

<210>  178
<211>  23
<212>  DNA
<213>  Artificial

<220>
<223>  Artificially synthesized primer sequence for RT-PCR

<400>  178
ggtctgtcat tgaccaaaac atc                                                23

<210>  179
<211>  23
<212>  DNA
<213>  Artificial

<220>
<223>  Artificially synthesized primer sequence for RT-PCR

<400>  179
tcctgaataa aggcctagta ccc                                              23


<210>  180
<211>  23
<212>  DNA
<213>  Artificial

<220>
<223>  Artificially synthesized primer sequence for RT-PCR

<400>  180
aaaccagaat ccaacactac cct                                              23


<210>  181
<211>  23
<212>  DNA
<213>  Artificial

<220>
<223>  Artificially synthesized primer sequence for RT-PCR

<400>  181
gagccctctc cacatttcta ttt                                              23


<210>  182
<211>  23
<212>  DNA
<213>  Artificial

<220>
<223>  Artificially synthesized primer sequence for RT-PCR

<400>  182
acactgaaac gtgatgggta act                                              23


<210>  183
<211>  23
<212>  DNA
<213>  Artificial

<220>
<223>  Artificially synthesized primer sequence for RT-PCR

<400>  183
gacatgagtc cgaaacaact acc                                              23


<210>  184
<211>  23
<212>  DNA

<213>  Artificial

<220>
<223>  Artificially synthesized primer sequence for RT-PCR

<400>  184
atgagactgt accaaatgat ggc                                              23


<210>  185
<211>  23
<212>  DNA
<213>  Artificial

<220>
<223>  Artificially synthesized primer sequence for RT-PCR

<400>  185
tgatacctgg aggcttatct gag                                              23


<210>  186
<211>  23
<212>  DNA
<213>  Artificial

<220>
<223>  Artificially synthesized primer sequence for RT-PCR

<400>  186
gactcagtag ccagttgaag gaa                                              23


<210>  187
<211>  23
<212>  DNA
<213>  Artificial

<220>
<223>  Artificially synthesized primer sequence for RT-PCR

<400>  187
tcttgcaggt ctggctattt tag                                              23


<210>  188
<211>  23
<212>  DNA
<213>  Artificial

<220>
<223>  Artificially synthesized primer sequence for RT-PCR

<400>  188
tatatttaag gagcacccct tcc                                              23


<210>  189
<211>  22
<212>  DNA
<213>  Artificial

<220>

<223>  Artificially synthesized primer sequence for RT-PCR

<400>  189
acaagcaagt gcattttcag tc                                              22


<210>  190
<211>  23
<212>  DNA
<213>  Artificial

<220>
<223>  Artificially synthesized primer sequence for RT-PCR

<400>  190
gaacagggta gccattaaca caa                                             23


<210>  191
<211>  23
<212>  DNA
<213>  Artificial

<220>
<223>  Artificially synthesized primer sequence for RT-PCR

<400>  191
tctatcatcg acgtctacca caa                                             23


<210>  192
<211>  23
<212>  DNA
<213>  Artificial

<220>
<223>  Artificially synthesized primer sequence for RT-PCR

<400>  192
gctactcttt gtggctttct tca                                             23


<210>  193
<211>  23
<212>  DNA
<213>  Artificial

<220>
<223>  Artificially synthesized primer sequence for RT-PCR

<400>  193
gcatgatcat agacgtcttt tcc                                             23


<210>  194
<211>  23
<212>  DNA
<213>  Artificial

<220>
<223>  Artificially synthesized primer sequence for RT-PCR

<400>  194

gatgaactca ctgaagtcca cct                                                23

<210>  195
<211>  21
<212>  DNA
<213>  Artificial

<220>
<223>  Artificially synthesized primer sequence for RT-PCR

<400>  195
tctccaggac aagatcaagg a                                                  21

<210>  196
<211>  22
<212>  DNA
<213>  Artificial

<220>
<223>  Artificially synthesized primer sequence for RT-PCR

<400>  196
gttttatttc cagcatgttc cc                                                 22

<210>  197
<211>  23
<212>  DNA
<213>  Artificial

<220>
<223>  Artificially synthesized primer sequence for RT-PCR

<400>  197
agagctgatc aagttcatgt gtg                                                23

<210>  198
<211>  23
<212>  DNA
<213>  Artificial

<220>
<223>  Artificially synthesized primer sequence for RT-PCR

<400>  198
acatagcaag ttcgagtttc tgc                                                23

<210>  199
<211>  23
<212>  DNA
<213>  Artificial

<220>
<223>  Artificially synthesized primer sequence for RT-PCR

<400>  199
gagctggagg taggaataca ggt                                                23

<210>  200
<211>  23
<212>  DNA
<213>  Artificial

<220>
<223>  Artificially synthesized primer sequence for RT-PCR

<400>  200
caatagtttg gcttggtgta agg                                              23


<210>  201
<211>  23
<212>  DNA
<213>  Artificial

<220>
<223>  Artificially synthesized primer sequence for RT-PCR

<400>  201
ctcctctgca tgcacttaac ttt                                              23


<210>  202
<211>  23
<212>  DNA
<213>  Artificial

<220>
<223>  Artificially synthesized primer sequence for RT-PCR

<400>  202
gagagtttaa tgttgtggga agg                                              23


<210>  203
<211>  22
<212>  DNA
<213>  Artificial

<220>
<223>  Artificially synthesized primer sequence for RT-PCR

<400>  203
ccgggcaata aagtaactct tg                                               22


<210>  204
<211>  25
<212>  DNA
<213>  Artificial

<220>
<223>  Artificially synthesized primer sequence for RT-PCR

<400>  204
gtatttgtct gtatgcctac atctg                                            25


<210>  205
<211>  23
<212>  DNA

<213>  Artificial

<220>
<223>  Artificially synthesized primer sequence for RT-PCR

<400>  205
tctgcgtatc ttgagtgctt aca                                              23


<210>  206
<211>  23
<212>  DNA
<213>  Artificial

<220>
<223>  Artificially synthesized primer sequence for RT-PCR

<400>  206
acagagatgt ggtggtgcta gtt                                              23


<210>  207
<211>  23
<212>  DNA
<213>  Artificial

<220>
<223>  Artificially synthesized primer sequence for RT-PCR

<400>  207
agcagaggat cagagctttc ttt                                              23


<210>  208
<211>  23
<212>  DNA
<213>  Artificial

<220>
<223>  Artificially synthesized primer sequence for RT-PCR

<400>  208
agaaaaggtg tgaacagagt tgc                                              23


<210>  209
<211>  23
<212>  DNA
<213>  Artificial

<220>
<223>  Artificially synthesized primer sequence for RT-PCR

<400>  209
agagccatag aaactgctcc tct                                              23


<210>  210
<211>  23
<212>  DNA
<213>  Artificial

<220>

<223>  Artificially synthesized primer sequence for RT-PCR

<400>  210
cataactgca tagacagcac gtc                                              23


<210>  211
<211>  21
<212>  DNA
<213>  Artificial

<220>
<223>  Artificially synthesized primer sequence for RT-PCR

<400>  211
tacctgctct atgtgggtgc t                                                21


<210>  212
<211>  25
<212>  DNA
<213>  Artificial

<220>
<223>  Artificially synthesized primer sequence for RT-PCR

<400>  212
cctcagaact ctcagtttat tcctg                                            25


<210>  213
<211>  23
<212>  DNA
<213>  Artificial

<220>
<223>  Artificially synthesized primer sequence for RT-PCR

<400>  213
ataagccaca gagacaaacc aga                                              23


<210>  214
<211>  23
<212>  DNA
<213>  Artificial

<220>
<223>  Artificially synthesized primer sequence for RT-PCR

<400>  214
gggaggttat tttcacagaa cac                                              23


<210>  215
<211>  22
<212>  DNA
<213>  Artificial

<220>
<223>  Artificially synthesized primer sequence for RT-PCR

<400>  215

gagttcctgt ctctctgcca ac 22

<210> 216
<211> 23
<212> DNA
<213> Artificial

<220>
<223> Artificially synthesized primer sequence for RT-PCR

<400> 216
taatatacaa gggctcaacc gag 23

<210> 217
<211> 21
<212> DNA
<213> Artificial

<220>
<223> Artificially synthesized primer sequence for RT-PCR

<400> 217
gtcatagctg tgtcctgggt c 21

<210> 218
<211> 23
<212> DNA
<213> Artificial

<220>
<223> Artificially synthesized primer sequence for RT-PCR

<400> 218
ctattttatc cccatggcag agt 23

<210> 219
<211> 23
<212> DNA
<213> Artificial

<220>
<223> Artificially synthesized primer sequence for RT-PCR

<400> 219
cagatattct gtatgctgga ggg 23

<210> 220
<211> 23
<212> DNA
<213> Artificial

<220>
<223> Artificially synthesized primer sequence for RT-PCR

<400> 220
ccatctcaga agggctttat ttc 23

<210>  221
<211>  23
<212>  DNA
<213>  Artificial

<220>
<223>  Artificially synthesized primer sequence for RT-PCR

<400>  221
gatttccata cttcgggaga aac                                              23


<210>  222
<211>  23
<212>  DNA
<213>  Artificial

<220>
<223>  Artificially synthesized primer sequence for RT-PCR

<400>  222
tatcagatgc cacacatacg aga                                              23


<210>  223
<211>  22
<212>  DNA
<213>  Artificial

<220>
<223>  Artificially synthesized primer sequence for RT-PCR

<400>  223
atggaacaaa gaagctgtga cc                                               22


<210>  224
<211>  22
<212>  DNA
<213>  Artificial

<220>
<223>  Artificially synthesized primer sequence for RT-PCR

<400>  224
gggtacatgc aaaccagtac ac                                               22


<210>  225
<211>  20
<212>  DNA
<213>  Artificial

<220>
<223>  Artificially synthesized primer sequence for RT-PCR

<400>  225
tgaacagttt gctggtcttg                                                  20


<210>  226
<211>  20
<212>  DNA

<213>  Artificial

<220>
<223>  Artificially synthesized primer sequence for RT-PCR

<400>  226
aatgtcaggt tggggagtta                                                    20


<210>  227
<211>  23
<212>  DNA
<213>  Artificial

<220>
<223>  Artificially synthesized primer sequence for RT-PCR

<400>  227
ttctggacag acggagagac tac                                                23


<210>  228
<211>  22
<212>  DNA
<213>  Artificial

<220>
<223>  Artificially synthesized primer sequence for RT-PCR

<400>  228
agtgatgaca taccccctggt tc                                                22


<210>  229
<211>  22
<212>  DNA
<213>  Artificial

<220>
<223>  Artificially synthesized primer sequence for RT-PCR

<400>  229
caagacttct cagatccttg gg                                                 22


<210>  230
<211>  23
<212>  DNA
<213>  Artificial

<220>
<223>  Artificially synthesized primer sequence for RT-PCR

<400>  230
actcacatgt ggaagtgttc ctt                                                23


<210>  231
<211>  23
<212>  DNA
<213>  Artificial

<220>

<223>  Artificially synthesized primer sequence for RT-PCR

<400>  231
tcaagcaata tgaagtaggg ctc                                              23


<210>  232
<211>  23
<212>  DNA
<213>  Artificial

<220>
<223>  Artificially synthesized primer sequence for RT-PCR

<400>  232
aacacaaatg tcccgtgtaa gtc                                              23


<210>  233
<211>  23
<212>  DNA
<213>  Artificial

<220>
<223>  Artificially synthesized primer sequence for RT-PCR

<400>  233
ctgcctctta ctcgtcacag ttt                                              23


<210>  234
<211>  22
<212>  DNA
<213>  Artificial

<220>
<223>  Artificially synthesized primer sequence for RT-PCR

<400>  234
tgacttcttt gaagtgaagg ct                                               22


<210>  235
<211>  23
<212>  DNA
<213>  Artificial

<220>
<223>  Artificially synthesized primer sequence for RT-PCR

<400>  235
ccctagtttt tgtagctgtc gaa                                              23


<210>  236
<211>  22
<212>  DNA
<213>  Artificial

<220>
<223>  Artificially synthesized primer sequence for RT-PCR

<400>  236

gatcacatgc caagaacaca at                                                22

<210>  237
<211>  23
<212>  DNA
<213>  Artificial

<220>
<223>  Artificially synthesized primer sequence for RT-PCR

<400>  237
ctacgtacct gggtgcctat atc                                               23

<210>  238
<211>  23
<212>  DNA
<213>  Artificial

<220>
<223>  Artificially synthesized primer sequence for RT-PCR

<400>  238
gtcctcttat aaggctcact ccc                                               23

<210>  239
<211>  23
<212>  DNA
<213>  Artificial

<220>
<223>  Artificially synthesized primer sequence for RT-PCR

<400>  239
gatgttagag actccttcac cca                                               23

<210>  240
<211>  23
<212>  DNA
<213>  Artificial

<220>
<223>  Artificially synthesized primer sequence for RT-PCR

<400>  240
cggtattctt aacacatctt gcc                                               23

<210>  241
<211>  23
<212>  DNA
<213>  Artificial

<220>
<223>  Artificially synthesized primer sequence for RT-PCR

<400>  241
gtgtctgcgt atctacctga acg                                               23

<210>  242
<211>  23
<212>  DNA
<213>  Artificial

<220>
<223>  Artificially synthesized primer sequence for RT-PCR

<400>  242
ataactctgt cttcgtgagc tgg                                              23


<210>  243
<211>  23
<212>  DNA
<213>  Artificial

<220>
<223>  Artificially synthesized primer sequence for RT-PCR

<400>  243
gtatttggct tactgtccca aac                                              23


<210>  244
<211>  23
<212>  DNA
<213>  Artificial

<220>
<223>  Artificially synthesized primer sequence for RT-PCR

<400>  244
ctaggaagaa atcatgctgg gtt                                              23


<210>  245
<211>  22
<212>  DNA
<213>  Artificial

<220>
<223>  Artificially synthesized primer sequence for RT-PCR

<400>  245
cagtttgagc aagcaaaaga tg                                               22


<210>  246
<211>  23
<212>  DNA
<213>  Artificial

<220>
<223>  Artificially synthesized primer sequence for RT-PCR

<400>  246
cggatatccc taatctattc cca                                              23


<210>  247
<211>  22
<212>  DNA

<213>  Artificial

<220>
<223>  Artificially synthesized primer sequence for RT-PCR

<400>  247
gacagtatag ctgcccttgc tc                                              22


<210>  248
<211>  23
<212>  DNA
<213>  Artificial

<220>
<223>  Artificially synthesized primer sequence for RT-PCR

<400>  248
aagcagtggg gtagagtcag aac                                             23


<210>  249
<211>  23
<212>  DNA
<213>  Artificial

<220>
<223>  Artificially synthesized primer sequence for RT-PCR

<400>  249
acagaagaag ctacctcagg tgt                                             23


<210>  250
<211>  22
<212>  DNA
<213>  Artificial

<220>
<223>  Artificially synthesized primer sequence for RT-PCR

<400>  250
ctagcggaag acaattcaga ac                                              22


<210>  251
<211>  23
<212>  DNA
<213>  Artificial

<220>
<223>  Artificially synthesized primer sequence for RT-PCR

<400>  251
taaccttgat agaagaacct tgg                                             23


<210>  252
<211>  22
<212>  DNA
<213>  Artificial

<220>

<223>  Artificially synthesized primer sequence for RT-PCR

<400>  252
gcaaatgaga caaaattggg ac                                              22


<210>  253
<211>  23
<212>  DNA
<213>  Artificial

<220>
<223>  Artificially synthesized primer sequence for RT-PCR

<400>  253
atctccactc tacggccttt tac                                             23


<210>  254
<211>  23
<212>  DNA
<213>  Artificial

<220>
<223>  Artificially synthesized primer sequence for RT-PCR

<400>  254
taatgactta aacaccagca cgg                                             23


<210>  255
<211>  23
<212>  DNA
<213>  Artificial

<220>
<223>  Artificially synthesized primer sequence for RT-PCR

<400>  255
gtgttctcct aatcccagaa cct                                             23


<210>  256
<211>  23
<212>  DNA
<213>  Artificial

<220>
<223>  Artificially synthesized primer sequence for RT-PCR

<400>  256
aagagttgtg gcctattacc tcc                                             23


<210>  257
<211>  23
<212>  DNA
<213>  Artificial

<220>
<223>  Artificially synthesized primer sequence for RT-PCR

<400>  257

tggtcctact aagagaatgc agc 23

<210> 258
<211> 23
<212> DNA
<213> Artificial

<220>
<223> Artificially synthesized primer sequence for RT-PCR

<400> 258
agccattagg aaaaagagca gag 23

<210> 259
<211> 23
<212> DNA
<213> Artificial

<220>
<223> Artificially synthesized primer sequence for RT-PCR

<400> 259
gactgctata ctccaactct ggg 23

<210> 260
<211> 24
<212> DNA
<213> Artificial

<220>
<223> Artificially synthesized primer sequence for RT-PCR

<400> 260
gccaaagaca tggtttagtc atac 24

<210> 261
<211> 23
<212> DNA
<213> Artificial

<220>
<223> Artificially synthesized primer sequence for RT-PCR

<400> 261
acactgagct ttaatggctg aag 23

<210> 262
<211> 23
<212> DNA
<213> Artificial

<220>
<223> Artificially synthesized primer sequence for RT-PCR

<400> 262
tccacagtga cctgacacaa tag 23

<210>  263
<211>  22
<212>  DNA
<213>  Artificial

<220>
<223>  Artificially synthesized primer sequence for RT-PCR

<400>  263
gtcctcattc cctttctgtt cc                                                22


<210>  264
<211>  23
<212>  DNA
<213>  Artificial

<220>
<223>  Artificially synthesized primer sequence for RT-PCR

<400>  264
ctgttttctt tcaacctgca ctc                                               23


<210>  265
<211>  21
<212>  DNA
<213>  Artificial

<220>
<223>  Artificially synthesized primer sequence for RT-PCR

<400>  265
aagagaggcc agaaactgag c                                                 21


<210>  266
<211>  23
<212>  DNA
<213>  Artificial

<220>
<223>  Artificially synthesized primer sequence for RT-PCR

<400>  266
aactagcagc tttattgccc ttc                                               23


<210>  267
<211>  20
<212>  DNA
<213>  Artificial

<220>
<223>  Artificially synthesized primer sequence for RT-PCR

<400>  267
gtggacatct aattgaggcc                                                   20


<210>  268
<211>  23
<212>  DNA

<213>  Artificial

<220>
<223>  Artificially synthesized primer sequence for RT-PCR

<400>  268
gaagatcttc cactagtaat att                                              23


<210>  269
<211>  23
<212>  DNA
<213>  Artificial

<220>
<223>  Artificially synthesized primer sequence for RT-PCR

<400>  269
cagaggactc tgatgaagaa agc                                              23


<210>  270
<211>  22
<212>  DNA
<213>  Artificial

<220>
<223>  Artificially synthesized primer sequence for RT-PCR

<400>  270
tttccacaaa cgctaagaga ac                                               22


<210>  271
<211>  21
<212>  DNA
<213>  Artificial

<220>
<223>  Artificially synthesized primer sequence for RT-PCR

<400>  271
atgtctgctc cgtgagtgtc t                                                21


<210>  272
<211>  23
<212>  DNA
<213>  Artificial

<220>
<223>  Artificially synthesized primer sequence for RT-PCR

<400>  272
gcaaatccta ctttcaactg cac                                              23


<210>  273
<211>  22
<212>  DNA
<213>  Artificial

<220>

<223>  Artificially synthesized primer sequence for RT-PCR

<400>  273
gccttaaagc tggacacaga ag                                              22


<210>  274
<211>  23
<212>  DNA
<213>  Artificial

<220>
<223>  Artificially synthesized primer sequence for RT-PCR

<400>  274
ctccagacac cattgcttaa atc                                             23


<210>  275
<211>  23
<212>  DNA
<213>  Artificial

<220>
<223>  Artificially synthesized primer sequence for RT-PCR

<400>  275
agactttaaa atcccacctg gac                                             23


<210>  276
<211>  23
<212>  DNA
<213>  Artificial

<220>
<223>  Artificially synthesized primer sequence for RT-PCR

<400>  276
cacccagcct tctctttatt ttc                                             23


<210>  277
<211>  21
<212>  DNA
<213>  Artificial

<220>
<223>  Artificially synthesized primer sequence for RT-PCR

<400>  277
aggggattct ggaactgaat g                                               21


<210>  278
<211>  23
<212>  DNA
<213>  Artificial

<220>
<223>  Artificially synthesized primer sequence for RT-PCR

<400>  278

ttataccgag gagatgggaa agt                                              23


<210>  279
<211>  23
<212>  DNA
<213>  Artificial

<220>
<223>  Artificially synthesized primer sequence for RT-PCR

<400>  279
gttgcagtac caatcctttc ttg                                              23


<210>  280
<211>  24
<212>  DNA
<213>  Artificial

<220>
<223>  Artificially synthesized primer sequence for RT-PCR

<400>  280
gtcctatgtt aatttccacc aagc                                             24


<210>  281
<211>  21
<212>  DNA
<213>  Artificial

<220>
<223>  Artificially synthesized primer sequence for RT-PCR

<400>  281
tatccagagg gtgtccctga c                                                21


<210>  282
<211>  24
<212>  DNA
<213>  Artificial

<220>
<223>  Artificially synthesized primer sequence for RT-PCR

<400>  282
gttctttaat gacagttcaa gggg                                             24


<210>  283
<211>  22
<212>  DNA
<213>  Artificial

<220>
<223>  Artificially synthesized primer sequence for RT-PCR

<400>  283
atcggatcga tattacacag ca                                               22

<210> 284
<211> 22
<212> DNA
<213> Artificial

<220>
<223> Artificially synthesized primer sequence for RT-PCR

<400> 284
cccatcaggg aaacaaagat ta 22


<210> 285
<211> 23
<212> DNA
<213> Artificial

<220>
<223> Artificially synthesized primer sequence for RT-PCR

<400> 285
tgcatctgta acttcaggag gat 23


<210> 286
<211> 23
<212> DNA
<213> Artificial

<220>
<223> Artificially synthesized primer sequence for RT-PCR

<400> 286
tccatcaact tacctatcga tgc 23


<210> 287
<211> 23
<212> DNA
<213> Artificial

<220>
<223> Artificially synthesized primer sequence for RT-PCR

<400> 287
aaacctacga acgccttttc tac 23


<210> 288
<211> 23
<212> DNA
<213> Artificial

<220>
<223> Artificially synthesized primer sequence for RT-PCR

<400> 288
ggtatcacag gagcaccaat aaa 23


<210> 289
<211> 23
<212> DNA

<213>  Artificial

<220>
<223>  Artificially synthesized primer sequence for RT-PCR

<400>  289
ctttctgttg ctttcccagt aga                                              23


<210>  290
<211>  23
<212>  DNA
<213>  Artificial

<220>
<223>  Artificially synthesized primer sequence for RT-PCR

<400>  290
ttgatacatt acactggtgg cag                                              23


<210>  291
<211>  21
<212>  DNA
<213>  Artificial

<220>
<223>  Artificially synthesized primer sequence for RT-PCR

<400>  291
acccacagaa ctgggagtga g                                                21


<210>  292
<211>  22
<212>  DNA
<213>  Artificial

<220>
<223>  Artificially synthesized primer sequence for RT-PCR

<400>  292
attttactgc agaaacgggt tg                                               22


<210>  293
<211>  24
<212>  DNA
<213>  Artificial

<220>
<223>  Artificially synthesized primer sequence for RT-PCR

<400>  293
gatggggaaa ctatgactaa tgac                                             24


<210>  294
<211>  25
<212>  DNA
<213>  Artificial

<220>

<223>  Artificially synthesized primer sequence for RT-PCR

<400>  294
ggtatcaata aagcccagat attcc                                          25


<210>  295
<211>  23
<212>  DNA
<213>  Artificial

<220>
<223>  Artificially synthesized primer sequence for RT-PCR

<400>  295
ttagtggatc tggctcttct tgt                                            23


<210>  296
<211>  21
<212>  DNA
<213>  Artificial

<220>
<223>  Artificially synthesized primer sequence for RT-PCR

<400>  296
caggcacatc acagttgtca c                                              21


<210>  297
<211>  22
<212>  DNA
<213>  Artificial

<220>
<223>  Artificially synthesized primer sequence for RT-PCR

<400>  297
gatttggaac ttggaaggag tg                                             22


<210>  298
<211>  23
<212>  DNA
<213>  Artificial

<220>
<223>  Artificially synthesized primer sequence for RT-PCR

<400>  298
acttcagtca cccaaaacaa cag                                            23


<210>  299
<211>  23
<212>  DNA
<213>  Artificial

<220>
<223>  Artificially synthesized primer sequence for RT-PCR

<400>  299

cgggaggatt gtaagatact gtg                                                23

<210>  300
<211>  23
<212>  DNA
<213>  Artificial

<220>
<223>  Artificially synthesized primer sequence for RT-PCR

<400>  300
acttctcatg agttcagcct cag                                                23

<210>  301
<211>  22
<212>  DNA
<213>  Artificial

<220>
<223>  Artificially synthesized primer sequence for RT-PCR

<400>  301
gtgagtattc ctccgttagc tt                                                 22

<210>  302
<211>  22
<212>  DNA
<213>  Artificial

<220>
<223>  Artificially synthesized primer sequence for RT-PCR

<400>  302
cagggagaag agaaaacatc ac                                                 22

<210>  303
<211>  20
<212>  DNA
<213>  Artificial

<220>
<223>  Artificially synthesized primer sequence for RT-PCR

<400>  303
agctgaagct gactgtgtct                                                    20

<210>  304
<211>  23
<212>  DNA
<213>  Artificial

<220>
<223>  Artificially synthesized primer sequence for RT-PCR

<400>  304
aggcacagac ggtattgttg tag                                                23

<210>  305
<211>  23
<212>  DNA
<213>  Artificial

<220>
<223>  Artificially synthesized primer sequence for RT-PCR

<400>  305
gactttcaaa caacccagtg tct                                              23


<210>  306
<211>  22
<212>  DNA
<213>  Artificial

<220>
<223>  Artificially synthesized primer sequence for RT-PCR

<400>  306
ctctagccag cttcttcctc ac                                               22


<210>  307
<211>  23
<212>  DNA
<213>  Artificial

<220>
<223>  Artificially synthesized primer sequence for RT-PCR

<400>  307
ggtcttcata cgctgtactt gct                                              23


<210>  308
<211>  23
<212>  DNA
<213>  Artificial

<220>
<223>  Artificially synthesized primer sequence for RT-PCR

<400>  308
tatgccttca ctgatccacc tac                                              23


<210>  309
<211>  23
<212>  DNA
<213>  Artificial

<220>
<223>  Artificially synthesized primer sequence for RT-PCR

<400>  309
tcctgtggaa atagaactgt cgt                                              23


<210>  310
<211>  23
<212>  DNA

<213>  Artificial

<220>
<223>  Artificially synthesized primer sequence for RT-PCR

<400>  310
cacaaagttc aaggaagcag tct                                              23


<210>  311
<211>  22
<212>  DNA
<213>  Artificial

<220>
<223>  Artificially synthesized primer sequence for RT-PCR

<400>  311
aaggttctct accgcctcaa gt                                               22


<210>  312
<211>  21
<212>  DNA
<213>  Artificial

<220>
<223>  Artificially synthesized primer sequence for RT-PCR

<400>  312
ctgaacacac cgtggcttta t                                                21


<210>  313
<211>  23
<212>  DNA
<213>  Artificial

<220>
<223>  Artificially synthesized primer sequence for RT-PCR

<400>  313
aagaagccac cactattcct ctc                                              23


<210>  314
<211>  23
<212>  DNA
<213>  Artificial

<220>
<223>  Artificially synthesized primer sequence for RT-PCR

<400>  314
cctgaaggac tgaaaaggtc ata                                              23


<210>  315
<211>  22
<212>  DNA
<213>  Artificial

<220>

<223>  Artificially synthesized primer sequence for RT-PCR

<400>  315
cctgtctcca aaggaaaaac aa                                                22


<210>  316
<211>  23
<212>  DNA
<213>  Artificial

<220>
<223>  Artificially synthesized primer sequence for RT-PCR

<400>  316
ctcagtttca tcaagtcctt tgc                                               23


<210>  317
<211>  22
<212>  DNA
<213>  Artificial

<220>
<223>  Artificially synthesized primer sequence for RT-PCR

<400>  317
agcgaggaga actcttgaaa tc                                                22


<210>  318
<211>  22
<212>  DNA
<213>  Artificial

<220>
<223>  Artificially synthesized primer sequence for RT-PCR

<400>  318
gtgtcccacc atagaaaact tc                                                22


<210>  319
<211>  23
<212>  DNA
<213>  Artificial

<220>
<223>  Artificially synthesized primer sequence for RT-PCR

<400>  319
gaagccagcc tactccttct tac                                               23


<210>  320
<211>  23
<212>  DNA
<213>  Artificial

<220>
<223>  Artificially synthesized primer sequence for RT-PCR

<400>  320

tagcattcac agagcaggag att                                                23

<210>  321
<211>  23
<212>  DNA
<213>  Artificial

<220>
<223>  Artificially synthesized primer sequence for RT-PCR

<400>  321
catatgtgga ggtgctgtgt aaa                                                23

<210>  322
<211>  23
<212>  DNA
<213>  Artificial

<220>
<223>  Artificially synthesized primer sequence for RT-PCR

<400>  322
gtctacagtt agacagggaa gcc                                                23

<210>  323
<211>  23
<212>  DNA
<213>  Artificial

<220>
<223>  Artificially synthesized primer sequence for RT-PCR

<400>  323
gacagctctt ggatccctat ttt                                                23

<210>  324
<211>  23
<212>  DNA
<213>  Artificial

<220>
<223>  Artificially synthesized primer sequence for RT-PCR

<400>  324
agagtgaact tgcatctgtt cct                                                23

<210>  325
<211>  22
<212>  DNA
<213>  Artificial

<220>
<223>  Artificially synthesized primer sequence for RT-PCR

<400>  325
gtgtgtgtac gtgtctccag gt                                                 22

<210>  326
<211>  23
<212>  DNA
<213>  Artificial

<220>
<223>  Artificially synthesized primer sequence for RT-PCR

<400>  326
cagacaagat agctgactct ccc                                              23


<210>  327
<211>  21
<212>  DNA
<213>  Artificial

<220>
<223>  Artificially synthesized primer sequence for RT-PCR

<400>  327
gaagtctggg ggtgttggtc t                                                21


<210>  328
<211>  27
<212>  DNA
<213>  Artificial

<220>
<223>  Artificially synthesized primer sequence for RT-PCR

<400>  328
ataaagactt gtctagactc cactggg                                          27


<210>  329
<211>  23
<212>  DNA
<213>  Artificial

<220>
<223>  Artificially synthesized primer sequence for RT-PCR

<400>  329
gaacagtgtt tggtctggaa tgt                                              23


<210>  330
<211>  23
<212>  DNA
<213>  Artificial

<220>
<223>  Artificially synthesized primer sequence for RT-PCR

<400>  330
ggatatgaga aaggaaggca agt                                              23


<210>  331
<211>  22
<212>  DNA

<213>  Artificial

<220>
<223>  Artificially synthesized primer sequence for RT-PCR

<400>  331
atcgtgagca tcatcagaga ag                                              22


<210>  332
<211>  23
<212>  DNA
<213>  Artificial

<220>
<223>  Artificially synthesized primer sequence for RT-PCR

<400>  332
agacacacag acaaacatgc aga                                             23


<210>  333
<211>  22
<212>  DNA
<213>  Artificial

<220>
<223>  Artificially synthesized primer sequence for RT-PCR

<400>  333
gcactaccca gacatcttcg ag                                              22


<210>  334
<211>  23
<212>  DNA
<213>  Artificial

<220>
<223>  Artificially synthesized primer sequence for RT-PCR

<400>  334
tgggtggcaa gtctaatcta ttc                                             23


<210>  335
<211>  22
<212>  DNA
<213>  Artificial

<220>
<223>  Artificially synthesized primer sequence for RT-PCR

<400>  335
gatccgaaga aactggctac tg                                              22


<210>  336
<211>  23
<212>  DNA
<213>  Artificial

<220>

<223>  Artificially synthesized primer sequence for RT-PCR

<400>  336
aggtcctgct ctctttgtcc tat                                              23


<210>  337
<211>  23
<212>  DNA
<213>  Artificial

<220>
<223>  Artificially synthesized primer sequence for RT-PCR

<400>  337
gagtcttccc cattttcagt cat                                              23


<210>  338
<211>  22
<212>  DNA
<213>  Artificial

<220>
<223>  Artificially synthesized primer sequence for RT-PCR

<400>  338
ctacatttat gtggcacgaa gg                                               22


<210>  339
<211>  23
<212>  DNA
<213>  Artificial

<220>
<223>  Artificially synthesized primer sequence for RT-PCR

<400>  339
ctttggctta tttacagagc tgg                                              23


<210>  340
<211>  23
<212>  DNA
<213>  Artificial

<220>
<223>  Artificially synthesized primer sequence for RT-PCR

<400>  340
aggaggctaa aggcaatgaa tag                                              23


<210>  341
<211>  23
<212>  DNA
<213>  Artificial

<220>
<223>  Artificially synthesized primer sequence for RT-PCR

<400>  341

gagtcttgaa gctctgtttg gtg                                              23

<210>  342
<211>  23
<212>  DNA
<213>  Artificial

<220>
<223>  Artificially synthesized primer sequence for RT-PCR

<400>  342
aacatcctga cagtcatcca cat                                              23

<210>  343
<211>  23
<212>  DNA
<213>  Artificial

<220>
<223>  Artificially synthesized primer sequence for RT-PCR

<400>  343
agtgtctgca accttgcttt aac                                              23

<210>  344
<211>  23
<212>  DNA
<213>  Artificial

<220>
<223>  Artificially synthesized primer sequence for RT-PCR

<400>  344
agtccagggc ataaaaccta aac                                              23

<210>  345
<211>  23
<212>  DNA
<213>  Artificial

<220>
<223>  Artificially synthesized primer sequence for RT-PCR

<400>  345
atgtgtgtgt gttcatcttc cag                                              23

<210>  346
<211>  22
<212>  DNA
<213>  Artificial

<220>
<223>  Artificially synthesized primer sequence for RT-PCR

<400>  346
atccattttc tcacaagcag tg                                               22

<210>  347
<211>  23
<212>  DNA
<213>  Artificial

<220>
<223>  Artificially synthesized primer sequence for RT-PCR

<400>  347
ggggaatata tatcctctgt ggc                                              23


<210>  348
<211>  22
<212>  DNA
<213>  Artificial

<220>
<223>  Artificially synthesized primer sequence for RT-PCR

<400>  348
aaaaacaact gaggtgatgg gt                                               22


<210>  349
<211>  21
<212>  DNA
<213>  Artificial

<220>
<223>  Artificially synthesized primer sequence for RT-PCR

<400>  349
atgaaggaca gcaaccagtt c                                                21


<210>  350
<211>  21
<212>  DNA
<213>  Artificial

<220>
<223>  Artificially synthesized primer sequence for RT-PCR

<400>  350
caatgctggt ttattcccca t                                                21


<210>  351
<211>  23
<212>  DNA
<213>  Artificial

<220>
<223>  Artificially synthesized primer sequence for RT-PCR

<400>  351
gtgaaaaagt ggaatgcagt agc                                              23


<210>  352
<211>  23
<212>  DNA

<213>  Artificial

<220>
<223>  Artificially synthesized primer sequence for RT-PCR

<400>  352
ttaggtaaca gcagggaaag tca                                              23


<210>  353
<211>  21
<212>  DNA
<213>  Artificial

<220>
<223>  Artificially synthesized primer sequence for RT-PCR

<400>  353
cagtcctgtg actcaactca a                                                21


<210>  354
<211>  22
<212>  DNA
<213>  Artificial

<220>
<223>  Artificially synthesized primer sequence for RT-PCR

<400>  354
cgagtttcac ctcagctctt ct                                               22


<210>  355
<211>  22
<212>  DNA
<213>  Artificial

<220>
<223>  Artificially synthesized primer sequence for RT-PCR

<400>  355
ggatgtagca atctccacca gt                                               22


<210>  356
<211>  23
<212>  DNA
<213>  Artificial

<220>
<223>  Artificially synthesized primer sequence for RT-PCR

<400>  356
gttcaaacac tcactgaaga gcc                                              23


<210>  357
<211>  21
<212>  DNA
<213>  Artificial

<220>

<223>  Artificially synthesized primer sequence for RT-PCR

<400>  357
ctccactcgc tcttccaaca c                                                21


<210>  358
<211>  22
<212>  DNA
<213>  Artificial

<220>
<223>  Artificially synthesized primer sequence for RT-PCR

<400>  358
ctttttacct tcgtgcacct tt                                               22


<210>  359
<211>  23
<212>  DNA
<213>  Artificial

<220>
<223>  Artificially synthesized primer sequence for RT-PCR

<400>  359
gacagcaaag tcttgactcc ttc                                              23


<210>  360
<211>  23
<212>  DNA
<213>  Artificial

<220>
<223>  Artificially synthesized primer sequence for RT-PCR

<400>  360
aaagtggctg ggagtaaggt atc                                              23


<210>  361
<211>  22
<212>  DNA
<213>  Artificial

<220>
<223>  Artificially synthesized primer sequence for RT-PCR

<400>  361
agggcacaca ttcatctttg ta                                               22


<210>  362
<211>  23
<212>  DNA
<213>  Artificial

<220>
<223>  Artificially synthesized primer sequence for RT-PCR

<400>  362

gttaccaaag acagacacat tgg                                              23

<210>  363
<211>  23
<212>  DNA
<213>  Artificial

<220>
<223>  Artificially synthesized primer sequence for RT-PCR

<400>  363
ctcagcaaga gaagaaccgt tta                                              23

<210>  364
<211>  23
<212>  DNA
<213>  Artificial

<220>
<223>  Artificially synthesized primer sequence for RT-PCR

<400>  364
ccacttagaa atcgaatacg tcc                                              23

<210>  365
<211>  23
<212>  DNA
<213>  Artificial

<220>
<223>  Artificially synthesized primer sequence for RT-PCR

<400>  365
tacccaagtc agaaagactc tgc                                              23

<210>  366
<211>  23
<212>  DNA
<213>  Artificial

<220>
<223>  Artificially synthesized primer sequence for RT-PCR

<400>  366
ggtggccttc tctcaaaatt agt                                              23

<210>  367
<211>  23
<212>  DNA
<213>  Artificial

<220>
<223>  Artificially synthesized primer sequence for RT-PCR

<400>  367
cgctgataat attcctcgtc cta                                              23

<210>  368
<211>  23
<212>  DNA
<213>  Artificial

<220>
<223>  Artificially synthesized primer sequence for RT-PCR

<400>  368
agtttttaga gtttcagggg gtc                                              23


<210>  369
<211>  23
<212>  DNA
<213>  Artificial

<220>
<223>  Artificially synthesized primer sequence for RT-PCR

<400>  369
ctccctaggg gtagactctt ctg                                              23


<210>  370
<211>  23
<212>  DNA
<213>  Artificial

<220>
<223>  Artificially synthesized primer sequence for RT-PCR

<400>  370
gagactaggc ctcttttctg gat                                              23


<210>  371
<211>  23
<212>  DNA
<213>  Artificial

<220>
<223>  Artificially synthesized primer sequence for RT-PCR

<400>  371
ttccagctat tcttcagatg ctc                                              23


<210>  372
<211>  23
<212>  DNA
<213>  Artificial

<220>
<223>  Artificially synthesized primer sequence for RT-PCR

<400>  372
tatatggcag gtttgtgtgt ctg                                              23


<210>  373
<211>  20
<212>  DNA

<213>  Artificial

<220>
<223>  Artificially synthesized primer sequence for RT-PCR

<400>  373
atgctgcgaa cagagagctg                                                    20


<210>  374
<211>  20
<212>  DNA
<213>  Artificial

<220>
<223>  Artificially synthesized primer sequence for RT-PCR

<400>  374
aatgaaccct gctgaccttc                                                    20


<210>  375
<211>  23
<212>  DNA
<213>  Artificial

<220>
<223>  Artificially synthesized primer sequence for RT-PCR

<400>  375
tgagtctcct cttggtgatt ctg                                                23


<210>  376
<211>  23
<212>  DNA
<213>  Artificial

<220>
<223>  Artificially synthesized primer sequence for RT-PCR

<400>  376
ggaagagcaa agagagcttc atc                                                23


<210>  377
<211>  21
<212>  DNA
<213>  Artificial

<220>
<223>  Artificially synthesized primer sequence for RT-PCR

<400>  377
gctcaagtcc aaacagcact c                                                  21


<210>  378
<211>  23
<212>  DNA
<213>  Artificial

<220>

<223>  Artificially synthesized primer sequence for RT-PCR

<400>  378
acatacacag ggacgctgta aac                                              23


<210>  379
<211>  23
<212>  DNA
<213>  Artificial

<220>
<223>  Artificially synthesized primer sequence for RT-PCR

<400>  379
tcctagggga ctcttgagct tag                                              23


<210>  380
<211>  22
<212>  DNA
<213>  Artificial

<220>
<223>  Artificially synthesized primer sequence for RT-PCR

<400>  380
ataaataggt acccgtgagc cc                                               22


<210>  381
<211>  22
<212>  DNA
<213>  Artificial

<220>
<223>  Artificially synthesized primer sequence for RT-PCR

<400>  381
tatgtgctac ccacaacacc tc                                               22


<210>  382
<211>  22
<212>  DNA
<213>  Artificial

<220>
<223>  Artificially synthesized primer sequence for RT-PCR

<400>  382
gtttgagagg aacaaccagg ag                                               22


<210>  383
<211>  23
<212>  DNA
<213>  Artificial

<220>
<223>  Artificially synthesized primer sequence for RT-PCR

<400>  383

agtcttggtt tacctgtggt gac                                              23

<210>  384
<211>  21
<212>  DNA
<213>  Artificial

<220>
<223>  Artificially synthesized primer sequence for RT-PCR

<400>  384
aaaacaaaac cccagaaacc c                                                21

<210>  385
<211>  23
<212>  DNA
<213>  Artificial

<220>
<223>  Artificially synthesized primer sequence for RT-PCR

<400>  385
gggactactg tgttttgctg ttc                                              23

<210>  386
<211>  23
<212>  DNA
<213>  Artificial

<220>
<223>  Artificially synthesized primer sequence for RT-PCR

<400>  386
tgaggtcata gatttcaagg cac                                              23

<210>  387
<211>  23
<212>  DNA
<213>  Artificial

<220>
<223>  Artificially synthesized primer sequence for RT-PCR

<400>  387
taatagtacc agccatcgct cag                                              23

<210>  388
<211>  23
<212>  DNA
<213>  Artificial

<220>
<223>  Artificially synthesized primer sequence for RT-PCR

<400>  388
atcctacggc tttattgaca cct                                              23

<210>  389
<211>  23
<212>  DNA
<213>  Artificial

<220>
<223>  Artificially synthesized primer sequence for RT-PCR

<400>  389
cagccagttc tcagacactt agg                                              23


<210>  390
<211>  21
<212>  DNA
<213>  Artificial

<220>
<223>  Artificially synthesized primer sequence for RT-PCR

<400>  390
gtactcgagc catctggcct t                                                21


<210>  391
<211>  22
<212>  DNA
<213>  Artificial

<220>
<223>  Artificially synthesized primer sequence for RT-PCR

<400>  391
acttttgtgg tgtccccaag ta                                               22


<210>  392
<211>  23
<212>  DNA
<213>  Artificial

<220>
<223>  Artificially synthesized primer sequence for RT-PCR

<400>  392
ctgtgtaccc tttacccatt cct                                              23


<210>  393
<211>  23
<212>  DNA
<213>  Artificial

<220>
<223>  Artificially synthesized primer sequence for RT-PCR

<400>  393
actagagaaa tgaggggcgt atc                                              23


<210>  394
<211>  23
<212>  DNA

<213>  Artificial

<220>
<223>  Artificially synthesized primer sequence for RT-PCR

<400>  394
atctctaacc aaacatcgta gcg                                              23


<210>  395
<211>  23
<212>  DNA
<213>  Artificial

<220>
<223>  Artificially synthesized primer sequence for RT-PCR

<400>  395
ctgaggcagc tttatttcct aca                                              23


<210>  396
<211>  23
<212>  DNA
<213>  Artificial

<220>
<223>  Artificially synthesized primer sequence for RT-PCR

<400>  396
actggtgggg ttacataacc ttt                                              23


<210>  397
<211>  25
<212>  DNA
<213>  Artificial

<220>
<223>  Artificially synthesized primer sequence for RT-PCR

<400>  397
ggtagtgaaa tatggacaaa ggaca                                            25


<210>  398
<211>  22
<212>  DNA
<213>  Artificial

<220>
<223>  Artificially synthesized primer sequence for RT-PCR

<400>  398
acttctgcca tgtcgtcttt tt                                               22


<210>  399
<211>  22
<212>  DNA
<213>  Artificial

<220>

<223>  Artificially synthesized primer sequence for RT-PCR

<400>  399
acaaagagga gaaggctgac ct                                                22


<210>  400
<211>  23
<212>  DNA
<213>  Artificial

<220>
<223>  Artificially synthesized primer sequence for RT-PCR

<400>  400
ctcctcgctg ggtagaacta act                                               23


<210>  401
<211>  23
<212>  DNA
<213>  Artificial

<220>
<223>  Artificially synthesized primer sequence for RT-PCR

<400>  401
ggaaggaaag gaactacgaa atc                                               23


<210>  402
<211>  23
<212>  DNA
<213>  Artificial

<220>
<223>  Artificially synthesized primer sequence for RT-PCR

<400>  402
gttaaaagga gcacagggac ata                                               23


<210>  403
<211>  23
<212>  DNA
<213>  Artificial

<220>
<223>  Artificially synthesized primer sequence for RT-PCR

<400>  403
ctccttactt gtgggatcaa atg                                               23


<210>  404
<211>  23
<212>  DNA
<213>  Artificial

<220>
<223>  Artificially synthesized primer sequence for RT-PCR

<400>  404

atgtgctaga attacagccc tga                                              23

<210>  405
<211>  23
<212>  DNA
<213>  Artificial

<220>
<223>  Artificially synthesized primer sequence for RT-PCR

<400>  405
aggagctgag tgtgttagag gtg                                              23

<210>  406
<211>  22
<212>  DNA
<213>  Artificial

<220>
<223>  Artificially synthesized primer sequence for RT-PCR

<400>  406
ataaacctgg atgctgacgc tc                                               22

<210>  407
<211>  23
<212>  DNA
<213>  Artificial

<220>
<223>  Artificially synthesized primer sequence for RT-PCR

<400>  407
agacctaagt ctggaacaga gcc                                              23

<210>  408
<211>  23
<212>  DNA
<213>  Artificial

<220>
<223>  Artificially synthesized primer sequence for RT-PCR

<400>  408
ctacagcact catttggaaa agg                                              23

<210>  409
<211>  23
<212>  DNA
<213>  Artificial

<220>
<223>  Artificially synthesized primer sequence for RT-PCR

<400>  409
ttggtcctcc tctgtttcat aga                                              23

<210>  410
<211>  23
<212>  DNA
<213>  Artificial

<220>
<223>  Artificially synthesized primer sequence for RT-PCR

<400>  410
gcttctcccc agttacaaga gac                                              23


<210>  411
<211>  21
<212>  DNA
<213>  Artificial

<220>
<223>  Artificially synthesized primer sequence for RT-PCR

<400>  411
gtactgaagg acctgccaag g                                                21


<210>  412
<211>  23
<212>  DNA
<213>  Artificial

<220>
<223>  Artificially synthesized primer sequence for RT-PCR

<400>  412
gggaaagcca gctttattga gta                                              23


<210>  413
<211>  23
<212>  DNA
<213>  Artificial

<220>
<223>  Artificially synthesized primer sequence for RT-PCR

<400>  413
agttttggat gactctgctc aag                                              23


<210>  414
<211>  23
<212>  DNA
<213>  Artificial

<220>
<223>  Artificially synthesized primer sequence for RT-PCR

<400>  414
ggcatttacg agcattatct gac                                              23


<210>  415
<211>  23
<212>  DNA

<213>  Artificial

<220>
<223>  Artificially synthesized primer sequence for RT-PCR

<400>  415
cagtttcagt cccaggtcat act                                              23


<210>  416
<211>  23
<212>  DNA
<213>  Artificial

<220>
<223>  Artificially synthesized primer sequence for RT-PCR

<400>  416
ggcatactct ttggtgagaa atg                                              23


<210>  417
<211>  22
<212>  DNA
<213>  Artificial

<220>
<223>  Artificially synthesized primer sequence for RT-PCR

<400>  417
ctaccctgaa ggggaagaaa ag                                               22


<210>  418
<211>  22
<212>  DNA
<213>  Artificial

<220>
<223>  Artificially synthesized primer sequence for RT-PCR

<400>  418
aacacaccct acatccaagg tc                                               22


<210>  419
<211>  22
<212>  DNA
<213>  Artificial

<220>
<223>  Artificially synthesized primer sequence for RT-PCR

<400>  419
cttcagagga aatctcccag tc                                               22


<210>  420
<211>  22
<212>  DNA
<213>  Artificial

<220>

<223>  Artificially synthesized primer sequence for RT-PCR

<400>  420
ggcgttatct cgttgtactc gt                                                22


<210>  421
<211>  23
<212>  DNA
<213>  Artificial

<220>
<223>  Artificially synthesized primer sequence for RT-PCR

<400>  421
aaagctgaat acagaaggca ctg                                               23


<210>  422
<211>  23
<212>  DNA
<213>  Artificial

<220>
<223>  Artificially synthesized primer sequence for RT-PCR

<400>  422
tttactgaca ggtggtgaaa ggt                                               23


<210>  423
<211>  18
<212>  DNA
<213>  Artificial

<220>
<223>  Artificially synthesized S-oligonucleotide sequence for antisense
       method

<400>  423
taaatcctcg gattccat                                                     18


<210>  424
<211>  18
<212>  DNA
<213>  Artificial

<220>
<223>  Artificially synthesized S-oligonucleotide sequence for antisense
       method

<400>  424
taccttaggc tcctaaat                                                     18


<210>  425
<211>  18
<212>  DNA
<213>  Artificial

<220>
<223>  Artificially synthesized S-oligonucleotide sequence for antisense

method

<400> 425
cgccgcgcgc ctcatgct                                                    18


<210> 426
<211> 18
<212> DNA
<213> Artificial

<220>
<223> Artificially synthesized S-oligonucleotide sequence for antisense
      method

<400> 426
tcgtactccg cgcgccgc                                                    18


<210> 427
<211> 18
<212> DNA
<213> Artificial

<220>
<223> Artificially synthesized S-oligonucleotide sequence for antisense
      method

<400> 427
cggccgcact caccggca                                                    18


<210> 428
<211> 18
<212> DNA
<213> Artificial

<220>
<223> Artificially synthesized S-oligonucleotide sequence for antisense
      method

<400> 428
acggccactc acgccggc                                                    18


<210> 429
<211> 18
<212> DNA
<213> Artificial

<220>
<223> Artificially synthesized S-oligonucleotide sequence for antisense
      method

<400> 429
acgactgctt gaaagagg                                                    18


<210> 430
<211> 18
<212> DNA
<213> Artificial

<220>
<223>  Artificially synthesized S-oligonucleotide sequence for antisense
       method

<400>  430
ggagaaagtt cgtcagca                                                    18


<210>  431
<211>  19
<212>  DNA
<213>  Artificial

<220>
<223>  Artificially synthesized S-oligonucleotide sequence for antisense
       method

<400>  431
agtgcactcg gatcttgct                                                   19


<210>  432
<211>  19
<212>  DNA
<213>  Artificial

<220>
<223>  Artificially synthesized S-oligonucleotide sequence for antisense
       method

<400>  432
tcgttctagg ctcacgtga                                                   19


<210>  433
<211>  18
<212>  DNA
<213>  Artificial

<220>
<223>  Artificially synthesized S-oligonucleotide sequence for antisense
       method

<400>  433
gcctcctgta ctggattt                                                    18


<210>  434
<211>  18
<212>  DNA
<213>  Artificial

<220>
<223>  Artificially synthesized S-oligonucleotide sequence for antisense
       method

<400>  434
tttaggtcat gtcctccg                                                    18


<210>  435
<211>  18
<212>  DNA

<213>  Artificial

<220>
<223>  Artificially synthesized S-oligonucleotide sequence for antisense
       method

<400>  435
ttgactggtt tcttatgt                                                    18


<210>  436
<211>  18
<212>  DNA
<213>  Artificial

<220>
<223>  Artificially synthesized S-oligonucleotide sequence for antisense
       method

<400>  436
tgtattcttt ggtcagtt                                                    18


<210>  437
<211>  18
<212>  DNA
<213>  Artificial

<220>
<223>  Artificially synthesized S-oligonucleotide sequence for antisense
       method

<400>  437
ctccgtaaac ggatccat                                                    18


<210>  438
<211>  18
<212>  DNA
<213>  Artificial

<220>
<223>  Artificially synthesized S-oligonucleotide sequence for antisense
       method

<400>  438
tacctaggca aatgcctc                                                    18


<210>  439
<211>  18
<212>  DNA
<213>  Artificial

<220>
<223>  Artificially synthesized S-oligonucleotide sequence for antisense
       method

<400>  439
cggatccatc gccccagg                                                    18


<210>  440

<211>  18
<212>  DNA
<213>  Artificial

<220>
<223>  Artificially synthesized S-oligonucleotide sequence for antisense
       method

<400>  440
ggaccccgct acctaggc                                                    18


<210>  441
<211>  18
<212>  DNA
<213>  Artificial

<220>
<223>  Artificially synthesized S-oligonucleotide sequence for antisense
       method

<400>  441
accaaagacg catcatca                                                    18


<210>  442
<211>  18
<212>  DNA
<213>  Artificial

<220>
<223>  Artificially synthesized S-oligonucleotide sequence for antisense
       method

<400>  442
actactacgc agaaacca                                                    18


<210>  443
<211>  18
<212>  DNA
<213>  Artificial

<220>
<223>  Artificially synthesized S-oligonucleotide sequence for antisense
       method

<400>  443
ccctcgattc ctccgagt                                                    18


<210>  444
<211>  18
<212>  DNA
<213>  Artificial

<220>
<223>  Artificially synthesized S-oligonucleotide sequence for antisense
       method

<400>  444
tgagcctcct tagctccc                                                    18

<210>  445
<211>  18
<212>  DNA
<213>  Artificial

<220>
<223>  Artificially synthesized S-oligonucleotide sequence for antisense
       method

<400>  445
aactgccaca cagtagta                                                    18


<210>  446
<211>  18
<212>  DNA
<213>  Artificial

<220>
<223>  Artificially synthesized S-oligonucleotide sequence for antisense
       method

<400>  446
atgatgacac accgtcaa                                                    18


<210>  447
<211>  18
<212>  DNA
<213>  Artificial

<220>
<223>  Artificially synthesized S-oligonucleotide sequence for antisense
       method

<400>  447
atcctcgctg tccagggc                                                    18


<210>  448
<211>  18
<212>  DNA
<213>  Artificial

<220>
<223>  Artificially synthesized S-oligonucleotide sequence for antisense
       method

<400>  448
cgggacctgt cgctccta                                                    18


<210>  449
<211>  18
<212>  DNA
<213>  Artificial

<220>
<223>  Artificially synthesized S-oligonucleotide sequence for antisense
       method

<400>  449

cgtccaggtg cagccact                                                    18

<210>  450
<211>  18
<212>  DNA
<213>  Artificial

<220>
<223>  Artificially synthesized S-oligonucleotide sequence for antisense
        method

<400>  450
tcaccgacgt ggacctgc                                                    18

<210>  451
<211>  18
<212>  DNA
<213>  Artificial

<220>
<223>  Artificially synthesized S-oligonucleotide sequence for antisense
        method

<400>  451
gttcccattc aagaacat                                                    18

<210>  452
<211>  18
<212>  DNA
<213>  Artificial

<220>
<223>  Artificially synthesized S-oligonucleotide sequence for antisense
        method

<400>  452
tacaagaact tacccttg                                                    18

<210>  453
<211>  18
<212>  DNA
<213>  Artificial

<220>
<223>  Artificially synthesized S-oligonucleotide sequence for antisense
        method

<400>  453
catgagtgat ggtggctc                                                    18

<210>  454
<211>  18
<212>  DNA
<213>  Artificial

<220>
<223>  Artificially synthesized S-oligonucleotide sequence for antisense
        method

<400>  454
ctcggtggta gtgagtac                                                    18


<210>  455
<211>  18
<212>  DNA
<213>  Artificial

<220>
<223>  Artificially synthesized S-oligonucleotide sequence for antisense
       method

<400>  455
cctctcccat ggcttcaa                                                    18


<210>  456
<211>  18
<212>  DNA
<213>  Artificial

<220>
<223>  Artificially synthesized S-oligonucleotide sequence for antisense
       method

<400>  456
aacttcggta ccctctcc                                                    18


<210>  457
<211>  18
<212>  DNA
<213>  Artificial

<220>
<223>  Artificially synthesized S-oligonucleotide sequence for antisense
       method

<400>  457
ggacaggaac caatgtac                                                    18


<210>  458
<211>  18
<212>  DNA
<213>  Artificial

<220>
<223>  Artificially synthesized S-oligonucleotide sequence for antisense
       method

<400>  458
catgtaacca aggacagg                                                    18


<210>  459
<211>  18
<212>  DNA
<213>  Artificial

<220>

<223>  Artificially synthesized S-oligonucleotide sequence for antisense
       method

<400>  459
acaatacaat gtgacaag                                                    18


<210>  460
<211>  18
<212>  DNA
<213>  Artificial

<220>
<223>  Artificially synthesized S-oligonucleotide sequence for antisense
       method

<400>  460
gaacagtgta acataaca                                                    18


<210>  461
<211>  18
<212>  DNA
<213>  Artificial

<220>
<223>  Artificially synthesized S-oligonucleotide sequence for antisense
       method

<400>  461
agattccatt ctgcaaac                                                    18


<210>  462
<211>  18
<212>  DNA
<213>  Artificial

<220>
<223>  Artificially synthesized S-oligonucleotide sequence for antisense
       method

<400>  462
caaacgtctt accttaga                                                    18


<210>  463
<211>  19
<212>  DNA
<213>  Artificial

<220>
<223>  Artificially synthesized S-oligonucleotide sequence for antisense
       method

<400>  463
gattaaaatt agattccat                                                   19


<210>  464
<211>  19
<212>  DNA
<213>  Artificial

<220>
<223>  Artificially synthesized S-oligonucleotide sequence for antisense
       method

<400>  464
taccttagat taaaattag                                                    19


<210>  465
<211>  18
<212>  DNA
<213>  Artificial

<220>
<223>  Artificially synthesized S-oligonucleotide sequence for antisense
       method

<400>  465
catcttgaga tcctattc                                                     18


<210>  466
<211>  18
<212>  DNA
<213>  Artificial

<220>
<223>  Artificially synthesized S-oligonucleotide sequence for antisense
       method

<400>  466
cttatcctag agttctac                                                     18


<210>  467
<211>  18
<212>  DNA
<213>  Artificial

<220>
<223>  Artificially synthesized S-oligonucleotide sequence for antisense
       method

<400>  467
tggggctttt ttactcat                                                     18


<210>  468
<211>  18
<212>  DNA
<213>  Artificial

<220>
<223>  Artificially synthesized S-oligonucleotide sequence for antisense
       method

<400>  468
tactcatttt tcggggt                                                      18


<210>  469
<211>  18

<212>  DNA
<213>  Artificial

<220>
<223>  Artificially synthesized S-oligonucleotide sequence for antisense
       method

<400>  469
aggtacttta aaccactt                                                    18


<210>  470
<211>  18
<212>  DNA
<213>  Artificial

<220>
<223>  Artificially synthesized S-oligonucleotide sequence for antisense
       method

<400>  470
ttcaccaaat ttcatgga                                                    18


<210>  471
<211>  18
<212>  DNA
<213>  Artificial

<220>
<223>  Artificially synthesized S-oligonucleotide sequence for antisense
       method

<400>  471
aggagccatg gcgctcgg                                                    18


<210>  472
<211>  18
<212>  DNA
<213>  Artificial

<220>
<223>  Artificially synthesized S-oligonucleotide sequence for antisense
       method

<400>  472
ggctcgcggt accgagga                                                    18


<210>  473
<211>  18
<212>  DNA
<213>  Artificial

<220>
<223>  Artificially synthesized S-oligonucleotide sequence for antisense
       method

<400>  473
gcaatccatg gctgtggc                                                    18

<210>  474
<211>  18
<212>  DNA
<213>  Artificial

<220>
<223>  Artificially synthesized S-oligonucleotide sequence for antisense
        method

<400>  474
cggtgtcggt acctaacg                                                    18


<210>  475
<211>  18
<212>  DNA
<213>  Artificial

<220>
<223>  Artificially synthesized S-oligonucleotide sequence for antisense
        method

<400>  475
aataattacc ttgtatta                                                    18


<210>  476
<211>  18
<212>  DNA
<213>  Artificial

<220>
<223>  Artificially synthesized S-oligonucleotide sequence for antisense
        method

<400>  476
tacctaacgt ttctatct                                                    18


<210>  477
<211>  18
<212>  DNA
<213>  Artificial

<220>
<223>  Artificially synthesized S-oligonucleotide sequence for antisense
        method

<400>  477
gaagttaccg tcctgcat                                                    18


<210>  478
<211>  18
<212>  DNA
<213>  Artificial

<220>
<223>  Artificially synthesized S-oligonucleotide sequence for antisense
        method

<400>  478
tacgtcctgc cattgaag                                                    18

<210>  479
<211>  18
<212>  DNA
<213>  Artificial

<220>
<223>  Artificially synthesized S-oligonucleotide sequence for antisense
       method

<400>  479
gcaggaagtt accgtcct                                                    18


<210>  480
<211>  18
<212>  DNA
<213>  Artificial

<220>
<223>  Artificially synthesized S-oligonucleotide sequence for antisense
       method

<400>  480
tcctgccatt gaaggacg                                                    18


<210>  481
<211>  18
<212>  DNA
<213>  Artificial

<220>
<223>  Artificially synthesized S-oligonucleotide sequence for antisense
       method

<400>  481
gttgttgagc acagctat                                                    18


<210>  482
<211>  18
<212>  DNA
<213>  Artificial

<220>
<223>  Artificially synthesized S-oligonucleotide sequence for antisense
       method

<400>  482
tatcgacacg agttgttg                                                    18


<210>  483
<211>  18
<212>  DNA
<213>  Artificial

<220>
<223>  Artificially synthesized S-oligonucleotide sequence for antisense
       method

<400>  483
gaagtcctcc ttccgata                                                    18


<210>  484
<211>  18
<212>  DNA
<213>  Artificial

<220>
<223>  Artificially synthesized S-oligonucleotide sequence for antisense
        method

<400>  484
atagccttcc tcctgaag                                                    18


<210>  485
<211>  18
<212>  DNA
<213>  Artificial

<220>
<223>  Artificially synthesized S-oligonucleotide sequence for antisense
        method

<400>  485
ccgtcgccat cttgcgtc                                                    18


<210>  486
<211>  18
<212>  DNA
<213>  Artificial

<220>
<223>  Artificially synthesized S-oligonucleotide sequence for antisense
        method

<400>  486
ctgcgttcta ccgctgcc                                                    18


<210>  487
<211>  18
<212>  DNA
<213>  Artificial

<220>
<223>  Artificially synthesized S-oligonucleotide sequence for antisense
        method

<400>  487
tatggtcgcc gtcgccat                                                    18


<210>  488
<211>  18
<212>  DNA
<213>  Artificial

<220>
<223>  Artificially synthesized S-oligonucleotide sequence for antisense

method

<400> 488
taccgctgcc gctggtat 18


<210> 489
<211> 18
<212> DNA
<213> Artificial

<220>
<223> Artificially synthesized S-oligonucleotide sequence for antisense
 method

<400> 489
ggactgcatg gtggagat 18


<210> 490
<211> 18
<212> DNA
<213> Artificial

<220>
<223> Artificially synthesized S-oligonucleotide sequence for antisense
 method

<400> 490
tagaggtggt acgtcagg 18


<210> 491
<211> 18
<212> DNA
<213> Artificial

<220>
<223> Artificially synthesized S-oligonucleotide sequence for antisense
 method

<400> 491
catggtggag atggcgac 18


<210> 492
<211> 18
<212> DNA
<213> Artificial

<220>
<223> Artificially synthesized S-oligonucleotide sequence for antisense
 method

<400> 492
cagcggtaga ggtggtac 18


<210> 493
<211> 18
<212> DNA
<213> Artificial

<220>
<223>  Artificially synthesized S-oligonucleotide sequence for antisense
       method

<400>  493
agcagggctg cagaatgg                                                    18


<210>  494
<211>  18
<212>  DNA
<213>  Artificial

<220>
<223>  Artificially synthesized S-oligonucleotide sequence for antisense
       method

<400>  494
ggtaagacgt cgggacga                                                    18


<210>  495
<211>  18
<212>  DNA
<213>  Artificial

<220>
<223>  Artificially synthesized S-oligonucleotide sequence for antisense
       method

<400>  495
tgctcttgaa gtcgggac                                                    18


<210>  496
<211>  18
<212>  DNA
<213>  Artificial

<220>
<223>  Artificially synthesized S-oligonucleotide sequence for antisense
       method

<400>  496
cagggatgaa gttctcgt                                                    18


<210>  497
<211>  18
<212>  DNA
<213>  Artificial

<220>
<223>  Artificially synthesized S-oligonucleotide sequence for antisense
       method

<400>  497
gcagttgaga tgattatt                                                    18


<210>  498
<211>  18
<212>  DNA

<213>  Artificial

<220>
<223>  Artificially synthesized S-oligonucleotide sequence for antisense
       method

<400>  498
ttattagtag agttgacg                                                    18


<210>  499
<211>  18
<212>  DNA
<213>  Artificial

<220>
<223>  Artificially synthesized S-oligonucleotide sequence for antisense
       method

<400>  499
caaaatcatt tcctcctc                                                    18


<210>  500
<211>  18
<212>  DNA
<213>  Artificial

<220>
<223>  Artificially synthesized S-oligonucleotide sequence for antisense
       method

<400>  500
ctcctccttt actaaaac                                                    18


<210>  501
<211>  18
<212>  DNA
<213>  Artificial

<220>
<223>  Artificially synthesized S-oligonucleotide sequence for antisense
       method

<400>  501
cgggccacca tcacggaa                                                    18


<210>  502
<211>  18
<212>  DNA
<213>  Artificial

<220>
<223>  Artificially synthesized S-oligonucleotide sequence for antisense
       method

<400>  502
aaggcactac caccgggc                                                    18


<210>  503

<211>  18
<212>  DNA
<213>  Artificial

<220>
<223>  Artificially synthesized S-oligonucleotide sequence for antisense
       method

<400>  503
acgattcatt gctgcctt                                                    18


<210>  504
<211>  18
<212>  DNA
<213>  Artificial

<220>
<223>  Artificially synthesized S-oligonucleotide sequence for antisense
       method

<400>  504
ttccgtcgtt acttagca                                                    18


<210>  505
<211>  18
<212>  DNA
<213>  Artificial

<220>
<223>  Artificially synthesized S-oligonucleotide sequence for antisense
       method

<400>  505
acacaagaca cgattcat                                                    18


<210>  506
<211>  18
<212>  DNA
<213>  Artificial

<220>
<223>  Artificially synthesized S-oligonucleotide sequence for antisense
       method

<400>  506
tacttagcac agaacaca                                                    18


<210>  507
<211>  18
<212>  DNA
<213>  Artificial

<220>
<223>  Artificially synthesized S-oligonucleotide sequence for antisense
       method

<400>  507
atccactctt ccgttcat                                                    18

<210>  508
<211>  18
<212>  DNA
<213>  Artificial

<220>
<223>  Artificially synthesized S-oligonucleotide sequence for antisense
       method

<400>  508
tacttgcctt ctcaccta                                                    18


<210>  509
<211>  18
<212>  DNA
<213>  Artificial

<220>
<223>  Artificially synthesized S-oligonucleotide sequence for antisense
       method

<400>  509
agccgccatc tccacagt                                                    18


<210>  510
<211>  18
<212>  DNA
<213>  Artificial

<220>
<223>  Artificially synthesized S-oligonucleotide sequence for antisense
       method

<400>  510
tgacacctct accgccga                                                    18


<210>  511
<211>  19
<212>  DNA
<213>  Artificial

<220>
<223>  Artificially synthesized S-oligonucleotide sequence for antisense
       method

<400>  511
cttgttcatg aacatctct                                                   19


<210>  512
<211>  19
<212>  DNA
<213>  Artificial

<220>
<223>  Artificially synthesized S-oligonucleotide sequence for antisense
       method

<400>  512

tctctacaag tacttgttc                                                    19

<210>  513
<211>  18
<212>  DNA
<213>  Artificial

<220>
<223>  Artificially synthesized S-oligonucleotide sequence for antisense
        method

<400>  513
tggcaggagg gttcttgt                                                     18

<210>  514
<211>  18
<212>  DNA
<213>  Artificial

<220>
<223>  Artificially synthesized S-oligonucleotide sequence for antisense
        method

<400>  514
tacttgttct tgggagga                                                     18

<210>  515
<211>  18
<212>  DNA
<213>  Artificial

<220>
<223>  Artificially synthesized S-oligonucleotide sequence for antisense
        method

<400>  515
caggcctacc tggcagga                                                     18

<210>  516
<211>  18
<212>  DNA
<213>  Artificial

<220>
<223>  Artificially synthesized S-oligonucleotide sequence for antisense
        method

<400>  516
aggacggtcc atccggac                                                     18

<210>  517
<211>  18
<212>  DNA
<213>  Artificial

<220>
<223>  Artificially synthesized S-oligonucleotide sequence for antisense
        method

<400>  517
accgcttacg gttggctg                                                    18


<210>  518
<211>  18
<212>  DNA
<213>  Artificial

<220>
<223>  Artificially synthesized S-oligonucleotide sequence for antisense
       method

<400>  518
gtcggttggc attcgcca                                                    18


<210>  519
<211>  18
<212>  DNA
<213>  Artificial

<220>
<223>  Artificially synthesized S-oligonucleotide sequence for antisense
       method

<400>  519
tctgaagaaa atagatca                                                    18


<210>  520
<211>  18
<212>  DNA
<213>  Artificial

<220>
<223>  Artificially synthesized S-oligonucleotide sequence for antisense
       method

<400>  520
actagataaa agaagtct                                                    18


<210>  521
<211>  18
<212>  DNA
<213>  Artificial

<220>
<223>  Artificially synthesized S-oligonucleotide sequence for antisense
       method

<400>  521
gtcaaccaga cccggcat                                                    18


<210>  522
<211>  18
<212>  DNA
<213>  Artificial

<220>

<223>  Artificially synthesized S-oligonucleotide sequence for antisense
       method

<400>  522
tacggcccag accaactg                                                    18


<210>  523
<211>  18
<212>  DNA
<213>  Artificial

<220>
<223>  Artificially synthesized S-oligonucleotide sequence for antisense
       method

<400>  523
tctcctttct cgatcata                                                    18


<210>  524
<211>  18
<212>  DNA
<213>  Artificial

<220>
<223>  Artificially synthesized S-oligonucleotide sequence for antisense
       method

<400>  524
atagtagctc tttcctct                                                    18


<210>  525
<211>  18
<212>  DNA
<213>  Artificial

<220>
<223>  Artificially synthesized S-oligonucleotide sequence for antisense
       method

<400>  525
attccttgtg ggcctcaa                                                    18


<210>  526
<211>  18
<212>  DNA
<213>  Artificial

<220>
<223>  Artificially synthesized S-oligonucleotide sequence for antisense
       method

<400>  526
aactccgggt gttcctta                                                    18


<210>  527
<211>  17
<212>  DNA
<213>  Artificial

<220>
<223>  Artificially synthesized S-oligonucleotide sequence for antisense
       method

<400>  527
cccatgcgag ctgcgcc                                                    17


<210>  528
<211>  17
<212>  DNA
<213>  Artificial

<220>
<223>  Artificially synthesized S-oligonucleotide sequence for antisense
       method

<400>  528
ccgcgtcgag cgtaccc                                                    17


<210>  529
<211>  18
<212>  DNA
<213>  Artificial

<220>
<223>  Artificially synthesized S-oligonucleotide sequence for antisense
       method

<400>  529
agtgataaac agaaagcg                                                   18


<210>  530
<211>  18
<212>  DNA
<213>  Artificial

<220>
<223>  Artificially synthesized S-oligonucleotide sequence for antisense
       method

<400>  530
gcgaaagaca aatagtga                                                   18


<210>  531
<211>  18
<212>  DNA
<213>  Artificial

<220>
<223>  Artificially synthesized S-oligonucleotide sequence for antisense
       method

<400>  531
tatcctcgac tttgactt                                                   18


<210>  532
<211>  18

<212>  DNA
<213>  Artificial

<220>
<223>  Artificially synthesized S-oligonucleotide sequence for antisense
        method

<400>  532
ttcagtttca gctcctat                                                    18


<210>  533
<211>  19
<212>  DNA
<213>  Artificial

<220>
<223>  Artificially synthesized oligonucleotide sequence for RNAi

<400>  533
ggaccaagct agacaagca                                                   19


<210>  534
<211>  19
<212>  DNA
<213>  Artificial

<220>
<223>  Artificially synthesized oligonucleotide sequence for RNAi

<400>  534
acagtgttcc gctaagtga                                                   19


<210>  535
<211>  19
<212>  DNA
<213>  Artificial

<220>
<223>  Artificially synthesized oligonucleotide sequence for RNAi

<400>  535
ccagttgagt cgacatctg                                                   19


<210>  536
<211>  19
<212>  DNA
<213>  Artificial

<220>
<223>  Artificially synthesized oligonucleotide sequence for RNAi

<400>  536
gcagcagata ccatcagtg                                                   19


<210>  537
<211>  20
<212>  DNA
<213>  Artificial

<220>
<223>  Artificially synthesized oligonucleotide sequence for RNAi

<400>  537
cgcagctgcg aagtgttgta                                                    20


<210>  538
<211>  19
<212>  DNA
<213>  Artificial

<220>
<223>  Artificially synthesized oligonucleotide sequence for RNAi

<400>  538
gatacgaaag cagctgcga                                                     19


<210>  539
<211>  19
<212>  DNA
<213>  Artificial

<220>
<223>  Artificially synthesized oligonucleotide sequence for RNAi

<400>  539
gagcgattca tcttcatca                                                     19


<210>  540
<211>  19
<212>  DNA
<213>  Artificial

<220>
<223>  Artificially synthesized oligonucleotide sequence for RNAi

<400>  540
ctgcaattga ggctccttc                                                     19


<210>  541
<211>  19
<212>  DNA
<213>  Artificial

<220>
<223>  Artificially synthesized oligonucleotide sequence for RNAi

<400>  541
gagtgtgctg gtgaagcag                                                     19


<210>  542
<211>  19
<212>  DNA
<213>  Artificial

<220>
<223>  Artificially synthesized oligonucleotide sequence for RNAi

<400>  542
gatcaagtcc tgcacactg                                                    19


<210>  543
<211>  19
<212>  DNA
<213>  Artificial

<220>
<223>  Artificially synthesized oligonucleotide sequence for RNAi

<400>  543
cgtgctagca gctgcgtgt                                                    19


<210>  544
<211>  19
<212>  DNA
<213>  Artificial

<220>
<223>  Artificially synthesized oligonucleotide sequence for RNAi

<400>  544
tgaggtgctc agcacagtg                                                    19


<210>  545
<211>  19
<212>  DNA
<213>  Artificial

<220>
<223>  Artificially synthesized oligonucleotide sequence for RNAi

<400>  545
cggaggatct catgaccac                                                    19


<210>  546
<211>  19
<212>  DNA
<213>  Artificial

<220>
<223>  Artificially synthesized oligonucleotide sequence for RNAi

<400>  546
gattcgcatc ctgccatcg                                                    19


<210>  547
<211>  19
<212>  DNA
<213>  Artificial

<220>
<223>  Artificially synthesized oligonucleotide sequence for RNAi

<400>  547
cagtattcgg acatagagg                                                    19

<210> 548
<211> 19
<212> DNA
<213> Artificial

<220>
<223> Artificially synthesized oligonucleotide sequence for RNAi

<400> 548
caccaagtac tgcttgtgc 19

<210> 549
<211> 19
<212> DNA
<213> Artificial

<220>
<223> Artificially synthesized oligonucleotide sequence for RNAi

<400> 549
ggagaagaac actgtggac 19

<210> 550
<211> 19
<212> DNA
<213> Artificial

<220>
<223> Artificially synthesized oligonucleotide sequence for RNAi

<400> 550
gacaaattga gtggcagca 19

<210> 551
<211> 19
<212> DNA
<213> Artificial

<220>
<223> Artificially synthesized oligonucleotide sequence for RNAi

<400> 551
gagattcaga gtggacgaa 19

<210> 552
<211> 19
<212> DNA
<213> Artificial

<220>
<223> Artificially synthesized oligonucleotide sequence for RNAi

<400> 552
gagagcaatg aggatgact 19

## Description

The present application is related to USSN 60/414,673 , filed September 30, 2002, USSN 60/451,374, filed February 28, 2003, and USSN 60/466,100, filed April 28,2003, which is incorporated herein by reference.

### Technical Field

The present invention relates to the field of biological science, more specifically to the field of cancer research. In particular, the invention relates to methods of diagnosing non-small cell lung cancers and genes with elevated or decreased expression in such cancerous cells.

### Background Art

Lung cancer is one of the most commonly fatal human tumors. Many genetic alterations associated with the development and progression of lung cancer has been reported. Although genetic changes can aid prognostic efforts and predictions of metastatic risk or response to certain treatments, information about a single or a limited number of molecular markers generally fails to provide satisfactory results for clinical diagnosis of non-small cell lung cancer (NSCLC)(Mitsudomi et al., Clin Cancer Res 6: 4055-63 (2000); Niklinski et aL, Lung Cancer. 34 Suppl 2: S53-8 (2001); Watine, Bmj 320: 379-80 (2000)). Non-small cell lung cancer (NSCLC) is by far the most common form, accounting for nearly 80% of lung tumors (Society, A. C. Cancer Facts and Figures 2001, 2001). The overall 10-year survival rate remains as low as 10% despite recent advances in multi-modality therapy, because the majority of NSCLCs are not diagnosed until advanced stages (Fry, W. A, Phillips, J. L., and Menck, H. R Ten-year survey of lung cancer treatment and survival in hospitals in the United States: a national cancer data base report, Cancer. 86: 1867-76., 1999.). Although chemotherapy regimens based on platinum are considered the reference standards for treatment of NSCLC, those drugs are able to extend survival of patients with advanced NSCLC only about six weeks (Chemotherapy in non-small cell lung cancer: a meta-analysis using updated data on individual patients from 52 randomised clinical trials. Non-small Cell Lung Cancer Collaborative Group, Bmj. 311: 899-909., 1995.). Numerous targeted therapies are being investigated for this disease, including tyrosine kinase inhibitors, but so far promising results have been achieved in only a limited number of patients and some recipients suffer severe adverse reactions (Kris M, N. R, Herbst RS A phase II trial of ZD1839 ('Iressa') in advanced non-small cell lung cancer (NSCLC) patients who had failed platinum and docetaxel-based regimens (IDEAL 2)., Proc Am Soc Clin Oncol. 21:292a(A1166), 2002).

cDNA microarray technologies have enabled to obtain comprehensive profiles of gene expression in normal and malignant cells, and compare the gene expression in malignant and corresponding normal cells (Okabe et al., Cancer Res 61:2129-37 (2001); Kitahara et al., Cancer Res 61: 3544-9 (2001); Lin et al., Oncogene 21:4120-8 (2002); Hasegawa et al., Cancer Res 62:7012-7 (2002)). This approach enables to disclose the complex nature of cancer cells, and helps to understand the mechanism of carcinogenesis. Identification of genes that are deregulated in tumors can lead to more precise and accurate diagnosis of individual cancers, and to develop novel therapeutic targets (Bienz and Clevers, Cell 103:311-20 (2000)). To disclose mechanisms underlying tumors from a genome-wide point of view, and discover target molecules for diagnosis and development of novel therapeutic drugs, the present inventors have been analyzing the expression profiles of tumor cells using a cDNA microarray of 23040 genes (Okabe et al., Cancer Res 61:2129-37 (2001); Kitahara et al., Cancer Res 61:3544-9 (2001); Lin et al., Oncogene 21:4120-8 (2002); Hasegawa et al., Cancer Res 62:7012-7 (2002)).

Studies designed to reveal mechanisms of carcinogenesis have already facilitated identification of molecular targets for anti-tumor agents. For example, inhibitors of farnexyltransferase (FTIs) which were originally developed to inhibit the growth-signaling pathway related to Ras, whose activation depends on posttranslational farnesylation, has been effective in treating Ras-dependent tumors in animal models (He et al., Cell 99:335-45 (1999)). Clinical trials on human using a combination or anti-cancer drugs and anti-HER2 monoclonal antibody, trastuzumab, have been conducted to antagonize the proto-oncogene receptor HER2/neu; and have been achieving improved clinical response and overall survival of breast-cancer patients (Lin et al., Cancer Res 61:6345-9 (2001)). A tyrosine kinase inhibitor, STI-571, which selectively inactivates bcr-abl fusion proteins, has been developed to treat chronic myelogenous leukemias wherein constitutive activation of bcr-abl tyrosine kinase plays a crucial role in the transformation of leukocytes. Agents of these kinds are designed to suppress oncogenic activity of specific gene products (Fujita et al., Cancer Res 61:7722-6 (2001)). Therefore, gene products commonly up-regulated in cancerous cells may serve as potential targets for developing novel anti-cancer agents.

It has been demonstrated that CD8+ cytotoxic T lymphocytes (CTLs) recognize epitope peptides derived from tumor-associated antigens (TAAs) presented on MHC Class I molecule, and lyse tumor cells. Since the discovery of MAGE family as the first example of TAAs, many other TAAs have been discovered using immunological approaches (Boon, Int J Cancer 54: 177-80 (1993); Boon and van der Bruggen, J Exp Med 183: 725-9 (1996); van der Bruggen et al., Science 254: 1643-7 (1991); Brichard et al., J Exp Med 178: 489-95 (1993); Kawakami et al., J Exp Med 180: 347-52 (1994)). Some of the discovered TAAs are now in the stage of clinical development as targets of immunotherapy. TAAs discovered so far include MAGE (van der Bruggen et al., Science 254: 1643-7 (1991)), gp100 (Kawakami et al., J Exp Med 180: 347-52 (1994)), SART (Shichijo et al., J Exp Med 187: 277-88 (1998)), and NY-ESO-1 (Chen et al., Proc Natl Acad Sci USA 94: 1914-8 (1997)). On the other hand, gene products which had been demonstrated to be specifically overexpressed in tumor cells, have been shown to be recognized as targets inducing cellular immune responses. Such gene products include p53 (Umano et al., Brit J Cancer 84: 1052-7 (2001)), HER2/neu (Tanaka et al., Brit J Cancer 84: 94-9 (2001)), CEA (Nukaya et al., Int J Cancer 80: 92-7 (1999)), and so on.

In spite of significant progress in basic and clinical research concerning TAAs (Rosenbeg et al., Nature Med 4: 321-7 (1998); Mukherji et al., Proc Natl Acad Sci USA 92: 8078-82 (1995); Hu et al., Cancer Res 56: 2479-83 (1996)), only limited number of candidate TAAs for the treatment of adenocarcinomas, including colorectal cancer, are available. TAAs abundantly expressed in cancer cells, and at the same time which expression is restricted to cancer cells would be promising candidates as immunotherapeutic targets. Further, identification of new TAAs inducing potent and specific antitumor immune responses is expected to encourage clinical use of peptide vaccination strategy in various types of cancer (Boon and can der Bruggen, J Exp Med 183: 725-9 (1996); van der Bruggen et al., Science 254: 1643-7 (1991); Brichard et al., J Exp Med 178: 489-95 (1993); Kawakami et al.,J Exp Med 180: 347-52 (1994); Shichijo et al., J Exp Med 187: 277-88 (1998); Chen et al., Proc Natl Acad Sci USA 94: 1914-8 (1997); Harris, J Natl Cancer Inst 88: 1442-5 (1996); Butterfield et al., Cancer Res 59: 3134-42 (1999); Vissers et al., Cancer Res 59: 5554-9 (1999); van der Burg et al., J Immunol 156: 3308-14 (1996); Tanaka et al., Cancer Res 57: 4465-8 (1997); Fujie et al., Int J Cancer 80: 169-72 (1999); Kikuchi et al., Int J Cancer 81: 459-66 (1999); Oiso et al., Int J Cancer 81: 387-94 (1999)).

It has been repeatedly reported that peptide-stimulated peripheral blood mononuclear cells (PBMCs) from certain healthy donors produce significant levels of IFN-γ in response to the peptide, but rarely exert cytotoxicity against tumor cells in an HLA-A24 or -A0201 restricted manner in ⁵¹Cr-release assays (Kawano et al., Cance Res 60: 3550-8 (2000); Nishizaka et al., Cancer Res 60: 4830-7 (2000); Tamura et al., Jpn J Cancer Res 92: 762-7 (2001)). However, both of HLA-A24 and HLA-A0201 are one of the popular HLA alleles in Japanese, as well as Caucasian (Date et al., Tissue Antigens 47: 93-101 (1996); Kondo et al., J Immunol 155: 4307-12 (1995); Kubo et al., J Immunol 152: 3913-24 (1994); Imanishi et al., Proceeding of the eleventh International Hictocompatibility Workshop and Conference Oxford University Press, Oxford, 1065 (1992); Williams et al., Tissue Antigen 49: 129 (1997)). Thus, antigenic peptides of cancers presented by these HLAs may be especially useful for the treatment of cancers among Japanese and Caucasian. Further, it is known that the induction of low-affinity CTL *in vitro* usually results from the use of peptide at a high concentration, generating a high level of specific peptide/MHC complexes on antigen presenting cells (APCs), which will effectively activate these CTL (Alexander-Miller et al., Proc Natl Acad Sci USA 93: 4102-7 (1996)).

### Summary of the Invention

The present invention is based on the discovery of a pattern of gene expression correlated with non-small cell lung cancer, *e.g*., squamous cell carcinoma, adenocarcinoma (*i.e*., acinar, papillary and bronchoalveolar), large cell carcinoma (*i.e*., giant cell and clear cell), adenosquamous carcinoma and undifferentiated carcinoma.

The genes that are differentially expressed in non-small cell lung cancer are collectively referred to herein as "non-small cell lung cancer-associated gene", "NSC nucleic acids" or "NSC polynucleotides", and polypeptides encoded by the genes are referred to as "NSC polypeptides" or "NSC proteins". Herein, differentially expressed in non-small cell lung cancer indicates that the expression level of a gene in a non-small cell lung cancer cell differs from that in a normal cell. A normal cell is one obtained from lung tissue.

Thus, the invention features a method of diagnosing or determining a predisposition to non-small cell lung cancer in a subject by determining an expression level of a non-small cell lung cancer-associated gene in a patient derived biological sample. A non-small cell lung cancer-associated gene includes, *e.g*., NSC 1-1448 (see Tables 1-3). An alteration, *e.g*., increase or decrease of the expression level of a gene compared to a normal control level of the gene indicates that the subject suffers from or is at risk of developing non-small cell lung cancer.

A "normal control level" indicates an expression level of a gene detected in a normal, healthy individual or in a population of individuals known not to be suffering from non-small cell lung cancer. A control level is a single expression pattern derived from a single reference population or from a plurality of expression patterns. In contrast to a "normal control level", the "control level" is an expression level of a gene detected in an individual or a population of individuals whose background of the disease state is known (i.e., cancerous or non-cancerous). Thus, the control level may be determined base on the expression level of a gene in a normal, healthy individual, in a population of individuals known not to be suffering from non-small cell lung cancer, a patient of non-small cell lung cancer or a population of the patients. The control level corresponding to the expression level of a gene in a patient of non-small cell lung cancer or a population of the patients are referred to as "non-small cell lung cancer control level". Furthermore, the control level can be a database of expression patterns from previously tested cells.

An increase in the expression level of any one or a panel of the genes of NSC 807-1448 detected in a test biological sample compared to a normal control level indicates that the subject (from which the sample was obtained) suffers from or is at risk of developing non-small cell lung cancer. In contrast, a decrease in the expression level of any one or a panel of the genes of NSC 1-806 detected in a test biological sample compared to a normal control level indicates that the subject suffers from or is at risk of developing non-small cell lung cancer. Alternatively, the expression level of any one or a panel of non-small cell lung cancer-associated genes in a biological sample may be compared to a non-small cell lung cancer control level of the same gene or the same panel of genes.

Gene expression is increased or decreased 10%, 25%, 50% or more compared to the control level. Alternatively, gene expression is increased or decreased 1, 2, 5 or more fold compared to the control level. Expression is determined by detecting hybridization, *e.g.*, on a chip or an array, of a non-small cell lung cancer-associated gene probe to a gene transcript of a patient-derived biological sample. The patient-derived biological sample may be any sample derived from a subject, *e.g*., a patient known to or suspected of having non-small cell lung cancer. For example, the biological sample may be tissue containing sputum, blood, serum, plasma or lung cell.

The invention also provides a non-small cell lung cancer reference expression profile comprising a pattern of gene expression levels of two or more of NSC 1-1448.

The invention further provides methods of identifying compounds that inhibit or enhance the expression or activity of a non-small cell lung cancer-associated gene (*e.g*., NSC 1-1448) by contacting a test cell expressing a non-small cell lung cancer-associated gene with a test compound and determining the expression level or activity of the non-small cell lung cancer-associated gene. The test cell may be a lung cell such as a lung epithelial cell. A decrease of the expression level compared to a normal control level of the gene indicates that the test compound is an inhibitor of the expression or function of the non-small cell lung cancer-associated gene. Therefore, if a compound suppresses the expression level of a non-small cell lung cancer-associated gene of NSC 807-1448 compared to a normal control level, the compound is expected to reduce a symptom of non-small cell lung cancer. Alternatively, an increase of the expression level or activity compared to a normal control level of the gene indicates that said test compound is an enhancer of the expression or function of the non-small cell lung cancer-associated gene. The compounds that increase the expression level of a non-small cell lung-associated gene of NSC 1-806 are expected to reduce a symptom of non-small cell lung cancer.

Alternatively, the present invention provides a method of screening for a compound for treating or preventing non-small cell lung cancer. The method includes contacting a NSC polypeptide with a test compound, and selecting the test compound that binds to or alters the biological activity of the NSC polypeptide. The invention further provides a method of screening for a compound for treating or preventing non-small cell lung cancer, which includes the steps of contacting a test compound with a cell that expresses the NSC protein or introduced with a vector comprising the transcriptional regulatory region of the NSC gene upstream of a reporter gene, and then selecting the test compound that alters the expression level of the NSC protein or protein encoded by the reporter gene. According to these screening methods, when a polypeptide encoded by NSC 807-1448 or a cell expressing the protein encoded by NSC 807-1448 or the transcriptional regulatory region of NSC 807-1448 is used, the test compound that suppresses the biological activity or the expression level compared to a normal control level is expected to reduce a symptom of non-small cell lung cancer. Alternatively, when a polypeptide encoded by NSC 1-806 or a cell expressing the protein encoded by NSC 1-806 or the transcriptional regulatory region of NSC 1-806 is used, the test compound that increases the expression level expected to reduce a symptom of non-small cell lung cancer.

The invention also provides a kit comprising two or more detection reagents which bind to one or more NSC nucleic acids or which binds to a gene product (e.g., mRNA and polypeptide) of the NSC nucleic acids. Also provided is an array of polynucleotides that binds to one or more NSC nucleic acids.

Methods for treating or preventing non-small cell lung cancer and compositions to be used for such methods are also provided. Therapeutic methods include a method of treating or preventing non-small cell lung cancer in a subject by administering to the subject a composition of an antisense, short interfering RNA (siRNA) or a ribozyme that reduce the expression of a gene of NSC 807-1448, or a composition comprising an antibody or fragment thereof that binds and suppresses the function of a polyepeptide encoded by the gene.

The invention also includes vaccines and vaccination methods. For example, a method of treating or preventing non-small cell lung cancer in a subject is carried out by administering to the subject a vaccine containing a polypeptide encoded by a polynucleotide of NSC 807-1448 or an immunologically active fragment of the polypeptide. An immunologically active fragment is a polypeptide that is shorter in length than the full-length naturally-occurring protein and which induces an immune response upon introduction into the body. For example, an immunologically active fragment includes a polypeptide at least 8 residues in length that stimulates an immune cell such as a T cell or a B cell *in vivo.* Immune cell stimulation can be measured by detecting cell proliferation, elaboration of cytokines (*e.g*., IL-2) or production of antibody.

Other therapeutic methods include those wherein a compound that increases the expression level of a gene of NSC 1-806 or the activity of a polypeptide encoded by the gene of NSC 1-806 is administered to the subject. Alternatively, non-small cell lung cancer may be treated or prevented by administering a polynucleotide (e.g., included in a vector) of NSC 1-806 or a polypeptide encoded by the polynucleotide. Furthermore, the present invention provides methods for treating or preventing non-small cell lung cancer wherein a compound selected by the screening method of the present invention is administered.

In a further aspect, the invention provides a substantially pure polypeptide comprising the amino acid sequence of SEQ ID NO: 2. The amino acid sequence may be mutated by substitution, deletion, insertion and/or addition of at least 1, 2, 3, 5, 10, 25, S0, 100 or 200 amino acids so long as the polypeptide having the amino acid sequence retains one or more biological activities of a protein consisting of the amino acid sequence of SEQ ID NO: 2. The mutated polypeptide is at least 85%, 90%, 95 % or 99% identical to a polypeptide that includes the amino acid sequences of SEQ ID NO: 2. Also included is a polypeptide encoded by a polynucleotide that hybridizes to the nucleic acid sequence of SEQ ID NO: 1. The polynucleotide hybridizes under stringent, moderately stringent or low stringent conditions to the nucleotide sequence of SEQ ID NO: 1.

As used herein, the phrase "stringent (hybridization) conditions" refers to conditions under which a probe, primer or oligonucleotide will hybridize to its target sequence, but to no other sequences. Stringent conditions are sequence-dependent and will be different under different circumstances. Specific hybridization of longer sequences are observed at higher temperatures than shorter sequences. Generally, the temperature of a stringent condition is selected to be about 5°C lower than the thermal melting point (Tₘ) for a specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (*e.g*., 10 to 50 nucleotides) and at least about 60°C for longer probes, primers and oligonucleotides. Stringent conditions may also be achieved with the addition of destabilizing agents, such as formamide.

The present invention further provides isolated polynucleotides encoding the above-described polypeptides of the present invention. As used herein, an isolated polynucleotide is a polynucleotide the structure of which is not identical to that of any naturally occurring polynucleotide or to that of any fragment of a naturally occurring genomic polynucleotide spanning more than three separate genes. The term therefore includes, for example, (a) a DNA which has the sequence of part of a naturally occurring genomic DNA molecule in the genome of the organism in which it naturally occurs; (b) a polynucleotide incorporated into a vector or into the genomic DNA of a prokaryote or eukaryote in a manner such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, *i.e*., a gene encoding a fusion polypeptide. Preferably, the isolated nucleic acid molecule is at least 65%, 70%, 75%, 80%, 85%, 90%, 91%, 92%, 93%, 94%, 95%, 96%, 97%, 98%, 99%, or more, identical to the nucleotide sequence shown in SEQ ID NO: 1. In the case of an isolated polynucleotide which is longer than or equivalent in length to the reference sequence, e.g., SEQ ID NO: 1, the comparison is made with the full length of the reference sequence. Where the isolated polynucleotide is shorter than the reference sequence, e.g., shorter than SEQ ID NO: 1, the comparison is made to segment of the reference sequence of the same length (excluding any loop required by the homology calculation).

Also included in the invention is a vector containing one or more of the nucleic acids described herein and a cell containing the vectors or nucleic acids of the invention. The invention is also directed to host cells transformed with a vector comprising any of the polynucleotides described above.

The invention also features methods for producing the polypeptides described herein by culturing a cell containing a vector comprising the isolated polynucleotide of SEQ ID NO: 1.

In still a further aspect, the invention provides an antibody that specifically binds to the polypeptides of SEQ ID NO: 2, or a fragment thereof. The antibody may be monoclonal or polyclonal. In part, a polynucleotide that is complementary to or an antisense polynucleotide (*e.g*., antisense DNA), ribozyme and siRNA (small interfering RNA) of the polynucleotides of the invention is also provided. Such polynucleotide constructs may be used for detecting the polynucleotide of the invention, *i.e*., diagnosing non-small cell lung cancer, or for treating or preventing the disease.

The present invention further provides antisense polynucleotides having the nucleotide sequence of SEQ ID NO: 423, 425, 427, 429, 431, 433, 435, 437, 439, 441, 443, 445, 447, 449, 451, 453, 455, 457, 459, 461, 463, 465, 467, 469, 471, 473, 475, 477, 479, 481, 483, 485, 487, 489, 491, 493, 495, 497, 499, 501, 503, 505, 507, 509, 511, 513, 515, 517, 519, 521, 523, 525, 527, 529, or 531. All of the polynucleotides having any of these nucleotide sequences were demonstrated to be effective for suppressing focus formation of NSCLC cell lines.

Furthermore, the present invention provides siRNAs having the nucleotide sequence of SEQ ID NO: 533, 534, 535, 536, 537, 538, 539, 540, 541, 542, 543, 544, 545, 546, 547, 548, 549, 550, 551,or 552 . All of the siRNAs having any of these nucleotide sequences were demonstrated to be effective for suppressing cell viability of NSCLC cell lines.

The present application also provides a pharmaceutical composition for treating non-small cell lung cancer using any of the antisense polynucleotides or siRNAs, as well as methods for treating or preventing non-small cell lung cancer using the composition.

The invention further provides pharmaceutical composition for treating non-small cell lung cancer, which contains a polypeptide having the amino acid sequence of SEQ ID NO: 2, functionally equivalents thereof, or polynucleotides encoding any of them. The polynucleotide included in the composition may be incorporated in a vector to be expressed *in vivo.*

The course of action of the pharmaceutical compositions of the present invention is desirably to inhibit growth of the cancerous cells. The pharmaceutical composition may be applied to mammals including humans and domesticated mammals.

In addition, the present invention provides methods for inducing anti tumor immunity by administering an upregulated NSC polypeptide (e.g., NSC 807-1448) or immunologically active fragment thereof, or polynucleotide encoding them.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents and other references mentioned herein are incorporated by reference in their entirety. In case of conflict, the present specification, including definitions, will control. In addition, the materials, methods and examples are illustrative only and not intended to be limiting.

It is to be understood that both the foregoing summary of the invention and the following detailed description are of a preferred embodiment, and not restrictive of the invention or other alternate embodiments of the invention.

### Brief Description of the Drawings

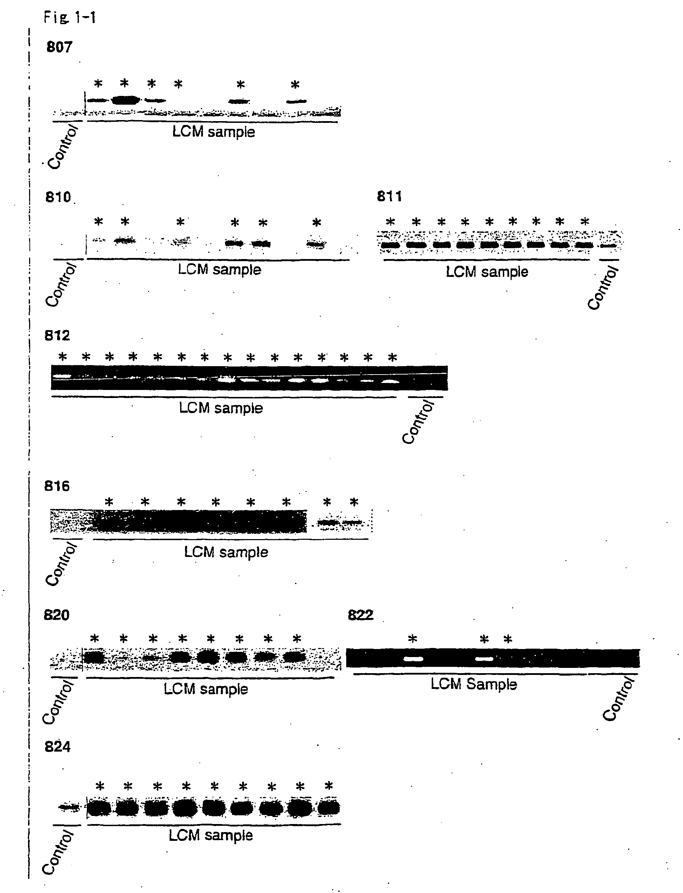

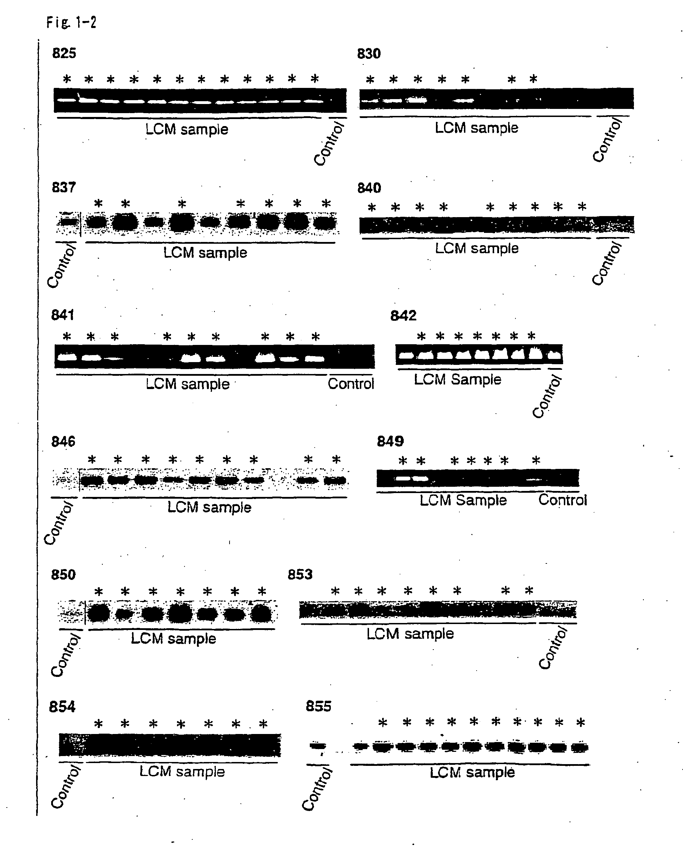

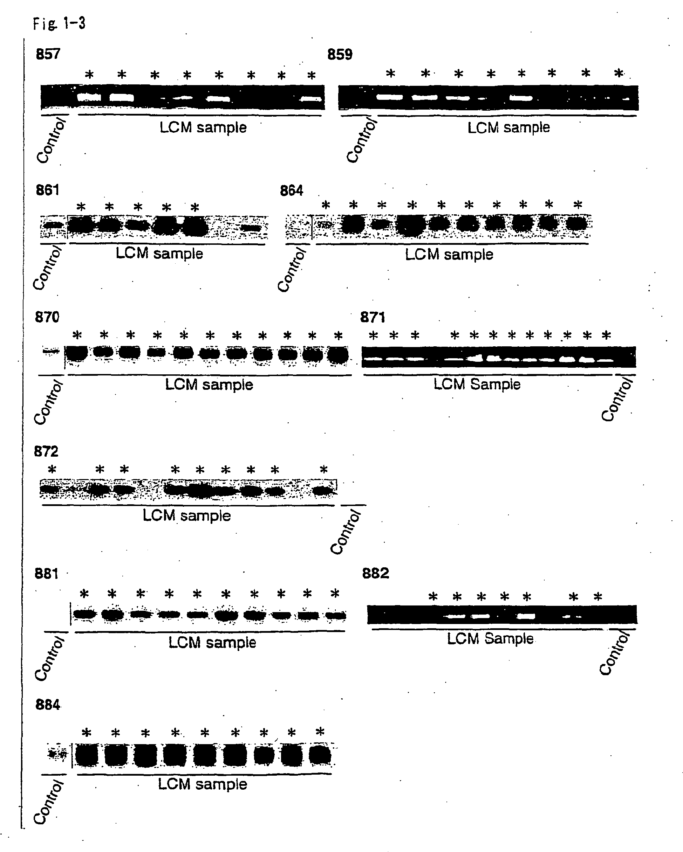

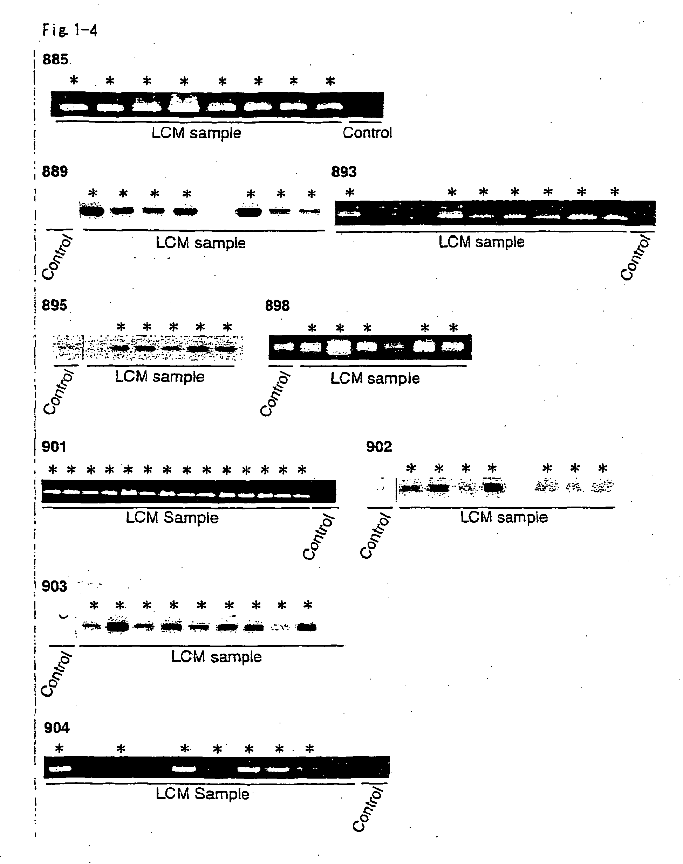

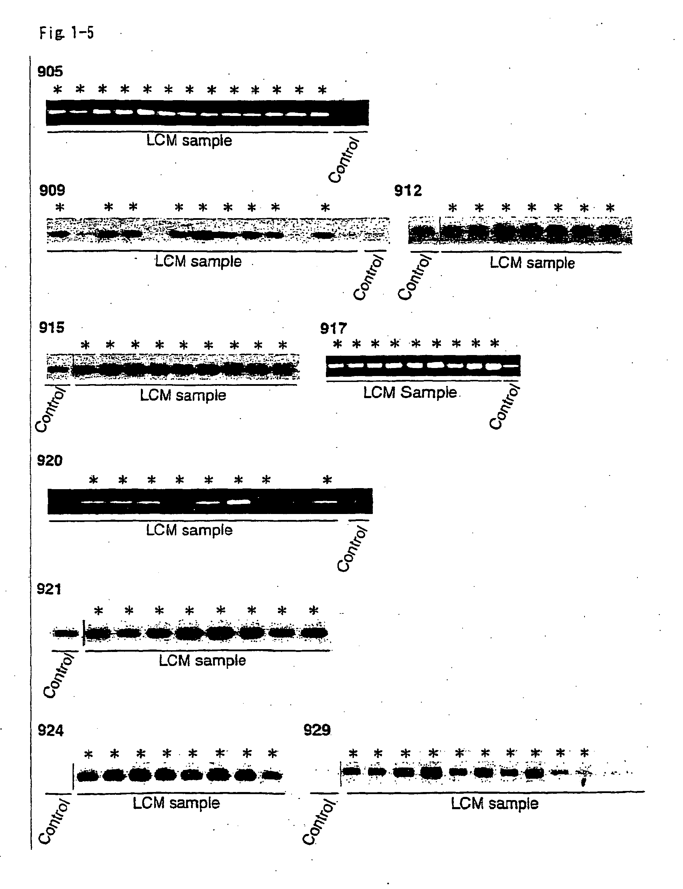

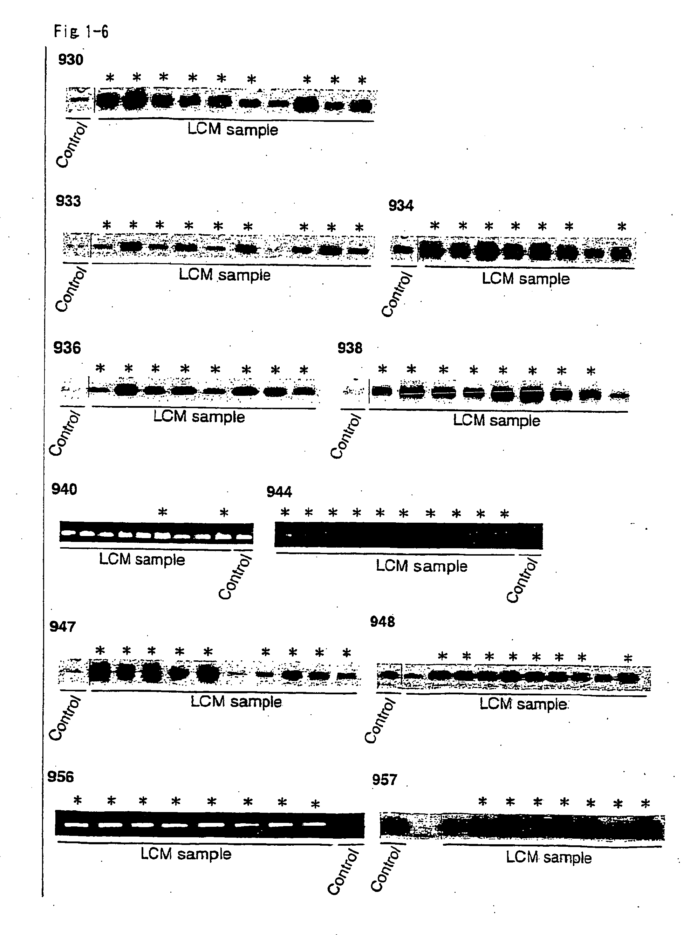

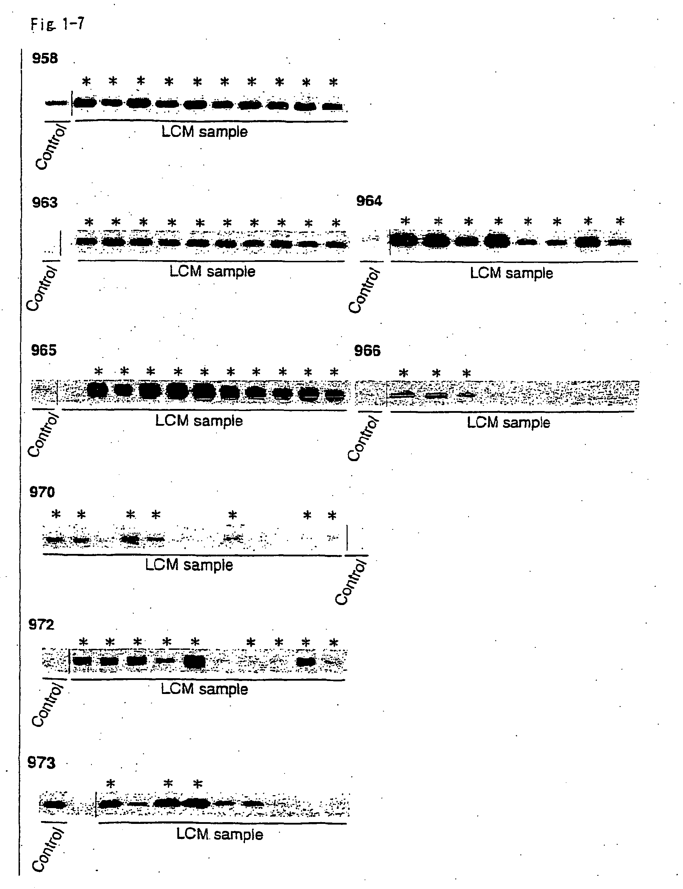

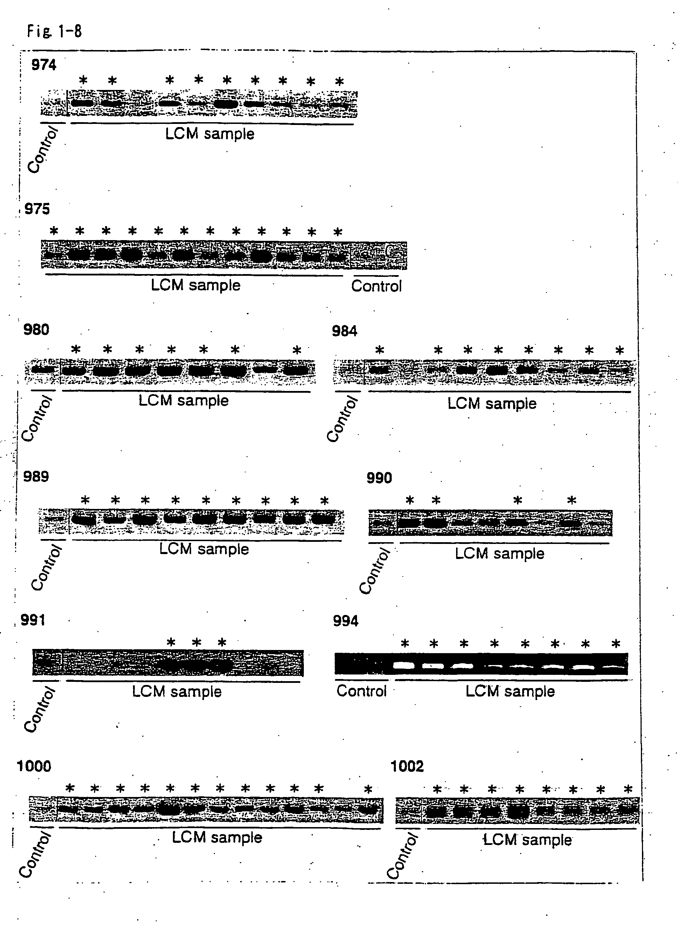

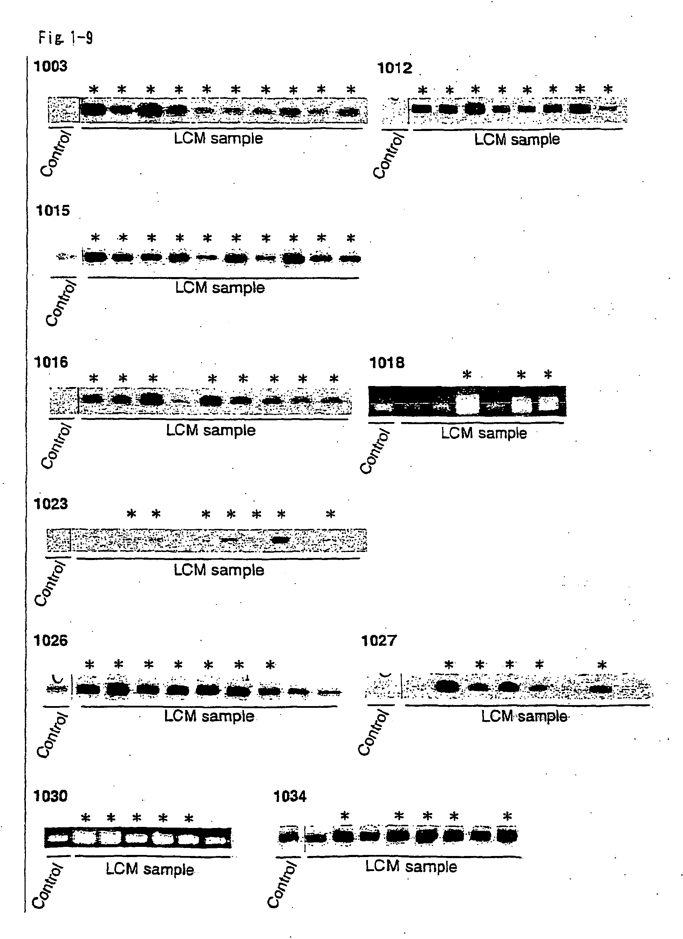

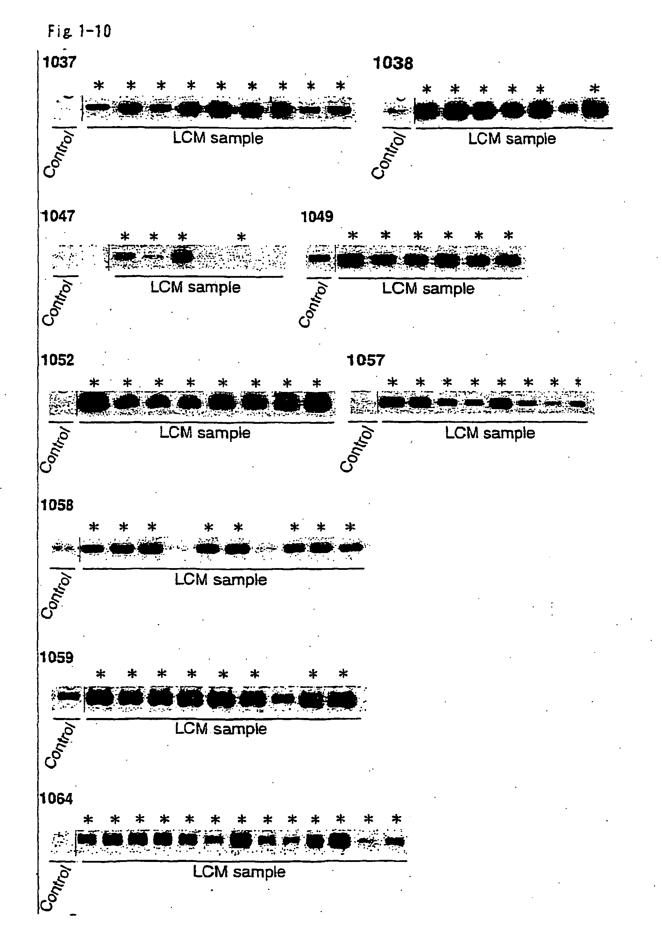

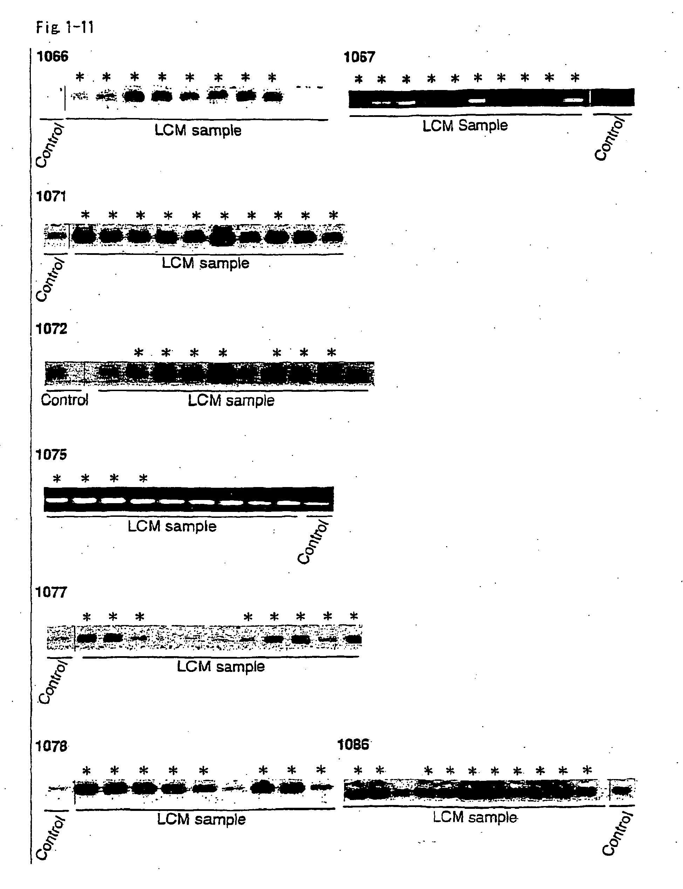

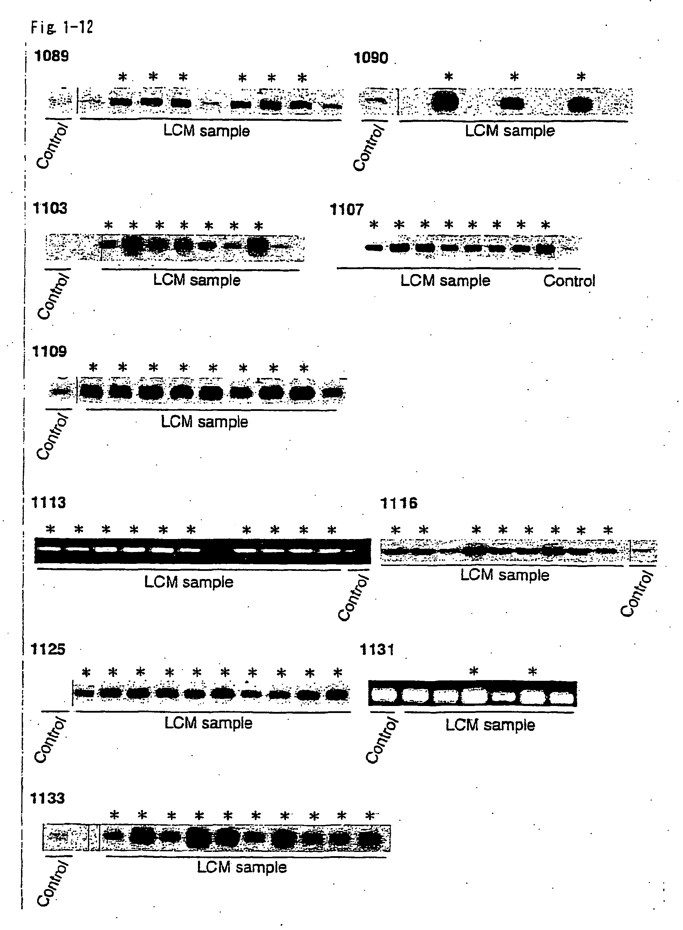

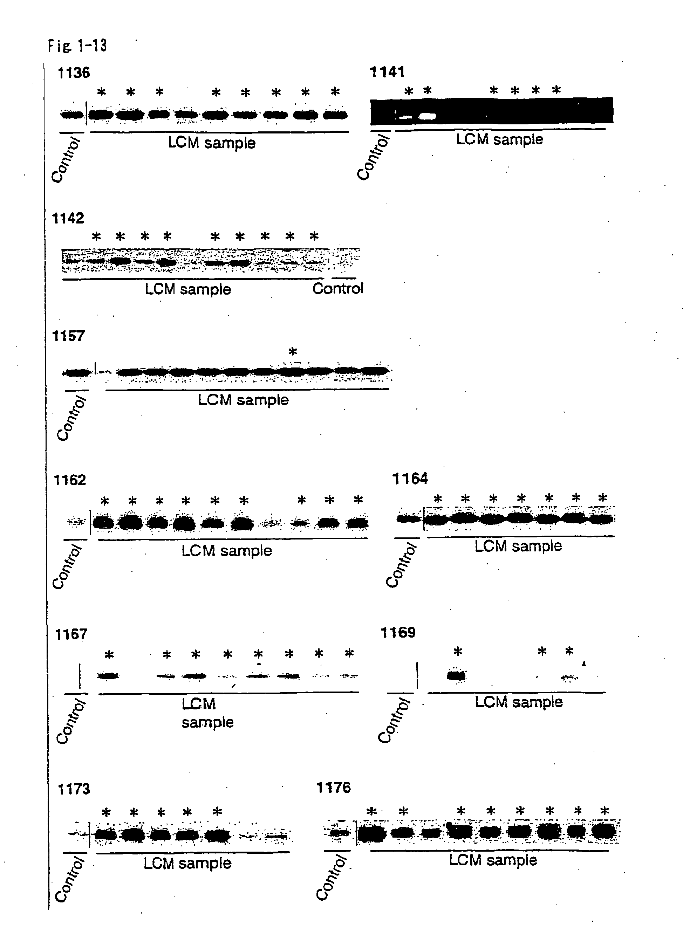

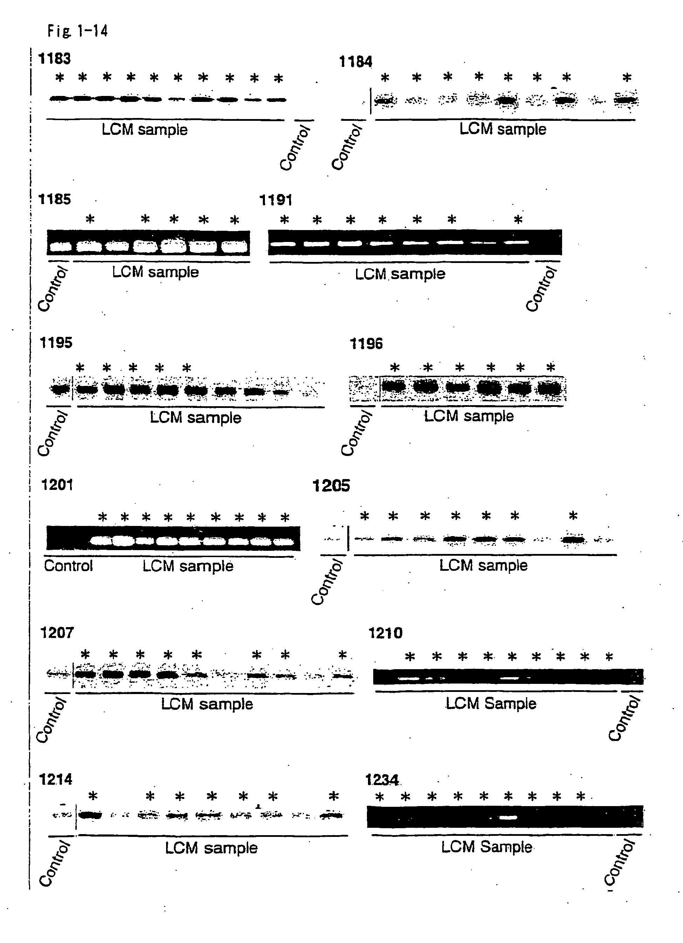

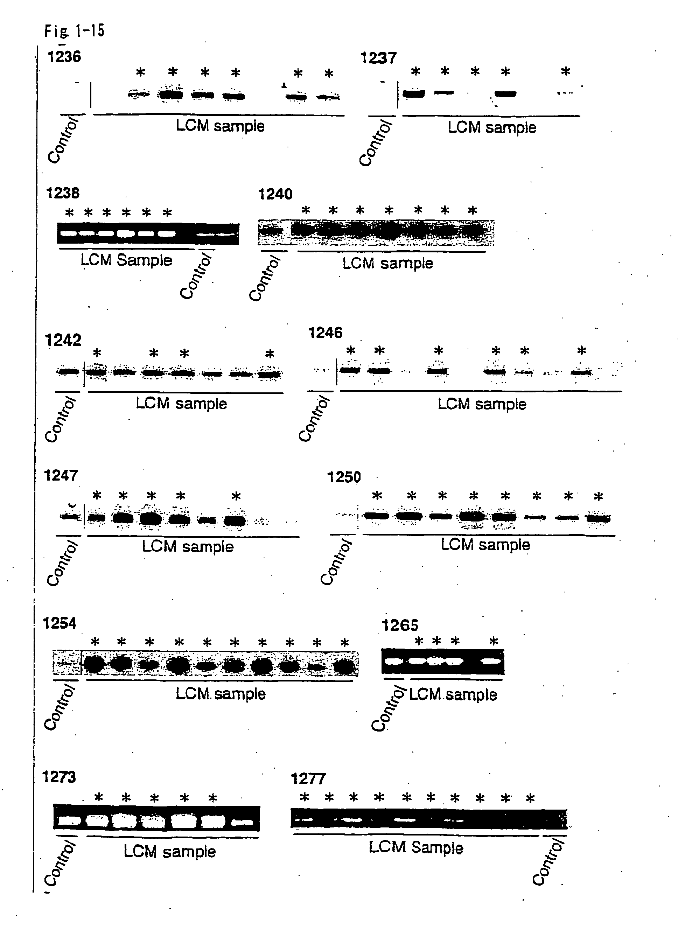

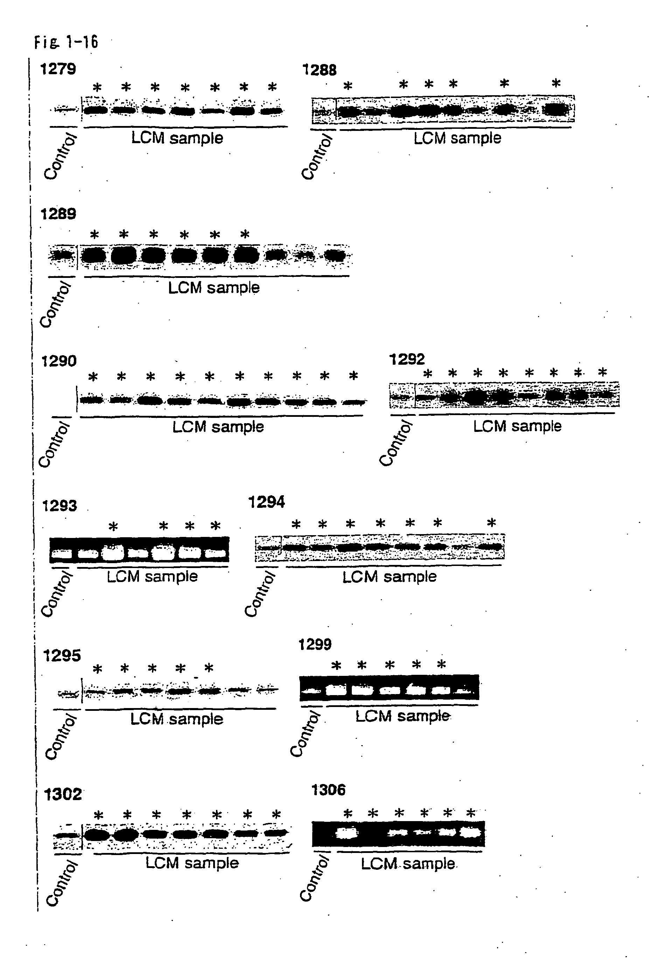

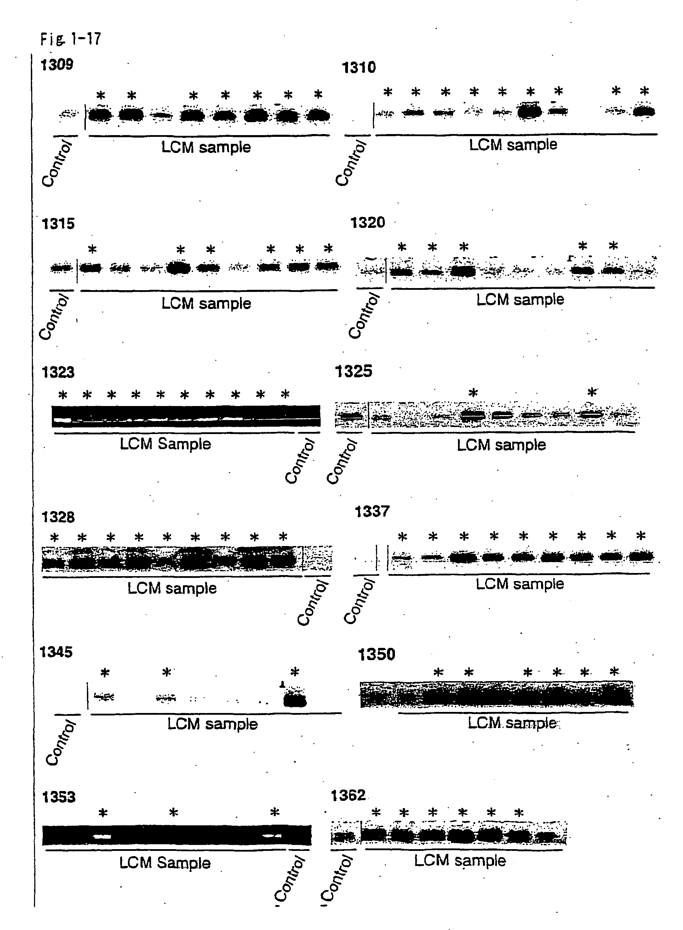

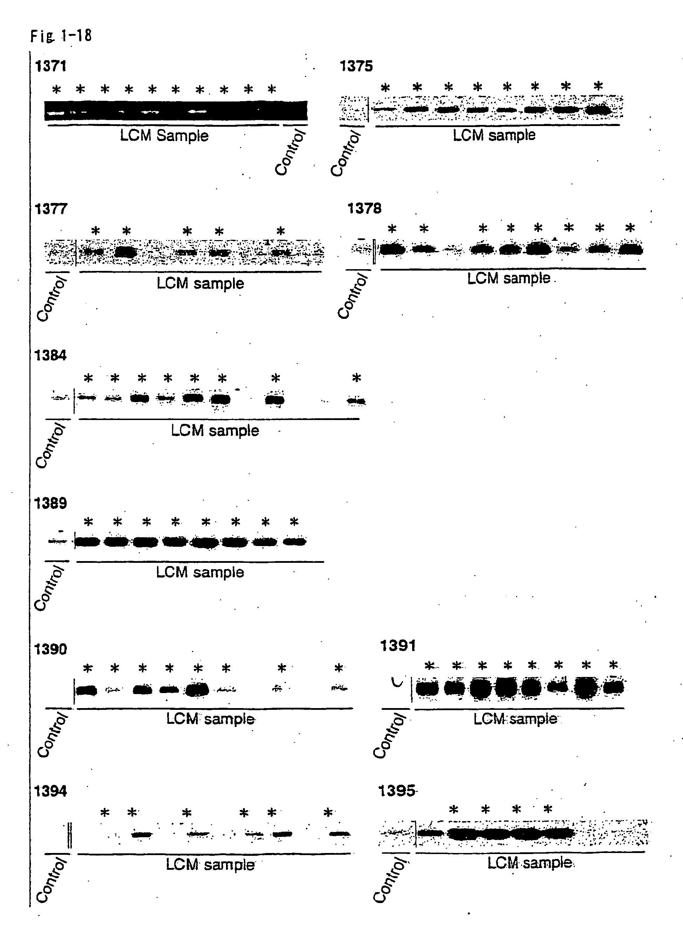

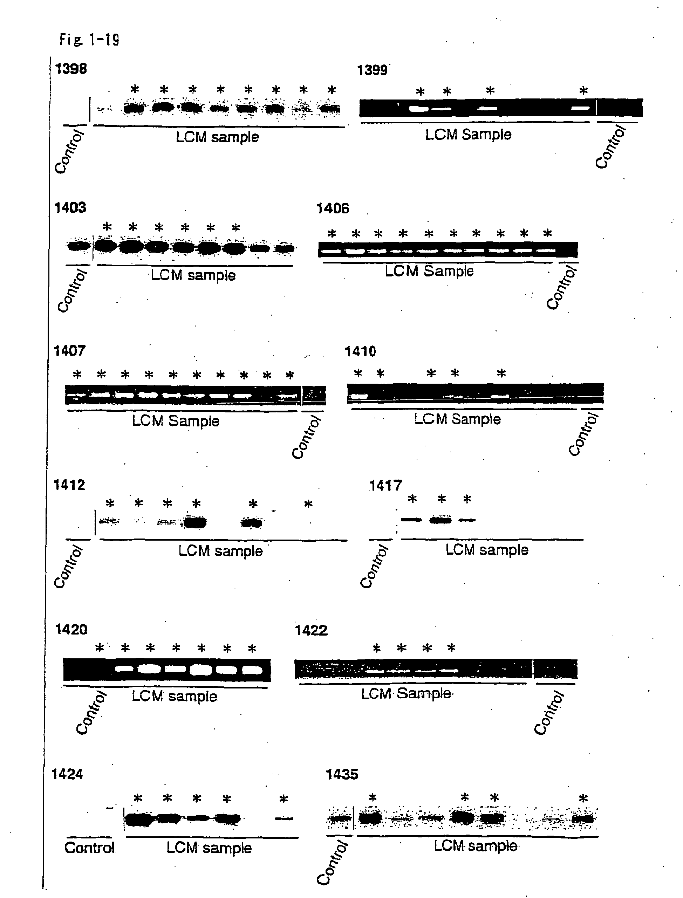

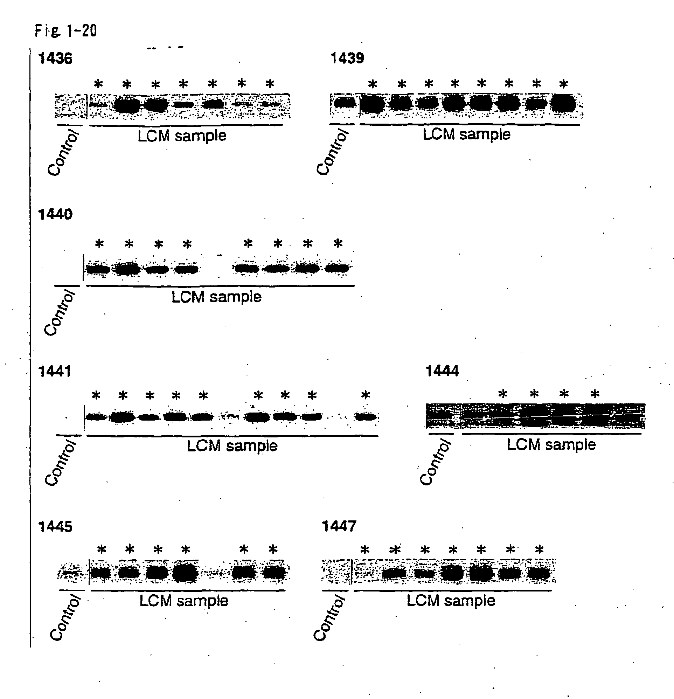

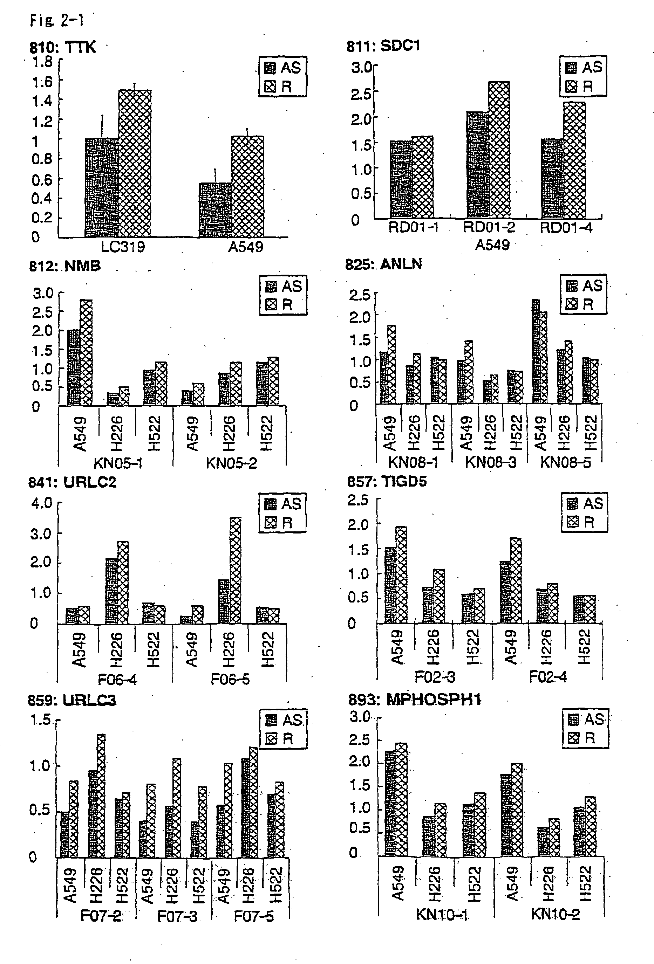

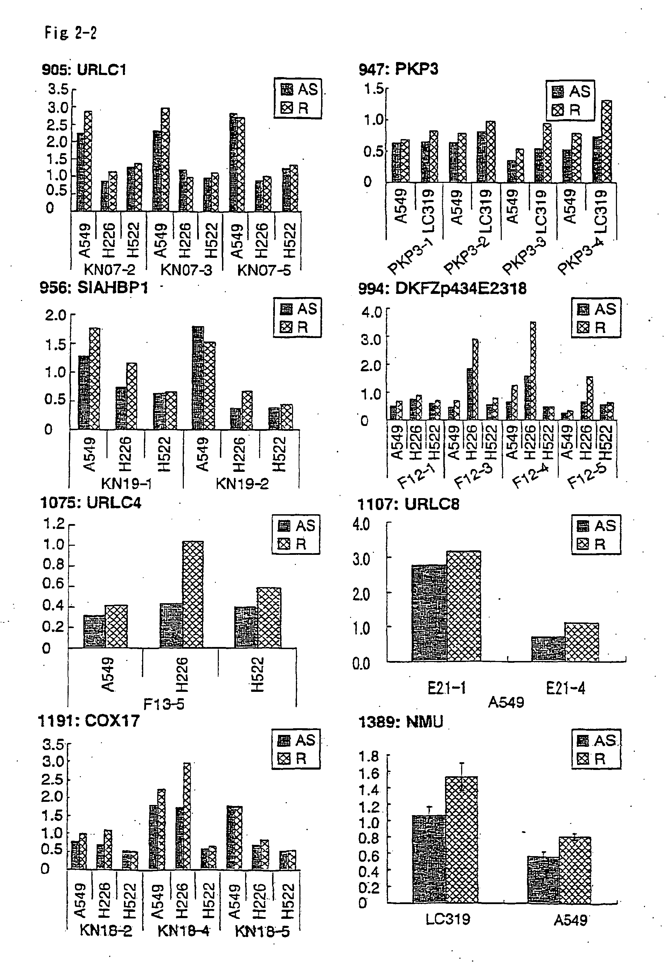

Fig. 1 depict photographs of blots showing over-expressed in lung cancer cell of 200 genes confirmed by semi-quantitative RT-PCR Lung cancer cells obtained from lung cancer patients by LCM methodsFig.2 depicts the growth-inhibitory effect of antisense S-origonucleotides designated to suppress NSC 810, NSC 811, NSC 812, NSC 825, NSC 841, NSC 857, NSC 859, NSC 893, NSC 905, NSC 947, NSC 956, NSC 994, NSC 1075, NSC 1107, NSC 1191 and NSC 1389 in lung cancer cell lines. Fig.2 depicts the results of MTT assay showing inhibition of cell growth by NSC 810-AS, NSC 811-AS1, NSC 811-AS2, NSC 811-AS4, NSC 812-AS1, NSC 812-AS2, NSC 825-AS1, NSC 825-AS3, NSC 825-AS5, NSC 841-AS4, NSC 841-AS5, NSC 857-AS3, NSC 857-AS4, NSC 859-AS2, NSC 859-AS3, NSC 859-AS5, NSC 893-AS1, NSC 893-AS2, NSC 905-AS2, NSC 905-AS3, NSC 905-AS5, NSC 947-AS1, NSC 947-AS2, NSC 947-AS3, NSC 947-AS4, NSC 956-AS1, NSC 956-AS2, NSC 994-AS1, NSC 994-AS3, NSC 994-AS4, NSC 994-AS5, NSC 1075-AS5, NSC 1107-AS1, NSC 1107-AS4, NSC 1191-AS2, NSC 1191-AS4, NSC 1191-AS5 and NSC 1389-AS.

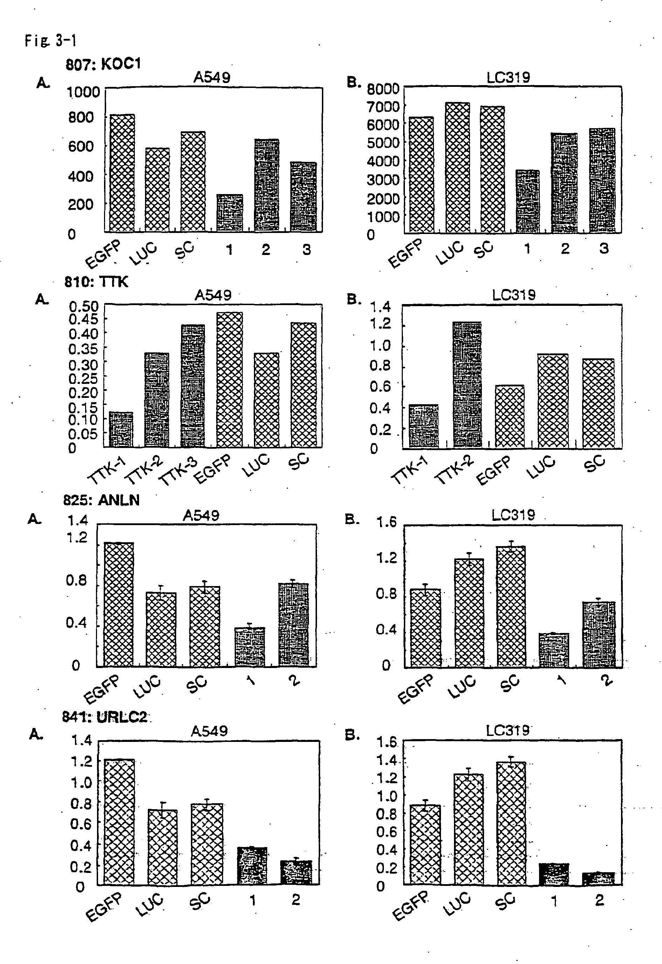

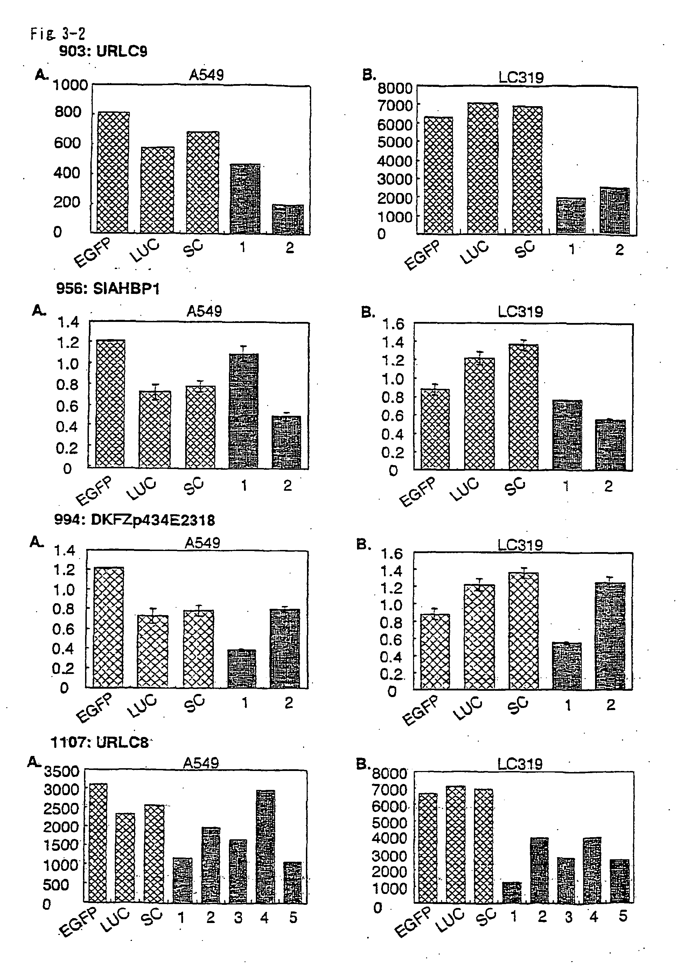

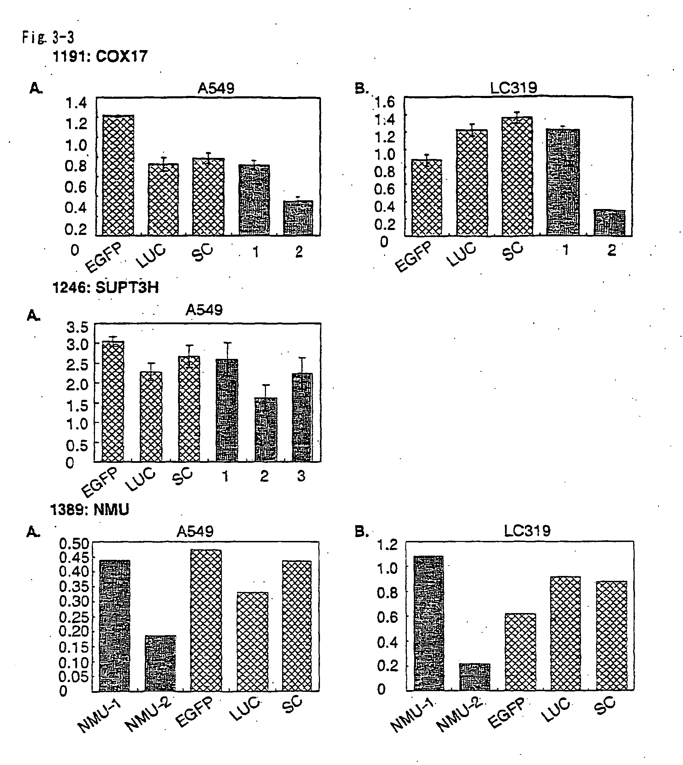

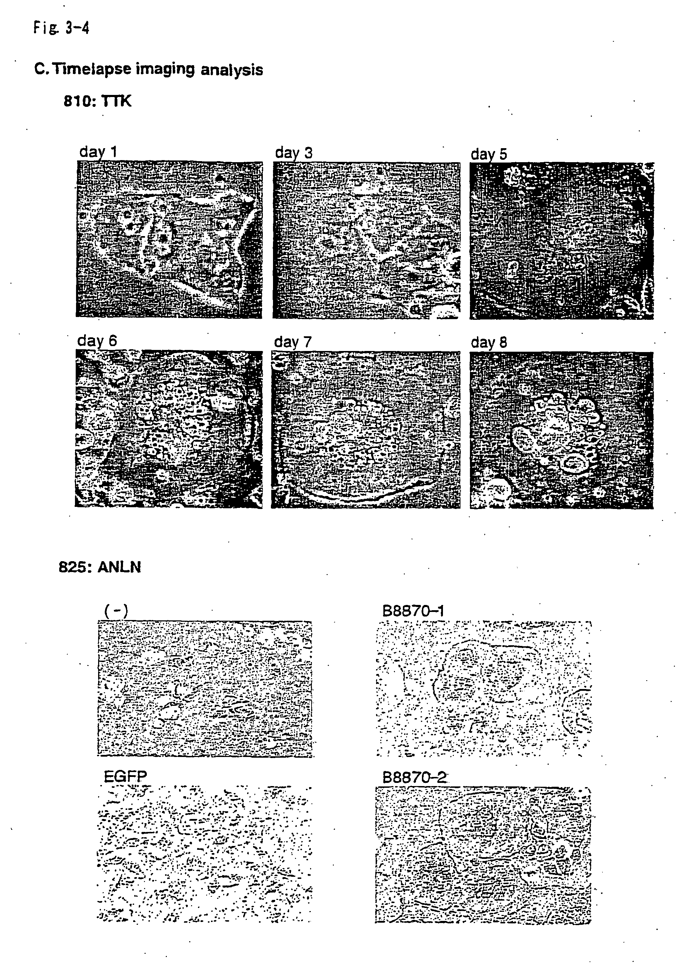

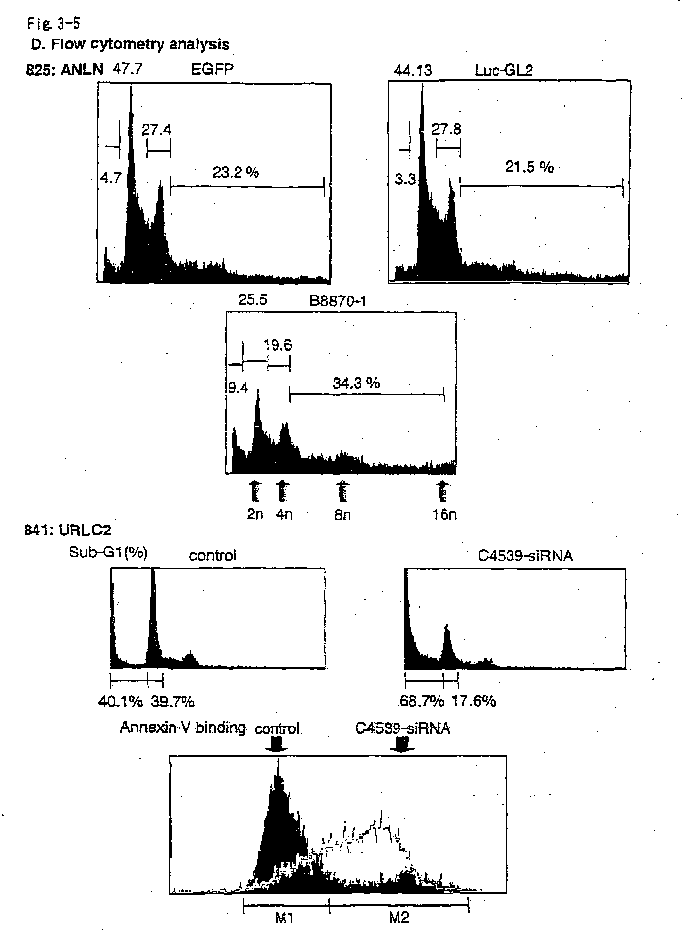

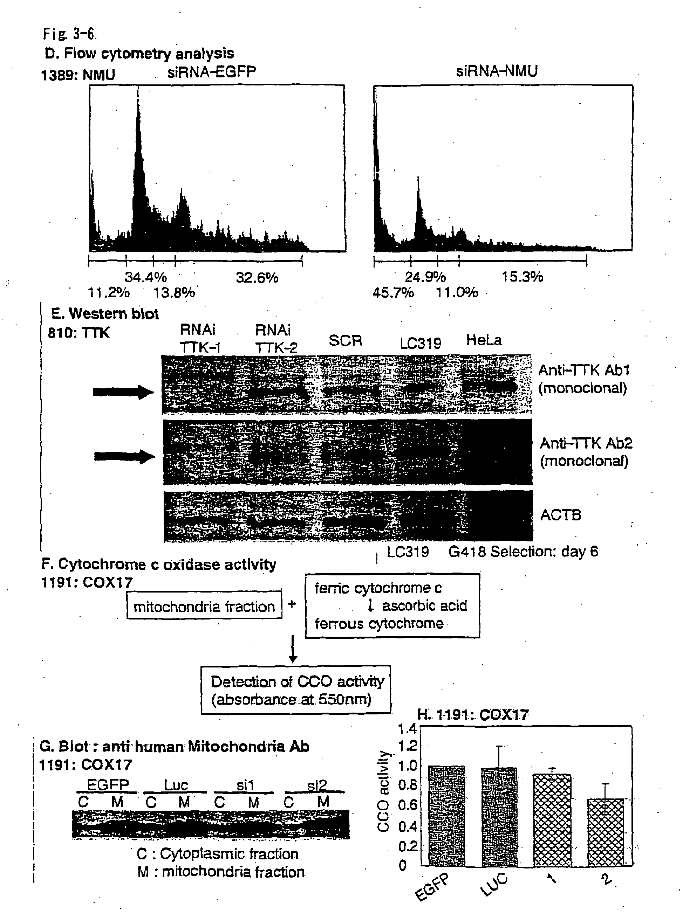

Fig. 3 depicts the growth suppressive effect of siRNAs (NSC 807-si1, NSC 810-si1, NSC 825-si1, NSC 825-si2, NSC 841-si1, NSC 841-si2, NSC 903-si1, NSC 903-si2, NSC 956-si1, NSC 956-si2, NSC 994-si1, NSC 1107-si1, NSC 1107-si2, NSC 1107-si3, NSC 1107-si4, NSC 1107-si5, NSC 1191-si2, NSC 1246-si2 and NSC 1389-si2) on lung cancer cell lines. Fig.3A depicts the results of MTT assay on A549 cells transfected with vectors expressing control-siRNA or target-siRNA. Fig.3B depicts the results of MTT assay on LC319 cells transfected with vectors expressing control-siRNA or target-siRNA. Fig.3C shows a microgram of timelapse imaging of the siRNA transfected-LC319 cells. Fig.3D depicts the results of Flow cytometry analysis showing the cell cycle profile of siRNA transfected cells. Fig.3E is a photograph showing the result of Western blotting illustrating the expression and inhibition by siRNA of native protein in LC319 cells detected by two different monoclonal antibodies. Fig.3F, G and H show the cytochrome c oxidase (CCO) activity and its inhibition by COX17 RNAi in A549 cells. Fig.3F depicts a schematic illustration of CCO activity measurement. Fig.3GFig. depicts the result of Western blotting confirming the fractionation of A549 cells transfected with COX17 RNAi, cytoplasmic and mitochondria fractions of the cells using mouse monoclonal antibody to human mitochondria (MAB1273; CHEMICON, Temecula, CA). Fig.3H shows the reduced CCO activity due to the suppression of the endogenous COX17 gene, 2 or 5 days after transfection.

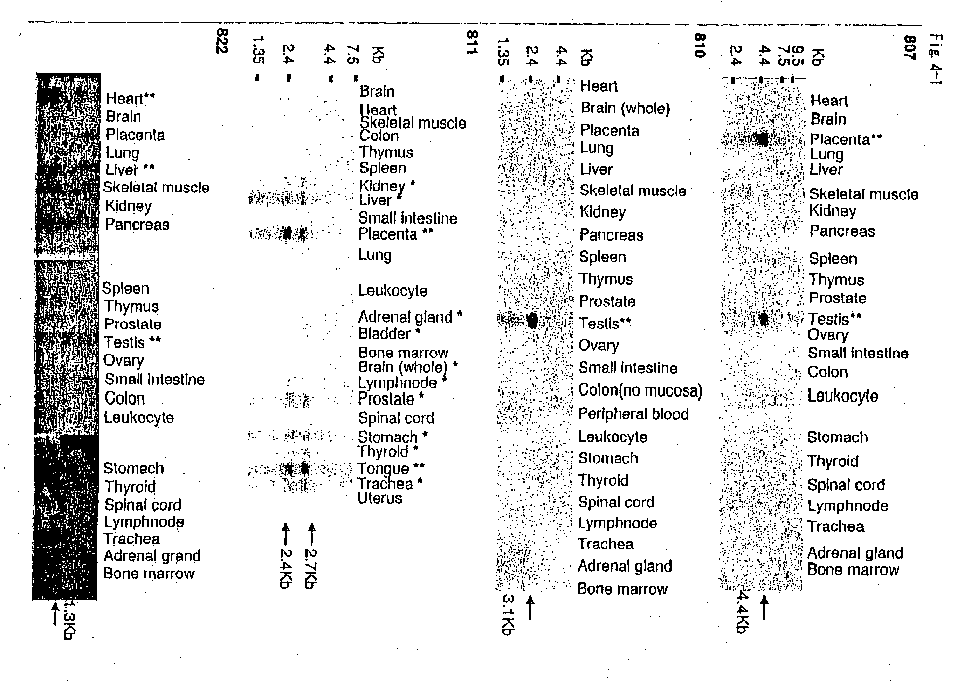

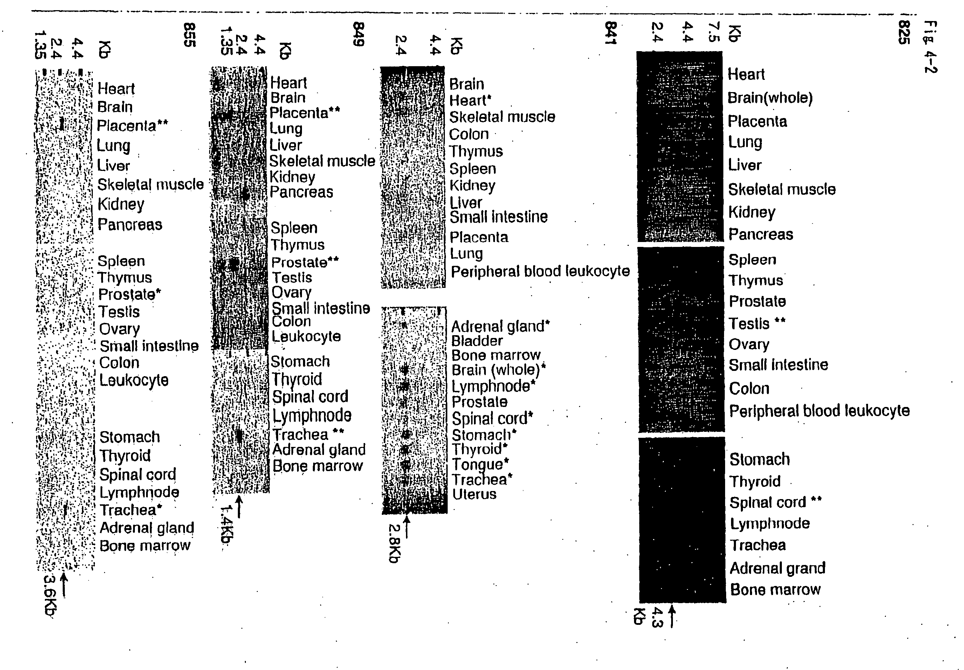

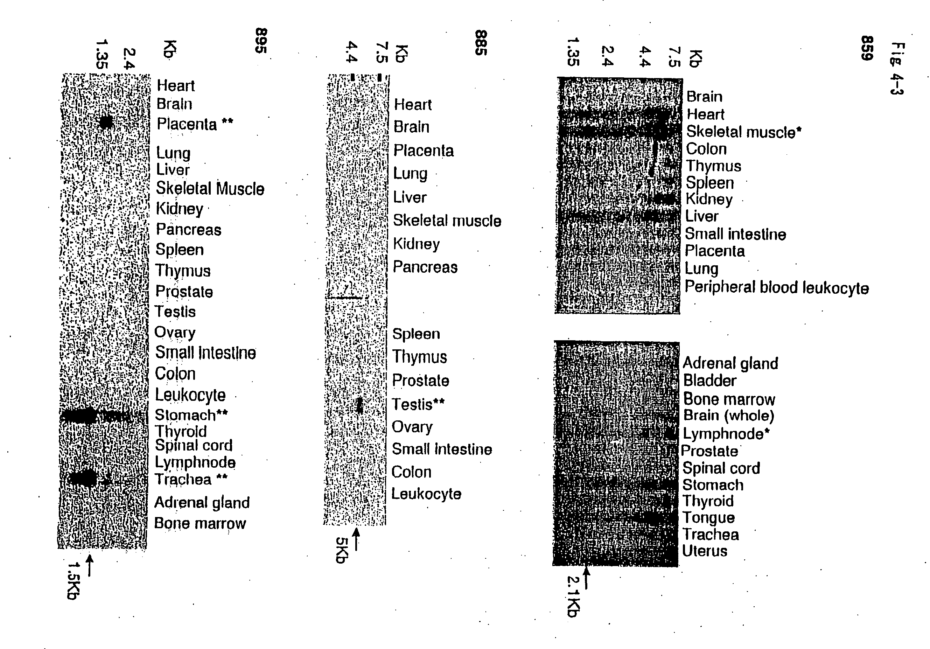

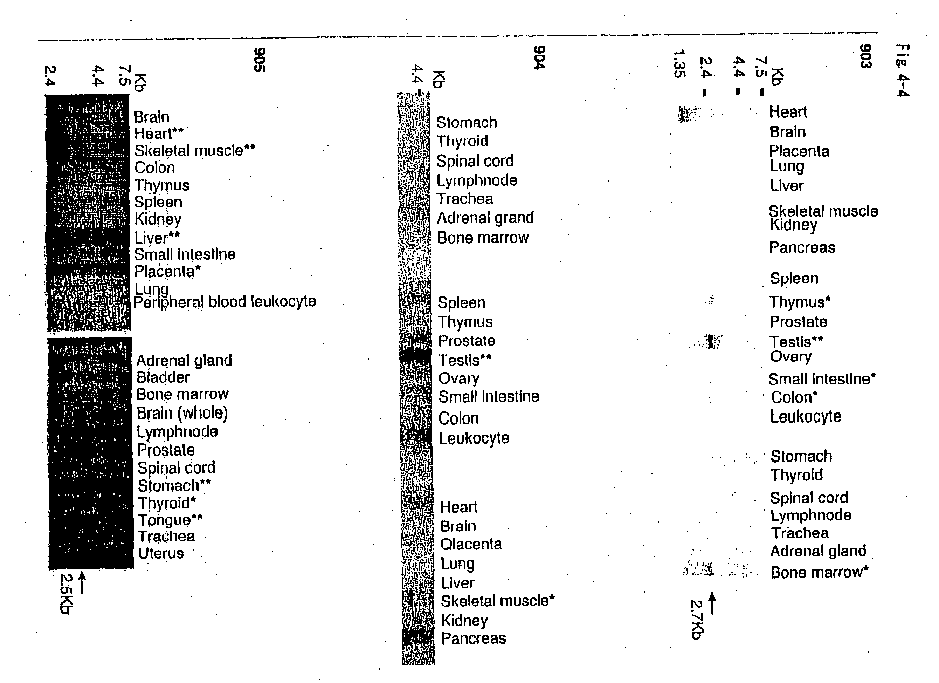

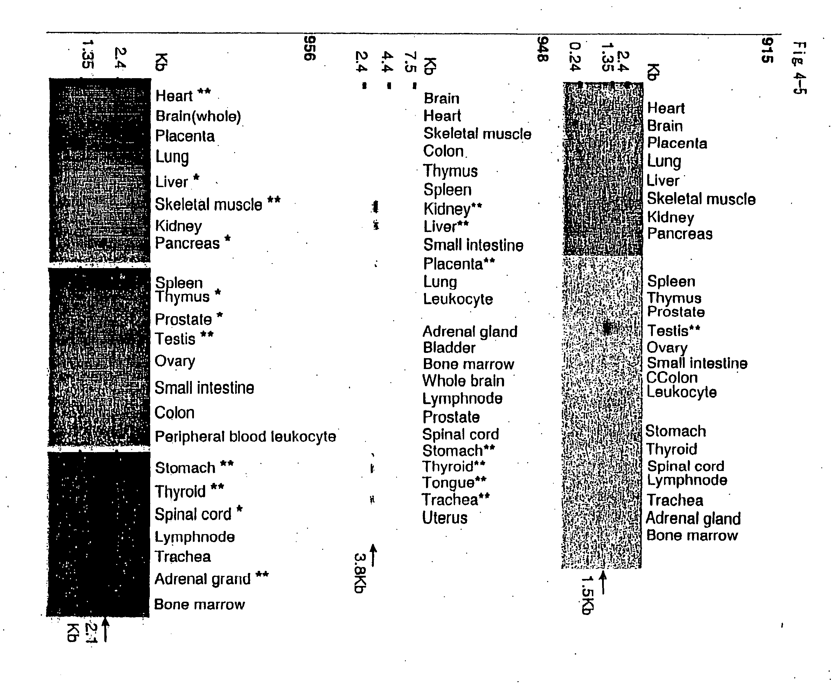

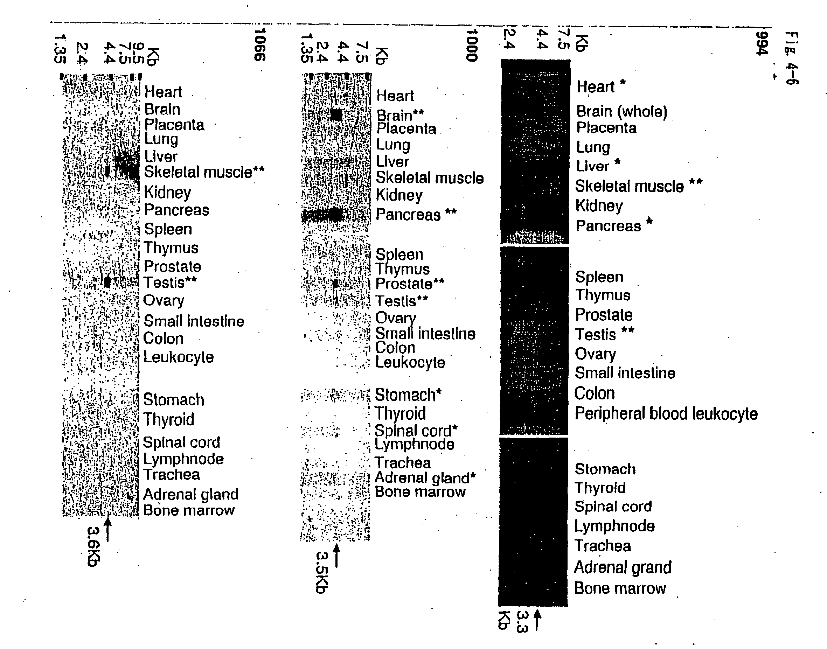

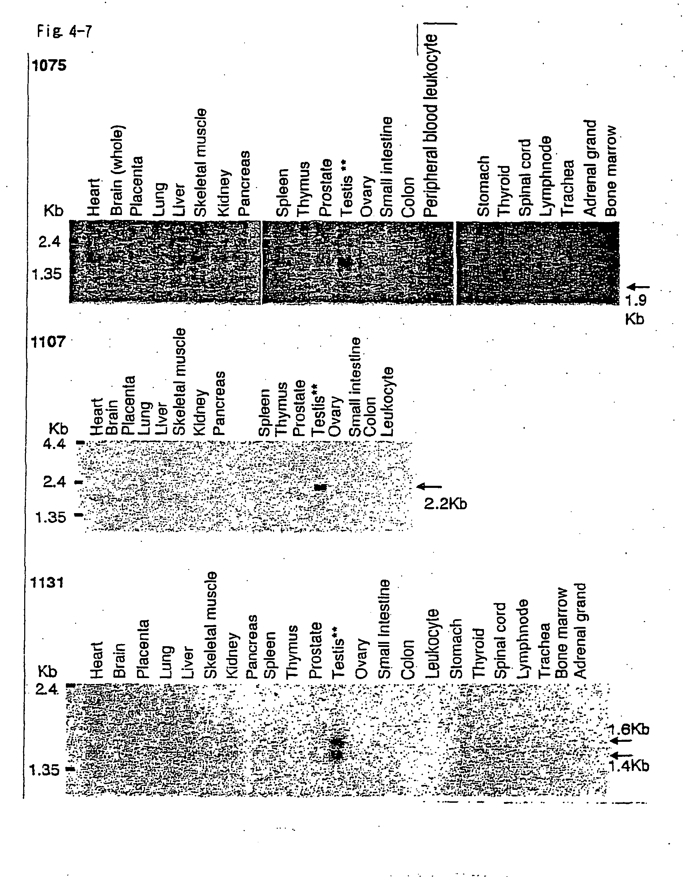

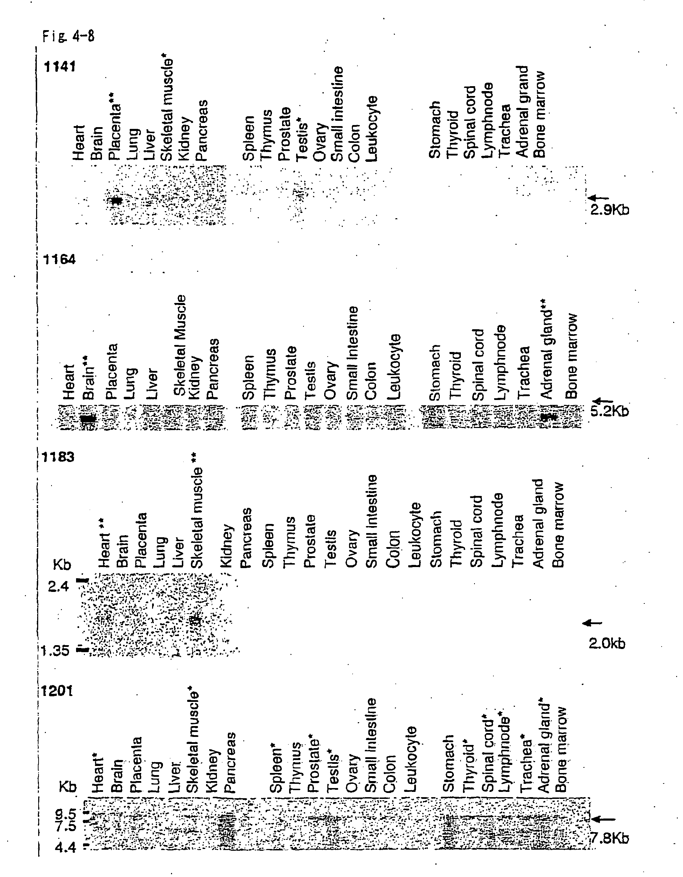

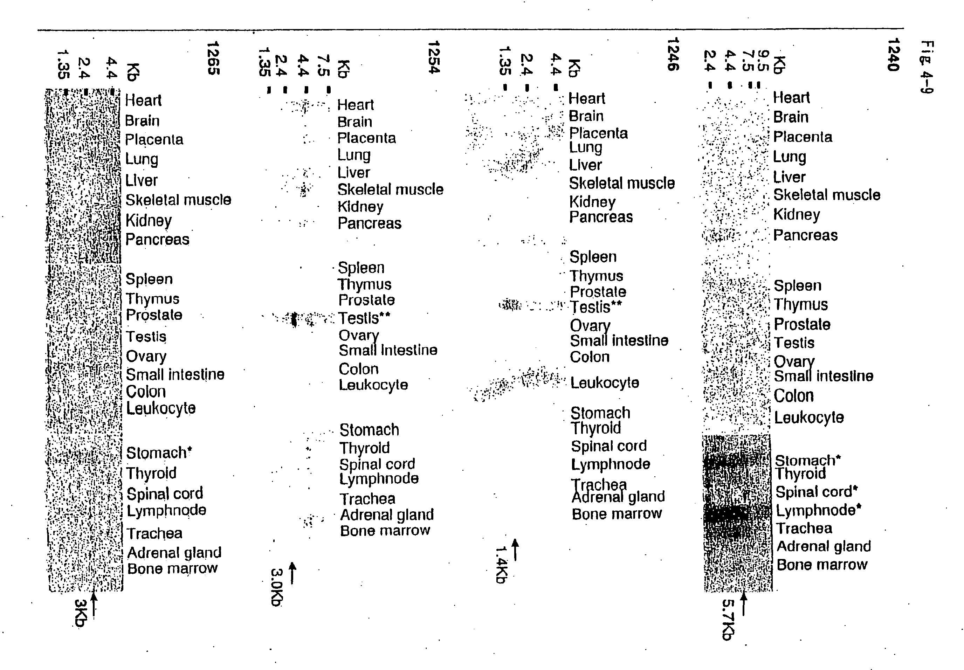

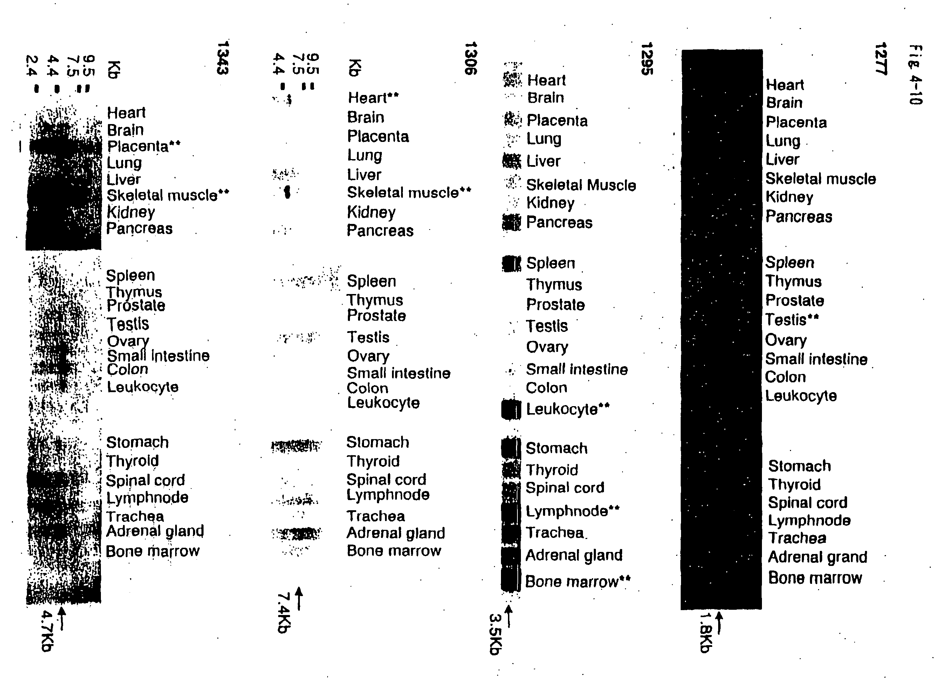

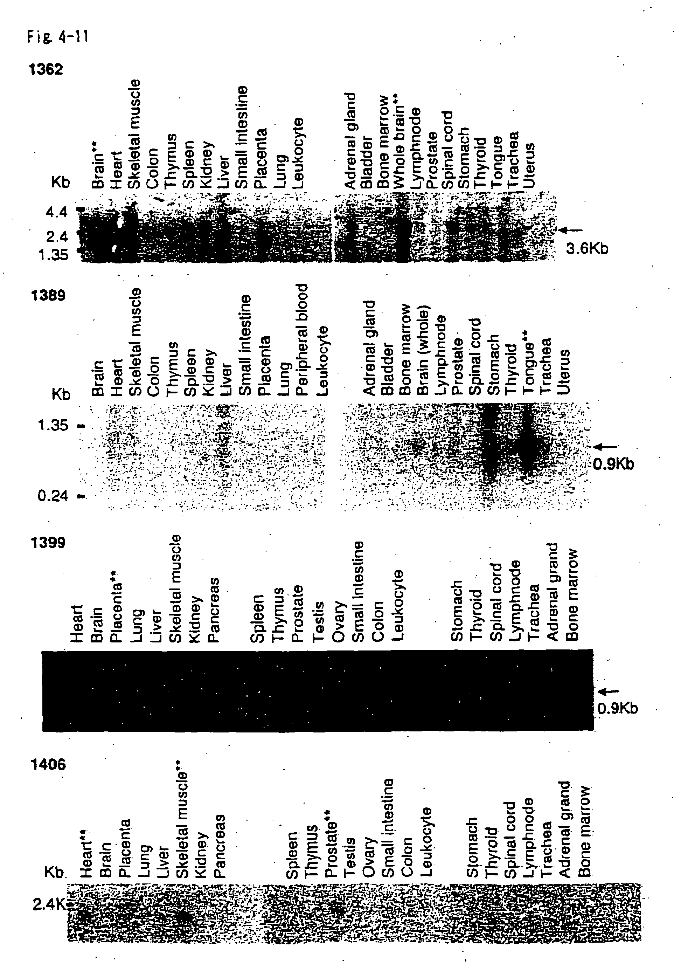

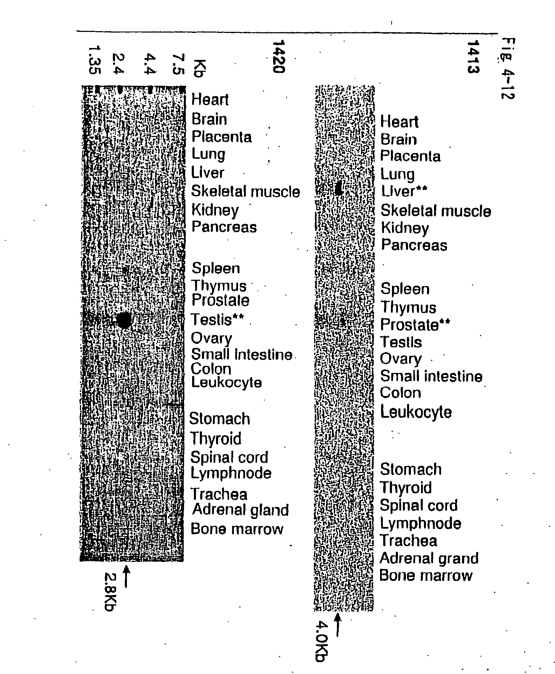

Fig. 4 shows a photograph depicting the expression of NSC 807,NSC 810, NSC 811, NSC 822, NSC 825, NSC 841, NSC 849, NSC 855, NSC 859, NSC 885, NSC 895, NSC 903, NSC 904, NSC 905, NSC 915, NSC 948, NSC 956, NSC 994, NSC 1000, NSC 1066, NSC 1075, NSC 1107, NSC 1113, NSC 1131, NSC 1141, NSC 1164, NSC 1183, NSC 1201, NSC 1240, NSC 1246, NSC 1254, NSC 1265, NSC 1277, NSC 1295, NSC 1306, NSC 1343, NSC 1362, NSC 1389, NSC 1399, NSC 1406, NSC 1413, and NSC 1420 in various human tissues analyzed by multiple-tissue northern blot analysis.

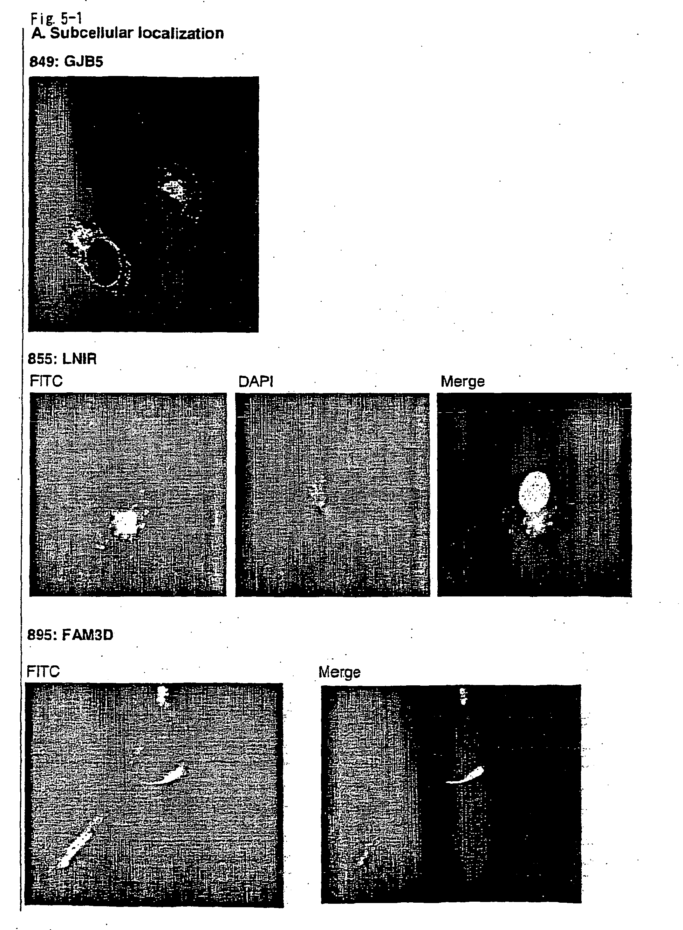

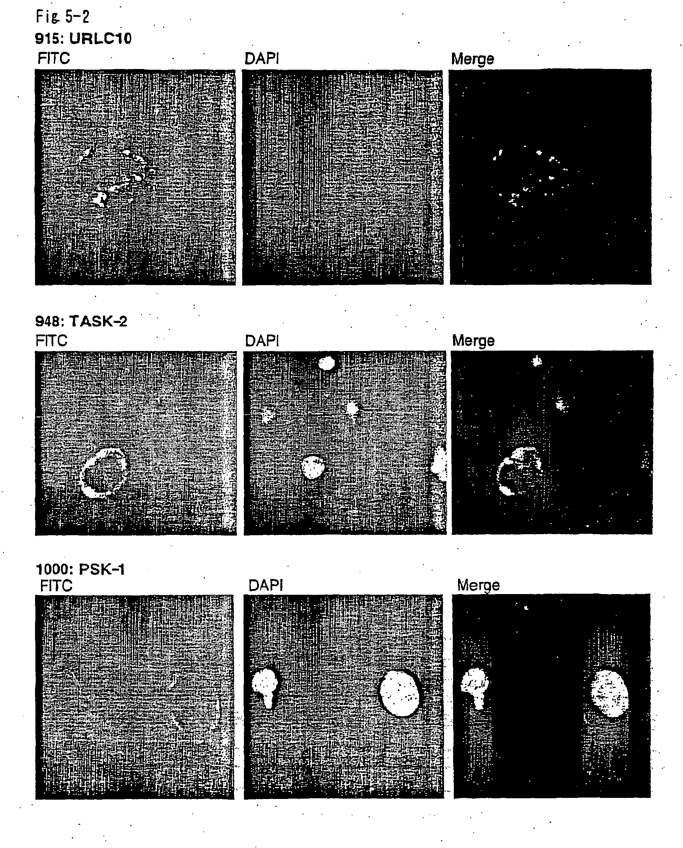

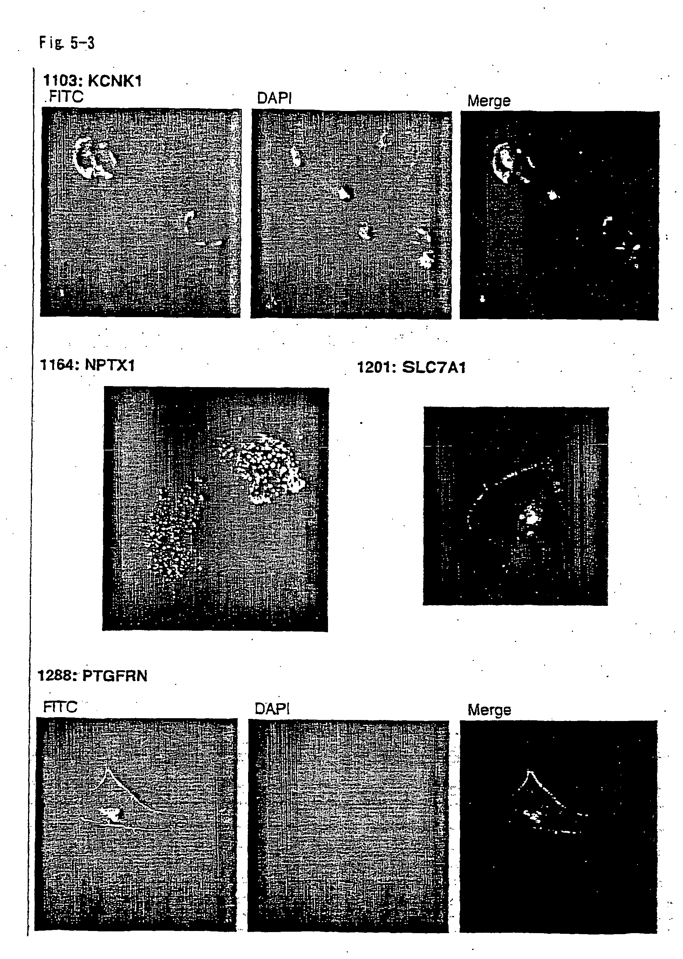

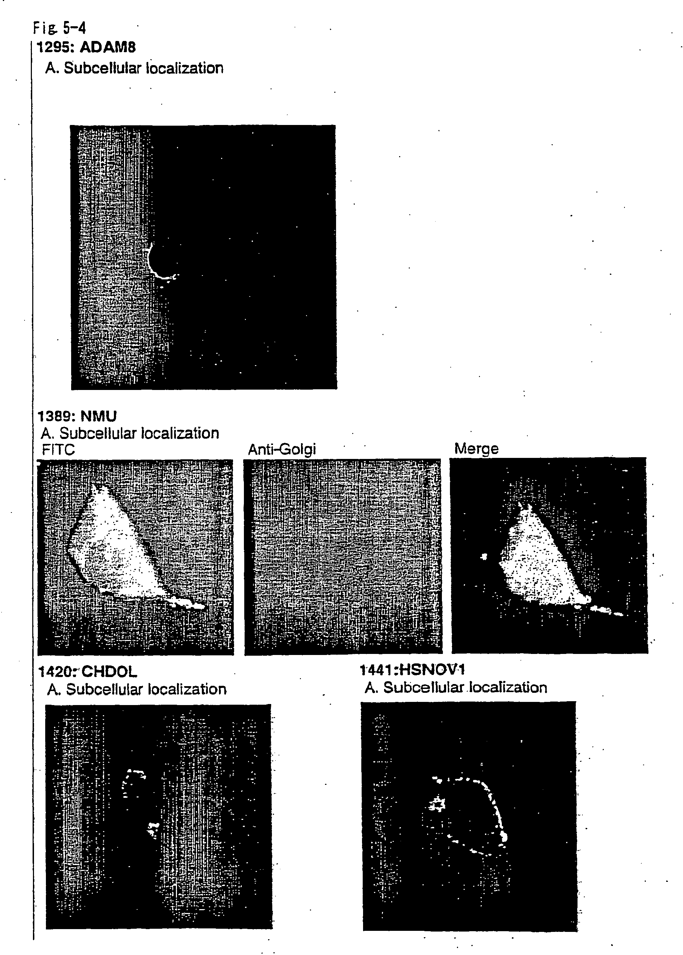

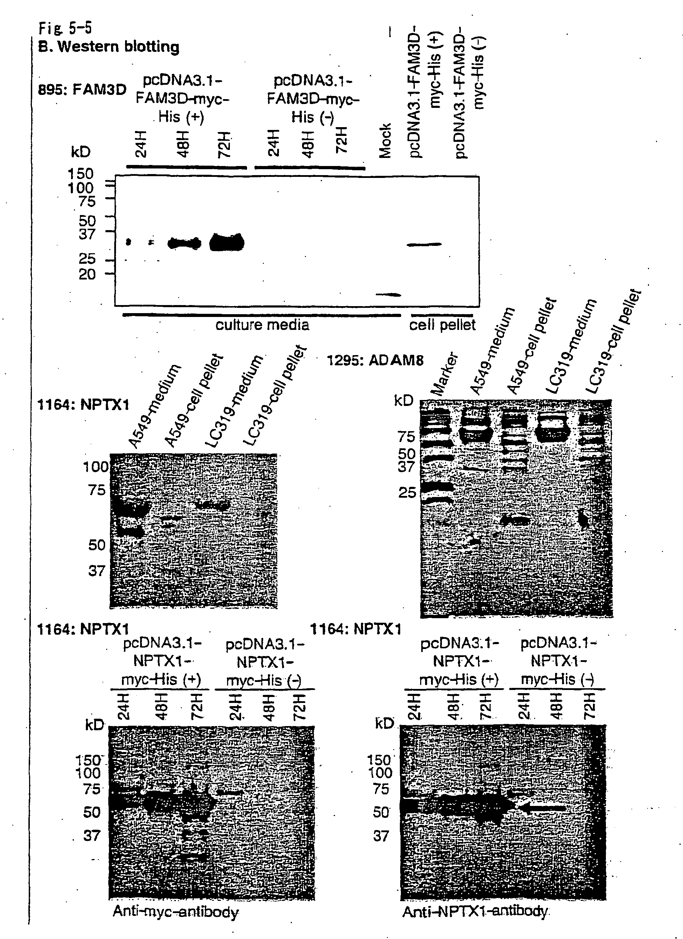

Fig. 5A depicts a photograph showing subcellular localization of NSC 849, NSC 855, NSC 895, NSC 915, NSC 948, NSC 1000, NSC 1103, NSC 1164, NSC 1201, NSC 1288, NSC 1295, NSC 1389, NSC 1420 and NSC 1441 observed by immunocytochemistry on COS-7 cells transfected with the c-myc-His tagged NSC-gene expression vector using anti-His monoclonal antibody and Rhodamine conjugated secondary anti-mouse IgG antibody for visualization. Nuclei were counter-stained with DAPI. Fig. 5B depicts a photograph showing the results of Western blot analysis of c-myc tagged NSC 895, NSC 1164 and NSC 1295 secreted in the culture medium.

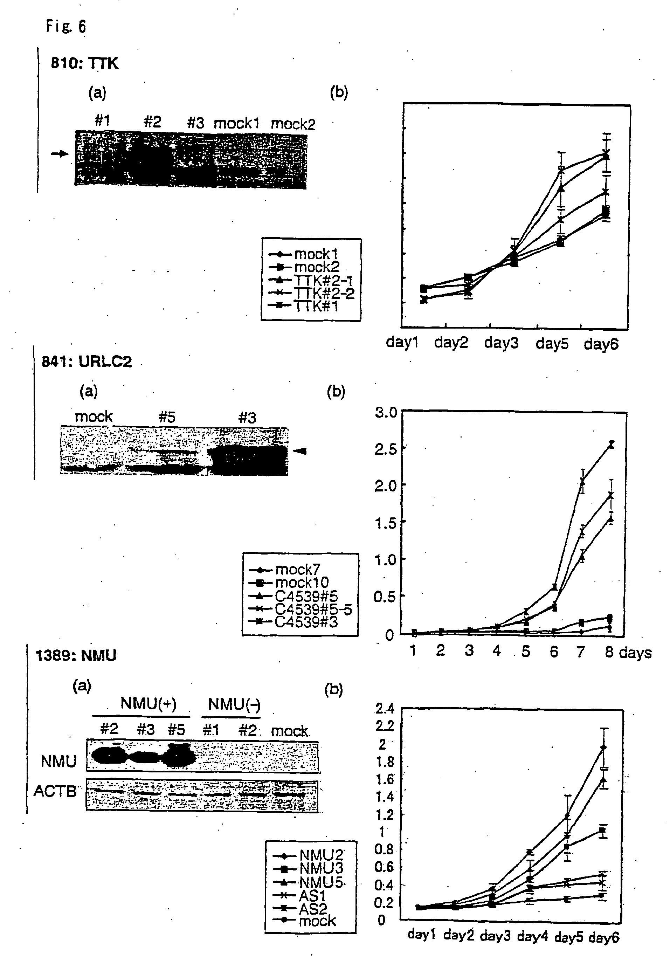

Fig. 6 depicts the effect of NSC-gene on cell growth in COS-7 cells stably transfected with c-myc-His tagged expression vector. Fig. 6a shows the expression of NSC 810; NSC 841 and NSC 1389 in stably transfected COS-7 cells detected by Western blotting. Fig. 6b shows the effect of NSC 810, NSC 841 and NSC 1389 on the growth of COS-7 cells. 2 or 3 independent transfectants expressing high levels of NSC 810(COS7-TTK-1 and 2), NSC 841(NIH3T3-URLC2-3 and 5) and NSC 1389 (COS-7-NMU-2, 3 and 5) and control (mock) were cultured in triplicate. Cell viability was measured by MTT assay.

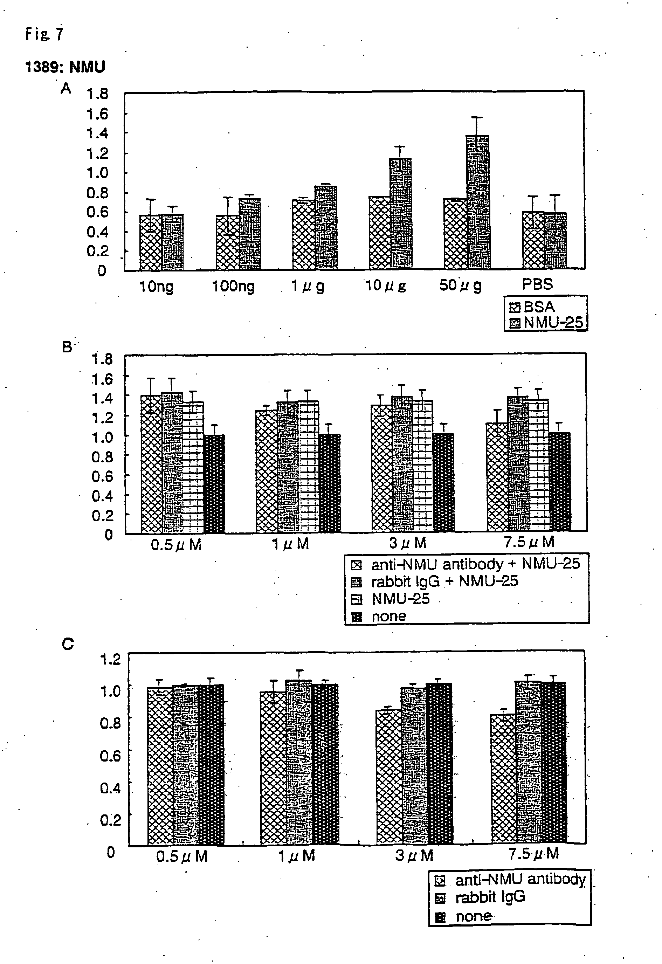

Fig. 7 shows the effect of NMU on cell growth examined by autocrine system.
   Fig 7A shows the result of autocrine assay of NMU. An active form of the 25 amino acid polypeptides of NMU (NMU-25) and BSA (control) protein were added to individual COS-7 cells every 48 hours. 7 days after the addition, cell numbers were counted by MTT assay. Fig 7B shows the growth-inhibitory effect of anti-NMU antibody on COS-7 cells treated with NMU-25. Fig 7C shows the growth-inhibitory effect of anti-NMU antibody on LC319 cells, which.endogenously overexpress NMU.

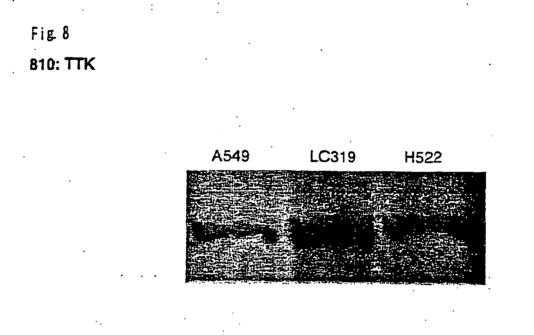

Fig. 8 depicts the result of Western blot analysis confirming overexpression of TTK protein in NSCLC cell lines, A549, LC319 and NCI-H522.

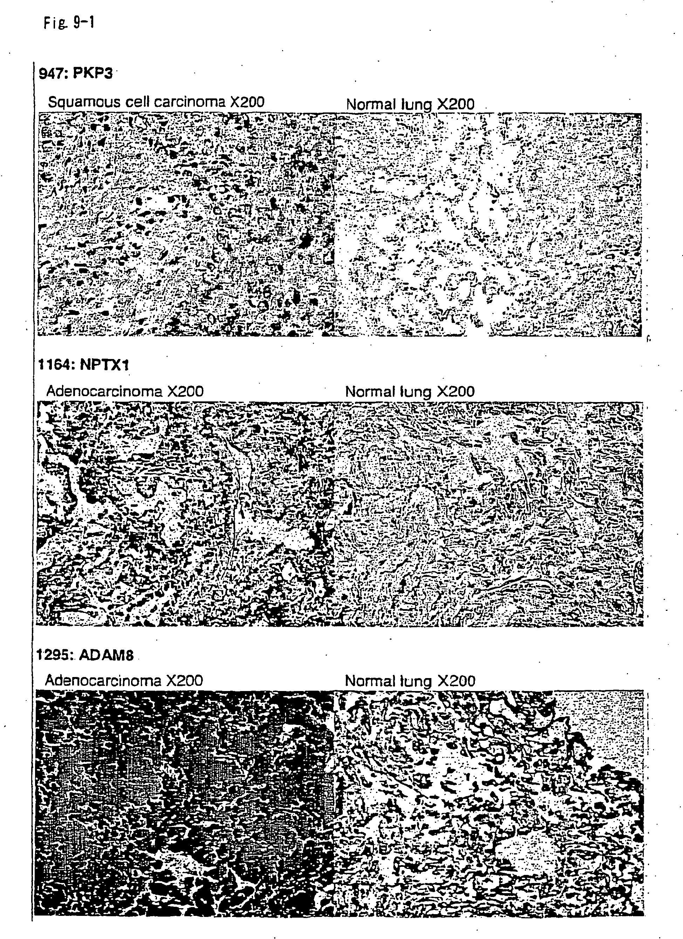

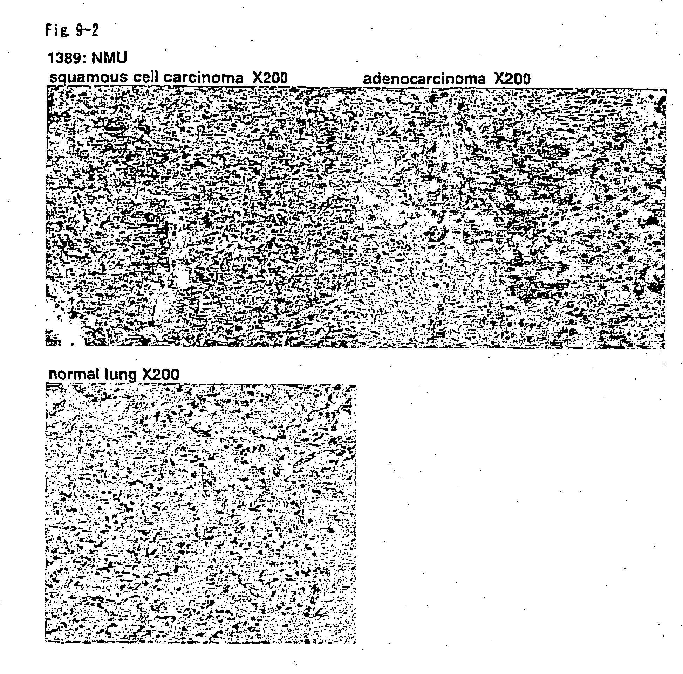

Fig. 9 shows the result of immunohistchemical staining of NSC 947, NSC 1164, NSC 1295 and NSC 1389 in clinical samples including adenocarcinoma, squamous cell carcinoma and normal lung with anti-NSC 947 antibody, anti-NSC 1164 antibody, anti-NSC 1295 antibody and anti- NSC 1389 antibody.

### Detailed Description of the Invention

The words "a", "an", and "the" as used herein mean "at least one" unless otherwise specifically indicated.

The present invention is based in part on the discovery of changes in the expression patterns of multiple genes in lung cells from primary lung cancer tissues of patients suffering lung cancer. The difference in the expression level of genes were identified by comprehensive cDNA microarray system.

The cDNA microarray analysis was performed on 23040 genes to select genes that are commonly over-expressed or suppressed among non-small cell lung cancer patients. 1448 genes were found differentially expressed according to the present invention. Among them, 642 genes were up-regulated and 806 genes were down-regulated.

The genes identified by the microarray analysis were further screened by antisense S-oligonucleotide and/or siRNA technique to identify candidate genes as targets for the development of therapeutic drugs or immunotherapy. Antisense S-oligonucleotides and siRNA are short, synthetic stretches of DNA/RNA which hybridize with specific mRNA strands that correspond to target genes (Jansen and Zangemeister-Wittke, Lancet Oncol 3: 672-83 (2002); Brummenlkamp et al., Science 296: 550-3 (2002)). By binding to the mRNA, antisense oligonucleotides prevent translation of target genes into proteins, as a result blocking the action of the genes (Jansen and Zangemeister-Wittke, Lancet Oncol 3: 672-83 (2002)). In contrast, siRNA is a sequence-specific double-stranded RNA which is introduced into cells to cause a nonheritable, epigenetic knockout of the gene function that phenocopies a null mutation in the targeted gene (Brummenlkamp et al., Science 296: 550-3 (2002)). This combined approach using an integrated gene-expression database of non-small cell lung cancers and epigenetic knock-down of up-regulated genes provides a powerful strategy for rapid identification and evaluation of target molecules for a personalized therapy. The genes have been identified, that regulate growth, proliferation and/or survival of NSCLC cells. These genes encode proteins which function in the autocrine, cell cycle/growth and signal transduction, or products with unknown function.

The differentially expressed genes identified herein can be used for diagnostic purposes and to develop gene targeted therapeutic approaches for inhibiting non-small cell lung cancer.

The genes whose expression levels are modulated (*i.e*., increased or decreased) in non-small cell lung cancer patients are summarized in Tables 1-3 and are collectively referred to herein as "non-small cell lung cancer-associated genes", "NSC genes ", "NSC nucleic acids" or "NSC polynucleotides" and polypeptides encoded by them are referred to as "NSC polypeptides" or "NSC proteins". Unless indicated otherwise, "NSC" refers to any of the sequences disclosed herein (*e.g*., NSC 1-1448). The genes have been previously described and are presented along with a database accession number.

**Table 1 down-regulated genes**

| NSC AssigN ment | LMMID | Acc | Symbol | TITLE |
|---|---|---|---|---|
| 1 | A2125 | M31452 | C4BPA | complement component 4-binding protein, alpha |
| 2 | A0386 | K02215 | SERPINA8 | serine (or cysteine) proteinase inhibitor, clade A (alpha antiproteinase, antitrypsin), member 8 |
| 3 | B3893 | AA573809 | ITLN | Intelectin |
| 4 | A0038N | W73825 | TCF21 | transcription factor 21 |
| 5 | C8088 | D87465 | KIAA0275 | KIAA0275 gene product |
| 6 | D1273 | AJ001015 | RAMP2 | receptor (calcitonin) activity modifying protein 2 |
| 7 | C7138 | X64559 | TNA | tetranectin (plasminogen-binding protein) |
| 8 | C7919 | X79981 | CDH5 | cadherin 5, type 2, VE-cadherin (vascular epithelium) |
| 9 | A2202 | AJ001016 | RAMP3 | receptor (calcitonin) activity modifying protein 3 |
| 10 | A0960 | U60115 | FHL1 | four and a half LIM domains 1 |
| 11 | A0760 | L05568 | SLC6A4 | solute carrier family 6 (neurotransmitter transporter, serotonin), member 4 |
| 12 | A2415 | M15856 | LPL | lipoprotein lipase |
| 13 | A8600 | AI200539 | | Homo sapiens cDNA: FLJ22690 fis, clone HS111134 |
| 14 | A4375N | AB008109 | RGS5 | regulator of G-protein signalling 5 |
| 15 | A0919N | X55635 | MRC1 | mannose receptor, C type 1 |
| 16 | A6696 | AA491502 | C1QR | complement component Clq receptor |
| 17 | B1090N | AA156022 | FLJ20798 | hypothetical protein |
| 18 | C0893 | AA122287 | GARP | glycoprotein A repetitions predominant |
| 19 | C1603 | U01317 | HBB | hemoglobin, beta |
| 20 | C0724 | AA573140 | | ESTs |
| 21 | C8046 | X54380 | PZP | pregnancy-zone protein |
| 22 | C6234 | AI247176 | | DKFZP586L2024 protein |
| 23 | B5155 | W84893 | AGTRL1 | angiotensin receptor-like 1 |
| 24 | A6358 | AA533191 | | ESTs, Weakly similar to ALU7_HUMAN ALU SUBFAMILY SQ SEQUENCE CONTAMINATION WARNING ENTRY [H.sapiens] |
| 25 | B3794N | N94777 | | ESTs |
| 26 | B9790 | AA054482 | LOC51267 | C-type lectin-like receptor |
| 27 | C8228 | L36033 | SDFI | stromal cell-derived factor 1 |
| 28 | E0733 | AI459767 | SPARCL1 | SPARC-like 1 (mast9, hevin) |
| 29 | C2324 | AA036631 | | ESTs |
| 30 | A2508 | X03350 | ADH2 | alcohol dehydrogenase 2 (class I), beta polypeptide |
| 31 | B7122 | AA480009 | | Homo sapiens cDNA FLJ13569 fis, clone PLACE1008369 |
| 32 | A7775 | AA922655 | FGL2 | fibrinogen-like 2 |
| 33 | A0702N | AA449301 | FLT1 | fins-related tyrosine kinase 1 (vascular endothelial growth factor/vascular permeability factor receptor) |
| 34 | A4630 | U89281 | RODH | oxidative 3 alpha hydroxysteroid dehydrogenase; retinol dehydrogenase; 3-hydroxysteroid epimerase |
| 35 | A1739 | J02761 | SFTPB | surfactant, pulmonary-associated protein B |
| 36 | A6712 | W76197 | DLC1 | Deleted in liver cancer 1 |
| 37 | A4829N | D63412 | AQP4 | aquaporin 4 |
| 38 | B5205N | AI096938 | KIAA0758 | KIAA0758 protein |
| 39 | D4204 | AA868130 | | ESTs, Moderately similar to C4BP_HUMAN C4B-BINDING PROTEIN ALPHA CHAIN PRECURSOR [H.sapiens] |
| 40 | C1604 | AA044381 | HSD11B1 | hydroxysteroid (11-beta) dehydrogenase 1 |
| 41 | A2460 | AF000959 | CLDN5 | claudin 5 (transmembrane protein deleted in velocardiofacial syndrome) |
| 42 | A3360 | S77410 | AGTR1 | angiotensin receptor 1 |
| 43 | A1423 | L38486 | MFAP4 | microfibrillar-associated protein 4 |
| 44 | B9634 | AI094298 | | ESTs |
| 45 | B8029 | AI090219 | | ESTs |
| 46 | D8515 | U21128 | LUM | lumican |
| 47 | A2195 | AF022813 | TM4SF7 | transmembrane 4 superfamily member 7 |
| 48 | B8384 | AA147582 | | ESTs |
| 49 | B8411 | AA122240 | | Homo sapiens cDNA FLJ13612 fis, clone PLACE1010833, weakly similar to CALTRACTIN |
| 50 | B9603 | AI347579 | | ESTs |
| 51 | A6717 | AA487952 | SYNEB | synaptic nuclei expressed gene 1b |
| 52 | D0946 | AA780308 | KSP37 | Ksp37 protein |
| 53 | C6387 | AI022180 | | ESTs |
| 54 | A2542 | J02874 | FABP4 | fatty acid binding protein 4, adipocyte |
| 55 | A3412 | M10321 | VWF | von Willebrand factor |
| 56 | A4043 | AA777648 | PMP22 | peripheral myelin protein 22 |
| 57 | A1818N | AA600048 | CALD1 | caldesmon 1 |
| 58 | A2633N | D13628 | ANGPT1 | angiopoietin 1 |
| 59 | C4884 | AA150200 | | ESTs, Weakly similar to tuftelin [M.musculus] |
| 60 | B7922 | Al004344 | | Homo sapiens cDNA: FLJ21042 fis, clone CAE11204 |
| 61 | D3758 | AI193122 | | ESTs |
| 62 | A3037 | D12763 | IL1RL1 | interleukin 1 receptor-like 1 |
| 63 | D9082 | AI123738 | | ESTs |
| 64 | A2403 | S53911 | CD34 | CD34 antigen |
| 65 | C7654 | AA142989 | | ESTs |
| 66 | A1610 | X58295 | GPX3 | glutathione peroxidase 3 (plasma) |
| 67 | A6545 | M98479 | TGM2 | transglutaminase 2 (C polypeptide, protein-glutamine-gamma-glutamyltransferase |
| 68 | A8531 | AA634913 | FBLN5 | fibulin 5 |
| 69 | A7230 | X03963 | COL4A1 | collagen, type IV, alpha 1 |
| 70 | B4240 | AI218211 | FXYD6 | FXYD domain-containing ion transport regulator 6 |
| 71 | B3933 | AA487977 | ETL | ETL protein |
| 72 | D8933 | AI239735 | | ESTs |
| 73 | E1622 | AI985921 | CAV1 | caveolin 1, caveolae protein, 22kD |
| 74 | B9616 | AI208877 | NYD-SP21 | Testes development-related NYD-SP21 |
| 75 | B7170N | AA604083 | PCDH18 | protocadherin 18 |
| 76 | A6237 | L05485 | SFTPD | surfactant, pulmonary-associated protein D |
| 77 | A6665 | AI279606 | LOC55885 | neuronal specific transcription factor DAT1 |
| 78 | B4320 | AA029815 | C5ORF4 | chromosome 5 open reading frame 4 |
| 79 | B4291 | T04932 | | Homo sapiens cDNA: FLJ21545 fis, clone COL06195 |
| 80 | B3695 | AI090213 | | Homo sapiens mRNA; cDNA DKFZp586E2023 (from clone DKFZp586E2023) |
| 81 | C9642 | AA493650 | | Homo sapiens cDNA: FLJ23494 fis, clone LNG01885 |
| 82 | C0250 | U20391 | FOLR1 | folate receptor 1 (adult) |
| 83 | A0701 | U05291 | FMOD | fibromodulin |
| 84 | A7247N | AA873533 | | Homo sapiens mRNA; cDNA DKFZp586N0121 (from clone DKFZp586N0121) |
| 85 | C1412 | AA446539 | | ESTs |
| 86 | A2418 | M96789 | GJA4 | gap junction protein, alpha 4, 37kD (connexin 37) |
| 87 | D3727 | AA843148 | | ESTs |
| 88 | B5421 | AA648414 | | ESTs |
| 89 | C8253 | AA599019 | MEOX2 | mesenchyme homeo box 2 (growth arrest-specific homeo box) |
| 90 | A0878 | L13288 | VIPR1 | vasoactive intestinal peptide receptor 1 |
| 91 | B5175N | AI350168 | KIAA0833 | KIAA0833 protein |
| 92 | D5870 | AA972840 | | ESTs |
| 93 | B8423 | R65585 | | ESTs |
| 94 | B8392 | AA971017 | | Homo sapiens cDNA FLJ12028 fis, clone HEMBB1001850 |
| 95 | A6099 | W60630 | FLJ21935 | hypothetical protein FLJ21935 |
| 96 | B4694 | AA436726 | DKFZP564D0764 | DKFZP564D0764 protein |
| 97 | B8366 | AI342255 | | Homo sapiens cDNA FLJ20767 fis, clone COL06986 |
| 98 | C8048 | X58840 | TCF2 | transcription factor 2, hepatic; LF-B3; variant hepatic nuclear factor |
| 99 | C7458 | AI272261 | MBP | myelin basic protein |
| 100 | C1959 | AA192426 | KIAA0717 | ESTs, Weakly similar to PEBP MOUSE PHOSPHATID YLETHANOLAMINE-BINDING PROTEIN [M.musculus] |
| 101 | A3519 | D82348 | ATIC | 5-aminoimidazole-4-carboxamide ribonucleotide formyltransferase/IMP cyclohydrolase |
| 102 | A2049 | X67292 | IGHM | immunoglobulin heavy constant mu |
| 103 | A0694 | M91211 | AGER | advanced glycosylation end product-specific receptor |
| 104 | A4491 | L15388 | GPRK5 | G protein-coupled receptor kinase 5 |
| 105 | A7286 | AI301935 | CFFM4 | high affinity immunoglobulin epsilon receptor beta subunit |
| 106 | B4137 | AA148493 | | Homo sapiens cDNA: FLJ22300 fis, clone HRC04759 |
| 107 | B5721N | AI075111 | | Homo sapiens cDNA FLJ14054 fis, clone HEMBB 1000240 |
| 108 | D5083 | AA649967 | | ESTs |
| 109 | B6555N | AA904865 | KIAA1912 | ESTs |
| 110 | A5690 | AA927075 | KIAA1029 | synaptopodin |
| 111 | A6436 | AB014609 | KIAA0709 | endocytic receptor (macrophage mannose receptor family) |
| 112 | B6700 | AI215600 | KIAA1300 | KIAA1300 protein |
| 113 | B0081N | D59339 | KIAA1529 | Homo sapiens mRNA; cDNA DKFZp434I2420 (from clone DKFZp434I2420) |
| 114 | C7592 | AA936619 | DOK2 | docking protein 2, 56kD |
| 115 | C1703 | W84753 | | Homo sapiens cDNA FLJ13510 fis, clone PLACE1005146 |
| 116 | D1811 | AA594318 | LOC51304 | DHHC1 protein |
| 117 | A2740 | D85777 | CDO1 | cysteine dioxygenase, type I |
| 118 | A1779N | AF025534 | LILRB5 | leukocyte immunoglobulin-like receptor, subfamily B (with TM and ITIM domains), member 5 |
| 119 | C7721 | AI333309 | | ESTs |
| 120 | A7094 | U33749 | TITF1 | thyroid transcription factor 1 |
| 121 | B1352 | M18786 | AMY1A | amylase, alpha 1A; salivary |
| 122 | A1871N | AA778308 | RNASE1 | ribonuclease, RNase A family, 1 (pancreatic) |
| 123 | A4798N | Y15724 | ATP2A3 | ATPase, Ca++ transporting, ubiquitous |
| 124 | B5442 | AA633352 | | Homo sapiens cDNA: FLJ23067 fis, clone LNG04993 |
| 125 | B7814 | AA455087 | | ESTs |
| 126 | A1617 | X69490 | TTN | titin |
| 127 | A3536 | J03040 | SPARC | secreted protein, acidic, cysteine-rich (osteonectin) |
| 128 | A1150 | M37033 | CD53 | CD53 antigen |
| 129 | B2148 | M61900 | PTGDS | prostaglandin D synthase gene |
| 130 | A8156 | AI148659 | | ESTs |
| 131 | C1520 | D79303 | COL14A1 | collagen, type XIV, alpha 1 (undulin) |
| 132 | C9503 | AA621124 | | ESTs, Weakly similar to ALU2_HUMAN ALU SUBFAMILY SB SEQUENCE CONTAMINATION WARNING ENTRY [H.sapiens] |
| 133 | D3086 | AA806358 | | ESTs |
| 134 | B4661 | AA425371 | PTPRD | protein tyrosine phosphatase, receptor type, D |
| 135 | A0593 | X76939 | LAMA4 | laminin, alpha 4 |
| 136 | A0184 | M59832 | LAMA2 | laminin, alpha 2 (merosin, congenital muscular dystrophy) |
| 137 | B0565 | AI090498 | PCDH12 | protocadherin 12 |
| 138 | B7930 | N21096 | | ESTs |
| 139 | B4396 | W58589 | | ESTs |
| 140 | C0505 | AA926639 | FLJ11110 | hypothetical protein FLJ11110 |
| 141 | C0219 | AA235013 | AKAP2 | A kinase (PRKA) anchor protein 2 |
| 142 | A1450 | M33906 | HLA-DQA1 | major histocompatibility complex, class II, DQ alpha 1 |
| 143 | B1689 | AA664472 | | Homo sapiens mRNA full length insert cDNA clone EUROIMAGE 1913076 |
| 144 | C9556 | N30188 | | ESTs |
| 145 | C7651 | AA142875 | | ESTs |
| 146 | D9990 | Z25109 | ZP3A | zona pellucida glycoprotein 3A (sperm receptor) |
| 147 | A1365 | D10653 | TM4SF2 | transmembrane 4 superfamily member 2 |
| 148 | A1147 | M14354 | F13A1 | coagulation factor XIII, A1 polypeptide |
| 149 | A9462 | AA055019 | | ESTs |
| 150 | A6567 | C05229 | PDK4 | pyruvate dehydrogenase kinase, isoenzyme 4 |
| 151 | A0774N | M27717 | CPA3 | carboxypeptidase A3 (mast cell) |
| 152 | B6631 | AA968840 | | Homo sapiens HSPC285 mRNA, partial cds |
| 153 | A9546N | AI076929 | | ESTs, Weakly similar to Homolog of rat Zymogen granule membrane protein [H.sapiens] |
| 154 | C5014 | AI185804 | FN1 | fibronectin 1 |
| 155 | D4936 | AI084457 | NPR3 | natriuretic peptide receptor C/guanylate cyclase C (atrionatriuretic peptide receptor C) |
| 156 | | D1758 AA368303 | TIMP3 | tissue inhibitor of metalloproteinase 3 (Sorsby fundus dystrophy, pseudoinflammatory) |
| 157 | E3918 | AF055460 | STC2 | stanniocalcin 2 |
| 158 | B9722 | AA029906 | | ESTs |
| 159 | B7441 | AA994299 | | Homo sapiens cDNA FIJ20898 fis, clone ADKA03584 |
| 160 | A4014 | D28769 | PBX2 | pre-B-cell leukemia transcription factor 2 |
| 161 | B9242 | R59292 | MS4A8B | Membrane-spanning 4-domains, subfamily A, member 8B |
| 162 | A4872 | U19568 | SSCA1 | squamous cell carcinoma antigen |
| 163 | A0100 | J04513 | FGF2 | fibroblast growth factor 2 (basic) |
| 164 | B4665N | AA045171 | | ESTs |
| 165 | B9172 | AI221059 | DKFZP566K192 4 | DKFZP566K1924 protein |
| 166 | B9957 | H39098 | KIAA0843 | KIAA0843 protein |
| 167 | A5176 | U37791 | MMP19 | matrix metalloproteinase 19 |
| 168 | D5160 | AI336306 | | ESTs |
| 169 | A0898 | Z22641 | CHN1 | chimerin (chimaerin) 1 |
| 170 | A5370 | R37540 | | ESTs |
| 171 | A9317 | AA429693 | | ESTs |
| 172 | B6831 | X72012 | ENG | endoglin (Osler-Rendu-Weber syndrome 1) |
| 173 | B3699 | AA864739 | | Homo sapiens cDNA: FLJ21841 fis, clone HEP01831 |
| 174 | B7996N | W73609 | | ESTs |
| 175 | D1274 | AI147089 | | ESTs |
| 176 | C4665 | AF009314 | | Homo sapiens clone TUA8 Cri-du-chat region mRNA |
| 177 | A4026 | D50312 | KCNJ8 | potassium inwardly-rectifying channel, subfamily J, member 8 |
| 178 | A0764 | L10320 | FBP1 | fructose,6-bisphosphatase 1 |
| 179 | A2188 | J02770 | IF | I factor (complement) |
| 180 | A2510 | X04481 | C2 | complement component 2 |
| 181 | A6248 | M15178 | HLA-DRB1 | major histocompatibility complex, class II, DR beta 1 |
| 182 | A1761 | K01171 | HLA-DRA | major histocompatibility complex, class II, DR alpha |
| 183 | A7689 | X00457 | | Human mRNA for SB classII histocompatibility antigen alpha-chain |
| 184 | A8152 | AA485172 | HLA-DNA | (major histocompatibility complex, class II, DN alpha |
| 185 | B7304N | AA777308 | | Homo sapiens cDNA FLJ13942 fis, clone Y79AA1000962, weakly similar to MYOSIN HEAVY CHAIN, NON-MUSCLE |
| 186 | D0766 | AA424762 | | ESTs |
| 187 | C4971 | U20971 | NNMT | nicotinamide N-methyltransferase |
| 188 | B4321 | AA256196 | RBM8B | RNA binding motif protein 8B |
| 189 | B3746 | AA976403 | | Homo sapiens pancreas tumor-related protein (FKSG12) mRNA, complete cds |
| 190 | A9373 | M34570 | COL6A2 | collagen, type VI, alpha 2 |
| 191 | A1810N | X72755 | MIG | monokine induced by gamma interferon |
| 192 | C0371 | AA411749 | | ESTs |
| 193 | A3733 | X04665 | THBS1 | thrombospondin 1 |
| 194 | C1466 | H03229 | GAB1 | GRB2-associated binding protein 1 |
| 195 | A2066 | M28443 | AMY2A | amylase, alpha 2A; pancreatic |
| 196 | C6547 | A774546 | NXF3 | nuclear RNA export factor 3 |
| 197 | A0401 | X00637 | HP | haptoglobin |
| 198 | B9211 | AI075316 | FLJ14033 | hypothetical protein FLJ14033 similar to hypoxia inducible factor 3, alpha subunit |
| 199 | A7978 | J04813 | CYP3A5 | cytochrome P450, subfamily IIIA (niphedipine oxidase), polypeptide 5 |
| 200 | B6827N | AA127856 | MPDZ | multiple PDZ domain protein |
| 201 | C4268 | AA885514 | | ESTs, Weakly similar to CAYP_HUMAN CALCYPHOSINE [H.sapiens] |
| 202 | A2487 | D10923 | HM74 | putative chemokine receptor; GTP-binding protein |
| 203 | C7059 | AA455044 | | ESTs, Weakly similar to AF257182 1 G-protein-coupled receptor 48 [H.sapiens] |
| 204 | A3250 | M14144 | VIM | vimentin |
| 205 | A5556 | AA310364 | TIMP2 | tissue inhibitor of metalloproteinase 2 |
| 206 | A6458 | H71292 | SLC21A9 | solute carrier family 21 (organic anion tansporter), member 9 |
| 207 | B3759 | AI366242 | | ESTs |
| 208 | B9826 | AA621350 | SLIT2 | slit (Drosophila) homolog 2 |
| 209 | E0336 | AI097529 | EPAS1 | endothelial PAS domain protein 1 |
| 210 | A6143 | AF035315 | | Homo sapiens clone 23664 and 23905 mRNA sequence |
| 211 | B7171 | H75419 | | Duodenal cytochrome b |
| 212 | C7813 | AI201273 | | ESTs |
| 213 | C9730 | AA030027 | | ESTs |
| 214 | D8827 | AA484891 | | ESTs |
| 215 | A1572 | U76421 | ADARB1 | adenosine deaminase, RNA-specific, B1 (homolog of rat RED1) |
| 216 | A1516 | U24488 | TNXA | tenascin XA |
| 217 | B4852N | X02530 | SCYB10 | small inducible cytokine subfamily B (Cys-X-Cys), member 10 |
| 218 | A0791 | L13923 | FBN1 | fibrillin 1 (Marfan syndrome) |
| 219 | B8265 | AA1 56792 | HEYL | hairy/enhancer-of-split related with YRPW motif-like |
| 220 | A1064 | M33492 | TPSB1 | tryptase beta 1 |
| 221 | A1708 | X85337 | MYLK | myosin, light polypeptide kinase |
| 222 | A4453 | AF027299 | EPB41L2 | erythrocyte membrane protein band 4.1-like 2 |
| 223 | B8354 | AA279159 | WASPIP | Wiskott-Aldrich syndrome protein interacting protein |
| 224 | C9565 | AA252389 | LHFP | lipoma HMGIC fusion partner |
| 225 | A3560 | L06797 | CXCR4 | chemokine (C-X-C motif), receptor 4 (fusin) |
| 226 | A2135 | U29091 | SELENBP1 | selenium binding protein 1 |
| 227 | A0578 | X68277 | DUSP1 | dual specificity phosphatase 1 |
| 228 | A0884 | U15085 | HLA-DMB | major histocompatibility complex, class II, DM beta |
| 229 | B1475 | AA918725 | ARRB1 | arrestin, beta 1 |
| 230 | B4085 | T34883 | AQP1 | aquaporin 1 (channel-forming integral protein, 28kD) |
| 231 | C4095 | K01505 | | DC classII histocompatibility antigen alpha-chain |
| 232 | B8722 | AB007923 | KIAA0477 | KIAA0477 gene product |
| 233 | B9564 | H85019 | KPNB1 | Karyopherin (importin) beta 1 |
| 234 | A1816N | M31158 | PRKAR2B | protein kinase, cAMP-dependent, regulatory, type II, beta |
| 235 | B8257 | AA426140 | | Homo sapiens cDNA FLJ11022 fis, clone PLACE1003771 |
| 236 | A9393N | W67577 | CD74 | CD74 antigen (invariant polypeptide of major histocompatibility complex, class II antigen-associated) |
| 237 | C6900 | AA707766 | | ESTs |
| 238 | B9368 | AI342469 | | ESTs |
| 239 | B3966 | AI038938 | | ESTs |
| 240 | B1004 | N87886 | MMP2 | matrix metalloproteinase 2 (gelatinase A, 72kD gelatinase, 72kD type IV collagenase) |
| 241 | A1807N | L76465 | HPGD | hydroxyprostaglandin dehydrogenase 15-(NAD) |
| 242 | C3775 | AW243357 | | Homo sapiens clone 24775 mRNA sequence |
| 243 | A1951 | L08895 | MEF2C | MADS box transcription enhancer factor 2, polypeptide C (myocyte enhancer factor 2C) |
| 244 | A7679 | U38894 | ROR1 | receptor tyrosine kinase-like orphan receptor 1 |
| 245 | B9803 | C02532 | COL21A1 | Collagen, type XXI, alpha 1 |
| 246 | D1219 | AA453640 | | ESTs, Weakly similar to KCC1_HUMAN CALCIUM/CALMODULIN-DEPENDENT PROTEIN KINASE TYPE I [H.sapiens] |
| 247 | A3061 | M83202 | LTF | lactotransferrin |
| 248 | A0875 | L13740 | NR4A1 | nuclear receptor subfamily 4, group A, member 1 |
| 249 | A4794 | AF064492 | LDB2 | LIM domain binding 2 |
| 250 | | A7232N AA669034 | | Homo sapiens cDNA: FLJ23125 fis, clone LNG08217 |
| 251 | C3653 | AL133415 | DNMT2 | DNA (cytosine-5-)-methyltransferase 2 |
| 252 | C8039 | Z22970 | CD163 | CD163 antigen |
| 253 | C8158 | K03431 | HPR | haptoglobin-related protein |
| 254 | B9924 | W52782 | LOC58514 | HUEL (C4orf1)-interacting protein |
| 255 | A1406 | L07555 | CD69 | CD69 antigen (p60, early T-cell activation antigen) |
| 256 | A3488 | U39050 | DAB2 | disabled (Drosophila) homolog 2 (mitogen-responsive phosphoprotein) |
| 257 | A1011 | M75106 | CPB2 | carboxypeptidase B2 (plasma) |
| 258 | A2427 | U69546 | CUGBP2 | CUG triplet repeat, RNA-binding protein 2 |
| 259 | B0337 | R37044 | MAPRE2 | microtubule-associated protein, RP/EB family, member 2 |
| 260 | A7942 | AA578712 | | ESTs |
| 261 | C0629 | H16793 | C8ORF4 | chromosome 8 open reading frame 4 |
| 262 | C7057 | H22566 | DACH | Dachshund homolog (Drosophila) |
| 263 | D5981 | AA974905 | FSCN3 | fascin (Strongylocentrotus purpuratus) homolog 3 (actin-bundling protein, testicular) |
| 264 | C5002 | AC002076 | GNG11 | guanine nucleotide binding protein 11 |
| 265 | A8488 | N75156 | | Homo sapiens cDNA FLJ11570 fis, clone HEMBA1003309 |
| 266 | B9013 | AA904456 | | ESTs |
| 267 | B9925 | AA993564 | | Homo sapiens mRNA; cDNADKFZp564E153 (from clone DKFZp564E1 53) |
| 268 | C4743 | AA699559 | NYD-5P15 | Protein kinase NYD-SP15 |
| | 269 A3015 | J04080 | C1S | complement component 1, s subcomponent |
| 270 | B6414N | AA429149 | C11ORF9 | chromosome 11 open reading frame 9 |
| 271 | E0523 | AA478501 | AHNAK | AHNAK nucleoprotein (desmoyokin) |
| 272 | A1414 | L25286 | COL15A1 | collagen, type XV, alpha 1 |
| 273 | C3724 | NP_055269 | PA26 | p53 regulated PA26 nuclear protein |
| 274 | B0267 | R78436 | GATA2 | GATA-binding protein 2 |
| 275 | A3189 | M16801 | NR3C2 | nuclear receptor subfamily 3, group C, member 2 |
| 276 | B8656 | AA398561 | FLJ30371 | hypothetical protein FLJ20371 |
| 277 | C8205 | AI276150 | TUCAN | Tumor up-re gulated CARD-containing antagonist of caspase nine |
| 278 | A9285 | AI027810 | KIAA1102 | KIAA1102 protein |
| 279 | C8636 | AA478752 | DKK3 | Dickkopf homolog 3 (Xenopus laevis) |
| 280 | A3178 | M29696 | IL7R | interleukin 7 receptor |
| 281 | C4735 | AA258282 | KIAA1474 | KIAA1474 protein |
| 282 | A1137 | L20688 | ARHGDIB | Rho GDP dissociation inhibitor (GDI) beta |
| 283 | A4414 | X97187 | ABCA3 | ATP-binding cassette, sub-family A (ABC1), member |
| 284 | A2404 | M15395 | ITGB2 | integrin, beta 2 (antigen CD18 (p95), lymphocyte function-associated antigen 1; macrophage antigen 1 (mac) beta subunit) |
| 285 | A8898 | AI022075 | FLJ13732 | hypothetical protein FLJ13732 similar to tensin |
| 286 | B3924 | AI079876 | HSPB7 | heat shock 27kD protein family, member 7 (cardiovascular) |
| 287 | B5776N | U51712 | | ESTs |
| 288 | B9533 | W44970 | SCA7 | spinocerebellar ataxia 7 (olivopontocerebellar atrophy with retinal degeneration) |
| 289 | B0830N | AA452493 | ID4 | inhibitor of DNA binding 4, dominant negative helix-loop-helix protein |
| 290 | A9067N | AI268375 | DDB1 | damage-specific DNA binding protein 1 (127kD) |
| 291 | C6721 | AA761358 | 753P9 | Chromosome X open reading frame 9 |
| 292 | B4440 | AA418784 | LOC64116 | up-regulated by BCG-CWS |
| 293 | A2644 | X04299 | ADH3 | alcohol dehydrogenase 3 (class I), gamma polypeptide |
| 294 | A2972 | X72475 | IGKC | immunoglobulin kappa constant |
| 295 | A1023 | X05610 | COL4A2 | collagen, type IV, alpha 2 |
| 296 | C6386 | W05570 | DKFZP586B0621 | DKFZP586B0621 protein |
| 297 | D8491 | X97324 | ADFP | adipose differentiation-related protein |
| 298 | AI275 | AF020543 | PPT2 | palmitoyl-protein thioesterase 2 |
| 299 | A6750 | U09608 | NKG7 | natural killer cell group 7 sequence |
| 300 | A3822 | AB001928 | CTSL2 | cathepsin L2 |
| 301 | C8282 | R84710 | ASAHL | N-acylsphingosine amidohydrolase (acid ceramidase)-like |
| 302 | B9777 | AA903451 | SRCL | Collectin sub-family member 12 |
| 303 | A0090 | D50683 | TGFBR2 | transforming growth factor, beta receptor II (70-80kD) |
| 304 | B9201 | AI083491 | WIF | Wnt inhibitory factor |
| 305 | B1531 | AA775224 | NPR1 | natriuretic peptide receptor A/guanylate cyclase A (atrionatriuretic peptide receptor A) |
| 306 | A7760N | M62324 | MRF | modulator recognition factor I |
| 307 | B9970 | H00903 | KIAA0640 | Homo sapiens mRNA; cDNA DKFZp586E0724 (from clone DKFZp586E0724) |
| 308 | A4238 | AI089249 | HK3 | hexokinase 3 (white cell) |
| 309 | C3772 | AW237266 | ASAH | N-acylsphingosine amidohydrolase (acid ceramidase) |
| 310 | A4709 | U62015 | CYR61 | cysteine-rich, angiogenic inducer, 61 |
| 311 | B4288 | AI078144 | HNOEL-iso | HNOEL-iso protein |
| 312 | A6266 | AA830322 | LMO2 | LIM domain only 2 (rhombotin-like 1) |
| 313 | A7233 | AA742701 | LCP1 | lymphocyte cytosolic protein 1 (L-plastin) |
| 314 | B8141 | AA431105 | | Homo sapiens cDNA: FIJ21310 fis, clone COL02160 |
| 315 | | A3200N AA419374 | COL5A1 | collagen, type V, alpha 1 |
| 316 | C0922 | AA136856 | | ESTs |
| 317 | C0787 | AA448082 | | ESTs |
| 318 | A4660 | M20681 | SLC2A3 | solute carrier family 2 (facilitated glucose transporter), member 3 |
| 319 | A5487 | AA256347 | KIAA1075 | KIAA1075 protein |
| 320 | A0597 | X79683 | LAMB2 | laminin, beta 2 (laminin S) |
| 321 | A3790 | X76104 | DAPK1 | death-associated protein kinase 1 |
| 322 | A1496 | U03688 | CYP1B1 | cytochrome P450, subfamily I (dioxin-inducible), polypeptide 1 (glaucoma 3, primary infantile) |
| 323 | C9716 | C17007 | | ESTs, Highly similar to C1QC_HUMAN COMPLEMENT C1Q SUBCOMPONENT, C CHAIN PRECURSO [H. sapiens] |
| 324 | D8527 | J03037 | CA2 | carbonic anhydrase II |
| 325 | B2696 | AA847136 | CSF2RB | Colony stimulating factor 2 receptor, beta, low-affinity (granulocyte-macrophage) |
| 326 | D8609 | AI052435 | | ESTs, Weakly similar to neuronal-STOP protein [M.musculus] |
| 327 | E0896 | AI141649 | NID | nidogen (enactin) |
| 328 | D7108 | AI005420 | | ESTs |
| 329 | A0055N | AF058925 | JAK2 | Janus kinase 2 (a protein tyrosine kinase) |
| 330 | A3613 | U20157 | PLA2G7 | phospholipase A2, group VII (platelet-activating factor acetylhydrolase, plasma) |
| 331 | A1879 | AF016004 | GPM6B | glycoprotein M6B |
| 332 | A0711 | AF068836 | pSC-DBP | pleckstrin homology, Sec7 and coiled/coil domains, binding protein |
| 333 | B9198 | AA609519 | MSRA | methionine sulfoxide reductase A |
| 334 | A9346N | AA317645 | PP2135 | PP2135 protein |
| 335 | B7497 | AA687507 | | ESTs |
| 336 | B5284 | A452079 | | Human DNA sequence from clone RP11-524D16 on chromosome X. Contains ESTs, STSs and GSSs. Contains the 3' part of the SRPX gene for a sushi-repeat containing protein and a novel gene for two protein isoforms similar to mouse granuphilin a und b |
| 337 | A2471 | J03578 | ANXA6 | annexin A6 |
| 338 | C4068 | AF005668 | PFC | properdin P factor, complement |
| 339 | A0968 | M74718 | TCF4 | transcription factor 4 |
| 340 | B8090 | AA152211 | KIAA1538 | KIAA1538 protein |
| 341 | A2257N | AA625532 | DDR2 | discoidin domain receptor family, member 2 |
| 342 | A1780N | AA449181 | ENPP2 | ectonucleotide pyrophosphatase/phosphodiesterase 2 (autotaxin) |
| 343 | A1859N | AA418167 | GATA3 | GATA-binding protein 3 |
| 344 | C3778 | BF060779 | MSTP032 | MSTP032 protein |
| 345 | A0654 | M15800 | MAL | mal, T-cell differentiation protein |
| 346 | B4400 | AI299106 | KIAA1500 | KIAA1500 protein |
| 347 | B5172N | AI288487 | CLIC2 | chloride intracellular channel 2 |
| 348 | A0745 | L19871 | ATF3 | activating transcription factor 3 |
| 349 | A7672 | U20982 | IGFBP4 | insulin-like growth factor-binding protein 4 |
| 350 | B1056N | AA757029 | DF | D component of complement (adipsin) |
| 351 | | A1397N M60484 | PPP2CB | protein phosphatase 2 (formerly 2A), catalytic subunit, beta isoform |
| 352 | C8146 | X53331 | MGP | matrix Gla protein |
| 353 | A0582 | X69819 | ICAM3 | intercellular adhesion molecule 3 |
| 354 | A2074 | K01396 | SERPINA1 | serine (or cysteine) proteinase inhibitor, clade A (alpha antiproteinase, antitrypsin), member 1 |
| 355 | A1092 | M57230 | IL6ST | interleukin 6 signal transducer (gp130, oncostatin M receptor) |
| 356 | A4819 | D17408 | CNN1 | calponin 1, basic, smooth muscle |
| 357 | A6251 | M25460 | IFNB1 | interferon, beta 1, fibroblast |
| 358 | C4126 | U55766 | HRB2 | HIV rev binding protein 2 |
| 359 | B8113 | AA263000 | RNASE6 | ribonuclease, RNase A family, k6 |
| 360 | B0297 | AA775440 | KIAA0909 | KIAA0909 protein |
| 361 | B7624 | AA434557 | LNK | lymphocyte adaptor protein |
| 362 | B9647 | AA688025 | | ESTs |
| 363 | B4246 | AA479071 | | Homo sapiens clone 24877 mRNA sequence |
| 364 | B5399 | N36581 | D2S448 | Melanoma associated gene |
| 365 | B7723 | AI140597 | LIFR | leukemia inhibitory factor receptor |
| 366 | A6595N | AA775497 | KIAA1095 | Homo sapiens mRNA; cDNA DKFZp564J0923 (from lone DKFZp564J0923) |
| 367 | B7465 | AI197941 | | Homo sapiens mRNA; cDNA DKFZp761K2024 (from clone DKFZp761K2024) |
| 368 | C8947 | AA805687 | | ESTs |
| 369 | C8186 | AA059489 | RGC32 | RGC32 protein |
| 370 | C1622 | AA807551 | | ESTs |
| 371 | C1422 | AA095034 | GK001 | GK001 protein |
| 372 | C4105 | AA494120 | ENPP4 | ectonucleotide pyrophosphatase/phosphodiesterase 4 (putative function) |
| 373 | C9836 | AA157832 | KIAA4844 | Homo sapiens cDNA: FLJ22841 fis, clone FAIA4844 |
| 374 | D9444 | AI367157 | | ESTs |
| 375 | D9939 | AI079987 | | ESTs |
| 376 | A4385N | X59770 | IL1R2 | interleukin 1 receptor, type II |
| 377 | B7869N | R42449 | FLJ10357 | hypothetical protein FLJ10357 |
| 378 | B9611 | AA427796 | KIAA1754 | ESTs |
| 379 | B5396 | AA446322 | FLJ11240 | hypothetical protein FLJ11240 |
| 380 | D0735 | AA740582 | ARL5 | ADP-ribosylation factor-like 5 |
| 381 | A1032 | M87790 | IGL@ | immunoglobulin lambda locus |
| 382 | A2530 | J02611 | APOD | apolipoprotein D |
| 383 | A4655N | L77864 | APBB1 | amyloid beta (A4) precursor protein-binding, family B, member 1 (Fe65) |
| 384 | C0475 | U57961 | 13CDNA73 | putative gene product |
| 385 | A9282 | AA889218 | OGN | osteoglycin (osteoinductive factor, mimecan) |
| 386 | B1451N | AI057161 | | ESTs |
| 387 | C7773 | AI300074 | | ESTs, Weakly similar to S43506 hypothetical protein - rat [R.norvegicus] |
| 388 | B3063 | T91708 | MD | MD, RP105-associated |
| 389 | A3903 | AF026692 | SFRP4 | secreted frizzled-related protein 4 |
| 390 | B8377 | N50822 | | ESTs |
| 391 | A5720 | AI218225 | SPON1 | spondin 1, (f-spondin) extracellular matrix protein |
| 392 | B7274 | AA777360 | KIAA1002 | ESTs |
| 393 | A0765 | M77477 | ALDH3 | aldehyde dehydrogenase 3 |
| 394 | A3563 | L10333 | RTN1 | reticulon 1 |
| 395 | B6062 | AA773223 | SLC16A3 | solute carrier family 16 (monocarboxylic acid transporters), member 3 |
| 396 | C8044 | AA987624 | EGR3 | early growth response 3 |
| 397 | B8707 | AA173755 | ROBO1 | roundabout (axon guidance receptor, Drosophila) homolog 1 |
| 398 | C7370 | AA961425 | EOMES | Eomesodermin homolog (Xenopus laevis) |
| 399 | D4501 | AA521117 | | ESTs |
| 400 | A1750 | D31716 | BTEB1 | basic transcription element binding protein 1 |
| 401 | A1522 | LT28369 | SEMA3B | sema domain, immunoglobulin domain (Ig), short basic domain, secreted, (semaphorin) 3B |
| 402 | A8482 | R79064 | | ESTs, Weakly similar to putative type III alcohol dehydrogenase [D.melanogaster] |
| 403 | B9053 | AA446948 | KIAA0941 | KIAA0941 protein |
| 404 | B4643 | AI332375 | FSTL3 | follistatin-like 3 (secreted glycoprotein) |
| 405 | C0825 | D61466 | | ESTs |
| 406 | C3648 | W79423 | | Homo sapiens mRNA; cDNA DKFZp586P1622 (from clone DKFZp586P1622) |
| 407 | D4020 | AA858162 | | Homo sapiens cDNA FLJ13005 fis, clone NT2RP3000441, weakly similar to Homo sapiens squamous cell carcinoma antigen recognized by T cell (SART-2) mRNA |
| 408 | E1621 | AL117515 | PLCE2 | phospholipase C, epsilon 2 |
| 409 | A5442 | AI290876 | KLF4 | Kruppel-like factor 4 (gut) |
| 410 | A9482 | AI160184 | LOC51673 | brain specific protein |
| 411 | A3867 | AF013249 | LAIR1 | leukocyte-associated Ig-like receptor 1 |
| 412 | A1510 | U16306 | CSPG2 | chondroitin sulfate proteoglycan 2 (versican) |
| 413 | B9132 | AA455877 | | Homo sapiens cDNA FLJ11177 fis, clone PLACE1007402 |
| 414 | A2291 | AF003341 | ALDH1 | aldehyde dehydrogenase 1, soluble |
| 415 | A1010 | X83378 | CLCN6 | chloride channel 6 |
| 416 | B8379 | D25869 | DKFZP434I1735 | DKFZP434I1735 protein |
| 417 | B6622 | AA369905 | | ESTs |
| 418 | C8388 | N92299 | FLJ21939 | hypothetical protein FLJ21939 similar to 5-azacytidine induced gene 2 |
| 419 | C4116 | AA242923 | DXS9928E | DNA segment on chromosome X (unique) 9928 expressed sequence |
| 420 | B8203 | D81610 | FLJ11109 | hypothetical protein FLJ11109 |
| 421 | A1431 | L43821 | HEF1 | enhancer of filamentation 1 (cas-like docking; Crk-associated substrate related) |
| 422 | B5949 | AA678263 | BIN2 | bridging integrator 2 |
| 423 | C7886 | AI270402 | INHBA | inhibin, beta A (activin A, activin AB alpha polypeptide) |
| 424 | A1405 | L01042 | TMF1 | TATA element modulatory factor 1 |
| 425 | B3940 | W45244 | C3 | complement component 3 |
| 426 | A1387 | D86479 | AEBP1 | AE-binding protein 1 |
| 427 | A1748 | U29089 | PRELP | proline arginine-rich end leucine-rich repeat protein |
| 428 | A3054 | U01839 | FY | Duffy blood group |
| 429 | | A2039N AA843756 | ID2 | inhibitor of DNA binding 2, dominant negative helix-loop-helix protein |
| 430 | B6319 | AA328385 | ICSBP1 | interferon consensus sequence binding protein 1 |
| 431 | B4364 | AI305201 | VRL | vanilloid receptor-like protein 1 |
| 432 | B4638 | AI122867 | | Homo sapiens cDNA FLJ12666 fis, clone NT2RM4002256 |
| 433 | D9799 | AI074177 | C1QA | complement component 1, q subcomponent, alpha polypeptide |
| 434 | A2523 | D21238 | GLRX | glutaredoxin (thioltransferase) |
| 435 | A5449 | U90654 | LMO7 | LIM domain only 7 |
| 436 | A3409 | L77564 | STK22B | serine/threonine kinase 22B (spermiogenesis associated) |
| 437 | A0174 | M37435 | CSF1 | colony stimulating factor 1 (macrophage) |
| 438 | B2439 | U04735 | STCH | stress 70 protein chaperone, microsome-associated, 60kD |
| 439 | B5470 | AA876372 | | ESTs, Weakly similar to ALU1_HUMAN ALU SUBFAMILY J SEQUENCE CONTAMINATION WARNING ENTRY [H.sapiens] |
| 440 | B4864 | X16665 | HOXB2 | homeo box B2 |
| 441 | B5800 | AA233243 | BM046 | uncharacterized bone marrow protein BM046 |
| 442 | C4170 | AB007884 | ARHGEF9 | Cdc42 guanine exchange factor (GEF) 9 |
| 443 | A5504N | AF052178 | | Homo sapiens cDNA: FLJ21897 fis, clone HEP03447, highly similar to AF052178 Homo sapiens clone 24523 mRNA sequence |
| 444 | B4574 | AI042204 | FLJ12895 | hypothetical protein FLJ12895 |
| 445 | B6998 | AA401227 | SEC31B-1 | Secretory pathway component Sec31B-1 |
| 446 | B9299 | N53090 | | Homo sapiens mRNA; cDNA DKFZp434I0835 (from clone DKFZp434I0835) |
| 447 | A3538 | J03464 | COL1A2 | collagen, type I, alpha 2 |
| 448 | A8508N | AA977227 | NET-6 | tetraspan NET-6 protein |
| 449 | A1887N | W76477 | JUN | v-jun avian sarcoma virus 17 oncogene homolog |
| 450 | B5459 | AA666119 | | ESTs, Highly similar to GBP1_HUMAN INTERFERON-INDUCED GUANYLATE-BINDING PROTEIN 1 [H.Sapiens] |
| 451 | B4646 | AI245038 | GLS | glutaminase |
| 452 | C3799 | BE873804 | | Homo sapiens mRNA; cDNA DKFZp564F053 (from clone DKFZp564F053) |
| 453 | C8119 | D87258 | PRSS11 | protease, serine, 11 (IGF binding) |
| 454 | D8494 | D16294 | ACAA2 | acetyl-Coenzyme A acyltransferase 2 (mitochondrial 3-oxoacyl-Coenzyme A thiolase) |
| 455 | E1456 | AB040951 | FLJ20004 | hypothetical protein FLJ20004 |
| 456 | B2119 | M33552 | LSP1 | lymphocyte-specific protein 1 |
| 457 | B0979 | AI361053 | | ESTs |
| 458 | A4702 | U53445 | DOC1 | downregulated in ovarian cancer 1 |
| 459 | D0737 | AA885279 | | ESTs |
| 460 | A0753 | L10918 | CCR1 | chemokine (C-C motif) receptor 1 |
| 461 | A3977 | AF069736 | PAF65B | PCAF associated factor 65 beta |
| 462 | A2839 | M36284 | GYPC | glycophorin C (Gerbich blood group) |
| 463 | A2019N | AA442410 | EMP1 | epithelial membrane protein 1 |
| 464 | A3203 | M64925 | MPP1 | membrane protein, palmitoylated 1 (55kD) |
| 465 | A0539 | U23946 | RBM5 | RNA binding motif protein 5 |
| 466 | A5899 | D61837 | KIAA1109 | KIAA1109 protein |
| 467 | A3119 | J04621 | SDC2 | syndecan 2 (heparan sulfate proteoglycan 1, cell surface-associated, fibroglycan) |
| 468 | A3745 | X16155 | NR2F1 | nuclear receptor subfamily 2, group F, member 1 |
| 469 | A7016 | U82108 | SLC9A3R2 | solute carrier family 9 (sodium/hydrogen exchanger), isoform 3 regulatory factor 2 |
| 470 | B2609 | N42862 | KIAA1434 | hypothetical protein FLJ11085 |
| 471 | B1966 | AA933908 | ROCK1 | Rho-associated, coiled-coil containing protein kinase 1 |
| 472 | A2214N | L37080 | FMO5 | flavin containing monooxygenase 5 |
| 473 | D4128 | W37848 | ARTS | type 1 tumor necrosis factor receptor shedding aminopeptidase regulator |
| 474 | A7678 | U32331 | RIG | regulated in glioma |
| 475 | B5489 | AI278652 | AP1S2 | adaptor-related protein complex 1, sigma 2 subunit |
| 476 | A0563 | X58288 | PTPRM | protein tyrosine phosphatase, receptor type, M |
| 477 | A4641 | J02854 | MYRL2 | myosin regulatory light chain 2, smooth muscle isoform |
| 478 | B6764 | AA313118 | DUSP10 | dual specificity phosphatase 10 |
| 479 | A6780 | M63262 | ALOX5AP | arachidonate 5-lipoxygenase-activating protein |
| 480 | A3161N | M92843 | ZFP36 | zinc finger protein homologous to Zfp-36 in mouse |
| 491 | B5367 | AA151153 | DPT | dermatopontin |
| 482 | A6156 | AA587167 | ARHE | ras homolog gene family, member E |
| 483 | A0127 | L24158 | ITGA9 | integrin, alpha 9 |
| 484 | B1524 | AI126293 | | ESTs |
| 485 | A6781 | M69199 | G0S2 | putative lymphocyte G0/G1 switch gene |
| 486 | B8775 | AA588212 | FLJ10128 | uveal autoantigen with coiled coil domains and ankyrin repeats |
| 487 | A0300 | U43142 | VEGFC | vascular endothelial growth factor C |
| 488 | A6530 | AI089584 | ADAMTS1 | a disintegrin-like and metalloprotease (reprolysin type) with thrombospondin type 1 motif, 1 |
| 489 | A0971 | D83407 | ZAKI4 | Down syndrome critical region gene 1-like 1 |
| 490 | D4142 | N93781 | TAX1BP1 | Tax1 (human T-cell leukemia virus type I) binding protein 1 |
| 491 | A1485N | S69790 | WASF3 | WAS protein family, member 3 |
| 492 | B8036 | R20340 | | ESTs |
| 493 | C9718 | W94051 | | ESTs |
| 494 | E0872 | AK025627 | | Homo sapiens cDNA: FLJ21974 fis, clone HEP05861 |
| 495 | B0243 | R76379 | LOC51316 | hypothetical protein |
| 496 | A1981 | U58514 | CHI3L2 | chitinase 3-like 2 |
| 497 | A2158 | Z11793 | SEPPI | selenoprotein P, plasma, 1 |
| 498 | A0975 | M14333 | FYN | FYN oncogene related to SRC, FGR, YES |
| 499 | B4849 | W74368 | | Homo sapiens cDNA: FLJ23324 fis, clone HEP12482, highly similar to HUMMYOHCB Human nonmuscle myosin heavy chain-B (MYH10) mRNA |
| 500 | A7640 | AA147751 | | Homo sapiens cDNA FLJ14146 fis, clone MAMMA1 002947 |
| 501 | C0830 | AA012832 | | ESTs |
| 502 | C6974 | AA679312 | HIBCH | 3-hydroxyisobutyryl-Coenzyme A hydrolase |
| 503 | E0289 | AI224952 | | ESTs |
| 504 | B4750 | AA769424 | VNN2 | vanin 2 |
| 505 | A3334 | M90696 | CTSS | cathepsin S |
| 506 | B1676 | AJ001563 | IGHG3 | immunoglobulin heavy constant gamma 3 (G3m marker) |
| 507 | C7731 | AI142828 | | Homo sapiens adlican mRNA, complete cds |
| 508 | C4700 | AA099820 | | ESTs |
| 509 | D0533 | AA234500 | ARHGEF12 | Rho guanine exchange factor (GEF) 12 |
| 510 | A4744 | AF020202 | UNC13 | UNC13 (C. elegans)-like |
| 511 | A1154 | M62401 | CYP27A1 | cytochrome P450, subfamily XXVIIA (steroid 27-hydroxrylase, cerebrotendinous xanthomatosis), polypeptide 1 |
| 512 | A2292 | X16832 | CTSH | cathepsin H |
| 513 | A1825 | X76775 | HLA-DMA | major histocompatibility complex, class II, DM alpha |
| 514 | A3841 | AF000984 | DBY | DEAD/H (Asp-Glu-Ala-Asp/His) box polypeptide, Y chromosome |
| 515 | A4645 | L13852 | UBE1L | ubiquitin-activating enzyme E1-like |
| 516 | A9003 | W39638 | FLJ10856 | hypothetical protein FLJ10856 |
| 517 | A7239 | AA523541 | GILZ | glucocorticoid-induced leucine zipper |
| 518 | A3308N | L23823 | ITGB7 | integrin beta 7 subunit |
| 519 | B8437 | Z20328 | DKFZp434C0328 hypothetical | protein DKFZp434C0328 |
| 520 | B4481 | AA857089 | DKFZP566G142 | hypothetical protein DKFZp566G1424 |
| | | | 4 | |
| 521 | B6014N | H09503 | KIAA0740 | KIAA0740 gene product |
| 522 | B6825 | AI290349 | C5 | complement component 5 |
| 523 | B9233 | AA211909 | | ESTs |
| 524 | B5381N | D42047 | KIAA0089 | KIAA0089 protein |
| 525 | B7003N | AF045584 | POV1 | prostate cancer overexpressed gene 1 |
| 526 | C8356 | AI265858 | | Human clone 23574 mRNA sequence |
| 527 | C4596 | AI344470 | | ESTs |
| 528 | C6906 | AA346311 | RAI3 | retinoic acid induced 3 |
| 529 | C8023 | M81141 | HLA-DQB1 | major histocompatibility complex, class Il, DQ beta 1 |
| 530 | D2661 | AA894447 | | Human BAC clone GS1-99H8 |
| 531 | B7659 | AB007924 | KIAA0455 | KIAA0455 gene product |
| 532 | A6593 | AI160213 | ANGPTL2 | Angiopoietin-like 2 |
| 533 | B7526 | R40594 | | Homo sapiens cDNA: FLJ22845 fis, clone KAIA5195 |
| 534 | B7796 | N52157 | | Homo sapiens mRNA; cDNA DKFZp76201615 (from clone DKFZp76201615) |
| 535 | A8525 | W67837 | AHSG | alpha-2-HS-glycoprotein |
| 536 | E0537 | AW276358 | DPYSL2 | dihydropyrimidinase-like 2 |
| 537 | A4254 | AI140851 | COL6A1 | collagen, type VI, alpha 1 |
| 538 | A0941 | S59049 | RGS1 | regulator of G-protein signalling 1 |
| 539 | A2122 | AB003476 | AKAP12 | A kinase (PRKA) anchor protein (gravin) 12 |
| 540 | A9501 | AA279817 | GADD45B | growth arrest and DNA-damage-inducible, beta |
| 541 | B8782 | U97067 | CTNNAL1 | catenin (cadherin-associated protein), alpha-like 1 |
| 542 | B9769 | AA156269 | | Homo sapiens mRNA; cDNA DKFZp434E2321 (from clone DKFZp434E2321); partial cds |
| 543 | A1567 | U70824 | BLu | BLu protein |
| 544 | A2444 | AF002672 | LOH11CR2A | loss of heterozygosity, 11, chromosomal region 2, gene A |
| 545 | B9317 | N35421 | | ESTs |
| 546 | | A5086N AA402615 | SELPLG | selectin P ligand |
| 547 | C6059 | AA699359 | | ESTs |
| 548 | A0399 | M20496 | CTSL | cathepsin L |
| 549 | A0325 | X03663 | CSF1R | colony stimulating factor 1 receptor, formerly McDonough feline sarcoma viral (v-fms) oncogene homolog |
| 550 | A0131 | L34155 | LAMA3 | laminin, alpha 3 (nicein (150kD), kalinin (165kD), BM600 (150kD), epilegrin) |
| 551 | | A8879N AA583491 | HCA112 | hepatocellular carcinoma-associated antigen 112 |
| 552 | E0691 | AL021917 | BTN2A3 | butyrophilin, subfamily 2, member A3 |
| 553 | A1051 | M33195 | FCER1G | Fc fragment of IgE, high affinity I, receptor for; gamma polypeptide |
| 554 | A9090 | AI306435 | DKPZPr586A052 | DKFZP586A0522 protein |
| 555 | A1471N | AL021026 | | Homo sapiens DNA sequence from PAC 127D3 on chromosome 1q23-25. Contains FMO2 and FMO3 genes for Elavin-containing Monooxygenase 2 and Flavin-containing Monooxygenase 3 (Dimethylaniline Monooxygenase (N-Oxide 3, EC1.14.13.8, Dimethylaniline Oxidase 3, FMO |
| 556 | B2937 | AA416820 | H2AFZ | H2A histone family, member Z |
| 557 | A1125 | J04127 | CYP19 | cytochrome P450, subfamily XIX (aromatization of androgens) |
| 558 | A6380 | T28620 | FGB | fibrinogen, B beta polypeptide |
| 559 | A4970 | AF062075 | LPXN | leupaxin |
| 560 | C9579 | N42267 | | Homo sapiens cDNA: FLJ22554 fis, clone HSI01092 |
| 561 | C7036 | U59289 | CDH13 | cadherin 13, H-cadherin (heart) |
| 562 | A9308 | AA452780 | GENX-3414 | genethonin 1 |
| 563 | A2638 | U20158 | LCP2 | lymphocyte cytosolic protein 2 (SH2 domain-containing leukocyte protein of 76kD) |
| 564 | C9620 | AI092721 | HEMBAI007319 | Homo sapiens cDNA FLJI1896 fis, clone |
| 565 | A5868 | AA418061 | SLC11A3 | solute carrier family 11 (proton-coupled divalent metal ion transporters), member 3 |
| 566 | A5900 | AI091372 | AXUD1 | AXIN1 up-regulated |
| 567 | A1453 | M37721 | PAM | peptidylglycine alpha-amidating monooxygenase |
| 568 | A9514 | Z39135 | | Homo sapiens cDNA: FLJ22735 fis, clone HUV00180 |
| 569 | B9504 | AA521163 | | Homo sapiens cDNA: FLJ21333 fis, clone COL02535 |
| 570 | B8028 | AA701478 | | Homo sapiens cDNA: FLJ23332 fis, clone HEP12754 |
| 571 | D0786 | AB011115 | KIAA0543 | KIAA0543 protein |
| 572 | B7289N | AA379112 | SBBI42 | BCM-like membrane protein precursor |
| 573 | A4367 | AF020043 | CSPG6 | chondroitin sulfate proteoglycan 6 (bamacan) |
| 574 | A3150 | M97370 | ADORA2A | adenosine A2a receptor |
| 575 | A5253 | AA261780 | | ESTs |
| 576 | B4938 | W56507 | KIAA0251 | KIAA0251 protein |
| 577 | A9295 | AI266286 | | ESTs, Weakly similar to IRX2_HUMAN IROQUOIS-CLASS HOMEODOMAIN PROTEIN IRX-2 [H.sapiens] |
| 578 | A6532 | AA449335 | | ESTs |
| 579 | A4597 | U97519 | PODXL | podocalyxin-like |
| 580 | B4053 | K03191 | CYP1A1 | cytochrome P450, subfamily I (aromatic compound-inducible), polypeptide 1 |
| 581 | B5138 | AI364974 | FCN3 | ficolin (collagen/fibrinogen domain-containing) 3 (Hakata antigen) |
| 582 | A6427 | AA402425 | | Homo sapiens cDNA: FLJ22343 fis, clone HRC06043 |
| 583 | A0970 | U44403 | SLA | Src-like-adapter |
| 584 | A4680 | U40282 | ILK | integrin-linked kinase |
| 585 | A5015 | U13219 | FOXF1 | forkhead box F1 |
| 586 | A4769 | AF004562 | STXBP1 | syntaxin binding protein 1 |
| 587 | A0056 | AF061836 | RASSF1 | Ras association (RalGDS/AF-6) domain family 1 |
| 588 | A7051 | AA429070 | ISLR | immunoglobulin superfamily containing leucine-rich repeat |
| 589 | A7795 | AA508749 | UBL3 | ubiquitia-like 3 |
| 590 | A8561 | AA699666 | | Melanophilin |
| 591 | A7764 | | | |
| 592 | B9056 | AI025137 | ARHGEF3 | Rho guanine nucleotide exchange factor (GEF) 3 |
| 593 | B4277 | AA147512 | STX7 | syntaxin 7 |
| 594 | B6265 | AA902739 | | ESTs |
| 595 | A0925N | Z69028 | PPP2R5B | protein phosphatase 2, regulatory subunit B (B56), beta isoform |
| 596 | B3833 | AI337078 | | MacGAP protein |
| 597 | B5623 | AA505359 | MYOlE | myosin IE |
| 598 | B7105 | AA707941 | | ESTs |
| 599 | B5917N | W23481 | FLJ20271 | hypothetical protein FLJ20271 |
| 600 | B5291 | AA481924 | TYROBP | TYRO protein tyrosine kinase binding protein |
| 601 | C0211 | AA306716 | FLJ11937 | hypothetical protein FLJ11937 |
| 602 | A4115 | AA290738 | GSTM4 | glutathione S-transferase M4 |
| 603 | A9993 | AB007903 | KIAA0443 | KIAA0443 gene product |
| 604 | D7150 | AA909959 | NESH | NESH protein |
| 605 | B4090 | M34175 | AP3B1 | adaptor-related protein complex 2, beta 1 subunit |
| 606 | B4352N | T46905 | | Homo sapiens clone 23649 and 23755 unknown mRNA, partial cds |
| 607 | A3390 | L35240 | ENIGMA | enigma (LIM domain protein) |
| 608 | B4076 | AA293636 | GJA1 | gap junction protein, alpha 1, 43kD (connexin 43) |
| 609 | B1535 | AI161137 | | Homo sapiens cDNA: FLJ22743 fis, clone HUV00901 |
| 610 | B8678 | AA759306 | KIAA1249 | KIAA1249 protein |
| 611 | A1445 | M27492 | IL1R1 | interleukin 1 receptor, type I |
| 612 | A6886 | W76482 | | ESTs |
| 613 | E0242 | AI093526 | | EST, Weakly similar to Fc gamma receptor I [H.sapiens] |
| 614 | A1710 | X06985 | HMOX1 | heme oxygenase (decycling) 1 |
| 615 | B4278 | AI198543 | | ESTs, Highly similar to KIAA1395 protein [H.sapiens] |
| 616 | B4497 | W88815 | LOC57406 | lipase protein |
| 617 | B7559 | N98940 | | ESTs |
| 618 | B5481 | AI274152 | LOC51762 | RAB-8b protein |
| 619 | E0721 | Z95331 | MLLT2 | myeloid/lymphoid or mixed-lineage leukemia (trithorax (Drosophila) homolog); translocated to. 2 |
| 620 | A7301N | W37605 | | ESTs |
| 621 | C9246 | AI348094 | KIAA0882 | KIAA0882 protein |
| 622 | B9394 | H59903 | DJI057B20.2 | hypothetical protein dJ1057B20.2 |
| 623 | C6040 | H05226 | | EST |
| 624 | E0880 | H12644 | NFRKB | nuclear factor related to kappa B binding protein |
| 625 | A2467 | AF035752 | CAV2 | caveolin 2 |
| 626 | A6234 | M12963 | ADH1 | alcohol dehydrogenase 1 (class I), alpha polypeptide |
| 627 | A2531 | V00493 | HBA2 | hemoglobin, alpha 2 |
| 628 | C4765 | N67091 | | ESTs |
| 629 | A5084 | M86511 | CD14 | CD14 antigen |
| 630 | A4545 | M22299 | PLS3 | plastin 3 (T isoform) |
| 631 | A2534 | M21119 | LYZ | lysozyme (renal amyloidosis) |
| 632 | B4633 | AA634261 | CLIC4 | chloride intracellular channel 4 |
| 633 | B8081 | AA528190 | | ESTs |
| 634 | C0533 | AA760720 | SPAG6 | sperm associated antigen 6 |
| 635 | A0323 | X03438 | CSF3 | colony stimulating factor 3 (granulocyte) |
| 636 | A8596 | AA632025 | | ESTs |
| 637 | A9093 | N80081 | | ESTs |
| 638 | B4285 | AA812063 | | Homo sapiens cDNA FLJ13698 fis, clone PLACE2000176 |
| 639 | A2363 | U03274 | BTD | biotinidase |
| 640 | A2518 | M62402 | IGFBP6 | insulin-like growth factor binding protein 6 |
| 641 | B0327 | A1038322 | | ESTs, Moderately similar to KIAA1058 protein [H.sapiens] |
| 642 | B4245 | AF052101 | | Homo sapiens clone 23872 mRNA sequence |
| 643 | B9433 | AA031379 | | ESTs |
| 644 | B9992 | AA191449 | KIAA1254 | KIAA1254 protein |
| 645 | C8175 | X00129 | RBP4 | retinol-binding protein 4, interstitial |
| 646 | A0890 | L11329 | DUSP2 | dual specificity phosphatase 2 |
| 647 | B4213 | X65460 | AIP5A1 | ATP synthase, H+ transporting, mitochondrial F1 complex, alpha subunit, isoform 1. cardiac muscle |
| 648 | B6035N | AA424407 | ZFP106 | zinc finger protein 106 |
| 649 | A6003 | AA678103 | FKBP5 | FK506-binding protein 5 |
| 650 | A7454 | AF007162 | CRYAB | crystallin, alpha B |
| 651 | B9457 | AA340728 | NR2F2 | nuclear receptor subfamily 2, group F, member 2 |
| 652 | B6552 | AA678830 | KIAA1035 | KIAA1035 protein |
| 653 | A2087N | X16940 | ACTG2 | actin, gamma 2, smooth muscle, enteric |
| 654 | A5785 | AA776284 | PSMB7 | Proteasome (prosome, macropain) subunit, beta type, 7 |
| 655 | A1891 | L13720 | GAS6 | growth arrest-specific 6 |
| 656 | A1183 | U28833 | DSCR1 | Down syndrome critical region gene 1 |
| 657 | A0905 | M64722 | CLU | clusterin (complement lysis inhibitor, SP-40,40, sulfated glycoprotein 2, testosterone-repressed prostate message 2, apolipoprotein J) |
| 658 | A2516 | M77016 | TMOD | tropomodulin |
| 659 | A6626 | AA197086 | | ESTs |
| 660 | A9357 | AA682274 | FLJ20093 | hypothetical protein FLJ20093 |
| 661 | B4077 | M81635 | EPB72 | erythrocyte membrane protein band 7.2 (stomatin) |
| 662 | A9451 | AF055066 | HLA-A | major histocompatibility complex, class I, A |
| 663 | A8883 | N24759 | LOC51170 | retinal short-chain deliydrogenase/reductase retSDR2 |
| 664 | A8209 | AA293061 | | Homo sapiens cDNA: FLJ21559 fis, clone COL06406 |
| 665 | A9564 | AI149131 | CDKN1C | cyclin-dependent kinase inhibitor 1C (p57, Kip2) |
| 666 | A1490N | AI097058 | | Homo sapiens cDNA: FLJ23538 fis, clone LNG08010, highly similar to BETA2 Human MEN1 region clone epsilon/beta mRNA |
| 667 | B9739 | X94770 | EMP2 | epithelial membrane protein 2 |
| 668 | A8504 | AI367368 | FACL5 long-chain | fatty acid coenzyme A ligase 5 |
| 669 | B3883 | AA121351 | RAI2 | retinoic acid induced 2 |
| 670 | B4335 | D59837 | KIAA1565 | KIAA1565 protein |
| 671 | A4360N | U83461 | SLC31A2 | solute 2 carrier family 31 (copper transporters), member |
| 672 | B9836 | R79561 | KIAA1376 | KIAA1376 protein |
| 673 | B5151 | AI189343 | | Homo sapiens CDNA FLJ13511 fis, clone PLACE1005331, highly similar to Homo sapiens 7h3 |
| | | | | protein mRNA |
| 674 | A6320N | AF070616 | HPCAL1 | hippocalcin-like 1 |
| 675 | B6752 | AA156797 | | Homo sapiens mRNA; cDNA DKFZp434E109 (from clone DKFZp434E109) |
| 676 | C8698 | AA903358 | CGGBP1 | CGG triplet repeat binding protein 1 |
| 677 | E0789 | AI969467 | | ESTs |
| 678 | A7498 | AA115280 | LOC55901 | TMTSP for transmembrane molecule with thrombospondin module |
| 679 | E0783 | AI146697 | MAPK7 | mitogen-activated protein kinase 7 |
| 680 | A6602 | W87690 | | Homo sapiens cDNA: FLJ23173 fis, clone LNG10019 |
| 681 | A7605 | R15801 | LOC51299 | neuritin |
| 682 | A1437N | W37188 | H2AFL | H2A histone family, member L |
| 683 | B9007 | AI203211 | | ESTs |
| 684 | D9915 | AI079175 | | Homo sapiens mRNA, cDNA DKFZp564F053 (from clone DKFZp564F053) |
| 685 | B2663 | AA953615 | ACTC | actin, alpha, cardiac muscle |
| 686 | B7193N | AI261663 | | ESTs |
| 687 | A1622N | X75918 | NR4A2 | nuclear receptor subfamily 4, group A, member 2 |
| 688 | C6826 | L02326 | | Homo sapiens clone Hu lambda7 lambda-like protein (IGLL2) gene, partial cds |
| 689 | A3739 | X14420 | COL3A1 | collagen, type III, alpha 1 (Ehlers-Danlos syndrome rype IV, autosomal dominant) |
| 690 | A3297 | X01410 | TRB@ | T cell receptor beta locus |
| 691 | A7293 | N48811 | KIAA0786 | latrophilin |
| 692 | B8295 | AI359344 | PCAF | P300/CBP-associated factor |
| 693 | A2429 | M61715 | WARS | tryptophanyl-tRNA synthetase |
| 694 | A9007 | AA037452 | KIAA0992 | palladin |
| 695 | B0176N | W56480 | SOS1 | son of sevenless (Drosophila) homolog 1 |
| 696 | C7506 | AI025678 | | Homo sapiens clone 25228 mRNA sequence |
| 697 | E0498 | AK025773 | | Homo sapiens cDNA: FLJ22120 fis, clone HEP18874 |
| 698 | A7291 | AA594600 | CTL2 | CTL2 gene |
| 699 | C8442 | AA910738 | KIAA0579 | KIAA0579 protein |
| 700 | A44 72 | AF042081 | SH3BGRL | SH3 domain binding glutamic acid-rich protein like |
| 701 | A1669 | M95787 | TAGLN | transgelin |
| 702 | A8155 | T34177 | LOC51255 | hypothetical protein |
| 703 | E0176 | AI090671 | | Homo sapiens cDNA FLJ12057 fis, clone HEMBB1002068 |
| 704 | A2452 | M33146 | CSRP1 | cysteine and glycine-rich protein 1 |
| 705 | A5016 | U13220 | FOXF2 | forkhead box F2 |
| 706 | A8843 | AA235920 | | ESTs |
| 707 | B4092 | AB011126 | KIAA0554 | KIAA0554 protein |
| 708 | A8493 | AA780301 | CTSF | cathepsin F |
| 709 | A9051 | AB007934 | ACF7 | actin binding protein; macrophin (microfilament and actin filament cross-linker protein) |
| 710 | B9813 | AI221110 | FLJ10980 | hypothetical protein FLJ10980 |
| 711 | B7487 | AA036947 | | Homo sapiens cDNA FLJ10229 fis, clone HEMBB1000136 |
| 712 | B9712 | AI002977 | | ESTs |
| 713 | B5430 | AA290920 | | ESTs |
| 714 | A2632N | D14665 | ADAM9 | a disintegrin and metalloproteinase domain 9 (meltrin gamma) |
| 715 | A0225N | M93426 | PTPRZ1 | protein tyrosine phosphatase, receptor-type, polypeptide 1 |
| 716 | A0704N | AA156840 | MAP3K8 | mitogen-activated protein kinase kinase kinase 8 |
| 717 | A8969 | AA039563 | KIAA1415 | KIAA1415 protein |
| 718 | B7478 | AA443202 | KIAA1053 | KIAA1053 protein |
| 719 | A3554 | K01160 | | ESTs |
| 720 | B9536 | AI333662 | | ESTs |
| 721 | C9685 | AI275584 | | Likely ortholog of rat proline rich synapse associated protein 2 |
| 722 | C0335 | X13839 | ACTA2 | actin, alpha 2, smooth muscle, aorta |
| 723 | C4163 | AA912674 | VE-JAM | vascular endothelial junction-associated molecule |
| 724 | D7516 | AI074524 | | ESTs |
| 725 | E1492 | R27799 | BMP6 | bone morphogenetic protein 6 |
| 726 | A7782 | N44246 | PRKCH | protein kinase C, eta |
| 727 | A5154 | M55543 | GBP2 | guanylate binding protein 2, interferon-inducible |
| 728 | C6278 | AA641454 | SART-2 | squamous cell carcinoma antigen recognized by T cell |
| 729 | A1693 | X94991 | ZYX | zyxin |
| 730 | A0808 | M58285 | HEM1 | hematopoietic protein 1 |
| 731 | A1704N | D21254 | CDH11 | cadherin 11, type 2, OB-cadherin (osteoblast) |
| 732 | B4614 | A1093734 | TAZ | Transcriptional co-activator with PDZ-binding motif (TAZ) |
| 733 | B5081N | AA419490 | | Homo sapiens mRNA full length insert cDNA clone EUROIMAGE 248114 |
| 734 | A0568 | X60957 | TIE | tyrosine kinase with immunoglobulin and epidermal growth factor homology domains |
| 735 | A8796 | AA479330 | SLC7A7 | solute carrier family 7 (cationic amino acid transporter, y+ system), member 7 |
| 736 | B9265 | AI346969 | TRIM14 | Tripartite motif-containing 14 |
| 737 | A9013N | D62275 | ITM2B | integral membrane protein 2B |
| 738 | B5202N | T78873 | | Homo sapiens cDNA: FLJ22290 fis, clone HRC04405 |
| 739 | C8299 | AA600814 | PTPN9 | protein tyrosine phosphatase, non-receptor type 9 |
| 740 | A2926 | X96719 | CLECSF2 | C-type (calcium dependent, carbohydrate-recognition domain) lectin, superfamily member 2 (activation-induced) |
| 741 | A4681 | U02020 | PBEF | pre-B-cell colony-enhancing factor |
| 742 | A1217 | X14454 | IRF1 | interferon regulatory factor 1 |
| 743 | C4981 | X05908 | ANXA1 | annexin A1 |
| 744 | B0293 | AA037349 | LAMR1 | laminin receptor I (67kD, nbosomal protein SA) |
| 745 | A8128 | M78933 | MY047 | MY047 protein |
| 746 | C8090 | AF052685 | PCDHGC3 | protocadherin gamma subfamily C, 3 |
| 747 | C4610 | N66498 | | ESTs |
| 748 | B7221N | AA706790 | | ESTs |
| 749 | A6623 | R64431 | RYBP | RING1 and YY1 binding protein |
| 750 | A8823 | N26005 | PPP1R5 | protein phosphatase 1, regulatory (inhibitor) subunit 5 |
| 751 | B3694 | AA745720 | | ESTs |
| 752 | A5459 | AA393478 | NFAT5 | Nuclear factor of activated T-cells 5, tonicity-responsive |
| 753 | A6360 | W69716 | | Homo sapiens mRNA; cDNA DKFZp761P06121 (from clone DKFZp761P06121) |
| 754 | A0944 | Z24725 | MIG2 | mitogen inducible 2 |
| 755 | A5484 | AA634825 | PINK1 | PTEN induced putative kinase 1 |
| 756 | A2503 | S60099 | APLP2 | amyloid beta (A4) precursor-like protein 2 |
| 757 | A0205 | M69066 | MSN | moesin |
| 758 | A5850 | AA282650 | SAC1 | Suppressor of actin 1 |
| 759 | A5423 | AA773731 | | Homo sapiens cDNA: FLJ21028 fis, clone CAE07155 |
| 760 | A2887 | M22865 | CYB5 | cytochrome b-5 |
| 761 | A6629 | AI366509 | HSMNP1 | uncharacterized hypothalamus protein HSMNP1 |
| 762 | A4543N | AB001636 | DDX15 | DEAD/H (Asp-Glu-Ala-Asp/His) box polypeptide 15 |
| 763 | B5480 | AA044842 | AHCP | Autosomal Highly Conserved Protein |
| 764 | B4084 | AA916826 | APP | amyloid beta (A4) precursor protein (protease nexin-II, Alzheimer disease) |
| 765 | A0224N | D13380 | PTPN12 | protein tyrosine phosphatase, non-receptor type 12 |
| 766 | B4552 | AA812671 | CDC14B | CDC14 (cell division cycle 14, S. cerevisiae) homolog |
| 767 | B3700 | AA443786 | SYTL2 | Synaptotagmin-like 2 |
| 768 | B4891 | W19216 | PKIG | protein kinase (cAMP-dependent, catalytic) inhibitor gamma |
| 769 | B5366N | AA291036 | KIAA0164 | KIAA0164 gene product |
| 770 | A8477N | W44716 | HSPC055 | HSPC055 protein |
| 771 | C8058 | N62855 | | ESTs |
| 772 | E1374 | AK000752 | KIAA1181 | KLAA1181 protein |
| 773 | A2287 | U09577 | HYAL2 | hyaluronoelucosaminidase 2 |
| 774 | A2118 | J04130 | SCYA4 | small inducible cytokine A4 (homologous to mouse Mipb) |
| 775 | A2511 | D49547 | HSPF1 | heat shock 40kD protein 1 |
| 776 | A6236 | L04656 | CA8 | carbonic anhydrase VIII |
| 777 | A1795 | J03004 | GNAI2 | guanine nucleotide binding protein (G protein), alpha inhibiting activity polypeptide 2 |
| 778 | A4171 | AA772230 | | Homo sapiens cDNA: FLJ23538 fis, clone LNG08010, highly similar to BETA2 Human MEN1 region clone epsilon/beta mRNA |
| 779 | A4766 | AF001434 | EHD1 | EH domain containing 1 |
| 780 | A1999 | D00172 | ANXA5 | annexin A5 |
| 781 | A6187 | AA412555 | KIAA1536 | KIAA1536 protein |
| 782 | A1452 | M35198 | ITGB6 | integrin, beta 6 |
| 783 | A3288 | M12670 | | TIMP1 tissue inhibitor of metalloproteinase 1 (erythroid potentiating activity, collagenase inhibitor) |
| 784 | A4279 | AI222322 | TOB2 | transducer of ERBB2, 2 |
| 795 | A8063 | H98203 | KIAA0987 | differentially expressed in adenocarcinoma of the lung |
| 786 | B0911 | W72053 | | Homo sapiens cDNA: FLJ21904 fis, clone HEP03585 |
| 787 | B4831 | M31210 | EDG1 | endothelial differentiation, sphingolipid G-protein-coupled receptor, 1 |
| 788 | B3748 | D88153 | HYA22 | HYA22 protein |
| 789 | A6715 | U83463 | SDCBP | syndecan binding protein (syntenin) |
| 790 | A9327 | AA447864 | KIAA1055 | KLAA1055 protein |
| 791 | B1490 | AI199405 | ZNF266 | zinc finger protein 266 |
| 792 | A9412 | AA523727 | | ESTs |
| 793 | A6411 | AA303231 | LOC64744 | hypothetical protein AL133206 |
| 794 | B1647 | AA242740 | SCEL | sciellin |
| 795 | A1992 | Z11697 | CD83 | CD83 antigen (activated B lymphocytes, immunoglobulin superfamily) |
| 796 | A8921 | R38569 | | ESTs |
| 797 | B6472 | AI288772 | DREV1 | CGI-81 protein |
| 798 | B7213N | D86982 | KIAA0229 | KIAA0229 protein |
| 799 | B7575 | W42910 | SEC22C | vesicle trafficking protein |
| 800 | B9287 | AA885480 | | Human DNA sequence from clone RP5-858B6 on chromosome 1q42.13-43 Contains ESTs, STSs, GSSs and a CpG island. Contains three novel genes |
| 801 | B3891 | C06051 | JAK1 | Janus kinase 1 (a protein tyrosine kinase) |
| 802 | B4491 | AA148566 | | Homo sapiens cDNA: FLJ22790 fis, clone KAIA2176, highly similar to HUMPMCA Human plasma membrane calcium-pumping ATPase (PMCA4) mRNA |
| 803 | C8127 | AA478197 | MAN2A2 | mannosidase, alpha, class 2A, member 2 |
| 804 | C8456 | U90912 | KIAA1128 | Human clone 23865 mRNA sequence |
| 805 | C0570 | H12297 | | Homo sapiens cDNA: FLJ22167 fis, clone HRC00584 |
| 806 | D1436 | A1341492 | RNB6 | RNB6 |

**Table2 up-regulated genes ( ≧x5, ≧50% of cases)**

| NSC Assign ment | LMMID | Acc | Symbol | TITLE |
|---|---|---|---|---|
| 807 | A1589 | NM_006547 | KOC1 | IGF-II mRNA-binding protein 3 |
| 808 | A0042 | AF029082 | SFN | stratifin |
| 809 | A1063 | M19888 | SPRRIB | small proline-rich protein 1B (cornifin) |
| 810 | A3243 | NM_003318 | TTK | TTK protein kinase |
| 811 | A0418 | NM_002997 | SDC1 | syndecan 1 |
| 812 | A2932 | M21551 | NMB | neuromedin B |
| 813 | A3547 | J04739 | BPI | bactericidal/permeability-increasing protein |
| 814 | A2282 | D79997 | KIAA0175 | KIAA0175 gene product |
| 815 | A4383 | Z97029 | RNASEHI | ribonuclease HI, large subunit |
| 816 | A1257 | AF006259 | PIR51 | RAD51-interacting protein |
| 817 | B4368 | AI082560 | FLJ20450 | hypothetical protein FLJ20450 |
| 818 | B7725 | C20910 | CCNB1 | cyclin B1 |
| 819 | A8043 | W72411 | TP63 | tumor protein 63 kDa with strong homology to p53 |
| 820 | B6769 | AA461217 | HMMR | hyaluronan-mediated motility receptor (RHAMM) |
| 821 | A6695 | AE035444 | TSSC3 | tumor suppressing subtransferable candidate 3 |
| 822 | C4330 | AA234722 | | ESTs (MGC12536), Moderately similar to CANS_HUMAN CALCIUM-DEPENDENT PROTEASE, SMALL SUBUNIT [H.sapiens] |
| 823 | B1406 | AA483082 | XAGE | XAGE protein |
| 824 | A0771N | M69325 | BPAG1 | bullous pemphigoid antigen 1 (230/240kD) |
| 825 | B8870 | NM_018685 - | ANLN | anillin (Drosophila Scraps homolog), actin binding protein |
| 826 | B9760 | R73030 | | ESTs, Weakly similar to ALU1_HUMAN ALU SUBFAMILY J SEQUENCE CONTAMINATION WARNING ENTRY [H.sapiens] |
| 827 | B5915 | T79582 | | ESTs, Weakly similar to KIAA0479 protein [H.sapiens] |
| 828 | A8893N | AA460182 | PPP1R16A | Protein phosphatase 1, regulatory (inhibitor) subunit 16A |
| 829 | B5301 | W44796 | | ESTs |
| 830 | B7439 | AI318098 | | ESTs |
| 831 | B8547 | AI125938 | | Homo sapiens X28 region near ALD locus containing dual specificity phosphatase 9 (DUSP9), ribosomal protein L18a (RPL18a), Ca2+/Calmodulin-dependent protein kinase I (CAMKI), creatine transporter (CRTR), CDM protein (CDM), adrenoleukodystrophy protein |
| 832 | B8743 | AI261284 | | ESTs |
| 833 | B7163 | R06794 | | ESTs |
| 834 | B8909 | AA552690 | | Homo sapiens cDNA: FLJ21274 fis, clone COL01781 |
| 835 | B4688 | AA411315 | FLJ10604 | hypothetical protein FLJ10604 |
| 836 | B4788N | AA776829 | | ESTs |
| 837 | B6264 | T91195 | | ESTs |
| 838 | B4186N | AI189587 | | ESTs |
| 839 | B7771 | AA427818 | HMGIC | high-mobility group (nonhistone chromosomal) protein isoform I-C |
| 840 | C1730 | AA847662 | GNAS | GNAS,complex locus |
| 841 | C4539 | AB101204 | URLC2 | up-regulated in lung cancer 2 |
| 842 | C8214 | AA047315 | KIAA0887 | KIAA0887 protein |
| 843 | C6987 | AI123912 | | Homo sapiens cDNA FLJ10041 fis, clone HEMBA1001022 |
| 844 | C7403 | AI359511 | | ESTs, Moderately similar to similar to smoothelin [H.sapiens] |
| 845 | D0773 | AA425730 | | ESTs, Weakly similar to ALU1_HUMAN ALU SUBFAMILY J SEQUENCE CONTAMINATION WARNING ENTRY [H.sapiens] |
| 846 | D0006 | AI015982 | | ESTs, Weakly similar to AF155135 1 novel retinal pigment epithelial cell protein [H.sapiens] |
| 847 | C0488 | AA781195 | PRAME | preferentially expressed antigen in melanoma |
| 848 | C6902 | AA479648 | | ESTs |
| 849 | C7601 | NM005268 | GJB5 | gap junction protein, beta 5 (connexin 31.1) |
| 850 | D1135 | AA447744 | | ESTs |
| 851 | C5005 | AA625553 | | ESTs |
| 852 | C6143 | AA678356 | | ESTs |
| 853 | C6664 | AI142832 | | ESTs |
| 854 | C1442 | AA807192 | | ESTs, Highly similar to unnamed protein product [H.sapiens] |
| 855 | C6719 | AB105191 | LAIR | Ig superfamily receptor LNIR |
| 856 | D0010 | AA358397 | | EST |
| 857 | C7630 | NM_032862 | TIGD5 | tigger transposable element derived5 |
| 858 | C6447 | AA079262 | | Homo sapiens mRNA; CDNA DKFZp566C0546 (from clone DKFZp566C0546) |
| 859 | C7444 | AB101205 | URLC3 | up-regulated in lung cancer 3 |
| 860 | D9683 | AI057353 | | ESTs |
| 861 | D9437 | W67209 | KIAA0251 | ESTs, Moderately similar to p53 regulated PA26-T2 nuclear protein [H.sapiens] |
| 862 | D3230 | AA780074 | | ESTs |
| 863 | E0904 | AI394016 | FLT20116 | hypothetical protein FLJ20116 |
| 864 | D5363 | AA954567 | | ESTs |
| 865 | A2691N | X63629 | CDH3 | cadherin 3, type 1, P-cadherin (placental) |
| 866 | A4693 | U42408 | LAD1 | ladinin 1 |
| 867 | C3760 | U75285 | BIRC5 | baculoviral IAP repeat-containing 5 (survivin) |
| 868 | | A2603N Z46629 | SOX9 | SRY (sex determining region Y)-box 9 (campomelic dysplasia, autosomal sex-reversal) |
| 869 | A3529N | D89016 | NBR | putative neuroblastoma protein |
| 870 | C8633 | AI161159 | | Homo sapiens mRNA; cDNA DKFZp566N034 (from clone DKFZp566N034); partial cds |
| 871 | D5753 | AA971042 | KIAA1929 | ESTs |
| 872 | A9044 | AA775667 | LOC51659 | HSPC037 protein |
| 873 | C8799 | AA219172 | | ESTs |
| 874 | B7197N | R07614 | | ESTs |
| 875 | B4414 | AA765913 | DEGR2 | 2,4-dienoyl CoA reductase 2, peroxisomal |
| 876 | C9030 | AI086906 | | ESTs, Highly similar to LRP1_HUMAN LOW-DENSITY LIPOPROTEIN RECEPTOR-RELATED PROTEIN 1 PRECURSOR [H.sapiens]/Highly similar to S02392 alpha-2-macroglobulin receptor precursor [H.sapiens] |
| 877 | A2709N | D85376 | | ESTs |
| 878 | D9621 | AI349804 | | ESTs, Weakly similar to IQGA_HUMAN RAS GTPASE-ACTIVATING-LIKE PROTEIN IQGAP1 [H.sapiens] |
| 879 | C7956 | AA172001 | FLJ10901 | hypothetical protein FLJ10901 |
| 880 | A2759N | X16260 | ITIH1 | inter-alpha (globulin) inhibitor, H1 polypeptide |
| 881 | C3813 | NM_017650 | FLJ20068 | hypothetical protein FLJ20068 |
| 882 | D4920 | AI247180 | GUCY1B2 | guanylate cyclase 1, soluble, beta 2 |
| 883 | A7675 | U26726 | HSD11B2 | hydroxysteroid (11-beta) dehydrogenase 2 |
| 884 | B8059 | AA625338 | RAD51 | RAD51 (S. cerevisiae) homolog (E coli RecA homolog) |
| 885 | C7616 | AB101211 | BAG5 | BCL2-associated athanogene 5 |
| 886 | D2176 | AI138545 | | ESTs |
| 887 | A2801 | X69314 | GPX2 | glutathione peroxidase 2 (gastrointestinal) |
| 888 | A3937 | AF044309 | STX11 | syntaxin 11 |
| 889 | A5657 | AA005074 | HSPC150 | HSPC150 protein similar to ubiquitin-conjugating enzyme |
| 890 | B4064 | X83573 | ARSE | arylsulfatase E (chondrodysplasia punctata 1) |
| 891 | C6559 | AA011131 | | ESTs |
| 892 | E0975 | AI816535 | | Homo sapiens cDNA FLJ12827 fis, clone NT2RP2002939, weakly similar to ZINC FINGER PROTEIN 136 |
| 893 | A3059 | NM_016195 | MPHOSPH1 | M-phase phosphoprotein 1 |
| 894 | A0480 | X54941 | CKS1 | CDC28 protein kinase 1 |
| 895 | C6675 | AB105189 | FAM3D | family with sequence similarity 3, member D |
| 896 | C7537 | AA121546 | PRO0971 | hypothetical protein PRO0971 |
| 897 | C4166 | M64247 | TNNI3 | troponin I, cardiac |
| 898 | C9393 | AB101209 | URLC7 | up-regulated in lung cancer 7 |
| 899 | D0182 | AA639491 | KRTHB6 | keratin, hair, basic, 6 (monilethrix) |
| 900 | B5912 | W04554 | FLJ20615 | Hypoxia-inducible factor 1, alpha subunit inhibitor |
| 901 | C3839 | AW166519 | MANIB1 | mannosidase, alpha, class 1B, member 1 |
| 902 | A2462 | AF054987 | ALDOC | aldolase C, fructose-bisphosphate |
| 903 | A8287 | AB105186 | URLC9 | up-regulated in lung cancer 9 |
| 904 | A8335 | AA448270 | | Homo sapiens, clone IMAGE:3690478, mRNA, partial cds |
| 905 | B6905 | AB101203 | URLC1 | up-regulated in lung cancer 1 |
| 906 | A2840 | M68867 | CRABP2 | cellular retinoic acid-binding protein 2 |
| 907 | C9468 | AA885242 | | Homo sapiens clone CDABP0014 mRNA sequence |
| 908 | B4239 | N51411 | PSA | phosphoserine aminotransferase |
| 909 | B3876 | NM_018101 | FLJ10468 | hypothetical protein FLJ10468 |
| 910 | C0709 | AA047768 | | ESTs |
| 911 | D3319 | AA768607 | | ESTs |
| 912 | A7432 | M32313 | SRD5A1 | steroid-5-alpha-reductase, alpha polypeptide 1 (3-oxo-5 alpha-steroid delta 4-dehydrogenase alpha 1) |
| 913 | A3962 | AF057034 | RODH-4 | microsomal NAD+-dependent retinol dehydrogenase 4 |
| 914 | A7908 | AA490691 | HOXD11 | homeo box D11 |
| 915 | C7457 | AB105187 | URLC10 | up-regulated in lung cancer 10 |
| 916 | B9429 | Z39229 | | EST |
| 917 | D7212 | AA132702 | KLAA1096 | KIAA1096 protein |
| 918 | A2254 | U63743 | KNSL6 | kinesin-like 6 (mitotic centromere-associated kinesin) |
| 919 | B4345 | AA576959 | | ESTs |
| 920 | A1957 | NM_005483 | CHAF1A | chromatin assembly factor 1, subunit A (p150) |
| 921 | A4076 | AF044961 | AKRIB11 | aldo-keto reductase family 1, member B11 (aldose reductase-like) |
| 922 | A9518N | AA570186 | | ESTs, ESTs, Weakly similar to MUC2_HUMAN MUCIN 2 PRECURSOR |
| 923 | B4593 | AA946930 | SNRPG | small nuclear ribonucleoprotein polypeptide G |
| 924 | C4878 | AA446064 | | ESTs |
| 925 | B1898 | AA496118 | | EST |
| 926 | C6506 | AA004412 | | ESTs |
| 927 | D6607 | A1000650 | | ESTs |
| 928 | E1138 | N80859 | ERBB2 | v-erb-b2 avian erythroblastic leukemia viral oncogene homolog 2 (neuro/glioblastoma derived oncogene homolog) |
| 929 | B8882 | D14657 | KIAA0101 | KIAA0101 gene product |
| 930 | A5223 | AA453716 | | ESTs |
| 931 | A0437 | AF047002 | ALY | transcriptional coactivator |
| 932 | B0303 | AA731891 | KIAA1517 | KIAA1517 protein |
| 933 | B2824 | AA115381 | | Homo sapiens cDNA FLT12640 fis, clone NT2RM4001940, highly similar to Homo sapiens timeless homolog mRNA |
| 934 | B6283 | AA677294 | CTT | citron (rho-interacting, serine/threonine kinase 21) |
| 935 | C0716 | AI097310 | | ESTs |
| 936 | C8776 | AA766028 | AF15Q14 | AF15q14 protein |
| 937 | C0671 | AI091125 | FZD10 | frizzled (Drosophila) homolog 10 |
| 938 | C6173 | W72182 | FLJ13852 | hypothetical protein FLJ13852 |
| 939 | D8061 | AA555306 | | ESTs |
| 940 | D5016 | AI191724 | KIAA1443 | KIAA1443 protein |
| 941 | C7353 | AA587766 | FLJ21935 | hypothetical protein FLJ21935 |
| 942 | A4139 | AA566069 | ARPC4 | actin related protein 2/3 complex, subunit 4 (20 kD) |
| 943 | B7138 | AA429262 | | ESTs |
| 944 | C6222 | W74371 | | ESTs |
| 945 | C0912 | AJ001014 | RAMP1 | receptor (calcitonin) activity modifying protein 1 |
| 946 | D9981 | N30381 | | ESTs |
| 947 | B0259 | NM_007183 | PKP3 | plakophilin 3 |
| 948 | D5142 | NM_003740 | KCNK5 | potassium channel, subfamily K, member 5 (TASK-2) |
| 949 | A2391 | L38961 | ITM1 | integral membrane protein 1 |
| 950 | B0323 | AI363295 | | ESTs |
| 951 | B6053 | AA916007 | | ESTs |
| 952 | C4825 | AA287862 | | ESTs |
| 953 | C6562 | AA012883 | | ESTs |
| 954 | D0684 | AA420960 | | EST |
| 955 | C9886 | AI034428 | | ESTs |
| 956 | D6488 - | NM_078480 | SIAHBP1 | siah binding protein 1; FBP interacting repressor; pyrimidine tract binding splicing factor; Ro ribonucleoprotein-binding protein 1 |
| 957 | E0502 | AI240520 | | ESTs |
| 958 | C4060 | N35250 | | ESTs |
| 959 | C9858 | AA748883 | DNMT3B | DNA (cytosine-5-)-methyltransferase 3 beta |
| 960 | C0903 | X81420 | KRTHB1 | keratin, hair, basic, 1 |
| 961 | C6634 | AA398740 | | ESTs |
| 962 | D9500 | AI361654 | | ESTs |
| 963 | G5995 | W58277 | | Homo sapiens mRNA; cDNA DKFZp586P2321 (from clone DKFZp586P2321) |
| 964 | A3765 | X60673 | AK3 | adenylate kinase 3 |
| 965 | A2448 | AF010314 | ENC1 | ectodermal-neural cortex (with BTB-like domain) |
| 966 | C0573 | H12479 | | ESTs |
| 967 | D0587 | AA872040 | INHBB | inhibin, beta B (activin AB beta polypeptide) |
| 968 | C7590 | AB005989 | CYP27B1 | cytochrome P450, subfamily XXVIIB (25-hydroxyvitamin D-alpha-hydroxylase), polypeptide 1 |
| 969 | B7749 | AI346758 | GYG2 | glycogenin 2 |
| 970 | C6959 | AA054259 | | EST |
| 971 | A0447 | U14973 | RPS29 | ribosomal protein S29 |
| 972 | C0247 | AI352156 | LOC51690 | U6 snRNA-associated Sm-like protein LSm7 |
| 973 | C8682 | AA227919 | HAS3 | hyaluronan synthase 3 |
| 974 | A2352 | M77836 | PYCR1 | pyrroline-oline-5-carboxylate reductase 1 |
| 975 | B7680 | AI342628 | FLJ12517 | hypothetical protein FLJ12517 |
| 976 | A1640 | X98400 | MASP2 | mannan-binding lectin serine protease 2 |
| 977 | A6282 | AI076128 | ARHD | ras homolog gene family, member |
| 978 | A6884 | AA523543 | CRABP1 | cellular retinoic acid-binding protein 1 |
| 979 | B4361 | AA989104 | NDLTFB2 | NADH dehydrogenase (ubiquinone) 1 beta subcomplex, 2 (8kD, AGGG) |
| 980 | B3971 | AI298472 | ANKT | Nucleolar protein ANKT |
| 981 | C6522 | AI249019 | | ESTs |
| 982 | C2298 | AA357675 | HES6 | hypothetical protein HES6 |
| 983 | C7939 | X17620 | NME1 | non-metastatic cells 1, protein (NM23A) expressed in |
| 984 | D3205 | AA077280 | MLL3 | myeloid/lymphoid or mixed-lineage leukemia3 |
| 985 | A1618 | X70693 | SOX4 | SRY (sex determining region Y)-box 4 |
| 986 | B4397 | AA873067 | | Homo sapiens cDNA: FLJ22940 fis, clone KAT08051 |
| 987 | A2755 | AF006043 | PHGDH | phosphoglycerate dehydrogenase |
| 988 | A4873 | U10688 | MAGEA4 | melanoma antigen, family A, 4 |
| 989 | A3058 | L16783 | FOXM1 | forkhead box M1 |
| 990 | B4217 | AA079060 | WFDC2 | WAP four-disulfide core domain 2 |
| 991 | B6595N | XM_209944 | DOLPP1 | linked to Surfeit genes in Fugu rubripes 2; LSFR2 gene 2 |
| 992 | A7411 | M12849 | SERPIND1 | serine (or cysteine) proteinase inhibitor, clade D (heparin cofactor), member 1 |
| 993 | A2753N | U26662 | | NPTX2 neuronalpentraxin II |
| 994 | C7028 | NM_032138 | DKFZp43 4E2318 | hypothetical protein DKFZp434E2318 |
| 995 | B8207 | AA411341 | | Homo sapiens 3 beta-hydroxy-delta 5-C27-steroid oxidoreductase mRNA, complete cds |
| 996 | A7691 | X04325 | GJB1 | gap junction protein, beta 1, 32kD (connexin 32, Charcot-Marie-Tooth neuropathy, X-linked) |
| 997 | B5904 | AA806630 | FIJ10540 | hypothetical protein FLJ10640 |
| 998 | B8643 | AA781393 | | ESTs |
| 999 | A0812 | M16937 | HOXB7 | homeo box B7 |
| 1000 | A5513 | AB105376 | PSK-1 | type I transmembrane receptor (seizure-related protein) |
| 1001 | A0725 | U02082 | ARHGEF5 | Rho guanine nucleotide exchange factor (GEF) 5 |
| 1002 | A7887 | AF070588 | LOC55565 | hypothetical protein LOC55565 |
| 1003 | B5018 | T47612 | | ESTs |
| 1004 | A0061 | AF068760 | BUB1B | budding uninhibited by benzimidazoles 1 (yeast homolog), beta |
| 1005 | C6805 | AA040734 | | Homo sapiens, clone MGC:16466 IMAGE:3952569, mRNA, complete cds |
| 1006 | C0811 | W69611 | | ESTs |
| 1007 | C9517 | AA586922 | POLR2J | polymerase (RNA) II (DNA directed) polypeptide J (13.3kD) |
| 1008 | C1590 | AI249914 | | ESTs |
| 1009 | C6086 | AA235149 | | ESTs |
| 1010 | D9504 | AA928656 | NTS | neurotensin |
| 1011 | A0329 | X07819 | MMP7 | matrix metalloproteinase 7 (matrilysin, uterine) |
| 1012 | C8953 | AA293513 | FLJ12428 | hypothetical protein FLJ12428 |
| 1013 | C0802 | H63947 | | ESTs |
| 1014 | B0864 | AI343440 | | ESTs, Weakly similar to Ydr472wp [S.cerevisiae] |
| 1015 | C6852 | AI335883 | PHB | prohibitin |
| 1016 | C6225 | W74482 | | ESTs, Weakly similar to KIAA1362 protein [H.sapiens] |
| 1017 | D8147 | AI142227 | CS | citrate synthase |
| 1018 | D4112 | AA648521 | | ESTs, Highly similar to pre-mRNA splicing SR protein rA4 [H.sapiens] |
| 1019 | A0490 | L10612 | MIF | macrophage migration inhibitory factor (glycosylation-inhibiting factor) |
| 1020 | C1555 | AI243620 | | ESTs |
| 1021 | A5740 | AI304392 | KIAA1436 | Prostaglandin F2 receptor negative regulator |
| 1022 | A7245 | AI275857 | | ESTs |
| 1023 | B1516 | AA885961 | CLDN2 | Claudin 2 |
| 1024 | B0436N | AA625794 | MTX1 | metaxin 1 |
| 1025 | B3987N | N30179 | PLAB | prostate differentiation factor |
| 1026 | B4030 | AA066180 | | Human DNA sequence from clone RP4-616B8 on chromosome 20q11.222 Contains a gene for an RNA helicase, NPM1P19 (nucleophosmin 1 (nucleolar phosphoprotein B23, numatrin) pseudogene 19), part of an RNA for KIAA0823 protein, ESTs, STSs, GSSs and CpG Islands |
| 1027 | B4587 | AA504314 | | ESTs |
| 1028 | C3815 | BE261922 | | Homo sapiens cDNAFLJ14154 fis, clone NT2RM1000341 |
| 1029 | A3534 | J00269 | KRT6A | keratin 6A |
| 1030 | C0778 | AI310465 | | Human putative ribosomal protein L36 mRNA |
| 1031 | C8481 | AA604841 | | ESTs |
| 1032 | A0238 | U01828 | MAP2 | microtubule-associated protein 2 |
| 1033 | A6127 | A1356291 | ST5 | Suppression of tumorigenicity 5 |
| 1034 | A0494 | M94556 | SSBP | single-stranded DNA-binding protein |
| 1035 | A2123 | K03515 | GPI | glucose phosphate isomerase |
| 1036 | A2954 | L05096 | RPL39 | Homo sapiens ribosomal protein L39 mRNA, complete cds |
| 1037 | A2822 | X05978 | CSTA | cystatin A (stefm A) |
| 1038 | B0292 | AA375432 | CLDN1 | claudin 1 |
| 1039 | A7172 | Y10043 | HMG4 | high-mobility group (nonhistone chromosomal) protein 4 |
| 1040 | B3500 | AA725807 | AA725807 | ESTs,Homo sapiens cDNAFU33104 fis, clone TRACH2000923 |
| 1041 | A6005N | AA531437 | MLLT4 | myeloid/lymphoid or mixed-lineage leukemia (trithorax (Drosophila) homolog); translocated to, 4 |
| 1042 | D0491 | AA815427 | SLC6A8 | solute carrier family 6 (neurotransmitter transporter, creatine), member 8 |
| 1043 | C2112 | AI022193 | A1BG | alpha-B glycoprotein |
| 1044 | C0844 | AA954657 | | ESTs, Weakly similar to collectin 34 [H.sapiens] |
| 1045 | C6180 | AA775500 | HsPOX2 | proline oxidase 2 |
| 1046 | D1375 | AA287860 | E2F5 | E2F transcription factor 5, p130-binding |
| 1047 | D7587 | AI096953 | SLC7A5 | Solute carrier family 7 (cationic amino acid transporter, y+ system), member 5 |
| 1048 | A2673 | X16135 | HNRPL | heterogeneous nuclear ribonucleoprotein L |
| 1049 | A2565 | Y00278 | S100A8 | S100 calcium-binding protein A8 (calgranulin A) |
| 1050 | A2088 | D38583 | S100A11 | S100 calcium-binding protein A11 (calgizzarin) |
| 1051 | A5518 | AA058761 | FLJ20550 | hypothetical protein FLJ20550 |
| 1052 | B4870 | AA308062 | S100P | S100 calcium-binding protein P |
| 1053 | A8597N | AA649986 | SNRPF | small nuclear ribouncleoprotein polypeptide F |
| 1054 | C1463 | AA001735 | | ESTs |
| 1055 | C0651 | AI086281 | | ESTs |
| 1056 | D0952 | A1014551 | | ESTs |
| 1057 | A2255 | 303826 | FDXR | ferredoxin reductase |
| 1058 | A5292 | AC004770 | FEN1 | flap structure-specific endonuclease 1 |
| 1059 | B6346 | AA235074 | TCF19 | transcription factor 19 (SC1) |
| 1060 | C6722 | AA977296 | | ESTs, Weakly similar to unknown [S.cerevisiae] |
| 1061 | C4081 | Z40760 | | Homo sapiens PIG-M mRNA for mannosyltransferase, complete cds |
| 1062 | D4812 | AA923368 | PTK2 | PTK2 protein tyrosine kinase 2 |
| 1063 | D9731 | AI056637 | | ESTs |
| 1064 | A6139 | AI356558 | PAFAH1B3 | platelet-activating factor acetylhydrolase, isoform Ib, gamma subunit (29kD) |
| 1065 | A8270 | AA501416 | | ESTs |
| 1066 | B5461 | AJ439063 | MCM8 | minichromosome maintenance 8 |
| 1067 | D6549 | AA994711 | FLJ10052 | hypothetical protein FLJ10052 |
| 1068 | A6202 | AA524968 | | ESTs, Weakly similar to T2D3_HUMAN TRANSCRIPTION INITIATION FACTOR TFIID 135 KDA SUBUNIT [H.sapiens] |
| 1069 | B4121 | AA877534 | GPRC5C | G protein-coupled receptor, family C, group 5, member C |
| 1070 | B4478 | AA910946 | AP1M2 | adaptor-related protein complex 1, mu 2 subunit |
| 1071 | C7114 | T16226 | | ESTs |
| 1072 | C7965 | AA173172 | FLJ13163 | hypodietical protein FLJ13163 |
| 1073 | C7122 | AA235710 | NJMU-R1 | protein kinase Njmu-R1 |
| 1074 | D4789 | AA921896 | | ESTs |
| 1075 | D6248 | AB101206 | URLC4 | up-regulated in lung cancer 4 |
| 1076 | A7409 | L41559 | PCBD | 6-pyruvoyl-tetrahydropterin synthase/dimerization cofactor of hepatocyte nuclear factor 1 alpha (TCF1) |
| 1077 | B6379 | AA443685 | | Homo sapiens mRNA; cDNA DKFZp564H142 (from clout DKFZp564H142) |
| 1078 | A0429 | U73379 | UBCH10 | ubiquitin carrier protein E2-C |
| 1079 | C4909 | W79821 | | Homo sapiens HSPC265 mRNA, partial cds |
| 1080 | A4146 | AA586974 | PI3 | protease inhibitor 3, skin-derived (SKALP) |
| 1081 | A1215 | X07696 | KRT15 | keratin 15 |
| 1082 | A2978 | X04741 | UCHL1 | ubiquitin carboxyl-terminal esterase L1 (ubiquitin thiolesterase) |
| 1083 | A4013 | D26485 | UQCRC1 | ubiquinol-cntochrome c reductase core protein I |
| 1084 | A5377 | AA339976 | TSSC1 | tumor suppressing subtransferable candidate 1 |
| 1085 | B4069 | AA128470 | DSP | desmoplakin (DPI, DPII) |
| 1086 | A7780 | AB006630 | TCF20 | transcription factor 20 (AR1) |
| 1087 | B2909 | AA568223 | TOP2A | topoisomerase (DNA) II alpha (170kD) |
| 1088 | A9040 | K03195 | SLC2A1 | solute carrier family 2 (facilitated glucose transporter), member 1 |
| 1089 | B9480 | W56303 | KIAA0802 | KIAA0802 protein |
| 1090 | B4932 | AA909294 | LOC51243 | hypothetical protein |
| 1091 | B5787 | AA514606 | FLJ10633 | hypothetical protein FLJ10633 |
| 1092 | B5534N | AA758653 | | ESTs |
| 1093 | A2694N | D31628 | HPD | 4-hydroxyphenylpyruvate dioxygenase |
| 1094 | B5382N | Y09836 | KIAA0374 | syntaphilin |
| 1095 | C6679 | AI168147 | | Homo sapiens HSPC289 mRNA, partial cds |
| 1096 | D1477 | T82181 | | EST |
| 1097 | B2980 | AI339770 | | ESTs |
| 1098 | C8586 | AI014673 | FLJ10709 | hypothetical protein FLJ10709 |
| 1099 | C1388 | AI244237 | H2BFB | H2B histone family, member B |
| 1100 | A3156 | L02870 | COL7A1 | collagen, type VII, alpha 1 (epidermolysis bullosa, dystrophic, dominant and recessive) |
| 1101 | A1677 | U58766 | TSTA3 | tissue specific transplantation antigen P35B |
| 1102 | B1887 | AA642480 | SMG1 | PI-3-kinase-related kinase SMG-1 |
| 1103 | A2013N | NM_002245 | KCNK1 | potassium channel, subfamily K, member 1 (TWIK) |
| 1104 | A2967 | X54473 | COX6B | cytochrome c oxidase subunit VIb |
| 1105 | A4366 | Y00503 | KRT19 | keratin 19 |
| 1106 | A6311 | A1090753 | SHMT2 | serine hydroxymethyltransferase 2 (mitochondrial) |
| 1107 | B4260 | AB101210 | URLC8 | up-regulated in lung cancer 8 |
| 1108 | A7435N | X16302 | IGFBP2 | insulin-like growth factor binding protein 2 (36kD) |
| 1109 | A6519N | AA703988 | ZNF259 | zinc finger protein 259 |
| 1110 | B4034 | AA523981 | | ESTs |
| 1111 | B7362 | AA579959 | CYP2S1 | cytochrome P540 family member predicted from ESTs |
| 1112 | B8716 | AA766315 | FLJ10461 | hypothetical protein FLJ10461 |
| 1113 | C6219 | AB101207 | URLC5 | up-regulated in lung cancer 5 |
| 1114 | C2132 | AA468538 | BRPF3 | bromodomain and PHD finger containing, 3 |
| 1115 | A2837 | L27711 | CDKN3 | cyclin-dependent kinase inhibitor 3 (CDK2-associated dual specificity phosphatase) |
| 1116 | C1434 | N22773 | KIAA0852 | Homo sapiens mRNA; cDNA DKFZp434J1618 (from clone DKFZp434J1618); partial cds |
| 1117 | C6321 | W86781 | | ESTs |
| 1118 | B7768 | AA683339 | GCNT3 | glucosaminyl (N-acetyl) transferase 3, mucin type |
| 1119 | AI379 | D49742 | HABP2 | hyaluronan-binding protein 2 |
| 1120 | A0623 | Y08302 | DUSP9 | dual specificity phosphatase 9 |
| 1121 | A6800 | AI223817 | | ESTs, Weakly similar to secreted cement gland protein XAG-2 homolog [H.sapiens] |
| 1132 | B4227 | AI189576 | FLJ10439 | hypothetical protein FLJ10439 |
| 1123 | B5890 | T78421 | | EST, Weakly similar to KIAA1498 protein [H.sapiens] |
| 1124 | B6599 | AI083771 | PFKFB2 | 6-phosphofnicto-2-kinase/fructose-2,6-biphosphatase 2 |
| 1125 | B5013 | T90472 | LOC51256 | hypothetical protein |
| 1126 | D1400 | AA481072 | | ESTs |
| 1127 | C8507 | N66159 | COX6C | cytochrome c oxidase subunit VIc |
| 1128 | D6136 | AA740188 | | ESTs |
| 1129 | D2882 | AA777954 | | ESTs |
| 1130 | C7435 | AA573892 | KIAA1359 | KIAA1359 protein |
| 1131 | A9323N | NM_018373 | SYNJ2BP | synaptojanin 2 binding protein |
| 1132 | C7257 | AI192528 | | ESTs |
| 1133 | B2900 | AI305234 | | ESTs |
| 1134 | C1890 | AA308562 | PLEK2 | pleckstrin 2 (mouse) homolog |
| 1135 | A0918 | U24183 | PFKM | phosphofructokinase, muscle |
| 1136 | A0516 | U12597 | TRAF2 | TNF receptor-associated factor 2 |
| 1137 | A9262 | AI160327 | MRPL12 | mitochondrial ribosomal protein L12 |
| 1138 | B0335N | R32035 | | Homo sapiens PAK2 mRNA, complete cds |
| 1139 | B8344 | AA164836 | | ESTs, Moderately similar to alternatively spliced product using exon 13A [H.sapiens] |
| 1140 | B4915N | AA459264 | CBFA2T2 | core-binding factor, runt domain, alpha subunit 2; translocated to, 2 |
| 1141 | C4362 | AB105188 | URLC11 | up-regulated in lung cancer 11 |
| 1142 | C6551 | NM_003826 | NAPG | N-ethylmaleimide-sensitive factor attachment protein, gamma |
| 1143 | E1497 | AA291604 | SLC16A3- | solute carrier family 16 (monocarboxylic acid transporters), member 3 |
| 1144 | D8920 | AM38231 | USP13 | Ubiquitin specific protease 13 (isopeptidase T-3) |
| 1145 | D4215 | AA883311 | | ESTs |
| 1146 | C3759 | | AVJ504047 SMARCA4 | SWI/SNF related, matrix associated, actin dependent regulator of chromatin, subfamily a, member 4 |
| 1147 | D3522 | AA813590 | RPLP2 | ribosomal protein, large P2 |
| 1148 | A2805 | Z49254 | MRPL23 | mitochondrial nbosomal protein L23 |
| 1149 | A0365 | U17077 | BENE | BENE protein |
| | 1150 B4616 | AA534943 | SCYB14 | small inducible cytokine subfamily B (Cys-X-Cys), member 14 (BRAK) |
| 1151 | C0986 | AA699979 | | ESTs |
| 1152 | A5623 | AF044588 | PRC1 | protein regulator of cytokinesis 1 |
| 1153 | A2678 | Z29074 | KRT9 | keratin 9 (epidermolytic palmoplantar keratoderma) |
| 1154 | A2498 | L11932 | SHNT1 | serine hydroxymethyltransferase 1 |
| 1155 | A6942 | AA521342 | | ESTs |
| 1156 | B1253N | D84557 | MCM6 | minichromosome maintenance deficient (mis5, S. pombe) 6 |
| 1157 | B7145N | AI088095 | NINJ2 | ninjurin 2 |
| 1158 | C1881 | H77737 | | EST |
| 1159 | C0969 | AI205093 | | ESTs |
| 1160 | D8285 | AA748613 | SMARCC1 | SWI/SNF related, matrix associated, actin dependent regulator of chromatin, subfamily c, member 1 |
| 1161 | C8167 | AA860277 | | ESTs, Weakly similar to TB2 [H.sapiens] |
| 1162 | C0400 | AA031695 | LMP-2 | IGF-II mRNA-binding protein 2 |
| 1163 | A7791 | AA578427 | | ESTs, Weakly similar to BGAM_HUMAN BETA-GALACTOSIDASE-RELATED PROTEIN PRECURSO [H.sapiens] |
| 1164 | A1198 | NM002522 | NPTX1 | neuronal pentraxin I |
| 1165 | A7165 | X92896 | DXS9879E | DNA segment on chromosome X (unique) 9879 expressed sequence |
| 1166 | B1194 | AA657405 | | ESTs |
| 1167 | A7724 | AA609417 | DKFZp762M hypothetical 136 | protein DKFZp762M136 |
| 1168 | B3984 | U69141 | GCDH | glutaryl-Coenzyme A dehydrogenase |
| 1169 | B3086 | AA743688 | FLJ12892 | hypothetical protein FLJ12892 |
| 1170 | B6535 | AA654506 | HLA-A | major histocompatibility complex, class I, A |
| 1171 | A8885N | H61951 | APMCF1 | APMCF1 protein |
| 1172 | B7360 | AA876375 | | ESTs, Highly similar to LB4D_HUMAN NADP-DEPENDENT LEUKOTRIENE B4 12-HYDROXYDEHYDROGENASE [H.sapiens] |
| 1173 | B7710 | AI341146 | | ESTs |
| 1174 | B9455 | AI299327 | | ESTs |
| 1175 | C7085 | AI139873 | KIAA0134 | KIAA0134 gene product |
| 1176 | C9720 | AA121245 | RANBP7 | RAN binding protein 7 |
| 1177 | C4408 | AA418644 | | ESTs, Weakly similar to C4HU complement C4A precursor [H.sapiens] |
| 1178 | D3747 | AA843607 | | ESTs |
| 1179 | D9933 | AI079544 | | ESTs |
| 1180 | D8458 | AA830668 | | EST |
| 1181 | C7801 | AI299827 | | Homo sapiens cDNA FL313782 fis, clone PLACE4000489, weakly similar to PROTEIN GRAINY-HEAD |
| 1182 | D9317 | AA532638 | | ESTs, Moderately similar to ALU2_HUMAN ALU SUBFAMILY SB SEQUENCE CONTAMINATION WARNING ENTRY [H.sapiens] |
| 1183 | B0203 | NM_004053 | BYSL | bystin-like |
| 1184 | B8260 | R27303 | SURF6 | Surfeit 6 |
| 1185 | B8930 | AB101208 | URLC6 | up-regulated in lung cancer 6 |
| 1186 | A2788 | X63187 | HE4 | epididymis-specific, whey-acidic protein type, four-disulfide core; putative ovarian carcinoma marker |
| 1187 | B8232 | AA666114 | | Homo sapiens pseudouridine synthase 1 (PUS1) mRNA, partial cds |
| 1188 | A7045 | AA096332 | | ESTs |
| 1189 | B8807 | AA214125 | NAP1L4 | nueleosome assembly protein 1-like 4 |
| 1190 | B9040 | R52161 | | Homo sapiens mRNA; cDNA DKFZp434A2410 (from clone DKEZp434A2410); partial cds |
| 1191 | A6241N | NM_005694 | COX17 | COX17 (yeast) homolog, cytochrome c oxidase assembly protein |
| 1192 | B8443 | AA602585 | | ESTs |
| 1193 | B3749 | AA394175 | | RAR (RAS like GTPASE) |
| 1194 | C0772 | AI215719 | KIAA0442 | KIAA0442 protein |
| 1195 | C1511 | AA905266 | LOC51250 | hypothetical protein |
| 1196 | C7658 | AA143060 | | ESTs, Highly similar to I38945 melanoma ubiquitous mutated protein [H.sapiens] |
| 1197 | C7479 | AF019413 | CYP21A2 | cytochrome P450, subfamily XXIA (steroid 21-hydroxylase, congenital adrenal hyperplasia), polypeptide 2 |
| 1198 | A0607N | AI347538 | BIK | BCL2-interacting killer (apoptosis-inducing) |
| 1199 | A2154 | X59617 | RRM1 | ribonucleotide reductase M1 polypeptide |
| 1200 | A6724 | AI193969 | FLJ22759 | hypothetical protein FLJ22759 |
| 1201 | A9581 | AB105377 | SLC7A1 | solute carrer family 7 (cationic amino acid transporter, y+ system), member 1 |
| 1202 | A7303 | N50517 | | ESTs |
| 1203 | B1397 | AI366215 | | Homo sapiens mRNA; cDNA DKFZp434C0126 (from clone DKFZp434C0126); partial cds |
| 1204 | B3912 | AA405413 | SLC25A10 | solute carrier family 25 (mitochondrial carrier, dicarboxylate transporter), member 10 |
| 1205 | B3198 | AI199919 | FLJ20657 | hypothetical protein FLJ20657 |
| 1206 | B4062 | X14640 | KRT13 | keratin 13 |
| 1207 | C0531 | N20321 | D19S1177E | DNA segment on chromosome 19 (unique) 1177 expressed sequence |
| 1208 | C0639 | H17516 | | ESTs |
| 1209 | C7434 | AI333599 | LOC56287 | CA11 |
| 1210 | D5382 | H90132 | | ESTs |
| 1211 | A1604 | X52186 | ITGB4 | integrin, beta 4 |
| 1212 | A0024 | AF017790 | HEC | highly expressed in cancer, rich in leucine heptad repeats |
| 1213 | A2728 | X87342 | LLGL2 | lethal giant larvae (Drosophila) homolog 2 |
| 1214 | A3410 | L77566 | DGSI | DiGeorge syndrome critical region gene DGSI |
| 1215 | B5730 | AI367310 | | ESTs, Weakly similar to dJ37E16.5 [H.sapiens] |
| 1216 | B6539 | AI239432 | | ESTs |
| 1217 | A4009 | D17793 | AKR1C3 | aldo-keto reductase family 1, member C3 (3-alpha hydroxysteroid dehydrogenase, type II) |
| 1218 | A2832 | D13118 | ATP5G1 | AIP synthase, H+ transporting, mitochondrial F0 complex, subunit c (subunit 9), isoform 1 |
| 1219 | A5666 | AA457022 | E2IG5 | hypothetical protein, estradiol-induced |
| 1220 | A1434 | M10036 | TPI1 | triosephosphate isomerase 1 |
| 1221 | A4593 | U94836 | ERPROT213 -21 | protein with polyglutamine repeat; calcium (can2+) homeostasis endoplasmic reticulum protein |
| 1222 | A2150 | D89618 | KPNA3 | karyopherin alpha 3 (importin alpha 4) |
| 1223 | A4536 | Y09723 | ZNF151 | zinc finger protein 151 (pHZ-67) |
| 1224 | B2589 | AA586814 | | ESTs |
| 1225 | A8643 | AA701659 | HUGT1 | UDP-glucose:glycoprotein glucosyltransferase 1 |
| 1226 | B9253 | R59595 | | ESTs |
| 1227 | B5279 | AA700186 | FST | follistatin |
| 1228 | B7214N | AA741058 | | ESTs |
| 1229 | A2411N | AI312652 | MRPS24 | Mitochondrial ribosomal protein S24 |
| 1230 | B7343N | AA521052 | | ESTs, Weakly similar to ALU1_HUMAN ALU SUBFAMILY J SEQUENCE CONTAMINATION WARNING ENTRY [H.sapiens] |
| 1231 | C2021 | N40918 | | Homo sapiens mRNA; cDNA DKFZp761G1111 (from clone DKFZp761G1111) |
| 1232 | C9732 | AA019655 | | EST |
| 1233 | C3905 | L48863 | | Homo sapiens mRNA; cDNA DKFZp434P0235 (from clone DKFZp434P0235) |
| 1234 | C9495 | R99122 | | ESTs, Highly similar to CBF_HUMAN CCAAT-BINDING FACTOR [H.sapiens] |
| 1235 | D8547 | AI018498 | FLJ20591 | hypothetical protein |
| 1236 | D5388 | AI301490 | HSPC135 | HSPC135 protein |
| 1237 | D3023 | AA781745 | | ESTs, Moderately similar to KIAA0638 protein [H.sapiens] |
| 1238 | D8837 | AI025916 | FSP-2 | fibrousheathin II |
| 1239 | A3311 | L04483 | RPS21 | ribosomal protein S21 |
| 1240 | C3979 | AK074088 | FLJ00159 | Homo sapiens cDNA: FLJ00159 |
| 1241 | B1719 | AA634294 | | ESTs |
| 1242 | B8220 | AF074264 | LRP6 | low density lipoprotein receptor-related protein 6 |
| 1243 | C9360 | AI366259 | | ESTs |
| 1244 | C7637 | AA491000 | | Homo sapiens mRNA; cDNA DKFZp586N1720 (from clone DKFZp586N1720) |
| 1245 | A4460 | AF037335 | CA12 | carbonic anhydrase XII |
| 1246 | A0060 | NM_003599 | SUPT3H | suppressor of Ty 3 homolog (S.cerevisiae) |
| 1247 | A5640 | AA047322 | MGC5585 | hypothetical protein MGC5585 |
| 1248 | B7466 | AA128378 | | ESTs |
| 1249 | B3907 | AA913298 | KIAA0969 | KIAA0969 protein |
| 1250 | B3698 | AA234475 | | PRIP-interacting protein with methyltransferase domain |
| 1251 | C8029 | M19309 | TNNT1 | troponin T1, skeletal, slow |
| 1252 | C9747 | AA420675 | | ESTs, Moderately similar to RL39_HUMAN 60S RIBOSOMAL PROTEIN L3 [H.sapiens] |
| 1253 | D6683 | AI361048 | | ESTs |
| 1254 | E1250 | NM_018231 | FLJ10815 | hypothetical protein FLJ10815 |
| 1255 | B5640 | AA759219 | | Homo sapiens cDNA FLJ13123 fis, clone NT2RP3002763 |
| 1256 | D8485 | AI277810 | PSMC2 | Proteasome (prosome, macropain) 26S subunit, ATPase, |
| 1257 | C1487 | N50938 | | Homo sapiens cDNA FLJ20428 fis, clone KAT03458, highly similar to Z184_HUMAN ZINC FINGER PROTEIN 184 |
| 1258 | C3716 | AK025906 | | Homo sapiens cDNA: FLJ22253 fis, clone HRC02763 |
| 1259 | C2294 | AI018174 | | ESTs |
| 1260 | E0161 | AI090079 | | EST |
| 1261 | D8466 | AA642343 | | ESTs |
| 1262 | A0458 | U14968 | RPL27A | ribosomal protein L27a |
| 1263 | A0345 | X52943 | ATF7 | activating transcription factor 7 |
| 1264 | A0333 | X13293 | MYBL2 | v-myb avian myeloblastosis viral oncogene homolog-like |
| 1265 | A3919 | NM 004212 | SLC28A2 | solute carrier family 28 (sodium-coupled nucleoside transporter), member 2 |
| 1266 | A3982 | AJ000553 | SH2D2A | SH2 domain protein 2A |
| 1267 | A2219 | M55265 | CSNK2A1 | casein kinase 2, alpha 1 polypeptide |
| 1268 | A4181 | AA847250 | SSR4 | signal sequence receptor, delta (translocon-associated protein delta) |
| 1269 | B4899 | AI366597 | | ESTs |
| 1270 | B0906 | H05704 | HCR | HCR (a-helix coiled-coil rod homologue) |
| 1271 | B4480 | W29089 | | ESTs, Moderately similar to unnamed protein product [H.sapiens] |
| 1272 | B4535 | AI125927 | FLJ13441 | hypothetical protein FLJ13441 |
| 1273 | B7505 | NM152440 | FLJ32549 | hypothetical protein FLJ32549 |
| 1274 | C4593 | AI192455 | | ESTs |
| 1275 | C6763 | AA032253 | | ESTs |
| 1276 | C1901 | AA649063 | FLJ21865 | hypothetical protein FLJ21865 |
| 1277 | C4573 | AA952902 | | ESTs |
| 1278 | C4172 | AA477870 | B4GALT7 | xsylprotein betal 1,4-galactosyltransferase, polypeptide 7 (galactosyltransferase I) |
| 1279 | E1007 | Z82244 | TOM1 | target of myb1 (chicken) homolog |
| 1280 | C6687 | AA043093 | | ESTs, Weakly similar to S10590 cysteine proteinase [H.sapiens] |
| 1281 | B9303 | AI271678 | | ESTs |
| 1282 | C4388 | H59788 | PBP | prostatic binding protein |
| 1283 | A3553 | J05581 | MUC1 | mucin 1, transmembrane |
| 1284 | A5644 | W76105 | | ESTs, Weakly similar to AF151840 1 CGI-82 protein [H.saplens] |
| 1285 | A4962 | S76474 | NTRK2 | neurotrophic tyrosine kinase, receptor, type 2 |
| 1286 | B3873N | AA703211 | FLJ20736 | hypothetical protein FLJ20736 |
| 1287 | B4531 | N62451 | | Homo sapiens cDNA FLJ11883 fis, clone HEMBA1007178 |
| 1288 | B5091 | AB037857 | PTGFRN | prostaglandin F2 receptor negative regulator |
| 1289 | C0691 | AA132089 | | ESTs, Highly similar to unnamed protein product [H.sapiens] |
| 1290 | E0560 | AA701308 | GALNT2 | UDP-N-acetyl-alpha-D-galactosamine:polypeptide N-acetylgalactosaminyltransferase 2 (GalNAc-T2) |
| 1291 | A0831 | M21389 | KRT5 | keratin 5 (epidermolysis bullosa simplex, Dowling-Meara/Kobner/Weber-Cockayne types) |
| 1292 | A7765 | BC035631 | C17orf26 | chromosome 17 open reading frame 26 |
| 1293 | A2507 | D13757 | PPAT | phosphoribosyl pyrophosphate amidotransferase |
| 1294 | A7115 | U78082 | MED6 | RNA polymerase II transcriptional regulation mediator (Med6, S. cerevisiae, homolog of) |
| 1295 | A4114N | NM001109 | ADAM8 | a disintegrin and metalloproteinase domain 8 |
| 1296 | B4017 | AA088857 | | ESTs |
| 1297 | B7165N | AA194384 | | ESTs |
| 1298 | C4548 | N64368 | | ESTs |
| 1299 | B8237 | H49431 | KIAA0720 | KIAA0720 protein |
| 1300 | B8883 | AF070546 | IL14 | interleukin 14 |
| 1301 | A2955 | L15203 | TFF3 | trefoil factor 3 (intestinal) |
| 1302 | A6673 | AA020936 | LOC51754 | NAG-5 protein |
| 1303 | A6979 | AI357616 | | Homo sapiens mRNA; cDNA DKFZp434C107 (from clone DKFZp434C107) |
| 1304 | B6651 | N47861 | PDP | pyruvate dehydrogenase phosphatase |
| 1305 | C1558 | AI201953 | | ESTs |
| 1306 | C2000 | AF000148 | ABCA4 | ATP-binding cassette, sub-family A (ABC1), member 4 |
| 1307 | C8101 | N47307 | NDUFA1 | NADH dehydrogenase (ubiquinone) 1 alpha subcomplex, 1 (7.5kD, MWFE) |
| 1308 | D1346 | A1091975 | | ESTs |
| 1309 | A7046 | W04300 | | ESTs, Highly similar to Unknown gene product [H.sapiens] |
| 1310 | C1849 | AI338625 | FJX1 | putative secreted ligand homologous to fjx1 |
| 1311 | A1259 | AF007170 | KIAA0452 | DEME-6 protein |
| 1312 | A4474 | AF047433 | ITGB4BP | integrin beta 4 binding protein |
| 1313 | B8016 | AA528243 | | ESTs |
| 1314 | B3530N | AI333192 | GJB2 | gap junction protein, beta 2, 26kD (connexin 26) |
| 1315 | C7582 | AA461250 | | ESTs |
| 1316 | D8905 | AI021894 | | ESTs, Weakly similar to ALU8_HUMAN ALU SUBFAMILY SX SEQUENCE CONTAMINATION WARNING ENTRY [H.sapiens] |
| 1317 | A1139 | L24203 | ATDC | ataxia-telangiectasia group D-associated protein |
| 1318 | A8614 | AA521149 | PSAP | prosaposin (variant Gaucher disease and variant metachromatic leukodystrophy) |
| 1319 | C7495 | D81606 | | Homo sapiens mRNA; cDNA DKFZp434M0531 (from clone DKFZp434M0531) |
| 1320 | C9099 | AA505974 | | ESTs |
| 1321 | A7204 | AA315827 | TXN | thioredoxin |
| 1322 | B7369 | AI289480 | | Homo sapiens cDNA FLJ13044 fis, clone NT2RP3001355, weakly similar to TRICARBOXYLATE TRANSPORT PROTEIN PRECURSOR |
| 1323 | C9024 | AA281364 | | DKFZp434D177 Hypothetical protein DKFZp434D177 |
| 1324 | B4647 | AA625270 | FLJ20640 | hypothetical protein FLJ20640 |
| 1325 | C7351 | AI357002 | FACL5 | long-chain fatty acid coenzyme A ligase 5 |
| 1326 | C0764 | AA045020 | FLJ13576 | hypothetical protein FLJ13576 |
| 1327 | C1018 | AA970651 | | Homo sapiens cDNA: FLJ22256 fis, clone HRC02860 |
| 1328 | E0465 | AA421724 | CDC20 | CDC20 (cell division cycle 20, S. cerevisiae, homolog) |
| 1329 | A0309 | U85658 | TFAP2C | transcription factor AP-2 gamma (activating ennancer-binding protein 2 gamma) |
| 1330 | B2602 | AA810725 | FLJ11273 | hypothetical protein FLJ11273 |
| 1331 | B2951 | L16785 | NME2 | non-metastatic cells 2, protein (NM23B) expressed in |
| 1332 | A5136N | AA029950 | ST14 | suppression of tumorigenicity 14 (colon carcinoma, matriptase, epithin) |
| 1333 | B9980 | AI284476 | | ESTs |
| 1334 | A3526 | D87957 | RQCD1 | RCD1 required for cell differentiation 1 homolog (S. pombe) |
| 1335 | A1054 | M13755 | ISG15 | interferon-stimulated protein, 15 kDa |
| 1336 | A1803 | M31159 | IGFBP3 | insulin-like growth factor binding protein 3 |
| 1337 | A4699 | U49260 | MVD | mevalonate (diphospho) decarboxylase |
| 1338 | A2108 | U05861 | AKR1C1 | aldo-keto reductase family 1, member C1 (dihydrodiol dehydrogenase 1; 20-alpha (3-alpha)-hydroxysteroid dehydrogenase) |
| 1339 | A4072 | AF040105 | RCL | putative c-Myc-responsive |
| 1340 | A5567 | AA236980 | | Homo sapiens cDNA FLJ11856 fis, clone HEMBA1006789 |
| 1341 | A4011 | D26125 | AKR1C4 | aldo-keto reductase family 1, member C4 (chlordecone reductase; 3-alpha hydroxysteroid dehydrogenase, type I; dihydrodiol dehydrogenase 4) |
| 1342 | A4122 | AA315816 | RBX1 | ring-box 1 |
| 1343 | A3970 | AB105190 | GPR49 | G protein-coupled receptor 49 |
| 1344 | A4695 | U44427 | TPD52L1 | tumor protein D52-like 1 |
| 1345 | A5660 | AA780068 | HT002 | HT002 protein; hypertension-related calcium-regulated gene |
| 1346 | A2595 | Y00281 | RPN1 | ribophorin I |
| 1347 | A2402 | M61831 | AHCY | S-adenosylhomocysteine hydrolase |
| 1348 | A3246 | M57899 | UGT1A1 | UDP glycosyltransferase 1 family, polypeptide A1 |
| 1349 | A0587 | X74795 | MCM5 | minichromosome maintenance deficient (S. cerevisiae) 5 (cell division cycle 46) |
| 1350 | A3009 | M30704 | AREG | amphiregulin (schwannoma-derived growth factor) |
| 1351 | A0374 | M61764 | TUBG1 | tubulin, gamma 1 |
| 1352 | A2323 | V00494 | ALB | albumin |
| 1353 | A7608 | AI338589 | | Homo sapiens mRNA; cDNA DKFZp434B0425 (from clone DKFZp434B0425) |
| 1354 | A6625 | AB002341 | NRCAM | neuronal cell adhesion molecule |
| 1355 | B5638 | AI242496 | | Homo sapiens cDNA FLJ12827 fis, clone NT2RP2002939, weakly similar to ZINC FINGER PROTEIN 136 |
| 1356 | A9077 | AA233853 | E1B-AP5 | E1B-55kDa-associated protein 5 |
| 1357 | B4311 | T55926 | | ESTs |
| 1358 | B3857 | AA418779 | POLR2F | polymerase (RNA) II (DNA directed) polypeptide F |
| 1359 | B8243 | AB011090 | KIAA0518 | Max-interacting protein |
| 1360 | B5455 | AA847227 | NUBP2 | nucleotide binding protein 2 (E.coli MinD like) |
| 1361 | B4495 | AI146846 | pAR3 | three-PDZ containing protein similar to C. elegans PAR3 (partitioning defect) |
| 1362 | A6636 | AB105192 | SCAMPS | secretory carrier membrane protein 5 |
| 1363 | B4430 | AI147455 | H17 | hypothetical protein |
| 1364 | B8276 | AI246699 | CATX-8 | CATX-8 protein |
| 1365 | A9313N | AA775810 | | ESTs, Moderately similar to ALUB_HUMAN !!!! ALU CLASS B WARNING ENTRY !!! [H.sapiens] |
| 1366 | B3935 | AA514317 | FLJ11090 | hypothetical protein FLJ11090 |
| 1367 | B3554 | AA720678 | | ESTs |
| 1368 | B4262 | A1066536 | | ESTs, Weakly similar to ALU8_HUMAN ALU SUBFAMILY SX SEQUENCE CONTAMINATION WARNING ENTRY [H.sapiens] |
| 1369 | B4094 | R47458 | K1AA1151 | KIAA1151 protein |
| 1370 | B6577N | AI086204 | TM4SF6 | transmembrane 4 superfamily member 6 |
| 1371 | B7305 | AA342649 | LOC56755 | hypothetical protein LOC56755 |
| 1372 | B4469 | N76634 | FLJ20315 | hypothetical protein FLJ20315 |
| 1373 | B5212 | AA468294 | | ESTs |
| 1374 | B4508 | R55793 | | ESTs |
| 1375 | B6879 | N72576 | | ESTs, Weakly similar to ALU8_HUMAN ALU SUBFAMILY SX SEQUENCE CONTAMINATION |
| 1376 | C2251 | AA923049 | | WARNING ENTRY [H.sapiens] ESTs, Weakly similar to ALU4_HUMAN ALU SUBFAMILY SB2 SEQUENCE CONTAMINATION WARNING ENTRY [H.sapiens] |
| 1377 | C9596 | AA930354 | | ESTs |
| 1378 | C0427 | AA402968 | LTBP3 | latent transforming growth factor beta binding protein 3 |
| 1379 | C8624 | AA827213 | AKAP8 | A kinase (PRKA) anchor protein 8 |
| 1380 | C1958 | W31174 | | ESTs |
| 1381 | D0767 | AA625387 | | ESTs, Moderately similar to ALU7_HUMAN ALU SUBFAMILY SQ SEQUENCE CONTAMINATION WARNING ENTRY [H.sapiens] |
| 1382 | C2226 | N20968 | | ESTs |
| 1383 | C8280 | D79995 | KIAA0173 | KIAA0173 gene product |
| 1384 | C6985 | AA055971 | KIAA0810 | Homo sapiens cDNA FLJ12407 fis, clone MAMMA1002843 |
| 1385 | E1371 | AI700180 | SES2 | Sestrin 2 |
| 1386 | D8019 | AA502265 | RRP4 | homolog of Yeast RRP4 (ribosomal RNA processing 4), 3'-5'-exonbonuclease |
| 1387 | D3571 | AI208033 | | ESTs |
| 1388 | D9210 | AA921763 | | ESTs |

**Table3 up-regulated genes(≧ x5, 33%-50% of cases)**

| NSC Assign ment | LMMID | Acc | Symbol | TTTLE |
|---|---|---|---|---|
| 1389 | A2796 | NM_006681 | NMU | neuromedin U |
| 1390 | A6122 | AA332510 | | MAGE-E1 protein |
| 1391 | A1564 | U70370 | PITX1 | paired-like homeodomain transcription factor 1 |
| 1392 | A4242 | AI094346 | LGALS7 | lectin, galactosde-binding, soluble, 7 (galectin 7) |
| 1393 | B1836 | AI093275 | | Homo sapiens cDNA FLJ14259 fis, clone PLACE1001076 |
| 1394 | B5412N | AA604379 | FLJ10156 | hypothetical protein |
| 1395 | A2033N | U03272 | FBN2 | fibrillin 2 (congenital contractural arachnodactyly) |
| 1396 | C1701 | H60869 | | ESTs |
| 1397 | C4786 | N72266 | | Homo sapiens mRNA; cDNA DKFZp564O2364 (from clone DKFZp564O2364) |
| 1398 | C7152 | AI338356 | DKFZP586C1324 | DKFZP586C1324 protein |
| 1399 | D1223 | AI278397 | DLX5 | distal-less homeo box 5 |
| 1400 | C7676 | AA148929 | | ESTs |
| 1401 | C7747 | AI282097 | | ESTs |
| 1402 | C6149 | W70242 | | ESTs |
| 1403 | C9574 1404 C6936 | AA813008 AI028661 | FOP | FGFR1 oncogene partner ESTs |
| 1405 | C2011 | AI087330 | | ESTs |
| 1406 | C3800 | AA122217 | LOC51654 | CGI-05 protein |
| 1407 | C4296 | AI193975 | | ESTs |
| 1408 | C6211 | AI127359 | HSPCA | heat shock 90kD protein 1, alpha |
| 1409 | C7751 | AA159920 | | ESTs, Weakly similar to ALU7_HUMAN ALU SUBFAMILY SQ SEQUENCE CONTAMINATION WARNING ENTRY [H.sapiens] |
| 1410 | C8372 | AI243594 | | ESTs |
| 1411 | C7048 | R43598 | | ESTs |
| 1412 | C0589 | N20480 | HSPC157 | HSPC157 protein |
| 1413 | C2309 | AI351898 | | ESTs |
| 1414 | C7610 | AA446866 | | ESTs |
| 1415 | C7681 | AA151182 | LOC58495 | putative zinc finger protein from EUROIMAGE 566589 |
| 1416 | D1352 | AA465341 | | ESTs |
| 1417 | C1938 | AI332412 | HOXC9 | homeo box C9 |
| 1418 | C7399 | AA195941 | | ESTs |
| 1419 | C9071 | AA423972 | | Homo sapiens cDNA: FLJ22562 fis, clone HSI01814 |
| 1420 | C8926 | NM_024944 | CHODL | chondrolectin |
| 1421 | C6055 | AA001450 | | ESTs |
| 1422 | C7422 | AA131918 | TMEM3 | transmembrane protein 3 |
| 1423 | D4376 | AA883952 | | ESTs |
| 1424 | E0451 | U10691 | MAGEA6 | melanoma antigen, family A, 6 |
| 1425 | D4637 | AA740747 | | ESTs |
| 1426 | D5215 | AA937589 | | ESTs |
| 1427 | D6767 | AA904882 | | ESTs |
| 1428 | D3103 | AA760780 | | Homo sapiens clone FLC0675 PRO2870 mRNA, complete cds |
| 1429 | E1110 | AW187989 | | ESTs |
| 1430 | B9320 | AI360163 | | ESTs |
| 1431 | B6707 | AA514538 | EIF2C2 | eukaryotic translation initiation factor 2C, 2 |
| 1432 | B6526 | AA634299 | PAK6 | p21-activated protein kinase 6 |
| 1433 | C1796 | AA019195 | | ESTs |
| 1434 | C4520 | N63600 | | ESTs |
| 1435 | C6421 | AI050743 | DKFZp586H0623 | hypothetical protein DKFZp586H0623 |
| 1436 | D9991 | AI074567 | FLJ10858 | hypothetical protein FLJ10858 |
| 1437 | C4449 | N62731 | | ESTs |
| 1438 | D1425 | T03044 | | EST |
| 1439 | A3477 | U30891 | PC | pyruvate carboxylase |
| 1440 | B6854 | AI243321 | | High-mobility group (nonhistone chromosomal) protein 2 |
| 1441 | B4301 | BC039195 | HSNOV1 | novel protein |
| 1442 | C6020 | AA863228 | KIAA0493 | KIAA0493 protein |
| 1443 | C9940 | AA923485 | | ESTs |
| 1444 | A5678N | AI219861 | TMPO | thymopoietin |
| 1445 | C3787 | AI439055 | RANBP3 | RAN binding protein 3 |
| 1446 | A0574 | X66363 | PCTK1 | PCTAIRE protein kinase 1 |
| 1447 | A6518 | AB009672 | ADAM23 | a disintegrin and metalloproteinase domain 23 |
| 1448 | B2579N | N70341 | KIAA0672 | ESTs |

### Diagnosing non-small cell lung cancer

By measuring the expression level of the various NSC genes, the occurrence of non-small cell lung cancer or a predisposition to develop non-small cell lung cancer in a subject can be determined using a biological sample derived from the subject.

The invention involves determining (*e.g.,* measuring) the expression level of at least one, and up to all the NSC sequences listed in Tables 1-3 in a biological sample.

According to the present invention, a gene transcript of the non-small cell lung cancer-associated gene is detected for determining the expression level of the gene.
Based on the sequence information provided by the GenBank™ database entries for the known sequences, the non-small cell lung cancer-associated genes can be detected and measured using techniques well known to one of ordinary skill in the art. The gene transcripts detected by the method include both the transcription and translation products, such as mRNA and proteins. For example, sequences within the sequence database entries corresponding to NSC polynucleotides can be used to construct probes for detecting NSC mRNAs by, *e.g*., Northern blot hybridization analyses. The hybridization of the probe to a gene transcript in a subject biological sample can be also carried out on a DNA array. The use of an array is preferable for detecting the expression level of a plurality of the NSC genes. As another example, the sequences can be used to construct primers for specifically amplifying the NSC polynucleotides in, *e.g.,* amplification-based detection methods such as reverse-transcription based polymerase chain reaction (RT-PCR). Furthermore, the expression level of the NSC genes can be analyzed based on the biological activity or quantity of proteins encoded by the genes. A method for determining the quantity of the protein includes immunoassay methods.

Any biological materials may be used as the biological sample for determining the expression level so long as NSC gene can be detected in the sample and includes test cell populations (*i.e.,* subject derived tissue sample). Preferably, the biological sample comprises a lung cell (a cell obtained from the lung). Gene expression may also be measured in blood, serum or other bodily fluids such as sputum. Furthermore, the test sample may be cells purified from a tissue.

The subject diagnosed for non-small cell lung cancer according to the method is preferably a mammal and includes human, non-human primate, mouse, rat, dog, cat, horse and cow.

The expression level of one or more of the NSC genes in the biological sample is compared to the expression level(s) of the same genes in a reference sample. The reference sample includes one or more cells with known parameters, *i.e.,* cancerous or non-cancerous. The reference sample should be derived from a tissue type similar to that of the test sample. Alternatively, the control expression level may be determined based on a database of molecular information derived from cells for which the assayed parameter or condition is known.

Whether or not a pattern of the gene expression levels in a biological sample indicates the presence of the NSCLC depends upon the composition of the reference cell population. For example, when the reference cell population is composed of non-cancerous cells, a similar gene expression level in the test biological sample to that of the reference indicates that the test biological sample is non-cancerous. On the other hand, when the reference cell population is made of cancerous cells, a similar gene expression profile in the biological sample to that of the reference indicates that the test biological sample includes cancerous cells.

The test biological sample may be compared to multiple reference samples. Each of the multiple reference samples may differ in the known parameter. Thus, a test sample may be compared to a reference sample known to contain, *e.g*., non-small cell lung cancer cells, and at the same time to a second reference sample known to contain, *e.g.,* non-non-small cell lung cancer cells (normal cells).

According to the invention, the expression of one or more of the non-small cell lung cancer-associated gene, *e.g*., NSC 1-1448 is determined in the biological sample and compared to the normal control level of the same gene. The phrase "normal control level" refers to an expression profile of the non-small cell lung cancer-associated gene(s) typically found in a biological sample of a population not suffering from non-small cell lung cancer. The expression level of the NSC genes in the biological samples from a control and test subjects may be determined at the same time or the normal control level may be determined by a statistical method based on the results obtained by analyzing the expression level of the gene(s) in samples previously collected from a control group. An increase or a decrease of the expression level of the non-small cell lung cancer-associated genes in the biological sample derived from a patient derived tissue sample indicates that the subject is suffering from or is at risk of developing non-small cell lung cancer. For example, an increase in the expression level of NSC 807-1448 in the test biological sample compared to the normal control level indicates that the subject is suffering from or is at risk of developing non-small cell lung cancer. On the other hand, a decrease in the expression level of NSC 1-806 in the test biological sample compared to the normal control level indicates that the subject is suffering from or is at risk of developing non-small cell lung cancer.

An expression level of a NSC gene in a test biological sample can be considered altered when the expression level differs from that of the reference by more than 1.0, 1.5, 2.0, 5.0, 10.0 or more fold. Alternatively, an expression level of a NSC gene in a test biological sample can be considered altered, when the expression level is increased or decreased to that of the reference at least 50%, 60%, 80%, 90% or more.

The difference in gene expression between the test sample and a reference sample may be normalized to a control, *e.g*., housekeeping gene. For example, a control polynucleotide includes those whose expression levels are known not to differ between the cancerous and non-cancerous cells. The expression levels of the control polynucleotide in the test and reference samples can be used to normalize the expression levels detected for the NSC genes. The control genes to be used in the present invention include β-actin, glyceraldehyde 3-phosphate dehydrogenase and ribosomal protein P1.

The differentially expressed NSC genes identified herein also allow for monitoring the course of treatment of non-small cell lung cancer. In this method, a test biological sample is provided from a subject undergoing treatment for non-small cell lung cancer. If desired, multiple test biological samples are obtained from the subject at various time points before, during or after the treatment. The expression of one or more of the NSC genes in the sample is then determined and compared to a reference sample with a known state of non-small cell lung cancer that has not been exposed to the treatment.

If the reference sample contains no non-small cell lung cancer cells, a similarity in the expression level of the NSC genes in the test biological sample and the reference sample indicates the efficaciousness of the treatment. However, a difference in the expression level of the NSC genes in the test and the reference samples indicates a less favorable clinical outcome or prognosis.

The term "efficacious" refers that the treatment leads to a reduction in the expression of a pathologically up-regulated gene (NSC 807-1448), increase in the expression of a pathologically down-regulated gene (NSC 1-806) or a decrease in size, prevalence or metastatic potential of non-small cell lung cancer in a subject. When a treatment is applied prophylactically, "efficacious" means that the treatment retards or prevents occurrence of non-small cell lung cancer or alleviates a clinical symptom of non-small cell lung cancer. The assessment of non-small cell lung cancer can be made using standard clinical protocols. Furthermore, the efficaciousness of a treatment is determined in association with any known method for diagnosing or treating non-small cell lung cancer. For example, non-small cell lung cancer is diagnosed histopathologically or by identifying symptomatic anomalies such as chronic cough, hoarseness, coughing up blood, weight loss, loss of appetite, shortness of breath, wheezing, repeated bouts of bronchitis or pneumonia and chest pain.

Moreover, the present method for diagnosing non-small cell lung cancer may also be applied for assessing the prognosis of a patient with the cancer by comparing the expression level of the (NSC gene(s) in the patient-derived biological sample. Alternatively, the expression level of the gene(s) in the biological sample may be measured over a spectrum of disease stages to assess the prognosis of the patient.

An increase in the expression level of the NSC 807-1448 or decrease in that of the NSC 1-806 compared to a normal control level indicates less favorable prognosis. A similarity in the expression level of the NSC 807-1448 or NSC 1-806 compared to a normal control level indicates a more favorable prognosis of the patient Preferably, the prognosis of a subject can be assessed by comparing the expression profile of NSC 807-1448 or NSC 1-806.

### Expression profile

The invention also provides a non-small cell lung cancer reference expression profile comprising a pattern of gene expression levels of two or more of NSC 1-1448. The expression profile serves as a control for the diagnosis of non-small cell lung cancer or predisposition to developing the disease, monitoring the course of treatment and assessing prognosis of a subject with the disease.

### Identifying compounds that inhibit or enhance non-small cel/ lung cancer-associated gene expression

A compound that inhibits the expression or activity of a non-small cell lung cancer-associated gene is identified by contacting a test cell expressing a non-small cell lung cancer-associated gene with a test compound and determining the expression level or activity of the non-small cell lung cancer-associated gene. A decrease in expression compared to the normal control level indicates that the compound is an inhibitor of the non-small cell lung cancer-associated gene. When the non-small cell lung cancer-associated gene expressed in the test cell is an up-regulated gene, the compound identified according to the method is useful for inhibiting non-small cell lung cancer.

Alternatively, a compound that enhances the expression or activity of a non-small cell lung cancer-associated gene may be identified as an enhancer of the gene by contacting a test cell population expressing a non-small cell lung cancer-associated gene with a test compound and determining the expression level or activity of the non-small cell lung cancer-associated gene. When the non-small cell lung cancer-associated gene expressed in the test cell is a down-regulated gene, the compound identified according to the method is suggested to be useful for inhibiting non-small cell lung cancer.

The test cell may be a population of cells and includes any cells as long as the cell expresses the target non-small cell lung cancer-associated gene(s). For example, the test cell contains an epithelial cell, such as a cell derived from the lung tissue, blood, serum or sputum. The test cell may be an immortalized cell line derived from a non-small cell lung cancer cell. Alternatively, the test cell may be a cell transfected with an NSC gene or which has been transfected with the regulatory sequence (*e.g*., promoter) of an NSC gene that is operably linked to a reporter gene.

### Screening compounds

Using the NSC gene, proteins encoded by the gene or transcriptional regulatory region of the gene, compounds can be screened that alter the expression of the gene or biological activity of a polypeptide encoded by the gene. Such compounds are expected to serve as pharmaceuticals for treating or preventing non-small cell lung cancer.

Therefore, the present invention provides a method of screening for a compound for treating or preventing non-small cell lung cancer using the polypeptide of the present invention. An embodiment of this screening method comprises the steps of: (a) contacting a test compound with a polypeptide of the present invention; (b) detecting the binding activity between the polypeptide of the present invention and the test compound; and (c) selecting the compound that binds to the polypeptide of the present invention.

The polypeptide to be used for the screening may be a recombinant polypeptide or a protein derived from the nature or a partial peptide thereof. The polypeptide to be contacted with a test compound can be; for example, a purified polypeptide, a soluble protein, a form bound to a carrier or a fusion protein fused with other polypeptides.

As a method of screening for proteins that bind to the NSC polypeptide, many methods well known by a person skilled in the art can be used. Such a screening can be conducted by, for example, immunoprecipitation method, specifically, in the following manner. A gene encoding any of the NSC polypeptides is expressed in animal cells and so on by inserting the gene into an expression vector for foreign genes, such as pSV2neo, pcDNA I, pcDNA3.1, pCAGGS and pCD8. The promoter to be used for the expression may be any promoter that can be used commonly and include, for example, the SV40 early promoter (Rigby in Williamson (ed.), Genetic Engineering, vol. 3. Academic Press, London, 83-141 (1982)), the EF-α promoter (Kim et al., Gene 91: 217-23 (1990)), the CAG promoter (Niwa et al., Gene 108: 193-200 (1991)), the RSV LTR promoter (Cullen, Methods in Enzymology 152: 684-704 (1987)) the SRα promoter (Takebe et al., Mol Cell Biol 8: 466 (1988)), the CMV immediate early promoter (Seed and Aruffo, Proc Natl Acad Sci USA 84: 3365-9 (1987)), the SV40 late promoter (Gheysen and Fiers, J Mol Appl Genet 1: 385-94 (1982)), the Adenovirus late promoter (Kaufman et al., Mol Cell Biol 9: 946 (1989)), the HSV TK promoter and so on. The introduction of the gene into animal cells to express a foreign gene can be performed according to any methods, for example, the electroporation method (Chu et al., Nucleic Acids Res 15: 1311-26 (1987)), the calcium phosphate method (Chen and Okayama, Mol Cell Biol 7: 2745-52 (1987)), the DEAE dextran method (Lopata et al., Nucleic Acids Res 12: 5707-17 (1984); Sussman and Milman, Mol Cell Biol 4: 1642-3 (1985)), the Lipofectin method (Derijard, B Cell 7: 1025-37 (1994); Lamb et al., Nature Genetics 5: 22-30 (1993): Rabindran et al., Science 259: 230-4 (1993)) and so on. The NSC polypeptide can also be expressed as a fusion protein comprising a recognition site (epitope) of a monoclonal antibody by introducing the epitope of the monoclonal antibody, whose specificity has been revealed, to the N- or C- terminus of the polypeptide. A commercially available epitope-antibody system can be used (Experimental Medicine 13: 85-90 (1995)). Vectors which can express a fusion protein with, for example, β-galactosidase, maltose binding protein, glutathione S-transferase, green florescence protein (GFP) and so on by the use of its multiple cloning sites are commercially available.

A fusion protein prepared by introducing only small epitopes consisting of several to a dozen amino acids so as not to change the property of the NSC polypeptide by the fusion is also reported. Epitopes, such as polyhistidine (His-tag), influenza aggregate HA, human c-myc, FLAG, Vesicular stomatitis virus glycoprotein (VSV-GP), T7 gene 10 protein (T7-tag), human simple herpes virus glycoprotein (HSV-tag), E-tag (an epitope on monoclonal phage) and such, and monoclonal antibodies recognizing them can be used as the epitope-antibody system for screening proteins binding to the NSC polypeptides (Experimental Medicine 13: 85-90 (1995)).

In immunoprecipitation, an immune complex is formed by adding these antibodies to cell lysate prepared using an appropriate detergent. The immune complex consists of the NSC polypeptide, a polypeptide comprising the binding ability with the polypeptide, and an antibody. Immunoprecipitation can be also conducted using antibodies against the NSC polypeptide, besides using antibodies against the above epitopes, which antibodies can be prepared as described above.

An immune complex can be precipitated, for example with Protein A sepharose or Protein G sepharose when the antibody is a mouse IgG antibody. If the NSC polypeptide is prepared as a fusion protein with an epitope, such as GST, an immune complex can be formed in the same manner as in the use of the antibody against the NSC polypeptide, using a substance specifically binding to these epitopes, such as glutathione-Sepharose 4B.

Immunoprecipitation can be performed by following or according to, for example, the methods in the literature (Harlow and Lane, Antibodies, 511-52, Cold Spring Harbor Laboratory publications, New York (1988)).

SDS-PAGE is commonly used for analysis of immunoprecipitated proteins and the bound protein can be analyzed by the molecular weight of the protein using gels with an appropriate concentration. Since the protein bound to the NSC polypeptide is difficult to detect by a common staining method, such as Coomassie staining or silver staining, the detection sensitivity for the protein can be improved by culturing cells in culture medium containing radioactive isotope, ³⁵S-methionine or ³⁵S-cystein, labeling proteins in the cells, and detecting the proteins. The target protein can be purified directly from the SDS-polyacrylamide gel and its sequence can be determined, when the molecular weight of the protein has been revealed.

As a method for screening proteins binding to any of the NSC polypeptides using the polypeptide, for example, West-Western blotting analysis (Skolnik et al., Cell 65: 83-90 (1991)) can be used. Specifically, a protein binding to an NSC polypeptide can be obtained by preparing a cDNA library from cells, tissues, organs (for example, tissues such as testis and prostate) or cultured cells (e.g., LNCaP, PC3, DU145) expected to express a protein binding to the NSC polypeptide using a phage vector (e.g., ZAP), expressing the protein on LB-agarose, fixing the protein expressed on a filter, reacting the purified and labeled NSC polypeptide with the above filter, and detecting the plaques expressing proteins bound to the NSC polypeptide according to the label. The NSC polypeptide may be labeled by utilizing the binding between biotin and avidin, or by utilizing an antibody that specifically binds to the NSC polypeptide, or a peptide or polypeptide (for example, GST) that is fused to the NSC polypeptide. Methods using radioisotope or fluorescence and such may be also used.

Alternatively, in another embodiment of the screening method of the present invention, a two-hybrid system utilizing cells may be used ("MATCHMAKER Two-Hybrid system", "Mammalian MATCHMAKER Two-Hybrid Assay Kit", "MATCHMAKER one-Hybrid system" (Clontech); "HybriZAP Two-Hybrid Vector System" (Stratagene); the references "Dalton and Treisman, Cell 68: 597-612 (1992)", "Fields and Sternglanz, Trends Genet 10: 286-92 (1994)").

In the two-hybrid system, the NSC polypeptide is fused to the SRF-binding region or GAL4-binding region and expressed in yeast cells. A cDNA library is prepared from cells expected to express a protein binding to the NSC polypeptide, such that the library, when expressed, is fused to the VP16 or GAL4 transcriptional activation region. The cDNA library is then introduced into the above yeast cells and the cDNA derived from the library is isolated from the positive clones detected (when a protein binding to the polypeptide of the invention is expressed in yeast cells, the binding of the two activates a reporter gene, making positive clones detectable). A protein encoded by the cDNA can be prepared by introducing the cDNA isolated above to *E. coli* and expressing the protein.

As a reporter gene, for example, Ade2 gene, lacZ gene, CAT gene, luciferase gene and such can be used in addition to the HIS3 gene.

A compound binding to an NSC polypeptide can also be screened using affinity chromatography. For example, the NSC polypeptide may be immobilized on a carrier of an affinity column, and a test compound, containing a protein capable of binding to the NSC polypeptide, is applied to the column. A test compound herein may be, for example, cell extracts, cell lysates, etc. After loading the test compound, the column is washed, and compounds bound to the NSC polypeptide can be prepared.

When the test compound is a protein, the amino acid sequence of the obtained protein is analyzed, an oligo DNA is synthesized based on the sequence, and cDNA libraries are screened using the oligo DNA as a probe to obtain a DNA encoding the protein.

A biosensor using the surface plasmon resonance phenomenon may be used as a mean for detecting or quantifying the bound compound in the present invention. When such a biosensor is used, the interaction between an NSC polypeptide and a test compound can be observed real-time as a surface plasmon resonance signal, using only a minute amount of polypeptide and without labeling (for example, BIAcore, Pharmacia). Therefore, it is possible to evaluate the binding between the NSC polypeptide and a test compound using a biosensor such as BIAcore.

The methods of screening for molecules that bind when an immobilized NSC polypeptide is exposed to synthetic chemical compounds, or natural substance banks or a random phage peptide display library, and the methods of screening using high-throughput based on combinatorial chemistry techniques (Wrighton et al., Science 273: 458-64 (1996); Verdine, Nature 384: 11-13 (1996); Hogan, Nature 384: 17-9 (1996)) to isolate not only proteins but chemical compounds that bind to the NSC protein (including agonist and antagonist) are well known to one skilled in the art.

Alternatively, the present invention provides a method of screening for a compound for treating or preventing non-small cell lung cancer using a NSC polypeptide comprising the steps as follows:
(a) contacting a test compound with a NSC polypeptide;
(b) detecting the biological activity of the NSC polypeptide of step (a); and
(c) selecting a compound that suppresses or enhances the biological activity of the NSC polypeptide in comparison with the biological activity detected in the absence of the test compound.

Since proteins encoded by any of the genes of NSC 1-1448 have the activity of promoting cell proliferation of non-small cell lung cancer cells, a compound which promotes or inhibits this activity of one of these proteins can be screened using this activity as an index.

Any polypeptides can be used for screening so long as they comprise the biological activity of the NSC proteins. Such biological activity includes cell-proliferating activity of the proteins encoded by a gene of NSC 807-1448. For example, a human protein encoded by NSC 807-1448 can be used and polypeptides functionally equivalent to these proteins can also be used. Such polypeptides may be expressed endogenously or exogenously by cells.

The compound isolated by this screening is a candidate for agonists or antagonists of the NSC polypeptide. The term "agonist" refers to molecules that activate the function of the NSC polypeptide by binding thereto. The term "antagonist" refers to molecules that inhibit the function of the NSC polypeptide by binding thereto. Moreover, a compound isolated by this screening is a candidate for compounds which inhibit the *in vivo* interaction of the NSC polypeptide with molecules (including DNAs and proteins).

When the biological activity to be detected in the present method is cell proliferation, it can be detected, for example, by preparing cells which express an NSC polypeptide (*e.g.,* NSC 807-1448), culturing the cells in the presence of a test compound, and determining the speed of cell proliferation, measuring the cell cycle and such, as well as by measuring the colony forming activity.

As discussed in detail above, by controlling the expression levels of an NSC gene, one can control the onset and progression of non-small cell lung cancer. Thus, compounds that may be used in the treatment or prevention of non-small cell lung cancer, can be identified through screenings that use the expression levels of one or more of the NSC genes as indices. In the context of the present invention, such screening may comprise, for example, the following steps:
a) contacting a test compound with a cell expressing one or more of the NSC genes;
   and
b) selecting a compound that reduces the expression level of one or more genes of NSC 807-1448, or elevates the expression level of one or more genes of NSC 1-806 in comparison with the expression level detected in the absence of the test compound.

Cells expressing at least one of the NSC genes include, for example, cell lines established from non-small cell lung cancer cells; such cells can be used for the above screening of the present invention (*e.g*., A549, NCI-H226, NCI-H522, LC319). The expression level can be estimated by methods well known to one skilled in the art. In the method of screening, a compound that reduces the expression level of at least one of the NSC genes can be selected as candidate agents to be used for the treatment or prevention of non-small cell lung cancer.

Alternatively, the screening method of the present invention may comprise the following steps:
a) contacting a test compound with a cell into which a vector comprising the transcriptional regulatory region of one or more marker genes and a reporter gene that is expressed under the control of the transcriptional regulatory region has been introduced, wherein the one or more marker genes are NSC 1-1448,
b) measuring the activity of said reporter gene; and
c) selecting a compound that reduces the expression level of said reporter gene as compared to a control when said marker gene is an up-regulated gene (*e.g*., NSC 807-1448) or that enhances the expression level when said marker gene is a down-regulated gene (*e.g.,* NSC 1-806).

Suitable reporter genes and host cells are well known in the art. The reporter construct required for the screening can be prepared using the transcriptional regulatory region of a marker gene. When the transcriptional regulatory region of a marker gene has been known to those skilled in the art, a reporter construct can be prepared using the previous sequence information. When the transcriptional regulatory region of a marker gene remains unidentified, a nucleotide segment containing the transcriptional regulatory region can be isolated from a genome library based on the nucleotide sequence information of the marker gene.

Any test compound, for example, cell extracts, cell culture supernatant, products of fermenting microorganism, extracts from marine organism, plant extracts, purified or crude proteins, peptides, non-peptide compounds, synthetic micromolecular compounds and natural compounds can be used in the screening methods of the present invention. The test compound of the present invention can be also obtained using any of the numerous approaches in combinatorial library methods known in the art, including (1) biological libraries, (2) spatially addressable parallel solid phase or solution phase libraries, (3) synthetic library methods requiring deconvolution, (4) the "one-bead one-compound" library method and (5) synthetic library methods using affinity chromatography selection. The biological library methods using affinity chromatography selection is limited to peptide libraries, while the other four approaches are applicable to peptide, non-peptide oligomer or small molecule libraries of compounds (Lam (1997) Anticancer Drug Des. 12: 145). Examples of methods for the synthesis of molecular libraries can be found in the art (DeWitt et al. (1993) Proc. Natl. Acad. Sci. USA 90: 6909; Erb et al. (1994) Proc. Natl. Acad. Sci. USA 91: 11422; Zuckermann et al. (1994) J. Med. Chem. 37: 2678; Cho et al. (1993) Science 261: 1303; Carell et al. (1994) Angew. Chem. Int. Ed. Engl. 33: 2059; Carell et al. (1994) Angew. Chem. Int. Ed. Engl. 33: 2061; Gallop et al. (1994) J. Med. Chem. 37: 1233). Libraries of compounds may be presented in solution (see Houghten (1992) Bio/Techniques 13: 412) or on beads (Lam (1991) Nature 354: 82), chips (Fodor (1993) Nature 364: 555), bacteria (US Pat. No. 5,223,409), spores (US Pat. No. 5,571,698;5,403,484, and 5,223,409), plasmids (Cull et al. (1992) Proc. Natl. Acad. Sci. USA 89: 1865) or phage (Scott and Smith (1990) Science 249: 386; Delvin (1990) Science 249: 404; Cwirla et al. (1990) Proc. Natl. Acad. Sci. USA 87: 6378; Felici (1991) J. Mol. Biol. 222: 301; US Pat Application 2002103360). The test compound exposed to a cell or protein according to the screening methods of the present invention may be a single compound or a combination of compounds. When a combination of compounds are used in the screening method of the invention, the compounds may be contacted sequentially or simultaneously.

A compound isolated by the screening methods of the present invention is a candidate for drugs which promote or inhibit the activity of a NSC polypeptide, for treating or preventing diseases attributed to, for example, cell proliferative diseases, such as non-small cell lung cancer. A compound in which a part of the structure of the compound obtained by the present screening methods of the present invention is converted by addition, deletion and/or replacement, is included in the compounds obtained by the screening methods of the present invention. A compound effective in stimulating the under-expressed genes (e.g., NSC 1-806) or in suppressing the expression of over-expressed genes (e.g., NSC 807-1448) is deemed to have a clinical benefit and can be further tested for its ability to prevent cancer cell growth in animal models or test subjects.

### Selecting a therapeutic agent for treating non-small cell lung cancer that is appropriate for a particular individual

Differences in the genetic makeup of individuals can result in differences in their relative abilities to metabolize various drugs. A compound that is metabolized in a subject to act as an anti-non-small cell lung cancer agent can manifest itself by inducing a change in gene expression pattern in the subject's cells from that characteristic of a cancerous state to a gene expression pattern characteristic of a non-cancerous state. Accordingly, the differentially expressed NSC genes disclosed herein allow for selection of a putative therapeutic or prophylactic inhibitor of non-small cell lung cancer specifically adequate for a subject by testing candidate compounds in a test cell (or test cell population) derived from the selected subject

To identify an anti-non-small cell lung cancer agent, that is appropriate for a specific subject, a test cell or test cell population derived from the subject is exposed to a therapeutic agent and the expression of one or more of the NSC 1-1448 genes is determined.

The test cell is or the test cell population contains a non-small cell lung cancer cell expressing a non-small cell lung cancer associated gene. Preferably, the test cell is or the test cell population contains an epithelial cell. For example, the test cell or test cell population is incubated in the presence of a candidate agent and the pattern of gene expression of the test cell or cell population is measured and compared to one or more reference profiles, *e.g*., a non-small cell lung cancer reference expression profile or an non-non-small cell lung cancer reference expression profile.

A decrease in the expression of one or more of NSC 807-1448 or an increase in the expression of one or more of NSC 1-806 in a test cell or test cell population relative to a reference cell population containing non-small cell lung cancer is indicative that the agent is therapeutic.

The test agent can be any compound or composition. For example, the test agent is an immunomodulatory agents.

### Kit

The invention also provides a kit comprising an NSC-detection reagent, *e.g.,* a nucleic acid that specifically binds to or identifies one or more NSC polynucleotides. Such nucleic acids specifically binding to or identifying one or more of NSC polynucleotides are exemplified by oligonucleotide sequences that are complementary to a portion of NSC polynucleotidesor antibodies which bind to polypeptides encoded by NSC polynucleotides. The reagents are packaged together in the form of a kit. The reagents, such as a nucleic acid or antibody (either bound to a solid matrix or packaged separately with reagents for binding them to the matrix), a control reagent (positive and/or negative) and/or a means of detection of the nucleic acid or antibody are preferably packaged in separate containers. Instructions (*e.g*., written, tape, VCR, CD-ROM, etc.) for carrying out the assay may be included in the kit. The assay format of the kit may be Northern hybridization or sandwich ELISA known in the art.

For example, an NSC detection reagent is immobilized on a solid matrix such as a porous strip to form at least one NSC detection site. The measurement or detection region of the porous strip may include a plurality of detection sites, each detection site containing an NSC detection reagent. A test strip may also contain sites for negative and/or positive controls. Alternatively, control sites are located on a separate strip from the test strip. Optionally, the different detection sites may contain different amounts of immobilized reagents, *i.e.,* a higher amount in the first detection site and lesser amounts in subsequent sites. Upon the addition of a test biological sample, the number of sites displaying a detectable signal provides a quantitative indication of the amount of NSC present in the sample. The detection sites may be configured in any suitably detectable shape and are typically in the shape of a bar or dot spanning the width of a teststrip.

Alternatively, the kit contains a nucleic acid substrate array comprising one or more NSC polynucleotide sequences. The nucleic acids on the array specifically identify one or more polynucleotide sequences represented by NSC 1-1448. The expression of 2, 3, 4, 5, 6, 7, 8, 9, 10, 15, 20, 25, 40 or 50 or more of the genes represented by NSC 1-1448 are identified by virtue of the level of binding to an array test strip or chip. The substrate array can be on, *e.g.,* a solid substrate, *e.g*., a "chip" as described in U.S. Patent No. 5,744,305.

### Array and pluralities

The invention also includes a nucleic acid substrate array comprising one or more NSC polynucleotides. The nucleic acids on the array specifically correspond to one or more polynucleotide sequences represented by NSC 1-1448. The level of expression of 2, 3, 4, 5, 6, 7, 8, 9, 10, 15, 20, 25, 40 or 50 or more of the genes represented by NSC 1-1448 are identified by detecting nucleic acid binding to the array.

The invention also includes an isolated plurality (*i.e*., a mixture of two or more nucleic acids) of nucleic acids. The nucleic acids are in a liquid phase or a solid phase, *e.g.,* immobilized on a solid support such as a nitrocellulose membrane. The plurality includes one or more of the polynucleotides represented by NSC 1-1448. According to a further embodiment of the present invention, the plurality includes 2, 3, 4, 5, 6, 7, 8, 9, 10, 15, 20, 25, 40 or 50 or more of the polynucleotides represented by NSC 1-1448.

### Chips

The DNA chip is a device that is convenient to compare the expression levels of a number of genes at the same time. DNA chip-based expression profiling can be carried out, for example, by the method as disclosed in "Microarray Biochip Technology " (Mark Schena, Eaton Publishing, 2000), etc.

A DNA chip comprises immobilized high-density probes to detect a number of genes. Thus, the expression levels of many genes can be estimated at the same time by a single-round analysis. Namely, the expression profile of a specimen can be determined with a DNA chip. The DNA chip-based method of the present invention comprises the following steps of:
(1) synthesizing aRNAs or cDNAs corresponding to the marker genes;
(2) hybridizing the aRNAs or cDNAs with probes for marker genes; and
(3) detecting the aRNA or cDNA hybridizing with the probes and quantifying the amount of mRNA thereof.

The aRNA refers to RNA transcribed from a template cDNA with RNA polymerase. An aRNA transcription kit for DNA chip-based expression profiling is commercially available. With such a kit, aRNA can be synthesized from T7 promoter-attached cDNA as a template using T7 RNA polymerase. On the other hand, by PCR using random primer, cDNA can be amplified using as a template a cDNA synthesized from mRNA.

Alternatively, the DNA chip comprises probes, which have been spotted thereon, to detect the marker genes of the present invention. There is no limitation on the number of marker genes spotted on the DNA chip. For example, it is allowed to select 5% or more, preferably 20% or more, more preferably 50% or more, still more preferably 70 % or more of the marker genes of the present invention. Any other genes as well as the marker genes can be spotted on the DNA chip. For example, a probe for a gene whose expression level is hardly altered may be spotted on the DNA chip. Such a gene can be used to normalize assay results when the assay results are intended to be compared between multiple chips or between different assays.

A probe is designed for each marker gene selected, and spotted on a DNA chip. Such a probe may be, for example, an oligonucleotide comprising 5-50 nucleotide residues. A method for synthesizing such oligonucleotides on a DNA chip is known to those skilled in the art. Longer DNAs can be synthesized by PCR or chemically. A method for spotting long DNA, which is synthesized by PCR or the like, onto a glass slide is also known to those skilled in the art. A DNA chip that is obtained by the method as described above can be used for diagnosing a non-small cell lung cancer according to the present invention.

The prepared DNA chip is contacted with aRNA, followed by the detection of hybridization between the probe and aRNA. The aRNA can be previously labeled with a fluorescent dye. A fluorescent dye such as Cy3(red) and Cy5 (green) can be used to label an aRNA. aRNAs from a subject and a control are labeled with different fluorescent dyes, respectively. The difference in the expression level between the two can be estimated based on a difference in the signal intensity. The signal of fluorescent dye on the DNA chip can be detected by a scanner and analyzed by using a special program. For example, the Suite from Affymetrix is a software package for DNA chip analysis.

### Methods for treating or preventing non-small cell lung cancer

The present invention provides a method for treating, alleviating or preventing a non-small cell lung cancer in a subject. Therapeutic compounds are administered prophylactically or therapeutically to subjects suffering from or at risk of (or susceptible to) developing non-small cell lung cancer. Such subjects are identified using standard clinical methods or by detecting an aberrant level of expression or activity of NSC 1-1448. Prophylactic administration occurs prior to the manifestation of overt clinical symptoms of disease, such that a disease or disorder is prevented or alternatively delayed in its progression.

The therapeutic method includes increasing the expression or function, or both of one or more gene products of genes whose expression is decreased ("under-expressed genes") in a non-small cell lung cancer cell relative to normal cells of the same tissue type from which the non-small cell lung cancer cells are derived. In these methods, the subject is treated with an effective amount of a compound, which increases the amount of one of more of the under-expressed genes (NSC 1-806) in the subject Administration can be systemic or local. Therapeutic compounds include a polypeptide product of an under-expressed gene, or a biologically active fragment thereof, a nucleic acid encoding an under-expressed gene downstream of expression control elements permitting expression I of the gene in the non-small cell lung cancer cells, and compounds that increase the expression level of such gene endogenously existing in the non-small cell lung cancer cells (*i.e*., compounds that up-regulate the expression of the under-expressed gene(s)). Administration of such therapeutic compounds counter the effects of aberrantly-under expressed gene(s) in the subjects' lung cells and improves the clinical condition of the subject. Such compounds can be obtained by the screening method of the present invention described above.

The method also includes decreasing the expression or function, or both, of one or more gene products of genes whose expression is aberrantly increased ("over-expressed gene") in a non-small cell lung cancer cell relative to normal cells of the same tissue type from which the non-small cell lung cancer cells are derived. The expression may be inhibited by any method known in the art. For example, a subject may be treated with an effective amount of a compound that decreases the amount of one or more of the over-expressed genes (NSC 807-1448) in the subject. Administration of the compound can be systemic or local. Such therapeutic compounds include compounds that decrease the expression level of such gene that endogenously exists in the non-small cell lung cancer cells (*i.e*., compounds that down-regulate the expression of the over-expressed gene(s)). The administration of such therapeutic compounds counter the effects of aberrantly-over expressed gene(s) in the subjects non-small cell lung cancer cells and are expected to improve the clinical condition of the subject. Such compounds can be obtained by the screening method of the present invention described above.

The compounds that modulate the activity of the protein (NSC 1-1448) that can be used for treating or preventing non-small cell lung cancer of the present invention include besides proteins, naturally-occurring cognate ligand of these proteins, peptides, peptidomimetics and other small molecules.

Alternatively, the expression of the over-expressed gene(s) (NSC 807-1448) can be inhibited by administering to the subject a nucleic acid that inhibits or antagonizes the expression of the over-expressed gene(s). Antisense oligonucleotide, siRNA or ribozymes which disrupts the expression of the over-expressed gene(s) can be used for inhibiting the expression of the over-expressed gene(s).

As noted above, antisense-oligonucleotides corresponding to any of the nucleotide sequence of NSC 807-1448 can be used to reduce the expression level of the NSC 807-1448. Antisense-oligonucleotides corresponding to NSC 807-1448 that are up-regulated in non-small cell lung cancer are useful for the treatment or prevention of non-small cell lung cancer. Specifically, the antisense-oligonucleotides of the present invention may act by binding to any of the polypeptides encoded by the NSC 807-1448, or mRNAs corresponding thereto, thereby inhibiting the transcription or translation of the genes, promoting the degradation of the mRNAs, and/or inhibiting the expression of proteins encoded by the NSC nucleotides, and finally inhibiting the function of the proteins. The term "antisense-oligonucleotides" as used herein encompasses both nucleotides that are entirely complementary to the target sequence and those having a mismatch of one or more nucleotides, so long as the antisense-oligonucleotides can specifically hybridize to the target sequence. For example, the antisense-oligonucleotides of the present invention include polynucleotides having the nucleotide sequence of SEQ ID NO: 423, 425, 427, 429, 431, 433, 435_{,} 437, 439, 441, 443, 445, 447, 449, 451, 453, 455, 457, 459, 461, 463, 465, 467, 469, 471, 473, 475, 477, 479, 481, 483, 485, 487, 489, 491, 493, 495, 497, 499, 501, 503, 505, 507, 509, 511, 513, 515, 517, 519, 521, 523, 525, 527, 529, and 531 which all proved to be effective for suppressing focus formation of NSCLC cell lines. In addition, the antisense-oligonucleotides of the present invention include polynucleotides that have a homology of at least 70% or higher, preferably at 80% or higher, more preferably 90% or higher, even more preferably 95% or higher over a span of at least 15 continuous nucleotides to any of the nucleotide sequence of NSC 807-1448. Algorithms known in the art can be used to determine the homology. Furthermore, derivatives or modified products of the antisense-oligonucleotides can also be used as antisense-oligonucleotides in the present invention. Examples of such modified products include lower alkyl phosphonate modifications such as methyl-phosphonate-type or ethyl-phosphonate-type, phosphorothioate modifications and phosphoroamidate modifications.

The antisense-oligonucleotides and derivatives thereof act on cells producing the proteins encoded by NSC 807-1448 by binding to the DNAs or mRNAs encoding the proteins, inhibiting their transcription or translation, promoting the degradation of the mRNAs and inhibiting the expression of the proteins, thereby resulting in the inhibition of the protein function.

An antisense-oligonucleotides and derivatives thereof can be made into an external preparation, such as a liniment or a poultice, by mixing with a suitable base material which is inactive against the derivative.

The antisense-oligonucleotides of the invention inhibit the expression of at least one NSC protein encoded by any one of NSC 807-1448, and thus is useful for suppressing the biological activity of the protein.

The polynucleotides that inhibit one or more gene products of over-expressed genes also include small interfering RNAs (siRNA) comprising a combination of a sense strand nucleic acid and an antisense strand nucleic acid of the nucleotide sequence encoding an over-expressed NSC protein, such as NSC 8.07-1448. The term "siRNA" refers to a double stranded RNA molecule which prevents translation of a target mRNA. Standard techniques of introducing siRNA into the cell can be used in the treatment or prevention of the present invention, including those in which DNA is a template from which RNA is transcribed. The siRNA is constructed such that a single transcript has both the sense and complementary antisense sequences from the target gene, *e.g*., a hairpin.

The method is used to suppress gene expression of a cell with up-regulated expression of an NSC gene. Binding of the siRNA to the NSC gene transcript in the target cell results in a reduction of NSC protein production by the cell. The length of the oligonucleotide is at least 10 nucleotides and may be as long as the naturally occurring transcript Preferably, the oligonucleotide is 19-25 nucleotides in length. Most preferably, the oligonucleotide is less than 75, 50 or 25 nucleotides in length. Preferable siRNA of the present invention include the polynucleotides having the nucleotide sequence of SEQ ID NO: 533, 534, 535, 536, 537, 538, 539, 540, 541, 542, 54-3, 544, 545, 546, 547, 548, 549, 550, 551, and 552 as the target sequence, which all proved to be effective for suppressing cell viability of NSCLC cell lines.

Furthermore, the nucleotide sequence of siRNAs may be designed using a siRNA design computer program available from the Arnbion website
(http://www.ambion.com/techlib/misc/siRNA_finder.html). The nucleotide sequences for the siRNA are selected by the computer program based on the following protocol:

Selection of siRNA Target Sites:
1. Beginning with the AUG start codon of the transcript, scan downstream for AA dinucleotide sequences. Record the occurrence of each AA and the 3' adjacent 19 nucleotides as potential siRNA target sites. Tuschl, et al. recommend not to design siRNA against the 5' and 3' untranslated regions (UTRs) and regions near the start codon (within 75 bases) as these may be richer in regulatory protein binding sites, and thus the complex of endonuclease and siRNAs that were designed against these regions may interfere with the binding of UTR-binding proteins and/or translation initiation complexes.
2. Compare the potential target sites to the human genome database and eliminate from consideration any target sequences with significant homology to other coding sequences. The homology search can be performed using BLAST, which can be found on the NCBI server at: www.ncbi.nlm.nih.gov/BLAST/
3. Select qualifying target sequences for synthesis. On the website of Ambion, several preferable target sequences can be selected along the length of the gene for evaluation.

The siRNAs inhibit the expression of over-expressed NSC protein and is thereby useful for suppressing the biological activity of the protein. Therefore, a composition comprising the siRNA is useful in treating or preventing non-small cell lung cancer.

The nucleic acids that inhibit one or more gene products of over-expressed genes also include ribozymes against the over-expressed gene(s) (NSC 807-1448).

The ribozymes inhibit the expression of over-expressed NSC protein and is thereby useful for suppressing the biological activity of the protein. Therefore, a composition comprising the ribozyme is useful in treating or preventing non-small cell lung cancer.

Generally, ribozymes are classified into large ribozymes and small ribozymes. A large ribozyme is known as an enzyme that cleaves the phosphate ester bond of nucleic acids. After the reaction with the large ribozyme, the reacted site consists of a 5'-phosphate and 3'-hydroayl group. The large ribozyme is further classified into (1) group I intron RNA catalyzing transesterification at the 5'-splice site by guanosine; (2) group II intron RNA catalyzing self-splicing through a two step reaction via lariat structure; and (3) RNA component of the ribonuclease P that cleaves the tRNA precursor at the 5' site through hydrolysis. On the other hand, small ribozymes have a smaller size (about 40 bp) compared to the large ribozymes and cleave RNAs to generate a 5'-hydroxyl group and a 2'-3' cyclic phosphate. Hammerhead type ribozymes (Koizumi et al. (1988) FEBS Lett. 228: 225) and hairpin type ribozymes (Buzayan (1986) Nature 323: 349; Kikuchi and Sasaki (1992) Nucleic Acids Res. 19: 6751) are included in the small ribozymes. Methods for designing and constructing ribozymes are known in the art (see Koizumi et al. (1988) FEBS Lett. 228: 225; Koizumi et al. (1989) Nucleic Acids Res. 17: 7059; Kikuchi and Sasaki (1992) Nucleic Acids Res. 19: 6751) and ribozymes inhibiting the expression of an over-expressed NSC protein can be constructed based on the sequence information of the nucleotide sequence encoding the NSC protein according to conventional methods for producing ribozymes.

The ribozymes inhibit the expression of over-expressed NSC protein and is thereby useful for suppressing the biological activity of the protein. Therefore, a composition comprising the ribozyme is useful in treating or preventing non-small cell lung cancer.

Alternatively, the function of one or more gene products of the over-expressed genes is inhibited by administering a compound that binds to or otherwise inhibits the function of the gene products. For example, the compound is an antibody which binds to the over-expressed gene product or gene products.

The present invention refers to the use of antibodies, particularly antibodies against a protein encoded by an up-regulated gene, or a fragment of the antibody. As used herein, the term "antibody" refers to an immunoglobulin molecule having a specific structure that interacts (binds) specifically with a molecule comprising the antigen used for synthesizing the antibody (i.e., the up-regulated gene product) or with an antigen closely related to it. An antibody that binds to the over-expressed NSC nucleotide may be in any form, such as monoclonal or polyclonal antibodies, and includes antiserum obtained by immunizing an animal such as a rabbit with the polypeptide, all classes of polyclonal and monoclonal antibodies, human antibodies and humanized antibodies produced by genetic recombination.

Furthermore, the antibody used in the method of treating or preventing non-small cell lung cancer of the present invention may be a fragment of an antibody or a modified antibody, so long as it binds to one or more of the proteins encoded by the marker genes. For instance, the antibody fragment may be Fab, F(ab')2, Fv or single chain Fv (scFv), in which Fv fragments from H and L chains are ligated by an appropriate linker (Huston et al. (1988) Proc. Natl. Acad. Sci. USA 85: 5879-83). More specifically, an antibody fragment may be generated by treating an antibody with an enzyme, such as papain or pepsin. Alternatively, a gene encoding the antibody fragment may be constructed, inserted into an expression vector, and expressed in an appropriate host cell (see, for example, Co et al. (1994) J. Immunol. 152: 2968-76; Better M. and Horwitz (1989) Methods Enzymol. 178:476-96; Pluckthun and Skerra (1989) Methods Enzymol. 178: 497-515; Lamoyi (1986) Methods Enzymol. 121: 652-63; Rousseaux et al. (1986) Methods Enzymol. 121:663-9; Bird and Walker (1991) Trends Biotechnol. 9: 132-7).

An antibody may be modified by conjugation with a variety of molecules, such as polyethylene glycol (PEG). The modified antibody can be obtained by chemically modifying an antibody. These modification methods are conventional in the field.

Alternatively, an antibody may be obtained as a chimeric antibody, between a variable region derived from nonhuman antibody and the constant region derived from human antibody, or as a humanized antibody, comprising the complementarity determining region (CDR) derived from nonhuman antibody, the frame work region (FR) derived from human antibody, and the constant region. Such antibodies can be prepared using known technology.

The present invention provides a method for treating or preventing non-small cell lung cancer, using an antibody against an over-expressed NSC polypeptide. According to the method, a pharmaceutically effective amount of an antibody against the NSC polypeptide is administered. An antibody against an over-expressed NSC polypeptide is administered at a dosage sufficient to reduce the activity of the NSC protein. Alternatively, an antibody binding to a cell surface marker specific for tumor cells can be used as a tool for drug delivery. Thus, for example, an antibody against an over-expressed NSC polypeptide conjugated with a cytotoxic agent may be administered at a dosage sufficient to injure tumor cells.

The present invention also relates to a method of treating or preventing non-small cell lung cancer in a subject comprising administering to said subject a vaccine comprising a polypeptide encoded by a nucleic acid selected from the group consisting of NSC 807-1448 or an immunologically active fragment of said polypeptide, or a polynucleotide encoding the polypeptide or the fragment thereof. Administration of the polypeptide induces an anti-tumor immunity in a subject Thus, the present invention further provides a method for inducing anti tumor immunity. The polypeptide or the immunologically active fragments thereof are useful as vaccines against non-small cell lung cancer. In some cases the proteins or fragments thereof may be administered in a form bound to the T cell receptor (TCR) or presented on an antigen presenting cell (APC), such as macrophage, dendritic cell (DC) or B-cells. Due to the strong antigen presenting ability of DC, the use of DC is most preferable among the APCs.

In the present invention, the phrase "vaccine against non-small cell lung cancer" refers to a substance that has the function to induce anti-tumor immunity or immunity to suppress non-small cell lung cancer upon inoculation into animals. In general, anti-tumor immunity includes immune responses such as follows:
- induction of cytotoxic lymphocytes against tumors,
- induction of antibodies that recognize tumors, and
- induction of anti-tumor cytokine production.

Therefore, when a certain protein induces any one of these immune responses upon inoculation into an animal, the protein is decided to have anti-tumor immunity inducing effect. The induction of the anti-tumor immunity by a protein can be detected by observing *in vivo* or *in vitro* the response of the immune system in the host against the protein.

For example, a method for detecting the induction of cytotoxic T lymphocytes is well known. A foreign substance that enters the living body is presented to T cells and B cells by the action of antigen presenting cells (APCs). T cells that respond to the antigen presented by APC in antigen specific manner differentiate into cytotoxic T cells (or cytotoxic T lymphocytes; CTLs) due to stimulation by the antigen, and then proliferate (this is referred to as activation of T cells). Therefore, CTL induction by a certain peptide can be evaluated by presenting the peptide to T cell by APC, and detecting the induction of CTL. Furthermore, APC has the effect of activating CD4+ T cells, CD8+ T cells, macrophage, eosinophils and NK cells. Since CD4+ T cells are also important in anti-tumor immunity, the anti-tumor immunity inducing action of the peptide can be evaluated using the activation effect of these cells as indicators.

A method for evaluating the inducing action of CTL using dendritic cells (DCs) as APC is well known in the art DC is a representative APC having the strongest CTL inducing action among APCs. In this method, the test polypeptide is initially contacted with DC and then this DC is contacted with T cells. Detection of T cells having cytotoxic effects against the cells of interest after the contact with DC shows that the test polypeptide has an activity of inducing the cytotoxic T cells. Activity of CTL against tumors can be detected, for example, using the lysis of ⁵¹Cr-labeled tumor cells as the indicator. Alternatively, the method of evaluating the degree of tumor cell damage using ³H-thymidine uptake activity or LDH (lactose dehydrogenase)-release as the indicator is also well known.

Apart from DC, peripheral blood mononuclear cells (PBMCs) may also be used as the APC. The induction of CTL is reported to be enhanced by culturing PBMC in the presence of GM-CSF and IL-4. Similarly, CTL has been shown to be induced by culturing PBMC in the presence of keyhole limpet hemocyanin (KLH) and IL-7.

The test polypeptides confirmed to possess CTL inducing activity by these methods are polypeptides having DC activation effect and subsequent CTL inducing activity. Therefore, polypeptides that induce CTL against tumor cells are useful as vaccines against non-small cell lung cancer. Furthermore, APC that acquired the ability to induce CTL against non-small cell lung cancer by contacting with the polypeptides are useful as vaccines against non-small cell lung cancer. Furthermore, CTL that acquired cytotoxicity due to presentation of the polypeptide antigens by APC can be also used as vaccines against non-small cell lung cancer. Such therapeutic methods for non-small cell lung cancer using anti-tumor immunity due to APC and CTL are referred to as cellular immunotherapy.

Generally, when using a polypeptide for cellular immunotherapy, efficiency of the CTL-induction is known to increase by combining a plurality of polypeptides having different structures and contacting them with DC. Therefore, when stimulating DC with protein fragments, it is advantageous to use a mixture of multiple types of fragments.

Alternatively, the induction of anti-tumor immunity by a polypeptide can be confirmed by observing the induction of antibody production against tumors. For example, when antibodies against a polypeptide are induced in a laboratory animal immunized with the polypeptide, and when growth, proliferation or metastasis of tumor cells is suppressed by those antibodies, the polypeptide can be determined to have an ability to induce anti-tumor immunity.

Anti-tumor immunity is induced by administering the vaccine of this invention, and the induction of anti-tumor immunity enables treatment and prevention of non-small cell lung cancer. Therapy against or prevention of the onset of non-small cell lung cancer includes any of the steps, such as inhibition of the growth of NSCLC cells, involution of NSCLC cells and suppression of occurrence of NSCLC cells. Decrease in mortality of individuals having non-small cell lung cancer, decrease of NSC markers in the blood, alleviation of detectable symptoms accompanying non-small cell lung cancer and such are also included in the therapy or prevention of non-small cell lung cancer. Such therapeutic and preventive effects are preferably statistically significant. For example, in observation, at a significance level of 5% or less, wherein the therapeutic or preventive effect of a vaccine against non-small cell lung cancer is compared to a control without vaccine administration. For example, Student's t-test, the Mann-Whitney U-test or ANOVA may be used for statistical analyses.

The above-mentioned protein having immunological activity, or a polynucleotide or vector encoding the protein may be combined with an adjuvant An adjuvant refers to a compound that enhances the immune response against the protein when administered together (or successively) with the protein having immunological activity. Examples of adjuvants include cholera toxin, salmonella toxin, alum and such, but are not limited thereto. Furthermore, the vaccine of this invention may be combined appropriately with a pharmaceutically acceptable carrier. Examples of such carriers are sterilized water, physiological saline, phosphate buffer, culture fluid and such. Furthermore, the vaccine may contain as necessary, stabilizers, suspensions, preservatives, surfactants and such. The vaccine is administered systemically or locally. Vaccine administration may be performed by single administration or boosted by multiple administrations.

When using APC or CTL as the vaccine of this invention, non-small cell lung cancer can be treated or prevented, for example, by the *ex vivo* method. More specifically, PBMCs of the subject receiving treatment or prevention are collected, the cells are contacted with the polypeptide *ex vivo,* and following the induction of APC or GTL, the cells may be administered to the subject. APC can be also induced by introducing a vector encoding the polypeptide into PBMCs *ex vivo.* APC or CTL induced *in vitro* can be cloned prior to administration. By cloning and growing cells having high activity of damaging target cells, cellular immunotherapy can be performed more effectively. Furthermore, APC and CTL isolated in this manner may be used for cellular immunotherapy not only against individuals from whom the cells are derived, but also against similar types of diseases in other individuals.

### Pharmaceutical compositions for treating or preventing non-small cell lung cancer

The present invention provides compositions for treating or preventing non-small cell lung cancer comprising a compound selected by the present method of screening for a compound that alters the expression or activity of a non-small cell lung cancer-associated gene.

When administering a compound isolated by the screening method of the present invention as a pharmaceutical for humans and other mammals, such as mice, rats, guinea-pig, rabbits, cats, dogs, sheep, pigs, cattle, monkeys, baboons or chimpanzees for treating a cell proliferative disease (e.g., non-small cell lung cancer), the isolated compoud can be directly administered or can be formulated into a dosage form using conventional pharmaceutical preparation methods. Such pharmaceutical formulations of the present compositions include those suitable for oral, rectal, nasal, topical (including buccal and sub-lingual), vaginal or parenteral (including intramuscular, sub-cutaneous and intravenous) administration, or for administration by inhalation or insufflation. The formulations are optionally packaged in discrete dosage units.

Pharmaceutical formulations suitable for oral administration include capsules, cachets or tablets, each containing a predetermined amount of the active ingredient. Formulations also include powders, granules, solutions, suspensions or emulsions. The active ingredient is optionally administered as a bolus electuary or paste. Tablets and capsules for oral administration may contain conventional excipients such as binding agents, fillers, lubricants, disintegrant or wetting agents. A tablet may be made by compression or molding, optionally with one or more formulational ingredients. Compressed tablets may be prepared by compressing in a suitable machine the active ingredients in a free-flowing form such as a powder or granules, optionally mixed with a binder, lubricant, inert diluent, lubricating, surface active or dispersing agent. Molded tablets may be made via molding in a suitable machine a mixture of the powdered compound moistened with an inert liquid diluent The tablets may be coated according to methods well known in the art. Oral fluid preparations may be in the form of, for example, aqueous or oily suspensions, solutions, emulsions, syrups or elixirs, or may be presented as a dry product for reconstitution with water or other suitable vehicle prior to use. Such liquid preparations may contain conventional additives such as suspending agents, emulsifying agents, non-aqueous vehicles (which may include edible oils) or preservatives. The tablets may optionally be formulated so as to provide slow or controlled release of the active ingredient *in vivo.* A package of tablets may contain one tablet to be taken on each of the month. The formulation or dose of medicament in these preparations makes a suitable dosage within the indicated range acquirable.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit dose or multi-dose containers, for example sealed ampoules and vials, and may be stored in a freeze-dried (lyophilized) condition requiring only the addition of the sterile liquid carrier, for example, saline, water-for-injection, immediately prior to use. Alternatively, the formulations may be presented for continuous infusion. Extemporaneous injection solutions and suspensions may be prepared from sterile powders, granules and tablets of the kind previously described.

Formulations for rectal administration include suppositories with standard carriers such as cocoa butter or polyethylene glycol. Formulations for topical administration in the mouth, for example, buccally or sublingually, include lozenges, which contain the active ingredient in a flavored base such as sucrose and acacia or tragacanth, and pastilles comprising the active ingredient in a base such as gelatin, glycerin, sucrose or acacia. For intra-nasal administration of an active ingredient, a liquid spray or dispersible powder or in the form of drops may be used. Drops may be formulated with an aqueous or non-aqueous base also comprising one or more dispersing agents, solubilizing agents or suspending agents.

For administration by inhalation the compositions are conveniently delivered from an insufflator, nebulizer, pressurized packs or other convenient means of delivering an aerosol spray. Pressurized packs may comprise a suitable propellant such as dichlorodifluoromethane, trichlorofluoromethane, dichiorotetrafluoroethane, carbon dioxide or other suitable gas. In the case of a pressurized aerosol, the dosage unit may be determined by providing a valve to deliver a metered amount.

Alternatively, for administration by inhalation or insufflation, the compositions may take the form of a dry powder composition, for example, a powder mix of an active ingredient and a suitable powder base such as lactose or starch. The powder composition may be presented in unit dosage form in, for example, capsules, cartridges, gelatin or blister packs from which the powder may be administered with the aid of an inhalator or insufflators.

Other formulations include implantable devices and adhesive patches; which release a therapeutic agent.

When desired, the above described formulations, adapted to give sustained release of the active ingredient, may be employed. The pharmaceutical compositions may also contain other active ingredients such as antimicrobial agents, immunosuppressants or preservatives.

It should be understood that in addition to the ingredients particularly mentioned above, the formulations of this invention may include other agents conventional in the art having regard to the type of formulation in question, for example, those suitable for oral administration may include flavoring agents.

Preferred unit dosage formulations are those containing an effective dose, as recited below, of the active ingredient or an appropriate fraction thereof

For each of the aforementioned conditions, the compositions, *e.g*., polypeptides and organic compounds are administered orally or via injection at a dose of from about 0.1 to about 250 mg/kg per day. The dose range for adult humans is generally from about 5 mg to about 17.5 g/day, preferably about 5 mg to about 10 g/day, and most preferably about 100 mg to about 3 g/day. Tablets or other unit dosage forms of presentation provided in discrete units may conveniently contain an amount which is effective at such dosage or as a multiple of the same, for instance, units containing about 5 mg to about 500 mg, usually from about 100 mg to about 500 mg.

The dose employed will depend upon a number of factors, including the age and sex of the subject, the precise disorder being treated, and its severity. Also the route of administration may vary depending upon the condition and its severity.

The present invention further provides a composition for treating or preventing non-small cell lung cancer comprising active ingredient that inhibits the expression of any one of the gene selected from the group of NSC 807-1448. Such active ingredient can be an antisense-oligonucleotide, siRNA or ribozyme against the gene, or derivatives, such as ex-pression vector, of the antisense-oligonucleotide, siRNA or ribozyme. The active ingredient may be made into an external preparation, such as liniment or a poultice, by mixing with a suitable base material which is inactive against the derivatives.

Also, as needed, the active ingredient can be formulated into tablets, powders, granules, capsules, liposome capsules, injections, solutions, nose-drops and freeze-drying agents by adding excipients, isotonic agents, solubilizers, preservatives, pain-killers and such. These can be prepared according to conventional methods for preparing nucleic acid containing pharmaceuticals.

Preferably, the antisense-oligonucleotide derivative, siRNAs derivative or ribozyme derivative is given to the patient by direct application to the ailing site or by injection into a blood vessel so that it will reach the site of ailment. A mounting medium can also be used in the composition to increase durability and membrane-permiability. Examples of mounting mediums include liposome, poly-L-lysine, lipid, cholesterol, lipofectin and derivatives thereof.

The dosage of such compositions can be adjusted suitably according to the patient's condition and used in desired amounts. For example, a dose range of 0.1 to 1.00 mg/kg, preferably 0.1 to 50 mg/kg can be administered.

Another embodiment of the present invention is a composition for treating or preventing non-small cell lung cancer comprising an antibody against a polypeptide encoded by any one of the genes selected from the group of NSC 807-1448 or fragments of the antibody that bind to the polypeptide.

Although there are some differences according to the symptoms, the dose of an antibody or fragments thereof for treating or preventing non-small cell lung cancer is about 0.1 mg to about 100 mg per day, preferably about 1.0 mg to about 50 mg per day and more preferably about 1.0 mg to about 20 mg per day, when administered orally to a normal adult (weight 60 kg).

When administering parenterally, in the form of an injection to a normal adult (weight 60 kg), although there are some differences according to the condition of the patient, symptoms of the disease and method of administration, it is convenient to intravenously inject a dose of about 0.01 mg to about 30 mg per day, preferably about 0.1 to about 20 mg per day and more preferably about 0.1 to about 10 mg per day. Also, in the case of other animals too, it is possible to administer an amount converted to 60 kg of body-weight.

### Polypeptides

According to the present invention, novel human genes *URLC 1* (NSC 905) whose expressions are markedly elevated in non-small cell lung cancer compared to corresponding non-cancerous tissues are provided.

*URLC 1* (NSC 905) encodes a TUDOR domain. The TUDOR domain is suggested to have the function of RNA binding and nucleic acid binding. The nucleotide sequence of this gene is shown in SEQ ID NO: 1 and the amino acid sequence encoded by the gene is shown in SEQ ID NO: 2.

These genes are suggested to render oncogenic activities to cancer cells, and that inhibition of the activity of these proteins could be a promising strategy for the treatment of cancer, specifically non-small cell lung cancer.

The present invention encompasses novel human genes including a nucleotide sequence selected from SEQ ID NO: 1, as well as degenerates and mutants thereof, to the extent that they encode a NSC protein, including the amino acid sequence set forth in SEQ ID NO: 2, or functional equivalents thereof. Hereinafter, the polypeptides encoded by these genes are collectively referred to as NSC protein(s). Examples of polypeptides functionally equivalent to NSC proteins include, for example, homologous proteins of other organisms corresponding to the human NSC protein, as well as mutants of human NSC proteins.

In the present invention, the term "functionally equivalent" means that the subject polypeptide has the activity to promote cell proliferation like any of the NSC proteins and to confer oncogenic activity to cancer cells. Whether the subject polypeptide has a cell proliferation activity or not can be judged by introducing the DNA encoding the subject polypeptide into a cell expressing the respective polypeptide, and detecting promotion of proliferation of the cells or increase in colony forming activity.

Methods for preparing polypeptides functionally equivalent to a given protein are well known by a person skilled in the art and include known methods of introducing mutations into the protein. For example, one skilled in the art can prepare polypeptides functionally equivalent to the human NSC protein by introducing an appropriate mutation in the amino acid sequence of either of these proteins by site-directed mutagenesis (Hashimoto-Gotoh et al., Gene 152:271-5 (1995); Zoller and Smith, Methods Enzymol 100: 468-500 (1983); Kramer et al., Nucleic Acids Res. 12:9441-9456 (1984); Kramer and Fritz, Methods Enzymol 154: 350-67 (1987); Kunkel, Proc Natl Acad Sci USA 82: 488-92 (1985); Kunkel, Methods Enzymol 85: 2763-6 (1988)). Amino acid mutations can occur in nature, too. The polypeptide of the present invention includes those proteins having the amino acid sequences of the human NSC protein in which one or more amino acids are mutated, provided the resulting mutated polypeptides are functionally equivalent to the human NSC protein. The number of amino acids to be mutated in such a mutant is generally 10 amino acids or less, preferably 6 amino acids or less, and more preferably 3 amino acids or less.

Mutated or modified proteins, proteins having amino acid sequences modified by substituting, deleting, inserting, and/or adding one or more amino acid residues of a certain amino acid sequence, have been known to retain the original biological activity (Mark et al., Proc Natl Acad Sci USA 81: 5662-6 (1984); Zoller and Smith, Nucleic Acids Res 10:6487-500 (1982); Dalbadie-McFarland et al., Proc Natl Acad Sci USA 79: 6409-13 (1982)).

The amino acid residue to be mutated is preferably mutated into a different amino acid in which the properties of the amino acid side-chain are conserved (a process known as conservative amino acid substitution). Examples of properties of amino acid side chains are hydrophobic amino acids (A, I, L, M, F, P, W, Y, V), hydrophilic amino acids (R, D, N, C, E, Q, G, H, K, S, T), and side chains having the following functional groups or characteristics in common: an aliphatic side-chain (G, A, V, L, I, P); a hydroxy group containing side-chain (S, T, Y); a sulfur atom containing side-chain (C, M); a carboxylic acid and amide containing side-chain (D, N, E, Q); a base containing side-chain (R, K, H); and an aromatic containing side-chain (H, F, Y, W). Note, the parenthetic letters indicate the one-letter codes of amino acids.

An example of a polypeptide to which one or more amino acids residues are added to the amino acid sequence of human NSC protein is a fusion protein containing the human NSC protein. Fusion proteins are, fusions of the human NSC protein and other peptides or proteins, and are included in the present invention. Fusion proteins can be made by techniques well known to a person skilled in the art, such as by linking the DNA encoding the human NSC protein of the invention with DNA encoding other peptides or proteins, so that the frames match, inserting the fusion DNA into an expression vector and expressing it in a host. There is no restriction as to the peptides or proteins fused to the protein of the present invention.

Known peptides that can be used as peptides that are fused to the NSC protein of the present invention include, for example, FLAG (Hopp et al., Biotechnology 6: 1204-10 (1988)), 6xHis containing six His (histidine) residues, 10xHis, Influenza agglutinin (HA), human c-myc fragment, VSP-GP fragment, p18HIV fragment, T7-tag, HSV-tag, E-tag, SV40T antigen fragment, lck tag, α-tubulin fragment, B-tag, Protein C fragment, and the like. Examples of proteins that may be fused to a protein of the invention include GST (glutathione-S-transferase), Influenza agglutinin (HA), immunoglobulin constant region, β-galactosidase, MBP (maltose-binding protein), and such.

Fusion proteins can be prepared by fusing commercially available DNA, encoding the fusion peptides or proteins discussed above, with the DNA encoding the NSC polypeptide of the present invention and expressing the fused DNA prepared.

An alternative method known in the art to isolate functionally equivalent polypeptides is, for example, the method using a hybridization technique (Sambrook et al., Molecular Cloning 2nd ed. 9.47-9.58, Cold Spring Harbor Lab. Press (1989)). One skilled in the art can readily isolate a DNA having high homology with NSC protein (i.e., SEQ ID NO: 1), and isolate functionally equivalent polypeptides to the human NSC protein from the isolated DNA The NSC proteins of the present invention include those that are encoded by DNA that hybridize with a whole or part of the DNA sequence encoding the human NSC protein and are functionally equivalent to the human NSC protein. These polypeptides include mammal homologues corresponding to the protein derived from human (for example, a polypeptide encoded by a monkey, rat, rabbit and bovine gene). In isolating a cDNA highly homologous to the DNA encoding the human NSC protein from animals, it is particularly preferable to use lung cancer tissues.

The condition of hybridization for isolating a.DNA encoding a polypeptide functionally equivalent to the human NSC protein can be routinely selected by a person skilled in the art. For example, hybridization may be performed by conducting prehybridization at 68°C for 30 min or longer using "Rapid-hyb buffer" (Amersham LIFE SCIENCE), adding a labeled probe, and warming at 68°C for 1 hour or longer. The following washing step can be conducted, for example, in a low stringent condition. A low stringent condition is, for example, 42°C, 2X SSC, 0.1% SDS, or preferably 50°C, 2X SSC, 0.1% SDS. More preferably, high stringent conditions are used. A high stringent condition is, for example, washing 3 times in 2X SSC, 0.01% SDS at room temperature for 20 min, then washing 3 times in 1x SSC, 0.1% SDS at 37°C for 20 min, and washing twice in 1x SSC, 0.1% SDS at 50°C for 20 min. However, several factors, such as temperature and salt concentration, can influence the stringency of hybridization and one skilled in the art can suitably select the factors to achieve the requisite stringency.

In place of hybridization, a gene amplification method, for example, the polymerase chain reaction (PCR) method, can be utilized to isolate a DNA encoding a polypeptide functionally equivalent to the NSC protein, using a primer synthesized based on the sequence information of the protein encoding DNA (SEQ ID NO: 1).

Polypeptides that are functionally equivalent to the human NSC protein encoded by the DNA isolated through the above hybridization techniques or gene amplification techniques, normally have a high homology to the amino acid sequence of the human NSC protein. "High homology" typically refers to a homology of 40% or higher, preferably 60% or higher, more preferably 80% or higher, even more preferably 95% or higher. The homology of a polypeptide can be determined by following the algorithm in "Wilbur and Lipman, Proc Natl Acad Sci USA 80: 726-30 (1983)".

A polypeptide of the present invention may have variations in amino acid sequence, molecular weight, isoelectric point, the presence or absence of sugar chains, or form, depending on the cell or host used to produce it or the purification method utilized. Nevertheless, so long as it has a function equivalent to that of the human NSC protein of the present invention, it is within the scope of the present invention.

The polypeptides of the present invention can be prepared as recombinant proteins or natural proteins, by methods well known to those skilled in the art. A recombinant protein can be prepared by inserting a DNA, which encodes the polypeptide of the present invention (for example, the DNA comprising the nucleotide sequence of SEQ ID NO: 1), into an appropriate expression vector, introducing the vector into an appropriate host cell, obtaining the extract, and purifying the polypeptide by subjecting the extract to chromatography, for example, ion exchange chromatography, reverse phase chromatography, gel filtration, or affinity chromatography utilizing a column to which antibodies against the protein of the present invention is fixed, or by combining more than one of aforementioned columns.

Also when the polypeptide of the present invention is expressed within host cells (for example, animal cells and *E. coli*) as a fusion protein with glutathione-S-transferase protein or as a recombinant protein supplemented with multiple histidines, the expressed recombinant protein can be purified using a glutathione column or nickel column. Alternatively, when the polypeptide of the present invention is expressed as a protein tagged with c-myc, multiple histidines, or FLAG, it can be detected and purified using antibodies to c-myc, His, or FLAG, respectively.

After purifying the fusion protein, it is also possible to exclude regions other than the objective polypeptide by cutting with thrombin or factor-Xa as required.

A natural protein can be isolated by methods Known to a person skilled in the art, for example, by contacting the affinity column, in which antibodies binding to the NSC protein described below are bound, with the extract of tissues or cells expressing the polypeptide of the present invention. The antibodies can be polyclonal antibodies or monoclonal antibodies.

The present invention also encompasses partial peptides of the NSC protein of the present invention. The partial peptide has an amino acid sequence specific to the polypeptide of the present invention and consists of at least 7 amino acids, preferably 8 amino acids or more, and more preferably 9 amino acids or more. The partial peptide can be used, for example, for preparing antibodies against the NSC protein of the present invention, screening for a compound that binds to the NSC protein of the present invention, and screening for accelerators or inhibitors of the NSC protein of the present invention.

A partial peptide of the invention can be produced by genetic engineering, by known methods of peptide synthesis, or by digesting the polypeptide of the invention with an appropriate peptidase. For peptide synthesis, for example, solid phase synthesis or liquid phase synthesis may be used.

Furthermore, the present invention provides polynucleotides encoding the NSC protein of the present invention. The NSC protein of the present invention can be used for the *in vivo* or *in vitro* production of the NSC protein of the present invention as described above, or can be applied to gene therapy for diseases attributed to genetic abnormality in the gene encoding the protein of the present invention. Any form of the polynucleotide of the present invention can be used so long as it encodes the NSC protein of the present invention or equivalents thereof, including mRNA, RNA, cDNA, genomic DNA, chemically synthesized polynucleotides. The polynucleotide of the present invention include a DNA comprising a given nucleotide sequences as well as its degenerate sequences, so long as the resulting DNA encodes the NSC protein of the present invention or equivalents thereof.

The polynucleotide of the present invention can be prepared by methods known to a person skilled in the art. For example, the polynucleotide of the present invention can be prepared by: preparing a cDNA library from cells which express the NSC protein of the present invention, and conducting hybridization using a partial sequence of the DNA of the present invention (for example, SEQ ID NO: 1) as a probe. A cDNA library can be prepared, for example, by the method described in Sarnbrook et al., Molecular Cloning, Cold Spring Harbor Laboratory Press (1989); alternatively, commercially available cDNA libraries may be used. A cDNA library can be also prepared by: extracting RNAs from cells expressing the NSC protein of the present invention, synthesizing oligo DNAs based on the sequence of the DNA of the present invention (for example, SEQ ID NO: 1), conducting PCR using the oligo DNAs as primers, and amplifying cDNAs encoding the NSC protein of the present invention.

In addition, by sequencing the nucleotides of the obtained cDNA, the translation region encoded by the cDNA can be routinely determined, and the amino acid sequence of the NSC protein of the present invention can be easily obtained. Moreover, by screening the genomic DNA library using the obtained cDNA or parts thereof as a probe, the genomic DNA can be isolated.

More specifically, mRNAs may first be prepared from a cell, tissue, or organ in which the object NSC protein of the invention is expressed. Known methods can be used to isolate mRNAs; for instance, total RNA may be prepared by guanidine ultracentrifugation (Chirgwin et al., Biochemistry 18:5294-9 (1979)) or AGPC method (Chomczynski and Sacchi, Anal Biochem 162:156-9 (1987)). In addition, mRNA may be purified from total RNA using mRNA Purification Kit (Pharmacia) and such or, alternatively, mRNA may be directly purified by QuickPrep mRNA Purification Kit (Pharmacia).

The obtained mRNA is used to synthesize cDNA using reverse transcriptase. cDNA may be synthesized using a commercially available kit, such as the AMV Reverse Transcriptase First-strand cDNA Synthesis Kit (Seikagaku Kogyo). Alternatively, cDNA may be synthesized and amplified following the 5'-RACE method (Frohman et al., Proc Natl Acad Sci USA 85: 8998-9002 (1988); Belyavsky et al., Nucleic Acids Res 17: 2919-32 (1989)), which uses a primer and such, described herein, the 5'-Ampli FINDER RACE Kit (Clontech), and polymerase chain reaction (PCR).

A desired DNA fragment is prepared from the PCR products and ligated with a vector DNA. The recombinant vectors are used to transform *E*. *coli* and such, and a desired recombinant vector is prepared from a selected colony. The nucleotide sequence of the desired DNA can be verified by conventional methods, such as dideoxynucleotide chain termination.

The nucleotide sequence of a polynucleotide of the invention may be designed to be expressed more efficiently by taking into account the frequency of codon usage in the host to be used for expression (Grantham et al., Nucleic Acids Res 9: 43-74 (1981)). The sequence of the polynucleotide of the present invention may be altered by a commercially available kit or a conventional method. For instance, the sequence may be altered by digestion with restriction enzymes, insertion of a synthetic oligonucleotide or an appropriate polynucleotide fragment, addition of a linker, or insertion of the initiation codon (ATG) and/or the stop codon (TAA, TGA, or TAG).

Specifically, the polynucleotide of the present invention encompasses the DNA comprising the nucleotide sequence of SEQ ID NO: 1.

Furthermore, the present invention provides a polynucleotide that hybridizes under stringent conditions with a polynucleotide having a nucleotide sequence of SEQ ID NO: 1, and encodes a polypeptide functionally equivalent to the NSC protein of the invention described above. One skilled in the art may appropriately choose stringent conditions. For example, low stringent condition can be used. More preferably, high stringent condition can be used. These conditions are the same as that described above. The hybridizing DNA above is preferably a cDNA or a chromosomal DNA.

### Vectors and host cells

The present invention also provides a vector into which the above polynucleotide of the present invention is inserted. A vector of the present invention is useful to keep a polynucleotide, especially a DNA, of the present invention in host cell, to express the NSC protein of the present invention, or to administer the polynucleotide of the present invention for gene therapy.

When *E. coli* is a host cell and the vector is amplified and produced in a large amount in *E*. *coli* (e.g., JM109, DH5α, HB101, or XLlBlue), the vector should have "ori" to be amplified in *E. coli* and a marker gene for selecting transformed *E. coli* (e.g., a drug-resistance gene selected by a drug such as ampicillin, tetracycline, kanamycin, chloramphenicol or the like). For example, M13-series vectors, pUC-series vectors, pBR322, pBluescript, pCR-Script, etc. can be used. In addition, pGEM-T, pDIRECT, and pT7 can also be used for subcloning and extracting cDNA as well as the vectors described above. When a vector is used to produce the NSC protein of the present invention, an expression vector is especially useful. For example, an expression vector to be expressed in *E. coli* should have the above characteristics to be amplified in *E. coli.* When E. *coli,* such as JM109, DH5α, HB101, or XL1 Blue, are used as a host cell, the vector should have a promoter, for example, lacZ promoter (Ward et al., Nature 341: 544-6 (1989); FASEB J 6: 2422-7 (1992)), araB promoter (Better et al., Science 240: 1041-3 (1988)), or T7 promoter or the like, that can efficiently express the desired gene in *E. coli.* In that respect, pGEX-5X-1 (Pharmacia), "QIAexpress system" (Qiagen), pEGFP and pET (in this case, the host is preferably BL21 which expresses T7 RNA polymerase), for example, can be used instead of the above vectors. Additionally, the vector may also contain a signal sequence for protein secretion. An exemplary signal sequence that directs the NSC protein to be secreted to the periplasm of the *E. coli* is the pelB signal sequence (Lei et al., J Bacteriol 169: 4379 (1987)). Means for introducing of the vectors into the target host cells include, for example, the calcium chloride method and the electroporation method.

In addition to *E*. *coli,* for example, expression vectors derived from mammals (for example, pcDNA3 (Invitrogen) and pEGF-BOS (Nucleic Acids Res 18(17): 5322 (1990)), pEF, pCDM8), expression vectors derived from insect cells (for example, "Bac-to-BAC baculovirus expression system" (GIBCO BRL), pBacPAK8), expression vectors derived from plants (e.g., pMH1, pMH2), expression vectors derived from animal viruses (e.g., pHSV, pMV, pAdexLcw), expression vectors derived from retroviruses (e.g., pZIpneo), expression vector derived from yeast (e.g., "Pichia Expression Kit" (Invitrogen), pNV11, SP-Q01), and expression vectors derived from *Bacillus subtilis* (e.g., pPL608, pKTH50) can be used for producing the polypeptide of the present invention.

In order to express the vector in animal cells, such as CHO, COS or NIH3T3 cells, the vector should have a promoter necessary for expression in such cells, for example, the SV40 promoter (Mulligan et al., Nature 277: 108 (1979)), the MMLV-LTR promoter, the EF1α, promoter (Mizushima et al., Nucleic Acids Res 18: 5322 (1990)), the CMV promoter, and the like, and preferably a marker gene for selecting transformants (for example, a drug resistance gene selected by a drug (e.g., neomycin, G418)). Examples of known vectors with these characteristics include, for example, pMAM, pDR2, pBh-RSV, pBK-CMV, pOPRSV and pOP13.

### Producing NSC proteins

In addition, the present invention provides methods for producing the NSC protein of the present invention. The NSC protein may be prepared by culturing a host cell which harbors a expression vector comprising a gene encoding the NSC protein. According to needs, methods may be used to express a gene stably and, at the same time, to amplify the copy number of the gene in cells. For example, a vector comprising the complementary DHFR gene (*e.g*., pCHO I) may be introduced into CHO cells in which the nucleic acid synthesizing pathway is deleted, and then amplified by methotrexate (MTX). Furthermore, in case of transient expression of a gene, the method wherein a vector comprising a replication origin of SV40 (pcD, etc.) is transformed into COS cells comprising the SV40 T antigen expressing gene on the chromosome can be used.

The NSC protein of the present invention obtained as above may be isolated from inside or outside (such as medium) of host cells, and purified as a substantially pure homogeneous polypeptide. The term "substantially pure" as used herein in reference to a given polypeptide means that the polypeptide is substantially free from other biological macromolecules. The substantially pure polypeptide is at least 75% (e.g., at least 80, 85, 95, or 99%) pure by dry weight. Purity can be measured by any appropriate standard method, for example by column chromatography, polyacrylamide gel electrophoresis, or HPLC analysis. The method for polypeptide isolation and purification is not limited to any specific method; in fact, any standard method may be used.

For instance, column chromatography, filter, ultrafiltration, salt precipitation, solvent precipitation, solvent extraction, distillation, immunoprecipitation, SDS-polyacrylamide gel electrophoresis, isoelectric point electrophoresis, dialysis, and recrystallization may be appropriately selected and combined to isolate and purify the NSC protein.

Examples of chromatography include, for example, affinity chromatography, ion-exchange chromatography, hydrophobic chromatography, gel filtration, reverse phase chromatography, adsorption chromatography, and such (Strategies for Protein Purification and Characterization: A Laboratory Course Manual. Ed. Daniel R Marshak et al., Cold Spring Harbor Laboratory Press (1996)). These chromatographies may be performed by liquid chromatography, such as HPLC and FPLC. Thus, the present invention provides for highly purified polypeptides prepared by the above methods.

The NSC protein of the present invention may be optionally modified or partially deleted by treating it with an appropriate protein modification enzyme before or after purification. Useful protein modification enzymes include, but are not limited to, trypsin, chymotrypsin, lysylendopeptidase, protein kinase, glucosidase and so on.

### Antibodies

The present invention provides an antibody that binds to the NSC protein of the invention. The antibody of the invention can be used in any form, such as monoclonal or polyclonal antibodies, and includes antiserum obtained by immunizing an animal such as a rabbit with the NSC protein of the invention, all classes of polyclonal and monoclonal antibodies, human antibodies, and humanized antibodies produced by genetic recombination.

The NSC protein of the invention used as an antigen to obtain an antibody may be derived from any animal species, but preferably is derived from a mammal such as a human, mouse, or rat, more preferably from a human. A human-derived NSC protein may be obtained from the nucleotide or amino acid sequences disclosed herein. According to the present invention, the polypeptide to be used as an immunization antigen may be a complete protein or a partial peptide of the NSC protein. A partial peptide may comprise, for example, the amino (N)-terminal or carboxy (C)-terminal fragment of the NSC protein of the present invention.

Herein, an antibody is defined as a protein that reacts with either the full length or a fragment of the NSC protein of the present invention.

A gene encoding the NSC protein of the invention or its fragment may be inserted into a known expression vector, which is then used to transform a host cell as described herein. The desired protein or its fragment may be recovered from the outside or inside of host cells by any standard method, and may subsequently be used as an antigen. Alternatively, whole cells expressing the NSC protein or their lysates, or a chemically synthesized polypeptide may be used as the antigen.

Any mammalian animal may be immunized with the antigen, but preferably the compatibility with parental cells used for cell fusion is taken into account. In general, animals of Rodentia, Lagomorpha or Primates are used. Animals of Rodentia include, for example, mouse, rat and hamster. Animals of Lagomorpha include, for example, rabbit. Animals of Primates include, for example, a monkey of Catarrhini (old world monkey) such as *Macaca fascicularis,* rhesus monkey, sacred baboon and chimpanzees.

Methods for immunizing animals with antigens are known in the art. Intraperitoneal injection or subcutaneous injection of antigens is a standard method for immunization of mammals. More specifically, antigens may be diluted and suspended in an appropriate amount of phosphate buffered saline (PBS), physiological saline, etc. If desired, the antigen suspension may be mixed with an appropriate amount of a standard adjuvant, such as Freund's complete adjuvant, made into emulsion, and then administered to mammalian animals. Preferably, it is followed by several administrations of antigen mixed with an appropriately amount of Freund's incomplete adjuvant every 4 to 21 days. An appropriate carrier may also be used for immunization. After immunization as above, serum is examined by a standard method for an increase in the amount of desired antibodies.

Polyclonal antibodies against the NSC protein of the present invention may be prepared by collecting blood from the immunized mammal examined for the increase of desired antibodies in the serum, and by separating serum from the blood by any conventional method. Polyclonal antibodies include serum containing the polyclonal antibodies, as well as the fraction containing the polyclonal antibodies may be isolated from the serum. Immunoglobulin G or M can be prepared from a fraction which recognizes only the NSC protein of the present invention using, for example, an affinity column coupled with the NSC protein of the present invention, and further purifying this fraction using protein A or protein G column.

To prepare monoclonal antibodies, immune cells are collected from the mammal immunized with the antigen and checked for the increased level of desired antibodies in the serum as described above, and are subjected to cell fusion. The immune cells used for cell fusion are preferably obtained from spleen. Other preferred parental cells to be fused with the above immunocyte include, for example, myeloma cells of mammalians, and more preferably myeloma cells having an acquired property for the selection of fused cells by drugs.

The above immunocyte and myeloma cells can be fused according to known methods, for example, the method of Milstein et al. (Galfre and Milstein, Methods Enzymol 73: 3-46 (1981)).

Resulting hybridomas obtained by the cell fusion may be selected by cultivating them in a standard selection medium, such as HAT medium (hypoxanthine, aminopterin, and thymidine containing medium). The cell culture is typically continued in the HAT medium for several days to several weeks, the time being sufficient to allow all the other cells, with the exception of the desired hybridoma (non-fused cells), to die. Then, the standard limiting dilution is performed to screen and clone a hybridoma cell producing the desired antibody.

In addition to the above method, in which a non-human animal is immunized with an antigen for preparing hybridoma, human lymphocytes such as those infected by EB virus may be immunized with the NSC protein, NSC protein expressing cells, or their lysates *in vitro.* Then, the immunized lymphocytes are fused with human-derived myeloma cells that are capable of indefinitely dividing, such as U266, to yield a hybridoma producing a desired human antibody that is able to bind to the NSC protein can be obtained (Unexamined Published Japanese Patent Application No. (JP-A) Sho 63-17688).

The obtained hybridomas are subsequently transplanted into the abdominal cavity of a mouse and the ascites are extracted. The obtained monoclonal antibodies can be purified by, for example, ammonium sulfate precipitation, a protein A or protein G column, DEAE ion exchange chromatography, or an affinity column to which the NSC protein of the present invention is coupled. The antibody of the present invention can be used not only for purification and detection of the NSC protein of the present invention, but also as a candidate for agonists and antagonists of the NSC prtotein of the present invention. In addition, this antibody can be applied to the antibody treatment for diseases related to the NSC protein of the present invention including non-small cell lung cancer. When the obtained antibody is to be administered to the human body (antibody treatment), a human antibody or a humanized antibody is preferable for reducing immunogenicity.

For example, transgenic animals having a repertory of human antibody genes may be immunized with an antigen such as the NSC protein, NSC protein expressing cells, or their lysates. Antibody producing cells are then collected from the animals and fused with myeloma cells to obtain hybridoma, from which human antibodies against the polypeptide can be prepared (see WO92-03918, WO93-2227, WO94-02602, WO94-25585, WO96-33735, and WO96-34096).

Alternatively, an immune cell, such as an immunized lymphocyte, producing antibodies may be immortalized by an oncogene and used for preparing monoclonal antibodies.

Monoclonal antibodies thus obtained can be also recombinantly prepared using genetic engineering techniques (see, for example, Borrebaeck and Larrick, Therapeutic Monoclonal Antibodies, published in the United Kingdom by MacMillan Publishers LTD (1990)). For example, a DNA encoding an antibody may be cloned from an immune cell, such as a hybridoma or an immunized lymphocyte producing the antibody, inserted into an appropriate vector, and introduced into host cells to prepare a recombinant antibody. The present invention also provides recombinant antibodies prepared as described above.

Furthermore, an antibody of the present invention may be a fragment of an antibody or modified antibody, so long as it binds to one or more of the NSC proteins of the invention. For instance, the antibody fragment may be Fab, F(ab')₂, Fv, or single chain Fv (scFv), in which Fv fragments from H and L chains are ligated by an appropriate linker (Huston et al., Proc Natl Acad Sci USA 85: 5879-83 (1988)). More specifically, an antibody fragment may be generated by treating an antibody with an enzyme, such as papain or pepsin. Alternatively, a gene encoding the antibody fragment may be constructed, inserted into an expression vector, and expressed in an appropriate host cell (see, for example, Co et al., J Immunol 152: 2968-76 (1994); Better and Horwitz, Methods Enzymol 178: 476-96 (1989); Pluckthun and Skerra, Methods Enzymol 178: 497-515 (1989); Lamoyi, Methods Enzymol 121: 652-63 (1986); Rousseaux et al., Methods Enzymol 121: 663-9 (1986); Bird and Walker, Trends Biotechnol 9: 132-7 (1991)).

An antibody may be modified by conjugation with a variety of molecules, such as polyethylene glycol (PEG). The present invention provides for such modified antibodies. The modified antibody can be obtained by chemically modifying an antibody. These modification methods are conventional in the field.

Alternatively, an antibody of the present invention may be obtained as a chimeric antibody, between a variable region derived from nonhuman antibody and the constant region derived from human antibody, or as a humanized antibody, comprising the complementarity determining region (CDR) derived from nonhuman antibody, the frame work region (FR) derived from human antibody, and the constant region. Such antibodies can be prepared by using known technology.

Antibodies obtained as above may be purified to homogeneity. For example, the separation and purification of the antibody can be performed according to separation and purification methods used for general proteins. For example, the antibody may be separated and isolated by the appropriately selected and combined use of column chromatographies, such as affinity chromatography, filter, ultrafiltration, salting-out, dialysis, SDS polyacrylamide gel electrophoresis, isoelectric focusing, and others (Antibodies: A Laboratory Manual. Ed Harlow and David Lane, Cold Spring Harbor Laboratory (1988)), but are not limited thereto. A protein A column and protein G column can be used as the affinity column. Exemplary protein A columns to be used include, for example, Hyper D, POROS, and Sepharose F.F. (Pharmacia).

Exemplary chromatography, with the exception of affinity includes, for example, ion-exchange chromatography, hydrophobic chromatography, gel filtration, reverse-phase chromatography, adsorption chromatography, and the like (Strategies for Protein Purification and Characterization: A Laboratory Course Manual. Ed Daniel R. Marshak et al., Cold Spring Harbor Laboratory Press (1996)). The chromatographic procedures can be carried out by liquid-phase chromatography, such as HPLC, and FPLC.

For example, measurement of absorbance, enzyme-linked immunosorbent assay (ELISA), enzyme immunoassay (EIA), radioimmunoassay (RIA), and/or immunofluorescence may be used to measure the antigen binding activity of the antibody of the invention. In ELISA, the antibody of the present invention is immobilized on a plate, the NSC protein of the invention is applied to the plate, and then a sample containing a desired antibody, such as culture supernatant of antibody producing cells or purified antibodies, is applied. Then, a secondary antibody that recognizes the primary antibody and is labeled with an enzyme, such as alkaline phosphatase, is applied, and the plate is incubated. Next, after washing, an enzyme substrate, such as *p*-nitrophenyl phosphate, is added to the plate, and the absorbance is measured to evaluate the antigen binding activity of the sample. A fragment of the NSC protein, such as a C-terminal or N-terminal fragment, may be used as the antigen to evaluate the binding activity of the antibody. BIAcore (Pharmacia) may be used to evaluate the activity of the antibody according to the present invention.

The above methods allow for the detection or measurement of the NSC protein of the invention, by exposing the antibody of the invention to a sample assumed to contain the NSC protein of the invention, and detecting or measuring the immune complex formed by the antibody and the protein.

Because the method of detection or measurement of the NSC protein according to the invention can specifically detect or measure the protein, the method may be useful in a variety of experiments in which the protein is used.

The following examples are presented to illustrate the present invention and to assist one of ordinary skill in making and using the same. The examples are not intended in any way to otherwise limit the scope of the invention.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. Any patents, patent applications, and publications cited herein are incorporated by reference.

### Best Mode for Carrying out the Invention

The present invention is illustrated in details by following Examples, but is not restricted to these Examples.

Tissue obtained from diseased tissue (e.g., epithelial cells from non-small cell lung cancer) and normal tissues was evaluated to identify genes which are differently expressed in a disease state, e.g., non-small cell lung cancer. The assays were carried out as follows.

### [Example 1] General methods

### (1) Patients and tissue samples

Primary lung cancer tissues were obtained with informed consent from 37 patients (15 female and 22 male of 46 to 79 years; median age 66.0) who underwent lobectomy. Clinical information was obtained from medical records and each tumor was diagnosed according to histopathological subtype and grade by pathologist; 22 of the 37 tumors were classified as adenocarcinomas, 14 as SCCs and one as adenosquamous carcinoma. The clinical stage for each tumor was judged according to the UICC TNM classification. All samples were immediately frozen and embedded in TissueTek OCT medium (Sakura, Tokyo, Japan) and stored at -80°C.

### (2) Laser-capture microdissection, extraction of RNA and T7-based RNA amplification

Cancer cells were selectively collected from the preserved samples using laser-capture microdissection (Kitahara et al., Cancer Res 61: 3544-9 (2001)). Extraction of total RNA and T7-based amplification were performed as described previously (Okabe et al., Cancer Res 61: 2129-37 (2001)). As a control probe, normal human lung poly(A) RNA (CLONTECH) was amplified in the same way. 2.5-µg aliquots of amplified RNAs (aRNAs) from each cancerous tissue and from the control were reversely transcribed in the presence of Cy5-dCTP and Cy3-dCTP, respectively.

### (3) Preparation of cDNA microarray

To obtain cDNAs for spotting on glass slides, RT-PCR was performed for each gene as described previously (Kitahara et al., Cancer Res 61: 3544-9 (2001)). The PCR products were spotted on type VII glass-slides (Amersham Biosciences) with Microarray Spotter Generation III (Amersham Biosciences). 4,608 genes were spotted in duplicate on a single slide. Five different sets of slides were prepared (total 23,040 genes), each spotted with the same 52 housekeeping genes and two negative-control genes.

### (4) Hybridization and acquisition of data

Hybridization, washing and detection of signals were carried out as described previously (Yanagawa et al., Neoplasia 3: 395-401 (2001)). The fluorescence intensities of Cy5 (tumor) and Cy3 (control) for each target spot were adjusted so that the mean Cy3/Cy5 ratio of the 52 housekeeping genes was equal to one. Data derived from low signal intensities are less reliable. Therefore, a cut-off value for signal intensities on each slide was determined. Genes were excluded from further analysis when both Cy3 and Cy5 dyes gave signal intensities lower than the cut-off.

### (5) Cluster analysis of 37 NSCLCs according to gene-expression profiles

A hierarchical clustering method to both genes and tumors was applied. To obtain reproducible clusters for classification of the 37 samples, 899 genes for which valid data had been obtained in 95% of the experiments, and whose expression ratios varied by standard deviations of more than 1.0 were selected. The analysis was performed using web-available software ("Cluster" and "TreeView") written by M. Eisen (http://genome-www5.stanford.edu/ MicroArray/SMD/restech.html). Before applying the clustering algorithm, the fluorescence ratio was log-transformed for each spot and then the data was median-centered for each sample to remove experimental biases.

### [Example 2] Identification of genes with clinically relevant expression patterns in non-small cell lung cancer cells

A two-dimensional hierarchical clustering algorithm was applied to analyze similarities among samples and genes, using data obtained from the expression profiles of all 37 NSCLC samples. Genes were excluded from further analysis when Cy3- or Cy5-fluorescence intensities were below the cut-off value, as described previously (Yanagawa et al., Neoplasia 3: 395-401 (2001)) and selected for which valid values could be obtained in more than 95% of the cases examined. Genes with observed standard deviations of <1.0 were also excluded. 899 genes that passed through this cutoff filter were further analyzed.

In the sample axis (horizontal), 39 samples (two cases were examined in duplicate to validate the reproducibility and reliability of the experimental procedure) from 37 cases were clustered into two major groups based on their expression profiles. The dendrogram represents similarities in the expression patterns among individual cases. The shorter the branches, the greater are the similarities. The two duplicated cases (Nos. 6 and 12) that were labelled and hybridized in independent experiments were clustered most closely within the same group. According to the analyses, the genes were clustered into adjacent rows of identical genes that were spotted on different positions of slide glasses (data not shown). Of the 37 cases, the 22 adenocarcinomas clustered into one major group and the 14 SCCs clustered into another. The single adenosquamous cell carcinoma (No. 25) fell into the SCC cluster. Clearly, adenocarcinoma and SCC appeared to have specific and different gene expression profiles that may disclose the molecular nature of etiological differences.

To search for down-regulated genes in NSCLCs, genes whose expression were decreased by < 0.2-fold or lower in more than 70% of NSCLCs were screened. 806 down-reaulated genes in NSCLCs were identified, which might have tumor suppressive function and thus may be potentially used for future gene therapy (see, Table1). In total, 582 up-regulated genes with Cy5/Cy3 ratios greater than 5.0 in more than 50% of NSCLCs (Table2) were identified. In the Tables, genes showing 5-fold expression in more than 70% of NSCLCs are potential diagnostic markers and those with 5-fold overexpression in more than 50% of cases are potential targets for drugs. As targets for drugs, genes for which data were present between 33%-50% of the cases were also selected and 60 genes which showed 5-fold higher expression in more than 90% of those NSCLCs were additionally determined (Table3). The criteria for further selection were as follows: (1) tumor markers detectable in serum: genes that showed expression only in human testis, ovary and 4 fetal tissues; (2) tumor markers detectable in sputum: genes that showed no expression in the tissues of airway (i.e., lung, trachea and salivary gland); and (3) therapeutic targets: genes that showed no expression in human vital organs like liver and kidney. The data of normal tissue distribution of these genes were obtained from the expression profiles in 25 adult and 4 fetal human tissues by means of a cDNA microarray containing 23,040 human genes.

### [Example 3] Identification and characterization of molecular targets for inhibiting non-small cell lung cancer cell growth

To identify and characterize new molecular targets that regulate growth, proliferation and survival of cancer cells, antisense S-oligonucleotide technique was applied to select target genes as follows.

### (1) Identification of full-length sequence

The full-length sequence of the genes that showed high signal intensity ratios of Cy5/Cy3 on the microarray was determined by database screening and 5' rapid amplification of cDNA ends using Marathon cDNA amplification kit (BD Biosciences Clontech, Palo Alto, CA, USA) according to the supplier's recommendations. A cDNA template was synthesized from human testis mRNA (BD Biosciences Clontech) with a gene-specific reverse primer and the AP1 primer supplied in the kit. Nucleotide sequences were determined with ABI PRISM 3700 DNA sequencer (Applied Biosystems, Foster City, CA, USA) according to the manufacturer's instructions.

### (2) Northern blot analysis

³²P-labeled PCR products corresponding to each of the genes selected for investigation on the microarray were hybridized with human multiple-tissue blots (BD Biosciences Clontech). Prehybridization, hybridization and washing were performed according to the supplier's recommendations. The blots were autoradiographed on intensifying screens at -80°C for 24-168 hours.

To determine tissue distribution and the size of each of the genes, human multiple tissue northern blot analysis was performed using human cDNA as a probe (Fig 4), and following results were obtained for respective genes:
NSC 807: a single 4.4kb mRNA was found in placenta and testis;
NSC 810: a single 3.1kbmRNA was found in testis;
NSC 811: 2.4 and 2.7kb mRNAs were found in placenta and tongue, and weak expression was detected in kidney, liver, adrenal gland, bladder, brain (whole), lymphnode, prostate, stomach, thyroid and trachea;
NSC 822: a single 1.3kb mRNA was found in heart, liver and testis;
NSC 825: a single 4.3kb mRNA was found in testis and spinal cord;
NSC 841: a weak expression of a transcript of 2.8kb was found in heart, adrenal gland, brain (whole), lymphnode, spinal code, stomach, thyroid, tongue and trachea;
NSC 849: a single 1.4kb mRNA was found in placenta, prostate and trachea;
NSC 855: a 3.6kb mRNA was found in placenta, prostate and trachea;
NSC 859: a weak expression of a transcript of 2.1kb was found in skeletal muscle and lymphnode;
NSC 885: a single 5.0kb mRNA was found in testis;
NSC 895: a single 1.5kb mRNA was found in placenta, stomach and trachea;
NSC 903: a single 2.7kb mRNA was found in testis, and weak expression was detected in thymus, small intestine, colon and bone marrow;
NSC 904: a single 4.4kb mRNA was found in testis and skeletal muscle;
NSC 905: a single 2.5kb mRNA was found in heart, skeletal muscle, liver, stomach and tongue, and weak expression was detected in placenta and thyroid;
NSC 915: a single 1.5kb mRNA was found in testis;
NSC 948: a single 3.8kb mRNA was found in kidney, liver, placenta, stomach, thyroid, tongue and trachea;
NSC 956: a single 2.1 kb mRNA was found in heart, skeletal muscle, testis, stomach, thyroid and adrenal grand, and weak expression was detected liver, pancreas, thymus, prostate and spinal code;
NSC 994: a single 3.3kb mRNA was found in skeletal muscle and testis, and weak expression was detected in heart, liver and pancreas;
NSC 1000: a single 3.5kb mRNA was found in brain, pancreas, prostate and testis, and weak expression was detected in stomach, spinal cord and adrenal grand;
NSC 1066: a single 3.6kb mRNA was found in skeletal muscle and testis;
NSC 1075: a single 1.9kb mRNA was found in testis;
NSC 1107: a single 2.2kb mRNA was found in testis;
NSC 1131: transcripts of 1.6 and 1.4kb were found in testis;
NSC 1141: a single 2.9kb mRNA was found in placenta, and weak expression of the transcript was detected in skeletal muscle and testis;
NSC 1164: a single 5.2kb mRNA was found in brain and adrenal grand;
NSC 1183: a single 2.0kb mRNA was found in skeletal muscle and heart;
NSC 1201: a weak expression of a transcript of 7.8kb was found in heart, skeletal muscle, spinal code, prostate, testis, thyroid, spleen, lymphnode, trachea and adrenal gland;
NSC 1240: a weak transcript of 5.7kb was found in stomach, spinal code and lymphnode;
NSC 1246: a single 1.4kb mRNA was found in testis;
NSC 1254: a single 3.0kb mRNA was found in testis;
NSC 1265: a weak expression of a transcript of 3.0kb was found in stomach;
NSC 1277: a single 1.8kb mRNA was found in testis;
NSC 1295: a single 3.5kb mRNA was found in leukocyte, lymphnode and bone marrow;
NSC 1306: a single 7.4kb mRNA was found in heart and skeletal muscle;
NSC 1343: a single 4.7kb mRNA was found in placenta and skeletal muscle;
NSC 1362: a single 3.6kb mRNA was found in brain and whole brain;
NSC 1389: a single 0.9kb mRNA was found in tongue;
NSC 1399: a single 0.9kb mRNA was found in placenta;
NSC 1406: a single 2.4kb mRNA was found in heart, skeletal muscle and prostate;
NSC 1413: a single 4.0kb mRNA was found in liver and prostate;
NSC 1420: a single 2.8kb mRNA was found in testis.

### (2) Semiquantitative RT-PCR analysis

The increase in the expression level of mRNAs by 5-fold or more in more than 50% of NSCLCs was confirmed by semiquantitative RT-PCR as described previously (Akashi et al. Int J Cancer 88: 873-80 (2000)). Total RNA was extracted from cultured cells and clinical tissues using Trizol reagent (Life Technologies, Inc., Gaithersburg, MD, USA) according to the manufacturer's protocol. Extracted RNA was treated with DNase I (Roche Diagnostics, Basel, Switzerland) and reverse transcribed to single-stranded cDNAs using oligo(dT)12-18 primer with Superscript II reverse transcriptase (Life Technologies, Inc.). Appropriate dilutions of each single-stranded cDNA for subsequent PCR amplification were prepared by monitoring the beta-actin (*ACTB*) or beta-2-microglobulin gene (*B2M*) as the quantitative controls. All reactions involved initial denaturation at 94 °C for 2 min, followed by 18 (for *ACTB* or *B2M*) or 25-30 cycles (for each gene of the present invention) of 94 °C for 30s, 58-62 °C for 30s and 72 °C for 45s on GeneAmp PCR system 9700 (Applied Biosystems). The primer sequences are listed in Table 4.

**Table4; Primer sequences for semi-quantitative RT-PCR experiments**

| **NSC Assign ment** | **Symbol** | | **RT-PCR primer** | **SEQ ID NO:** |
|---|---|---|---|---|
| 807 | KOC1 | F | 5'-TAAATGGCTTCAGGAGACTTCAG-3' | 3 |
| | | R | 5-GGTTTTAAATGCAGCTCCTATGTG-3' | 4 |
| 810 | TTK | F | 5'-ATGGAATCCGAGGATTTAAGTGGCAGAGAATTGA-3' | 5 |
| | | R | 5'-TTTTTTTCCCCTTTTTTTTTCAAAAGTCTTGGAGGAT-3' | 6 |
| 811 | SDC1 | F | 5'-GCTTCCTCCTGGAAATTGAC-3' | 7 |
| | | R | 5'-TCTACTGTACAGGGAAAAACCCA-3' | 8 |
| 812 | NMB | F | 5'-AGTCGTGGTTCAGAAGTTACAGC-3' | 9 |
| | | R | 5'-TCTCTTACCAAATGCTGTTGAGC-3' | 10 |
| 816 | PIR51 | F | 5'-CATCTGGCATTTCTGCTCTCTAT-3' | 11 |
| | | R | 5'-CTCAGGGAAAGGAGAATAAAAGAAC-3' | 12 |
| 820 | HMMR | F | 5'-GAAGTATCAAAACTCCGCTGTCA-3' | 13 |
| | | R | '-ATGCTGAGTAGACATGCAGATGA-3' | 14 |
| 822 | | F | 5'-CGGTATGCTAATGAAGATGGAGA-3' | 15 |
| | | R | 5'-CACAGGGTATCAGCAACTGTGTA-3' | 16 |
| 824 | BPAG1 | F | 5'-AGAAGTATCTGAGCCCCTGATG-3' | 17 |
| | | R | 5'-GTCTAACCTCCCAGCTGTTCC-3' | 18 |
| 825 | ANLN | F | 5'-GCTGCGTAGCTTACAGACTTAGC-3' | 19 |
| | | R | 5'-AAGGCGTTTAAAGGTGATAGGTG-3' | 20 |
| 830 | | F | 5'-GTTTGCAACCAGGAGATACAAAG-3' | 21 |
| | | R | 5'-GCTGTGAGGTACAACAAATCACA-3' | 22 |
| 837 | | F | 5'-CCTCCTTTCCCTAGAGACTCAAT-3' | 23 |
| | | R | 5'-AGAAGCAACAGCAAGACCACTAC-3' | 24 |
| 840 | GNAS | F | 5'-TTGCCTATGAAAGATAGGTCCTG-3' | 25 |
| | | R | 5'-GTTTTAATGCCCAGATAGCACAG-3' | 26 |
| 841 | URLC2 | F | 5'-AGGAGAAGTTGGAGGTGGAAA-3' | 27 |
| | | R | 5'-CAGATGAAAGATCCAAATTCCAA-3' | 28 |
| 842 | KIAA0887 | F | 5'-TCCACGACTTCTTATTCTCCTTG-3' | 29 |
| | | R | 5'-CATTTCTTTTAGGGACTGGGGTA-3' | 30 |
| 846 | | F | 5'-GAGAAACTGAAGTCCCAGGAAAT-3' | 31 |
| | | R | 5'-CTGATACTTCCATTCGCTTCAAC-3' | 32 |
| 849 | GJB5 | F | 5'-AGCTAAGCCATGAGGTAGGG-3' | 33 |
| | | R | 5'-CGCATGTGTGTTCTTCTATGA-3' | 34 |
| 850 | | F | 5'-CCAAGACAGGCAGAGTAGGTAAA-3' | 35 |
| | | R | 5'-CATTTTCATTGTGATCAGCCAG-3' | 36 |
| 853 | | F | 5'-TGTATGGGGGATTACCTACACAC-3' | 37 |
| | | R | 5'-AAAGGAGCACAACAAACATGC-3' | 38 |
| 854 | | F | 5'-TGTCCAAGGAGTCTGAAGTTCTC-3' | 39 |
| | | R | 5'-CTTGCCACCATACCTTTATTCTG-3' | 40 |
| 855 | LNIR | F | 5'-CGAGAGAGTAGGAGTTGAGGTGA-3' | 41 |
| | | R | 5'-CAGAAATCCAGCAGATTTCAGAC-3' | 42 |
| 857 | TIGD5 | F | 5'-GAACAGGTGGCTGTGTTCCT-3' | 43 |
| | | R | 5'-ATAGAATCAAGTGGTGTGCTTCG-3' | 44 |
| 859 | URLC3 | F | 5'-CTGAGACTTTGAGTCCTTGGGAG-3' | 45 |
| | | R | 5'-TTCCTCATTTCTCTCAGTAACCG-3' | 46 |
| 861 | KIAA0251 | F | 5'-AACAATGCAAAGTAGTGCTCCTC-3' | 47 |
| | | R | 5'-GCTGAACTTCTTTATGCTCTTCG-3' | 48 |
| 864 | R | F | 5'-ACCTTTGATTTTAGACTGAGGGC-3' | 49 |
| | | R | 5'-ACACTGGGTTGTGTGTTATTTCC-3' | 50 |
| 870 | | F | 5'-ATGAGCCTCTCATCCATGTCTTT-3' | 51 |
| | | R | 5'-AGTAAGAGTCTGCCTGAGACACG-3' | 52 |
| 871 | KIAA1929 | F | '-AGAAAATGGGGGTGCAAGTAG-3' | 53 |
| | | R | 5'-TAACCAAATTAACACGTGCTGG-3' | 54 |
| 872 | LOC51659 | f | 5'-AGAAAAGTTGGAGAAGATGAGGG-3' | 55 |
| | | R | 5'-GCCACCTCTGTGAGAGAGTCTAA-3' | 56 |
| 881 | FLJ20068 | F | 5'-AGAACTAGTGTGACCCCACCC-3' | 57 |
| | | R | 5'-GCTTGCCTTTTCCCTTAGTAGG-3' | 58 |
| 882 | GUCY1B2 | F | 5'-AGGGAAATGAAGACAGGAGAACT-3' | 59 |
| | | R | 5'-GAGACACGGCTTAAGAAGTTTTG-3' | 60 |
| 884 | RAD51 | F | 5'-GCTTGTAAAGTCCTCGGAAAGTT-3' | 61 |
| | | R | 5'-ATCTCAACTCTGCATCATCTGGT-3' | 62 |
| 885 | BAG5 | F | 5'-ATAAGAGAAATATTGGCCATCG-3' | 63 |
| | | R | 5'-GCAAGCGTAAGAGACTGGTTTTA-3' | 64 |
| 889 | HSPC150 | F | 5'-CAAATATTAGGTGGAGCCAACAC-3' | 65 |
| | | R | 5'-TAGATCACCTTGGCAAAGAACAC-3' | 66 |
| 892 | | F | 5'-ACACACAGAGAGGAGGAAGTCT-3' | 67 |
| | | R | 5'-GAGTCTTTATGGAGCTGTGTCA-3' | 68 |
| 893 | MPHOSPH 1 | F | 5'-CAGGCCAAGTGATTTTAATGG-3' | 69 |
| | | R | 5'-CAATACAGGATGCAAGTTCCAA-3' | 70 |
| 895 | FAM3D | F | 5'-ACAGCCCAGACACAAACAAATAC-3' | 71 |
| | | R | 5'-ACCCCATTCTCTCCACAGAC-3' | 72 |
| 896 | PRO0971 | F | 5'-TACAGGCCAGGATAGAAACACTC-3' | 73 |
| | | R | 5'-GTTCAAATATTGAAAGGGCCAC-3' | 74 |
| 898 | URLC7 | F | 5'-AGTTATGGGTTCCTGTGTGCTTA-3' | 75 |
| | | R | 5'-AAAGGCCTGTTCACAAGCTAAGT-3' | 76 |
| 901 | MAN1B1 | F | 5'-CTCGTGAAGCCTCAGATGTCC-3' | 77 |
| | | R | 5'-CTCCACCGAAAAGACCCATTC-3' | 78 |
| 902 | ALDOC | F | '-AGCGTACACCCTGTGCACTTG-3' | 79 |
| | | R | 5'-TTTGCTGTATGGTATGTACTCAAGG-3' | 80 |
| 903 | URLC9 | F | 5'-CAGAAGAGAGAGGAGAGAACACG-3' | 81 |
| | | R | 5'-GAGGTTATCTCTGATGGAACCAA-3' | 82 |
| 904 | | F | 5'-CTTGAAGAAGAACTTCCAGACGA-3' | 83 |
| | | R | 5'-AATGTTCTAAAGATGAGAGGGGG-3' . | 84 |
| 905 | URLC1 | F | 5'-AGGAGGCTGCTGGTACAAATACT-3' | 85 |
| | | R | 5'-GCAGGAAATACAGCAGGAACATA-3' | 86 |
| 909 | FLJ10468 | F | '-ATTCATTCTGGACCAAAGATCC-3' | 87 |
| | | R | 5'-TCTACTGTGGACAAGAAGCCTGT-3' | 88 |
| 912 | SRD5A1 | F | 5'-GTGATCTCTTCAAGGTCAACTGC-3' | 89 |
| | | R | 5'-CCAGATGAGATGATAAGGCAAAG-3' | 90 |
| 915 | URLC10 | F | 5'-ATTCGCTACTGCAATTTAGAGG-3' | 91 |
| | | R | 5'-GTTTAATGCAACAGGTGACAACG-3' | 92 |
| 917 | KIAA1096 | F | 5'-CACTTGGATTCCTTGCTTGTTAC-3' | 93 |
| | | R | 5'-GGGAAAAAGTATGCAACACTCAG-3' | 94 |
| 920 | CHAF1A | F | 5'-AGGCGATGACCTGAAGGTACTG-3' | 95 |
| | | R | 5'-CAATAGGCCAGCAATCTCAATA-3' | 96 |
| 921 | AKR1B11 | F | 5'-AGGTTCTGATCCGTTTCCATATC-3' | 97 |
| | | R | 5'-ATCTTTACATCCTCAGTGTTGGC-3' | 98 |
| 924 | | F | 5'-GAAGACAAATGGTGTCCACAAA-3' | 99 |
| | | R | 5'-CCACTGGAAGTTTTCTTCGTACA-3' | 100 |
| 929 | KIAA0101 | F | 5'-TTCGTTCTCTCCTCTCCTCTCTT-3' | 101 |
| | | R | 5'-GGCAGCAGTACAACAATCTAAGC-3' | 102 |
| 930 | | F | 5'-CAGCACAGAGTAGGTGAACACAG-3' | 103 |
| | | R | 5'-CCTCAGTACATTTTCAACCCATC-3' | 104 |
| 933 | | F | 5'-AGGATGATGAGGATGACTGAAGA-3' | 105 |
| | | R | 5'-GAATGGGCCTCTATCTGGTATCT-3' | 106 |
| 934 | CIT | F | 5'-TGTGTCTCATCTGTGAACTGCTT-3' | 107 |
| | | R | 5'-TTCGTGTTACGGTATATCCTGCT-3' | 108 |
| 936 | AF15Q14 | F | 5'-CTTCTGTTCCGTAAACTCCTTGA-3' | 109 |
| | | R | 5'-CAATTGTGTACTCCAAACCCAA-3' | 110 |
| 938 | FLJ13852 | F | 5'-GCCCTTCCAACTTGTCCTTAAC-3' | 111 |
| | | R | 5'-GCCTCTTTATTCCCATCTCCTTA-3' | 112 |
| 940 | KIAA1443 | F | 5'-GAACAGATCACTGGTTTACCTCG-3' | 113 |
| | | R | 5'-ATCTTTCAGTAACAGACCTCCCC-3' | 114 |
| 944 | | F | 5'-ACAAGATGGCTAGCTCAAAAGTG-3' | 115 |
| | | R | 5'-CAACACGTGGTGGTTCTAATTT-3' | 116 |
| 947 | PKP3 | F | 5'-ATGCAGGACGGTAACTTCCTGC-3' | 117 |
| | | R | 5'-TGGGCCCAGGAAGTCCTCCTT-3' | 118 |
| 948 | KCNK5 | F | 5'-CCCAACATGTGAAGACAGTGAT-3' | 119 |
| | | R | 5'-CCTGTCCACCTCATGTTTTATTG-3' | 120 |
| 956 | SIAHBP1 | F | 5'-GCTGAAGTGTACGACCAGGAG-3' | 121 |
| | | R | 5'-CACCTTTATCCGCACTGTAGG-3' | 122 |
| 957 | | F | 5'-AAAGCTGATGAGGACAGACCAG-3' | 123 |
| | | R | 5'-GGCAGAGGCACAATCATTTTAG-3' | 124 |
| 958 | | F | 5'-GAAGAGAATGCAGGTGTTGAGTT-3' | 125 |
| | | R | 5'-GTCCACAGCATTCATAAAACAGG-3' | 126 |
| 963 | | F | 5'-CTCCTCAGTGTCCACACTTCAA-3' | 127 |
| | | R | 5'-GTTACTTGCAGCCAAAAGCAG-3' | 128 |
| 964 | AK3 | F | 5'-AGTCTCTCCTTTTCAGACATCCC-3' | 129 |
| | | R | 5'-TCCATAAAGTCAGACCAGCAGTT-3' | 130 |
| 965 | ENC1 | F | 5'-CCTTCTGGGAGGACAGACTTT-3' | 131 |
| | | R | 5'-TTTCTCTTCATTAGACTTGGCCTCT-3' | 132 |
| 966 | | F | 5'-AACCTAGCCTCCCTTCAAACTTA-3' | 133 |
| | | R | 5'-GAGACAGGATGGAAAAATCTGTG-3' | 134 |
| 970 | | F | 5'-CCTTTCCTGACCCTTTTAGTCTT-3' | 135 |
| | | R | 5'-CAAATCCTGTATTTCTCACAGGC-3' | 136 |
| 972 | LOC51690 | F | 5'-GAAAAAGGAGAGCATCTTGGACT-3' | 137 |
| | | R | 5'-AAAGGAAAATGCTTCCGTTCC-3' | 138 |
| 973 | HAS3 | F | 5'-TAATGTAGGATGACAGGCTCTCC-3' | 139 |
| | | R | 5'-CCAATTGTATAAAGGCTCTTCCC-3' | 140 |
| 974 | PYCR1 | F | 5'-AGGACAACGTCAGCTCTCCTG-3' | 141 |
| | | R | 5'-TCCACTATTCCACCCACAGTAAC-3' | 142 |
| 975 | FLJI2517 | F | 5'-GACCGAGAGTCCAGCATTTTT-3' | 143 |
| | | R | 5'-ACTGAACAGAGCAGACAGAAACC-3' | 144 |
| 980 | ANKT | F | 5'-CTGCTGTTATTACCCCATTCAAG-3' | 145 |
| | | R | 5'-GTGAGTGACAGATGGCAATTACA-3' | 146 |
| 984 | MLL3 | F | 5'-CTCGGGTAGAATTTGATGACAAC-3' | 147 |
| | | R | 5'-GCTGGTAAAGCAGGTGTAAAAGA-3' | 148 |
| 989 | FOXM1 | F | 5'-CCCTGACAACATCAACTGGTC-3' | 149 |
| | | R | 5'-GTCCACCTTCGCTTTTATTGAGT-3' | 150 |
| 990 | WFDC2 | F | 5'-CTCTCTGCCCAATGATAAGGAG-3' | 151 |
| | | R | 5'-GAAACTTTCTCTCCTCACTGCTC-3' | 152 |
| 991 | DOLPP1 | F | 5'-CAGAAGTTTTGAGGACTGAACTG-3' | 153 |
| | | R | 5'-CCGACCTACCTTCCCTAGAAAT-3' | 154 |
| 994 | DKFZp43 4E2318 | F | 5'-GGGGTTTTGAAGGATGTGTACTT-3' | 155 |
| | | R | 5'-TATGAGGCCATTCTGCACATTA-3' | 156 |
| 1000 | ASK-1 | F | 5'-GGGAGTATGAAGTTTCCATCTG-3' | 157 |
| | | R | 5'-GGATGCTGGTTTATTTACTGTAGG-3' | 158 |
| 1002 | LOC55565 | F | 5'-AATATGGAATCCCTACCCACAGT-3' | 159 |
| | | R | 5'-TTTGACTTCACAACTTCATGGG-3' | 160 |
| 1003 | | F | 5'-GAGGCCATTTTAGTTCTGAGGTT-3' | 161 |
| | | R | 5'-CTTTACTGCATATGGATTCTGGG-3' | 162 |
| 1004 | BUB1B | F | 5'-TCAACCTCAAGTTAAAGGAACG-3' | 163 |
| | | R | 5'-AGGGAAAAGTAGAGACAAATGGG-3' | 164 |
| 1005 | | F | 5'-TCTAGGCAAAGTGGAAGTCAAAG-3' | 165 |
| | | R | 5'-CTCCTAGAGAAATGGGTTGGATT-3' | 166 |
| 1012 | FLJ12428 | F | 5'-ATACACTGAATGTGGAAGAACCG-3' | 167 |
| | | R | 5'-GGGCACACAATTTCATGTAGTCT-3' | 168 |
| 1015 | PHB | F | 5'-AGACATTGCATACCAGCTCTCAT-3' | 169 |
| | | R | 5'-CCTTTACTTCCTTCACTTTAAGCC-3' | 170 |
| 1016 | | F | 5'-GTAACAAACGCCACCTTACACTC-3' | 171 |
| | | R | 5'-TTCTGTTCTTGCAACTGAGTCGT-3' | 172 |
| 1018 | | F | 5'-ACCTCCAGTAAAAGTTTCTTCCG-3' | 173 |
| | | R | 5'-GTAAATTCAGCTTCAAACCCTGG-3' | 174 |
| 1023 | CLDN2 | F | 5'-CATTGAGCCTTCTCTGATCACTC-3' | 175 |
| | | R | 5'-GCACTGTTACAGATAG-TCTGGGG-3' | 176 |
| 1026 | | F | 5'-TATCAGTAACTGCTCCGTGTTCA-3' | 177 |
| | | R | 5'-GGTCTGTCATTGACCAAAACATC-3' | 178 |
| 1027 | | F | 5'-TCCTGAATAAAGGCCTAGTACCC-3' | 179 |
| | | R | 5'-AAACCAGAATCCAACACTACCCT-3' | 180 |
| 1030 | | F | 5'-GAGCCCTCTCCACATTTCTATTT-3' | 181 |
| | | R | 5'-ACACTGAAACGTGATGGGTAACT-3' | 182 |
| 1034 | SSBP | F | 5'-GACATGAGTCCGAAACAACTACC-3' | 183 |
| | | R | 5'-ATGAGACTGTACCAAATGATGGC-3' | 184 |
| 1037 | CSTA | F | 5'-TGATACCTGGAGGCTTATCTGAG-3' | 185 |
| | | R | 5'-GACTCAGTAGCCAGTTGAAGGAA-3' | 186 |
| 1038 | CLDN1 | F | 5'-TCTTGCAGGTCTGGCTATTTTAG-3' | 187 |
| | | R | 5'-TATATTTAAGGAGCACCGCTTCC-3' . | 188 |
| 1047 | SLC7A5 | F | 5'-ACAAGCAAGTGCATTTTCAGTC-3' | 189 |
| | | R | 5'-GAACAGGGTAGCCATTAACACAA-3' | 190 |
| 1049 | S100A8 | F | 5'-TCTATCATCGACGTCTACCACAA-3' | 191 |
| | | R | 5'-GCTACTCTTTGTGGCTTTCTTCA-3' | 192 |
| 1052 | S100P | F | 5'-GCATGATCATAGACGTCTTTTCC-3' | 193 |
| | | R | 5'-GATGAACTCACTGAAGTCCACCT-3' | 194 |
| 1057 | FDXR | F | 5'-TCTCCAGGACAAGATCAAGGA-3' | 195 |
| | | R | 5'-GTTTTATTTCCAGCATGTTCCC-3' | 196 |
| 1058 | FEN1 | F | 5'-AGAGCTGATCAAGTTCATGTGTG-3' | 197 |
| | | R | 5'-ACATAGCAAGTTCGAGTTTCTGC-3' | 198 |
| 1059 | TCF19 | F | 5'-GAGCTGGAGGTAGGAATACAGGT-3' | 199 |
| | | R | 5'-CAATAGTTTGGCTTGGTGTAAGG-3' | 200 |
| 1064 | PAFAH1B3 | F | 5'-CTCCTCTGCATGCACTTAACTTT-3' | 201 |
| | | R | 5'-GAGAGTTTAATGTTGTGGGAAGG-3' | 202 |
| 1066 | MCM8 | F | 5'-CCGGGCAATAAAGTAACTCTTG-3' | 203 |
| | | R | 5'-GTATTTGTCTGTATGCCTACATCTG-3' | 204 |
| 1067 | FLJ10052 | F | 5'-TCTGCGTATCTTGAGTGCTTACA-3' | 205 |
| | | R | 5'-ACAGAGATGTGGTGGTGCTAGTT-3' | 206 |
| 1071 | | F | 5'-AGCAGAGGATCAGAGCTTTCTTT-3' | 207 |
| | | R | 5'-AGAAAAGGTGTGAACAGAGTTGC-3' | 208 |
| 1072 | FLJ13163 | F | 5'-AGAGCCATAGAAACTGCTCCTCT-3' | 209 |
| | | R | 5'-CATAACTGCATAGACAGCACGTC-3' | 210 |
| 1075 | URLC4 | F | 5'-TACCTGCTCTATGTGGGTGCT-3' | 211 |
| | | R | 5'-CCTCAGAACTCTCAGTTTATTCCTG-3' | 212 |
| 1077 | | F | 5'-ATAAGCCACAGAGACAAACCAGA-3' | 213 |
| | | R | 5'-GGGAGGTTATTTTCACAGAACAC-3' | 214 |
| 1078 | UBCH10 | F | 5'-GAGTTCCTGTCTCTCTGCCAAC-3' | 215 |
| | | R | 5'-TAATATACAAGGGCTCAACCGAG-3' | 216 |
| 1086 | TCF20 | F | 5'-GTCATAGCTGTGTCCTGGGTC-3' | 217 |
| | | R | 5'-CTATTTTATCCCCATGGCAGAGT-3' | 218 |
| 1089 | KLkAO902 F | F | 5'-CAGATATTCTGTATGCTGGAGGG-3' | 219 |
| | | R | 5'-CCATCTCAGAAGGGCTTTATTTC-3' | 220 |
| 1090 | LOC51243 | F | 5'-GATTTCCATACTTCGGGAGAAAC-3' | 221 |
| | | R | 5'-TATCAGATGCCACACATACGAGA-3' | 222 |
| 1103 | KCNK1 | F | 5'-ATGGAACAAAGAAGCTGTGACC-3' | 223 |
| | | R | 5'-GGGTACATGCAAACCAGTACAC-3' | 224 |
| 1107 | URLC8 | F | 5'-TGAACAGTTTGCTGGTCTTG-3' | 225 |
| | | R | 5'-AATGTCAGGTTGGGGAGTTA-3' | 226 |
| 1109 | ZNF259 | F | 5'-TTCTGGACAGACGGAGAGAGTAC-3' | 227 |
| | | R | 5'-AGTGATGACATACCCCTGGTTC-3' | 228 |
| 1113 | URLC5 | F | 5'-CAAGACTTCTCAGATCCTTGGG-3' | 229 |
| | | R | 5'-ACTCACATGTGGAAGTGTTCCTT-3' | 230 |
| 1116 | KIAA0832 | F | 5'-TCAAGCAATATGAAGTAGGGCTC-3' | 231 |
| | | R | 5'-AACACAAATGTCCCGTGTAAGTC-3' | 232 |
| 1121 | | F | 5'-CTGCGTCTTACTCGTCACAGTTT-3' | 233 |
| | | R | 5'-TGACTTCTTTGAAGTGAAGGCT-3' | 234 |
| 1125 | LOC51256 | F | 5'-CCCTAGTTTTTGTAGCTGTCGAA-3' | 235 |
| | | R | 5'-GATCACATGCCAAGAACACAAT-3' | 236 |
| 1131 | SYNJ2BP | F | 5'-CTACGTACCTGGGTGCCTATATC-3' | 237 |
| | | R | 5'-GTCCTCTTATAAGGCTCACTCCC-3' | 238 |
| 1133 | | F | 5'-GATGTTAGAGACTCCTTCACCCA-3' | 239 |
| | | R | 5'-CGGTATTCTTAACACATCTTGCC-3' | 240 |
| 1136 | TRAF2 | F | 5'-GTGTCTGCGTATCTACCTGAACG-3' | 241 |
| | | R | 5'-ATAACTCTGTCTTCGTGAGCTGG-3' | 242 |
| 1141 | URLC11 | F | 5'-GTATTTGGCTTACTGTCCCAAAC-3' | 243 |
| | | R | 5'-CTAGGAAGAAATCATGCTGGGTT-3' | 244 |
| 1142 | NAPG | F | 5'-CAGTTTGAGCAAGCAAAAGATG-3' | 245 |
| | | R | 5'-CGGATATCCCTAATCTATTCCCA-3' | 246 |
| 1157 | NINJ2 | F | 5'-GACAGTATAGCTGCCCTTGCTC-3' | 247 |
| | | R | 5'-AAGCAGTGGGGTAGAGTCAGAAC-3' | 248 |
| 1162 | IMP-2 | F | 5'-ACAGAAGAAGCTACCTCAGGTGT-3' | 249 |
| | | R | 5'-CTAGCGGAAGACAATTCAGAAC-3' | 250 |
| 1164 | NPTX1 | F | 5'-TAACCTTGATAGAAGAACCTTGG-3' | 251 |
| | | R | 5'-GCAAATGAGACAAAATTGGGAC-3' | 252 |
| 1167 | DKFZp762 M136 | F | 5'-ATCTCCACTCTACGGCCTTTTAC-3' | 253 |
| | | R | 5'-TAATGACTTAAACACCAGCACGG-3' | 254 |
| 1169 | FLJ12892 | F | 5'-GTGTTCTCCTAATCCCAGAACCT-3' | 255 |
| | | R | 5'-AAGAGTTGTGGCCTATTACCTCC-3' | 256 |
| 1173 | | F | 5'-TGGTCCTACTAAGAGAATGCAGC-3' | 257 |
| | | R | 5'-AGCCATTAGGAAAAAGAGCAGAG-3' | 258 |
| 1176 | RANBP7 | F | 5'-GACTGCTATACTCCAACTCTGGG-3' | 259 |
| | | R | 5'-GCCAAAGACATGGTTTAGTCATAC-3' | 260 |
| 1183 | BYSL | F | 5'-ACACTGAGCTTTAATGGCTGAAG-3' | 261 |
| | | R | 5'-TCCACAGTGACCTGACACAATAG-3' | 262 |
| 1184 | SURF6 | F | 5'-GTCCTCATTCCCTTTCTCTTTCC-3' | 263 |
| | | R | R 5'-CTGTTTTCTTTCAACCTGCACTC-3' | 264 |
| 1185 | URLC6 | F | 5'-AAGAGAGGCCAGAAACTGAGC-3' | 265 |
| | | R | 5'-AACTAGCAGCTTTATTGCCCTTC-3' | 266 |
| 1191 | COX17 | F | 5'-GTGGACATCTAATTGAGGCC-3' | 267 |
| | | R | 5'-GAAGATCTTCCACTAGTAATATT-3' | 268 |
| 1195 | LOC51250 | F | 5'-CAGAGGACTCTGATGAAGAAAGC-3' | 269 |
| | | R | 5'-TTTCCACAAAGGCTAAGAGAAC-3' | 270 |
| 1196 | | F | 5'-ATGTCTGCTCCGTGAGTGTCT-3' | 271 |
| | | R | 5'-GCAAATCCTACTTTCAACTGCAC-3' | 272 |
| 1201 | SLC7A1 | F | 5'-GCCTTAAAGCTGGACACAGAAG-3' | 273 |
| | | R | 5'-CTCCAGACACCATTGCTTAAATC-3' | 274 |
| 1205 | FLJ20657 | F | 5'-AGACTTTAAAATCCCACCTGGAC-3' | 275 |
| | | R | 5'-CACCCAGCCTTCTCTTTATTTTC-3' | 276 |
| 1207 | D19S1177E | F | 5'-AGGGGATTCTGGAACTGAATG-3' | 277 |
| | | R | 5'-T'TATACCGAGGAGATGGGAAAGT-3' | 278 |
| 1210 | | F | 5'-GTTGCAGTACCAATCCTTTCTTG-3' | 279 |
| | | R | 5'-GTCCTATGTTAATTTCCACCAAGC-3' | 280 |
| 1214 | DGSI | F | 5'-TATCCAGAGGGTGTCCCTGAC-3' | 281 |
| | | R | 5'-GTTCTTTAATGACAGTTCAAGGGG-3' | 282 |
| 1234 | | F | 5'-ATCGGATCGATATTACACAGCA-3' | 283 |
| | | R | 5'-CCCATCAGGGAAACAAAGATTA-3' | 284 |
| 1236 | HSPC135 | F | 5'-TGCATCTGTAACTTCAGGAGGAT-3' | 285 |
| | | R | 5'-TCCATCAACTTACCTATCGATGC-3' | 286 |
| 1237 | | F | 5'-AAACCTACGAACGCCTTTTCTAC-3' | 287 |
| | | R | 5'-GGTATCACAGGAGCACCAATAAA-3' | 288 |
| 1238 | FSP-2 | F | 5'-CTTTCTGTTGCTTTCCCAGTAGA-3' | 289 |
| | | R | 5'-TTGATACATTACACTGGTGGCAG-3' | 290 |
| 1240 | FLJ00159 | F | 5'-ACCCACAGAACTGGGAGTGAG-3' | 291 |
| | | R | 5'-ATTTTACTGCAGAAACGGGTTG-3' | 292 |
| 1242 | LRP6 | F | 5'-GATGGGGAAACTATGACTAATGAC-3' | 293 |
| | | R | 5'-GGTATCAATAAAGCCCAGATATTCC-3' | 294 |
| 1246 | SUPT3H | F | 5'-TTAGTGGATCTGGCTCTTCTTGT-3' | 295 |
| | | R | 5'-CAGGCACATCACAGTTGTCAC-3' | 296 |
| 1247 | MGC5585 | F | 5'-GATTTGGAACTTGGAAGGAGTAGTG-3' | 297 |
| | | R | 5'-ACTTCAGTCACCCAAAACAACAG-3' | 298 |
| 1250 | | F | 5'-CGGGAGGATTGTAAGATACTGTG-3' | 299 |
| | | R | 5'-ACTTCTCATGAGTTCAGCCTCAG-3' | 300 |
| 1254 | FLJ10815 | F | 5'-GTGAGTATTCCTCCGTTAGCTT-3' | 301 |
| | | R | 5'-CAGGGAGAAGAGAAAACATCAC-3' | 302 |
| 1265 | SLC28A2 | F | 5'-AGCTGAAGCTGACTGTGTCT-3' | 303 |
| | | R | 5'-AGGCACAGACGGTATTGTTGTAG-3' | 304 |
| 1271 | | F | 5'-GACTTTCAAACAACCCAGTGTCT-3' | 305 |
| | | R | 5'-CTCTAGCCAGCTTCTTCCTCAC-3' | 306 |
| 1273 | FIJ32549 | F | 5'-GGTCTTCATACGCTGTACTTGCT-3' | 307 |
| | | R | 5'-TATGCCTTCACTGATCCACCTAC-3' | 308 |
| 1277 | | F | 5'-TCCTGTGGAAATAGAACTGTCGT-3' | 309 |
| | | R | 5'-CACAAAGTTCAAGGAAGCAGTCT-3' | 310 |
| 1279 | TOM1 | F | 5'-AAGGTTCTCTACCGCCTCAAGT-3' | 311 |
| | | R | 5'-CTGAACACACCGTGGCTAT-3' | 312 |
| 1288 | PTGFRN | F | 5'-AAGAAGCCACCACTATTCCTCTC-3' | 313 |
| | | R | 5'-CCTGAAGGACTGAAAAGGTCATA-3' | 314 |
| 1289 | | F | 5'-CCTGTCTCCAAAGGAAAAACAA-3' | 315 |
| | | R | 5'-CTCAGTTTCATCAAGTCCTTTGC-3' | 316 |
| 1290 | GALNT2 | F | 5'-AGCGAGGAGAACTCTTGAAATC-3' | 317 |
| | | R | 5'-GTGTCCCACCATAGAAAACTTC-3' | 318 |
| 1292 | C17orf26 | F | 5'-GAAGCCAGCCTACTCCTTCTTAC-3' | 319 |
| | | R | 5'-TAGCATTCACAGAGCAGGAGATT-3' | 320 |
| 1293 | PPAT | F | 5'-CATATGTGGAGGTGCTGTGTAAA-3' | 321 |
| | | R | 5'-GTCTACAGTTAGACAGGGAAGCC-3' | 322 |
| 1294 | MED6 | F | 5'-GACAGGTCTTGGATCCCTATTTT-3' | 323 |
| | | R | 5'-AGAGTGAACTTGCATGTGTTCCT-3' | 324 |
| 1295 | ADAM8 R | F | 5'-GTGTGTGTACGTGTCTCCAGGT-3' | 325 |
| | | | 5'-CAGACAAGATAGCTGACTCTCCC-3' | 326 |
| 1299 | KIAA0720 | F | 5'-GAAGTCTGGGGGTGTTGGTCT-3' | 327 |
| | | R | 5'-ATAAAGACTTGTCTAGACTCCACTGGG-3' | 328 |
| 1302 | LOC51754 | F | 5'-GAACAGTGTTTGGTCTGGAATGT-3' | 329 |
| | | R | 5'-GGATATGAGAAAGGAAGGCAAGT-3' | 330 |
| 1306 | ABCA4 | F | 5'-ATCGTGAGCATCATCAGAGAAG-3' | 331 |
| | | R | 5'-AGACACACAGACAAACATGCAGA-3' | 332 |
| 1309 | | F | 5'-GCACTACCCAGACATCTTCGAG-3' | 333 |
| | | R | 5'-TGGGTGGCAAGTCTAATCTATTC-3' | 334 |
| 1310 | FJX1 | F | 5'-GATCCGAAGAAACTGGCTACTG-3' | 335 |
| | | R | 5'-AGGTCCTGCTCTCTTTOTCCTATi3' | 336 |
| 1315 | | F | 5'-GAGTCTTCCCCATTTTCAGTCAT-3' | 337 |
| | | R | 5'-CTACATTTATGTGGCACGAAGG-3' | 338 |
| 1320 | | F | 5'-CTTTGGCTTATTTACAGAGCTGG-3' | 339 |
| | | R | 5'-AGGAGGCTAAAGGCAATGAATAG-3' | 340 |
| 1321 | TXN | F | 5'-GAGTCTTGAAGCTCTGTTTGGTG-3' | 341 |
| | | R | 5'-AACATCCTGACAGTCATCCACAT-3' | 342 |
| 1323 | | F | 5'-AGTGTCTGCAACCTTGCTTTAAC-3' | 343 |
| | | R | 5'-AGTCCAGGGCATAAAACCTAAAC-3' | 344 |
| 1325 | FACL5 | F | 5'-ATGTGTGTGTGTTCATCTTCCAG-3' | 345 |
| | | R | 5'-ATCCATTTTCTCACAAGCAGTG-3' | 346 |
| 1328 | CDC20 | F | 5'-GGGGAATATATATCCTCTGTGGC-3' | 347 |
| | | R | 5'-AAAAACAACTGAGGTGATGGGT-3' | 348 |
| 1337 | MVD | F | 5'-ATGAAGGACAGCAACCAGTTC-3' | 349 |
| | | R | 5'-CAATGCTGGTTTATTCCCCAT-3' | 350 |
| 1342 | RBX1 | F | 5'-GTGAAAAAGTGGAATGCAGTAGC-3' | 351 |
| | | R | 5'-TTAGGTAACAGCAGGGAAAGTCA-3' | 352 |
| 1343 | GPR49 | F | 5'-CAGTCCTGTGACTCAACTCAA-3' | 353 |
| | | R | 5'-CGAGTTTCACCTCAGCTCTTCT-3' | 354 |
| 1345 | HT002 | F | 5'-GGATGTAGCAATCTCCACCAGT-3' | 355 |
| | | R | 5'-GTTCAAACACTCACTGAAGAGCC-3' | 356 |
| 1350 | AREG | F | 5'-CTCCACTCGCTCTTCCAACAC-3' | 357 |
| | | R | 5'-CTTTTTACCTTCGTGCACCTTT-3' | 358 |
| 1353 | | F | 5'-GACAGCAAAGTCTTGACTCCTTC-3' | 359 |
| | | R | 5'-AAAGTGGCTGGGAGTAAGGTATC-3' | 360 |
| 1362 | SCAMP5 | F | 5'-AGGGCACACATTCATCTTTGTA-3' | 361 |
| | | R | 5'-GTTACCAAAGACAGACACATTGG-3' | 362 |
| 1371 | LOC56755 | F | 5'-CTCAGCAAGAGAAGAACCGTTTA-3' | 363 |
| | | R | 5'-CCACTTAGAAATCGAATACGTCC-3' | 364 |
| 1375 | | F | 5'-TAGCCAAGTCAGAAAGACTCTGC-3' | 365 |
| | | R | 5'-GGTGGCCTTCTCTCAAAATTAGT-3' | 366 |
| 1377 | | F | 5'-CGCTGATAATATTCCTCGTCCTA-3' | 367 |
| | | R | 5'-AGTTTTTAGAGTTTCAGGGGGTC-3' | 368 |
| 1378 | LTBP3 | F | 5'-CTCCCTAGGGGTAGACTCTTCTG-3' | 369 |
| | | R | 5'-GAGACTAGGCCTCTTTTCTGGAT-3' | 370 |
| 1384 | KIAA0810 | F | 5'-TTCCAGCTATTCTTCAGATGCTC-3' | 371 |
| | | R | 5'-TATATGGCAGGTTTGTGTGTCTG-3' | 372 |
| 1389 | NMU | F | 5'-ATGCTGCGAACAGAGAGCTG-3' | 373 |
| | | R | 5'-AATGAACCCTGCTGACCTTC-3' | 374 |
| 1390 | | F | 5'-TGAGTCTCCTCTTGGTGATTCTG-3' | 375 |
| | | R | 5'-GGAAGAGCAAAGAGAGCTTCATC-3' | 376 |
| 1391 | PITX1 | F | 5'-GCTCAAGTCCAAACAGCACTC-3' | 377 |
| | | R | 5'-ACATACACAGGGAGGCTGTAAAC-3' | 378 |
| 1394 | FLJ10156 | F | 5'-TCCTAGGGGACTCTTGAGCTTAG-3' | 379 |
| | | R | 5'-ATAAATAGGIACCCGIGAGCCC-3' | 380 |
| 1395 | FBN2 | F | 5'-TAT'GTGCTACCCACAACACCTC-3' | 381 |
| | | R | 5'-GTTTGAGAGGAACAACCAGGAG-3' | 382 |
| 1398 | DKFZP586 C1324 | F | 5'-AGTCTTGGTTTACCTGTGGTGAC-3' | 383 |
| | | R | 5'-AAAACAAAACCCCAGAAACCC-3' | 384 |
| 1399 | DLX5 | F | 5'-GGGACTACTGTGTTTTGCTGTTC-3' | 385 |
| | | R | 5'-TGAGGTCATAGATTTCAAGGCAC-3' | 386 |
| 1403 | FOP | F | 5'-TAATAGTACCAGCCATCGCTCAG-3' | 387 |
| | | R | 5'-ATCCTACGGCTTTATTGACACCT-3' | 388 |
| 1406 | LOC51654 | F | 5'-CAGCCAGTTCTCAGACACTTAGG-3' | 389 |
| | | R | 5'-GTACTCGAGCCATCTGGCCTT-3' | 390 |
| 1407 | | F | 5'-ACTTTTGTGGTGTCCCCAAGTA-3' | 391 |
| | | R | 5'-CTGTGTACCCTTTACCCATTCCT-3' | 392 |
| 1410 | | F | 5'-ACTAGAGAAATGAGGGGCGTATC-3' | 393 |
| | | R | 5'-ATCTCTAACCAAACATCGTAGCG-3' | 394 |
| 1412 | HSPC157 | F | 5'-CTGAGGCAGCTTTATTTCCTACA-3' | 395 |
| | | R | 5'-ACTGGTGGGGTTACATAACCTTT-3' | 396 |
| 1413 | | F | 5'-GGTAGTGAAATATGGACAAAGGACA-3' | 397 |
| | | R | 5'-ACTTCTGCCATGTCGTCTTTTT-3' | 398 |
| 1417 | HOXC9 | F | 5'-ACAAAGAGGAGAAGGCTGACCT-3' | 399 |
| | | R | 5'-CTCCTCGCTGGGTAGAACTAACT-3' | 400 |
| 1420 | CHODL | F | 5'-GGAAGGAAAGGAACTACGAAATC-3' | 401 |
| | | R | 5'-GTTAAAAGGAGCACAGGGACATA-3' | 402 |
| 1422 | TMEM3 | F | 5'-CTCCTTACTTGTGGGATCAAAAG-3' | 403 |
| | | R | 5'-ATGTGCTAGATTACAGCCCTGA-3' | 404 |
| 1424 | MAGEA6 | F | 5'-AGGAGCTGAGTGTGTTAGAGGTG-3' | 405 |
| | | R | 5'-ATAAACCTGGATGCTGACGCTC-3' | 406 |
| 1435 | DKFZp586 H0623 | F | 5'-AGACCTAAGTCTGGAACAGAGCC-3' | 407 |
| | | R | 5'-CTACAGCACTCATTTGGAAAAGG-3' | 408 |
| 1436 | FLJ10858 | F | 5'-TTGGTCCTCCTCTGTTTCATAGA-3' | 409 |
| | | R | 5'-GCTTCTCCCCAGTTACAAGAGAC-3' | 410 |
| 1439 | PC | F | 5'-GTACTGAAGGACCTGCCAAGG-3' | 411 |
| | | R | 5'-GGGAAAGCCAGCTTTATTGAGTA-3' | 412 |
| 1440 | | F | 5'-AGTTTTGGATGACTCTGCTCAAG-3' | 413 |
| | | R | 5'-GGCATTTACGAGCATTATCTGAC-3' | 414 |
| 1441 | HSNOV1 | F | 5'-CAGTTTCAGTCCCAGGTCATACT-3' | 415 |
| | | R | 5'-GGCATACTCTTTGGTGAGAAATG-3' | 416 |
| 1444 | | F | 5'-CTACCCTGAAGGGGAAGAAAAG-3' | 417 |
| | | R | 5'-AACACACCCTACATCCAAGGTC-3' | 418 |
| 1445 | PANBP3 | F | 5'-CTTCAGAGGAAACTCCCAGTC-3' | 419 |
| | | R | 5'-GGCGTTATCTCGTTGTACTCGT-3' | 420 |
| 1447 | ADAM23 | F | 5'-AAAGCTGAATACAGAAGGCACTG-3' | 421 |
| | | R | 5'-TTTACTGACAGGTGGTGAAAGGT-3' | 422 |

The expression of each of the genes in cancer tissue obtained from the lung cancer patient (Fig.2) was confirmed by semi-quantitative RT-PCR. The result was as follows:
NSC 807: the expression of NSC 807 was up-regulated in 6 of 9 NSCLC cases;
NSC 810: the expression of NSC 810 was up-regulated in 6 of 10 NSCLC cases;
NSC 811: the expression of NSC 811 was up-regulated in all of 9 NSCLC cases;
NSC 812: the expression of NSC 812 was up-regulated in all of 15 NSCLC cases;
NSC 816: the expression of NSC 816 was up-regulated in all of 8 NSCLC cases;
NSC 820: the expression of NSC 820 was up-regulated in 8 of 9 NSCLC cases;
NSC 822: the expression of NSC 822 was up-regulated in 3 of 10 NSCLC cases;
NSC 824: the expression of NSC 824 was up-regulated in all of 9 NSCLC cases;
NSC 825: the expression of NSC 825 was up-regulated in all of 12 NSCLC cases;
NSC 830: the expression of NSC 830 was up-regulated in 7 of 10 NSCLC cases;
NSC 837: the expression of NSC 837 was up-regulated in 7 of 9 NSCLC cases;
NSC 840: the expression of NSC 840 was up-regulated in 9 of 10 cases of NSCLCs:
NSC 841: the expression of NSC 841 was up-regulated in 9 of 11 NSCLC cases;
NSC 842: the expression of NSC 842 was up-regulated in 7 of 8 NSCLC cases;
NSC 846: the expression of NSC 846 was up-regulated in 9 of 10 NSCLC cases;
NSC 849: the expression of NSC 849 was up-regulated in 7 of 10 NSCLC cases;
NSC 850: the expression of NSC 850 was up-regulated in all of 7 NSCLC cases;
NSC 853: the expression of NSC 853 was up-regulated in 8 of 10 NSCLC cases;
NSC 854: the expression of NSC 854 was up-rsgulated in all of 7 NSCLC cases;
NSC 855: the expression of NSC 855 was up-regulated in 10 of 11 NSCLC cases;
NSC 857: the expression of NSC 857 was up-repulated in all of 8 NSCLC cases;
NSC 859: the expression of NSC 859 was up-regulated in all of 8 NSCLC cases;
NSC 861: the expression of NSC 861 was up-regulated in 5 of 7 NSCLC cases;
NSC 864: up-regulation of NSC 864 was confirmed by semi-quantitative RT-PCR in all of 10 NSCLC cases;
NSC 870: the expression of NSC 870 was up-regulated in 10 of 11 NSCLC cases;
NSC 871: the expression of NSC 871 was up-regulated in 12 of 13 NSCLC cases;
NSC 872: the expression of NSC 872 was up-regulated in 9 of 12 NSCLC cases;
NSC 881: the expression of NSC 881 was up-regulated in all of 10 NSCLC cases;
NSC 882: the expression of NSC 882 was up-regulated in 7 of 10 NSCLC cases;
NSC 884: the expression of NSC 884 was up-regulated in all of 9 NSCLC cases;
NSC 885: the expression of NSC 885 was up-regulated in all of 8 NSCLC cases;
NSC 889: the expression of NSC 889 was up-regulated in and 7 of 8 NSCLC cases;
NSC 893: the expression of NSC 893 was up-regulated in 7 of 9 NSCLC cases;
NSC 895: the expression of NSC 895 was up-regulated in 5 of 6 NSCLC cases;
NSC 898: the expression of NSC 898 was up-regulated in 5 of 6 NSCLC cases;
NSC 901: the expression of NSC 901 was up-regulated in all of 14 NSCLC cases;
NSC 902: the expression of NSC 902 was up-regulated in 7 of 8 NSCLC cases;
NSC 903: the expression of NSC 903 was up-regulated in 9 of 10 NSCLC cases;
NSC 904: the expression of NSC 904 was up-regulated in 7 of 10 NSCLC cases;
NSC 905: the expression of NSC 905 was up-regulated in all of 13 NSCLC cases;
NSC 909: the expression of NSC 909 was up-regulated in 9 of 13 NSCLC cases;
NSC 912: the expression of NSC 912 was up-regulated in all of 7 NSCLC cases;
NSC 915: the expression of NSC 915 wasup-regulated in all of 9 NSCLC cases;
NSC 917: the expression of NSC 917 was up-regulated in all of 9 NSCLC cases;
NSC 920: the expression of NSC 920 was up-regulated in 8 of 10 NSCLC cases;
NSC 921: the expression of NSC 921 was up-regulated in all of 8 NSCLC cases;
NSC 924: the expression of NSC 924 was up-regulated in all of 8 NSCLC cases;
NSC 929: the expression of NSC 929 was up-regulated in 10 of 12 NSCLC cases;
NSC 930: the expression of NSC 930 was up-regulated in 9 of 10 NSCLC cases;
NSC 933: the expression of NSC 933 was up-regulated in 9 of 10 NSCLC cases;
NSC 934:CIT. the expression of NSC 934 was up-regulated in 7 of 8 NSCLC cases;
NSC 936: the expression of NSC 936 was up-regulated in all of 8 NSCLC cases;
NSC 938: the expression of NSC 938 was up-regulated in 9 of 10 NSCLC cases;
NSC 940: the expression of NSC 940 was up-regulated in 2 of 10 NSCLC cases;
NSC 944: the expression of NSC 944 was up-regulated in all of 10 NSCLC cases;
NSC 947: the expression of NSC 947 was up-regulated in 9 of 10 NSCLC cases;
NSC 948: the expression of NSC 948 was up-regulated in 8 of 10 NSCLC cases;
NSC 956: the expression of NSC 956 was up-regulated in all of 8 NSCLC cases;
NSC 957: the expression of NSC 957 was up-regulated in 7 of 8 NSCLC cases;
NSC 958: the expression of NSC 958 was up-regulated in all of 10 NSCLC cases;
NSC 963: the expression of NSC 963 was up-regulated in all of 10 NSCLC cases;
NSC 964: the expression of NSC 964 was up-regulated in all of 8 NSCLC cases;
NSC 965: the expression of NSC 965 was up-regulated in 10 of 11 NSCLC cases;
NSC 966: the expression of NSC 966 was up-regulated in 3 of 8 NSCLC cases;
NSC 970: the expression of NSC 970 was up-regulated in 7 of 12 NSCLC cases;
NSC 972: the expression of NSC 972 was up-regulated in 9 of 10 NSCLC cases;
NSC 973: the expression of NSC 973 was up-regulated in 3 of 9 NSCLC cases;
NSC 974: the expression of NSC 974 was up-regulated in 9 of 10 NSCLC cases;
NSC 975: the expression of NSC 975 was up-regulated in 12 NSCL cases;
NSC 980: the expression of NSC 980 was up-regulated in 7 of 8 NSCLC cases;
NSC 984: the expression of NSC 984 was up-regulated in 8 of 9 NSCLC cases;
NSC 989: the expression of NSC 989 was up-regulated in all of 9 NSCLC cases;
NSC 990: the expression of NSC 990 was up-regulated in 4 of 8 NSCLC cases;
NSC 991: the expression of NSC 991 was up-regulated in 3 of 10 NSCLC cases;
NSC 994: the expression of NSC 994 was up-regulated in all of 8 NSCLC cases;
NSC 1000: the expression of NSC1000 was up-regulated in 12 of 13 cases of NSCLCs.
NSC 1002: the expression of NSC 1002 was up-regulated in all of 8 NSCLC cases;
NSC 1003: the expression of NSC 1003 was up-regulated in all of 10 NSCLC cases;
NSC 1012: the expression of NSC 1012 was up-regulated in all of 8 NSCLC cases;
NSC 1015: the expression of NSC 1015 was up-regulated in 10 NSCLC cases;
NSC 1016: the expression of NSC 1016 was up-regulated in 8 of 9 NSCLC cases;
NSC 1018: the expression of NSC 1018 was up-regulated in 3 of 6 NSCLC cases;
NSC 1023: the expression of NSC 1023 was up-regulated in 7 of 12 NSCLC cases;
NSC 1026: the expression of NSC 1026 was up-regulated in 7 of 9 NSCLC cases;
NSC 1027: the expression of NSC 1027 was up-regulated in 5 of 8 NSCLC cases;
NSC 1030: the expression of NSC 1030 was up-regulated in 5 of 6 NSCLC cases;
NSC 1034: the expression of NSC 1034 was up-regulated in 5 of 8 NSCLC cases;
NSC 1037: the expression of NSC 1037 was up-regulated in all of 9 NSCLC cases;
NSC 1038: the expression of NSC 1038 was up-regulated in 6 of 7 NSCLC cases;
NSC 1047: the expression of NSC 1047 was up-regulated in 4 of 6 NSCLC cases;
NSC 1049: up-regulation of NSC 1049 was detected in all of 6 NSCLC cases;
NSC 1052: the expression of NSC 1052 was up-regulated in all of 8 NSCLC cases;
NSC 1057: the expression of NSC 1057 was up-regulated in all of 8 NSCLC cases;
NSC 1058: the expression of NSC 1058 was up-regulated in 8 of 10 NSCLC cases;
NSC 1059: the expression of NSC 1059 was up-regulated in 8 of 9 NSCLC cases;
NSC 1064: the expression of NSC 1064 was up-regulated in all of 13 NSCLC cases;
NSC 1066: the expression of NSC 1066 was up-regulated in 8 of 10 NSCLC cases;
NSC 1067: the expression of NSC 1067 was up-regulated in all of 10 NSCLC cases;
NSC 1071: the expression of NSC 1071 was up-regulated in all of 10 NSCLC cases;
NSC 1072: the expression of NSC 1072 was up-regulated in 7 of 10 NSCLC cases;
NSC 1075: the expression of NSC 1075 was up-regulated in all of 9 NSCLC cases;
NSC 1077: the expression of NSC 1077 was up-regulated in 8 of 11 NSCLC cases;
NSC 1078: the expression of NSC 1078 was up-regulated in 8 of 9 NSCLC cases;
NSC 1086: the expression of NSC 1086 was up-regulated in 10 of 11 NSCLC cases;
NSC 1089: the expression of NSC 1089 was up-regulated in 6 of 9 NSCLC cases;
NSC 1090: the expression of NSC 1090 was up-regulated is of 7 NSCLC cases;
NSC 1103: the expression of NSC 1103 was up-regulated in 7 of 8 NSCLC cases;
NSC 1107: the expression of NSC 1107 was up-regulated in 8 of the 9 NSCLC cases;
NSC 1109: the expression of NSC 1109 was up-regulated in 8 of 9 NSCLC cases;
NSC 1113: The expression of NSC 1113 was up-regulated in 10 of the 11 NSCLC cases;
NSC 1116: the expression of NSC 1116 was up-regulated in 8 of 9 NSCLC cases;
NSC 1125: the expression of NSC 1125 was up-regulated in 10 NSCLC cases;
NSC 1131: up-reglation of NSC 1131 was detected in 2 of 6 NSCLC cases;
NSC 1133: the expression of NSC 1133 was up-regulated in all of 10 NSCLC cases;
NSC 1136: the expression of NSC 1136 was up-regulated in 8 of 9 NSCLC cases;
NSC 1141: the expression of NSC 1141 was up-regulated in 6 of 10 NSCLC cases;
NSC 1142: up-regulation of NSC 1142 was detected in 9 of 11 NSCLC cases;
NSC 1157: the expression of NSC 1157 was up-regulated in 1 of 11 NSCLC cases;
NSC1162: the expression of NSC1162 was yp-regulated in 9 of 10 cases of NSCLCs
NSC 1164: the expression of NSC 1164 was up-regulated in all of 7 NSCLC cases;
NSC 1167: the expression of NSC 1167 was up-regulated in 8 of 9 NSCLC cases;
NSC 1169: the expression of NSC 1169 was up-regulated in 3 of 7 NSCLC cases;
NSC 1173: the expression of NSC 1173 was up-regulated in 5 of 7 NSCLC cases;
NSC 1176: the expression of NSC 1176 was up-regulated in 8 of 9 NSCLC cases;
NSC 1183: up-regulation of NSC 1183 was detected in all of 10 NSCLC cases;
NSC 1184: the expression of NSC 1184 was up-regulated in 8 of 9 NSCLC cases;
NSC 1185: up-regulation of NSC 1185 was detected in 5 of 6 NSCLC cases;
NSC 1191: the expression of NSC 1191 was up-regulated in 7 of 8 NSCLC cases;
NSC 1195: the expression of NSC 1195 was up-regulated in 5 of 9 NSCLC cases;
NSC 1196: the expression of NSC 1196 was up-regulated in all of 6 NSCLC cases;
NSC 1201: the expression of NSC 1201 was up-regulated in all of 9 NSCLC cases;
NSC 1205: the expression of NSC 1205 was up-regulated in 7 of 9 NSCLC cases;
NSC 1207: the expression of NSC 1207 was up-regulated in 8 of 10 NSCLC cases;
NSC 1210: the expression of NSC 1210 was and up-regulated in 9 of 10 NSCLC cases;
NSC 1214: the expression of NSC 1214 was up-regulated in 7 of 9 NSCLC cases;
NSC 1234: the expression of NSC 1234 was up-regulated in 9 of 10 NSCLC cases;
NSC 1236: the expression of NSC 1236 was up-regulated in all of 6 of 8 NSCLC cases;
NSC 1237: the expression of NSC 1237 was up-regulated in 5 of 6 NSCLC cases;
NSC 1238: the expression of NSC 1238 was up-regulated in 6 of 7 NSCLC cases;
NSC 1240: the expression of NSC 1240 was up-regulated in all of 7 NSCLC cases;
NSC 1242: the expression of NSC 1242 was up-regulated in 4 of 7 NSCLC cases;
NSC 1246: the expression of NSC 1246 was up-regulated in 6 of 10 NSCLC cases;
NSC 1247: the expression of NSC 1247 was up-regulated in 5 of 8 NSCLC cases;
NSC 1250: the expression of NSC 1250 was up-regulated in 8 NSCLC cases;
NSC 1254: the expression of NSC 1254 was up-regulated in 10 NSCLC cases;
NSC 1265: up-regulation of NSC 1265 was detected in 4 of 5 NSCLC cases;
NSC 1273: up-regulation of NSC 1273 was detected in 5 of 6 NSCLC cases;
NSC 1277: the expression of NSC 1277 was up-regulated in all of 10 NSCLC cases;
NSC 1279: the expression of NSC 1279 was up-regulated in all of 7 NSCLC cases;
NSC 1288: the expression of NSC 1288 was up-regulated in 6 of 9 NSCLC cases;
NSC 1289: the expression of NSC 1289 was up-regulated in 6 of 9 NSCLC cases;
NSC 1290: the expression of NSC 1290 was up-regulated in all of 10 NSCLC cases;
NSC 1292: the expression of NSC 1292 was up-regulated in all of 8 NSCLC cases;
NSC 1293: up-regulation of NSC 1293 was detected in 4 of 6 NSCLC cases;
NSC 1294: the expression of NSC 1294 was up-regulated in 7 of 8 NSCLC cases;
NSC 1295: the expression of NSC 1295 was up-regulated in 5 of 7 NSCLC cases;
NSC 1299: up-regulation of NSC 1299 was detected in 5 of 6 NSCLC cases;
NSC 1302: the expression of NSC 1302 was up-regulated in all of 7 NSCLC cases;
NSC 1306: up-regulation of NSC 1306 was detected in 5 of 6 NSCLC cases;
NSC 1309: the expression of NSC 1309 was up-regulated in 7 of 8 NSCLC cases;
NSC 1310: the expression of NSC 1310 was up-regulated in 9 of 10 NSCLC cases;
NSC 1315: the expression of NSC 1315 was up-regulated in 6 of 9 NSCLC cases;
NSC 1320: the expression of NSC 1320 was up-regulated in 5 of 9 NSCLC cases;
NSC 1323: the expression of NSC 1323 was up-regulated in all of 10 NSCLC cases;
NSC 1325: the expression of NSC 1325 was up-regulated in 2 of 9 NSCLC cases;
NSC 1328: the expression of NSC 1328 was up-regulated in all of 9 NSCLC cases;
NSC 1337: the expression of NSC 1337 was up-regulated in all of 9 NSCLC cases;
NSC 1345: the expression of NSC 1345 was up-regulated in 3 of 8 NSCLC cases;
NSC 1350: the expression of NSC 1350 was up-regulated in 6 of 8 NSCLC cases;
NSC 1353: the expression of NSC 1353 was up-regulated in 3 of 10 NSCLC cases;
NSC 1362: the expression of NSC 1362 was up-regulated in 6 of 7 NSCLC cases;
NSC 1371: the expression of NSC 1371 was up-regulated in all of 10 NSCLC cases;
NSC 1375: the expression of NSC 1375 was up-regulated in all of 8 NSCLC cases;
NSC 1377: the expression of NSC 1377 was up-regulated in 5 of 8 NSCLC cases;
NSC 1378: the expression of NSC 1378 was up-regulated in 8 of 9 NSCLC cases;
NSC 1384: the expression of NSC 1384 was up-regulated in 8 of 11 NSCLC cases;
NSC 1389: the expression of NSC 1389 was up-regulated in 8 of 9 NSCLC cases;
NSC 1390: the expression of NSC 1390 was up-regulated in 8 of 10 NSCLC cases;
NSC 1391: the expression of NSC 1391 was up-regulated in all of 8 NSCLC cases;
NSC 1394: the expression of NSC 1394 was up-regulated in 6 of 10 NSCLC cases;
NSC 1395: the expression of NSC 1395 was up-regulated in 4 of 7 NSCLC cases;
NSC 1398: the expression of NSC 1398 was up-regulated in 8 of 9 NSCLC cases;
NSC 1399: The expression of NSC 1399 was up-regulated in 4 of 10 NSCLC cases;
NSC 1403: the expression of NSC 1403 was up-regulated in 6 of 8 NSCLC cases;
NSC 1406: the expression of NSC 1406 was up-regulated in all of 10 NSCLC cases;
NSC 1407: the expression of NSC 1407 was up-regulated in all of 10 NSCLC cases;
NSC 1410: the expression of NSC 1410 was up-regulated in 5 of 10 NSCLC cases;
NSC 1412: the expression of NSC 1412 was up-regulated in 6 of 9 NSCLC cases;
NSC 1417: the expression of NSC 1417 was up-regulated in 3 of 7 NSCLC cases;
NSC 1420: up-regulation of NSC 1420 was detected in all of 7 NSCLC cases;
NSC 1422: the expression of NSC 1422 was up-regulated in 4 of 10 NSCLC cases;
NSC 1424: the expression of NSC 1424 was up-regulated in 5 of 6 NSCLC cases;
NSC 1435: the expression of NSC 1435 was up-regulated in 4 of 8 NSCLC cases;
NSC 1436: the expression of NSC 1436 was up-regulated in all of 7 NSCLC cases;
NSC 1439: the expression of NSC 1439 was up-regulated in all of 8 NSCLC cases;
NSC 1440: the expression of NSC 1440 was up-regulated in 8 of 9 NSCLC cases;
NSC 1441: the expression of NSC 1441 was up-regulated in 9 of 11 NSCLC cases;
NSC 1444: the expression of NSC 1444 was up-regulated in 4 of 6 NSCLC cases;
NSC 1445: the expression of NSC 1445 was up-regulated in 6 of 7 NSCLC cases;
NSC 1447: the expression of NSC 1447 was up-regulated in all of 7 NSCLC cases.

### (3) Antisense S-oligonucleotide assay

Three to five pairs of reverse (control) and antisense S-oligonucleotides corresponding to each of the genes were prepared. Four NSCLC cell lines A549, NCI-H226, NCI-H522 and/or LC319 plated on 6-well or 10cm dishes were transfected with synthetic S-oligonucleotides corresponding to each of the genes using Lipofectin reagent (Life Technologies, Inc.) and maintained in media containing 10% fetal bovine serum for 2 days. The cells were then fixed with 100% methanol and stained with Giemsa solution. Antisense S-oligonucleotides against 26genes suppressed focus formation compared with control. Thus, suppression of these genes were demonstrated to reduce growth, proliferation and/or survival of the transfected cells. The sequences of the effective antisense S-oligonucleotides and the control reverse oligonucleotides are shown in Table 5. A MTT assay was performed in triplicate as by methods known in the art (Akashi et al. (2000) Int. J. Cancer 88: 873-80). Methods and results for each of the genes were as follows:
NSC 810:*TTK*; an effective antisense S-oligonucleotide (SEQ ID NO:423) and reverse S-oligonucleotide (control) (SEQ ID NO:424) corresponding to *TTK* were synthesized. Each of the S-oligonucleotides was transfected into NSCLC cell line A549 and LC319, which showed highest expression level for *TTK.* Two days after transfection, the antisense S-oligonucleotide clearly were demonstrated to suppress cell proliferation compared with control by the MTT assay (Fig.2). Thus, the suppression of *TTK* was suggested to reduce growth, proliferation and/or survival of cells. The result was also confirmed by focus formation (staining by Giemsa) (data not shown).
NSC 811:*SDC1*; three effective antisense S-oligonucleotides (AS (SEQ ID NO:425), AS2 (SEQ ID NO:427) and AS4 (SEQ ID NO:429)) and reverse S-oligonucleotides (control)(R1(SEQ ID NO:426), R2(SEQ ID N:428) and R4(SEQ ID NO:430)) corresponding to *SDC1* were synthesized and transfected respectively into NSCLC cell line A549, which showed highest expression for *SDC1.* Two days after transfection, these antisense S-oligonucleotides were clearly shown to suppress cell proliferation compared with control by the MTT assay (Fig.2). Therefore, the suppression of *SDC1* was suggested to reduce growth, proliferation and/or survival of cells. The result was also confirmed by focus formation (staining by Giemsa) (data not shown).
NSC 812:*NMB*; two effective antisense S-oligonucleotides (AS1 (SEQ ID NO:431) and AS2 (SEQ ID NO:433)) and reverse S-oligonucleotides (control)(R1(SEQ ID NO:432) and R2(SEQ ID NO:434)) corresponding to *NMB* were synthesized. Each of the S-oligonucleotides was transfected into NSCLC cell lines A549, NCI-H226 and NCI-H522, which showed highest expression for *NMB.* Two days after transfection, these antisense S-oligonucleotides were demonstrated to clearly suppress cell proliferation compared with control by the MTT assay (Fig.2). Therefore, the suppression of *NMB* was suggested to reduce growth, proliferation and/or survival of cells. These results were also confirmed by focus formation (staining by Giemsa) (data not shown).
NSC 816:*PIR51*; an effective antisense S-oligonucleotide AS1 (SEQ ID NO:435) and reverse S-oligonucleotide (control) R1(SEQ ID NO:436) corresponding to *PIR51* were synthesized. Each of the S-oligonucleotides was transfected into NSCLC cell lines A549, NCI-H226 and NCI-H522, which showed highest expression for *NMB.* Two days after transfection, the antisense S-oligonucleotide was demonstrated to clearly suppress cell proliferation compared with control by focus formation (stained by Giemsa)(data not shown). Thus, the suppression of *PIR51* was suggested to reduce growth, proliferation and/or survival of cells.
NSC 825:*ANLN*; three effective antisense S-oligonucleotides (AS1(SEQ ID NO:437), AS3(SEQ ID NO:439) and AS5(SEQ ID NO:441)) and reverse S-oligonucleotides (control)(R1(SEQ ID NO:438), R3(SEQ ID NO:440) and R5(SEQ ID NO:442)) corresponding to *ANLN* were synthesized. Each of the S-oligonucleotides was transfected into NSCLC cell lines A549, NCI-H226 and NCI-H522, which showed highest expression for *ANLN.* Two days after transfection, these antisense S-oligonucleotides were demonstrated to clearly suppress cell proliferation compared with control by the MTT assay (Fig.2). Thus, the suppression of *ANLN* was suggested to reduce growth, proliferation and/or survival of cells.
NSC 841:*URLC2*; two effective antisense S-oligonucleotides, AS4 (SEQ ID NO:443) and AS5 (SEQ ID NO:445), and reverse S-oligonucleotides (control) R4 (SEQ ID NO:444) and R5 (SEQ ID NO:446) corresponding to *URLC2* were synthesized. Each of the S-oligonucleotides was transfected into NSCLC cell lines A549, NCI-H226 and NCI-H522, which showed highest expression for *URLC2.* Two days after transfection, these antisense S-oligonucleotides were shown to clearly suppress cell proliferation compared with control by the MTT assay (Fig.2). Thus, the suppression of *URLC2* was suggested to reduce growth, proliferation and/or survival of cells. The result was also confirmed by focus formation (staining by Giemsa)(data not shown).
NSC 857:*TIGD5*; two effective antisense S-oligonucleotides, AS3 (SEQ ID NO:447) and AS4 (SEQ ID NO:449), and reverse S-oligonucleotides(control), R3 (SEQ ID NO:448) and R4 (SEQ ID NO:450), corresponding to *TIGD5* were synthesized. Each of the S-oligonucleotides were transfected into NSCLC cell lines A549, NCI-H226 and NCI-H522, which showed highest expression for *TIGD5.* Two days after transfection, these antisense S-oligonucleotides were shown to clearly suppress cell proliferation compared with control by the MTT assay (Fig.2). Thus, the suppression of *TIGD5* was suggested to reduce growth, proliferation and/or survival of cells. The result was also confirmed by focus formation (staining by Giemsa)(data not shown).
NSC 859: *URLC3*; three pairs effective antisense S-oligonucleotides, AS2 (SEQ ID NO:451), AS3 (SEQ ID NO:453) and AS5 (SEQ ID NO:455), and reverse S-oligonucleotides (control), R2 (SEQ ID NO:452), R3 (SEQ ID NO:454) and R5 (SEQ ID NO:456), corresponding to *URLC3* were synthesized. Each of the S-oligonucleotides was transfected into NSCLC cell lines A549, NCI-H226 and NCI-H522, which showed highest expression for *URLC3.* Two days after transfection, these antisense 5-oligonucleotides were shown to clearly suppress cell proliferation compared with control by the MTT assay (Fig.2). Thus, the suppression of *URLC3* was suggested to reduce growth, proliferation and/or survival of cells. The result was also confirmed by focus formation (staining by Giemsa)(data not shown).
NSC 885:*BAG5*; two effective antisense S-oligonucleotides, AS1 (SEQ ID NO:457) and AS2 (SEQ ID NO:459), and reverse S-oligonucleotides (control), R1 (SEQ ID NO: 458) and R2 (SEQ ID NO: 460), corresponding to *BAG5* were synthesized. Each of the S-oligonucleotides was transfected into NSCLC cell lines A549, NCI-H226 and NCI-H522, which showed highest expression for *BAG5.* Two days after transfection, the antisense S-oligonucleotides were shown to clearly suppress cell proliferation compared with controls by focus formation (staining by Giemsa)(data not shown). Thus, the suppression of *BAG5* was suggested to reduce growth, proliferation and/or survival of cells.
NSC 893:*MPHOSPH1*; two effective antisense S-oligonucleotides, AS1 (SEQ ID NO:461) and AS2 (SEQ ID NO:463), and reverse S-oligonucleotides (control) R1(SEQ ID NO:462) and R2 (SEQ ID NO:464) corresponding to *MPHOSPH1* were synthesized. Each of the S-oligonucleotides was transfected into NSCLC cell lines A549, NCI-H226 and NCI-H522, which showed highest expression for *MPHOSPH1.* Two days after transfection, these antisense S-oligonucleotides were demonstrated to clearly suppress cell proliferation compared with control by the MTT assay (Fig.2). Thus, the suppression of *MPHOSPH1* was suggested to reduce growth, proliferation and/or survival of cells. The result was also confirmed by focus formation (staining by Giemsa)(data not shown).
NSC 905: *URLC1*; three effective antisense S-oligonucleotides, AS2 (SEQ ID NO:465), AS3 (SEQ ID NO:467) and AS5 (SEQ ID NO:469), and reverse S-oligonucleotides(control), R2 (SEQ ID NO:466), R3 (SEQ ID NO:468) and R5(SEQ ID NO:470), corresponding to *URLC1* were synthesized. Each of the S-oligonucleotides was transfected into NSCLC cell lines A549, NCI-H226 and NCI-H522, which showed highest expression for *URLC1.* Two days after transfection, these antisense S-oligonucleotides were shown to clearly suppress cell proliferation compared with control by the MTT assay (Fig.2). Thus, the suppression of *URLC1* was suggested to reduce growth, proliferation and/or survival of cells. The result was also confirmed by focus formation (staining by Giemsa)(data not shown).
NSC 909:*FLJ10468*; an effective antisense S-oligonucleotide AS1 (SEQ ID NO:471) and reverse S-oligonucleotide (control) R1 (SEQ ID NO:472) corresponding to *FLJ10468* were synthesized. Each of the S-oligonucleotides was transfected into NSCLC cell lines A549 and NCI-H522, which showed highest expression for *FLJ10468.* Two days after transfection, the antisense S-oligoncleotide was shown to clearly suppress cell proliferation compared with control by focus formation (staining by Giemsa)(data not shown). Thus, the suppression of *FLJ10468* was suggested to reduce growth, proliferation and/or survival of cells.
NSC 920:*CHAF1A*; two effective antisense S-oligonucleotides, AS1 (SEQ ID NO:473) and AS4 (SEQ ID NO:475), and reverse S-oligonucleotides(control), R1 (SEQ ID NO:474) and R4 (SEQ ID NO:476) corresponding to *CHAF1A* were synthesized. Each of the S-oligonucleotides was transfected into NSCLC cell lines A549 and NCI-H522, which showed highest expression for *CHAF1A.* Two days after transfection, these antisense S-oligonucleotides were shown to clearly suppress cell proliferation compared with control by focus formation (stained by Giemsa)(data not shown). Thus, the suppression of *CHAF1A* was suggested to reduce growth, proliferation and/or survival of cells.
NSC 947:*PKP3*; four effective antisense S-oligonucleotides (AS1 (SEQ ID NO:477), AS2 (SEQ ID NO:479), AS3 (SEQ ID NO:481) and AS4 (SEQ ID NO:483)) and reverse S-oligonucleotides(control)(R1(SEQ ID NO:478), R2 (SEQ ID NO:480), R3 (SEQ ID NO: 482) and R4 (SEQ ID NO:484) corresponding to *PKP3* were synthesized. Each of the S-oligonucleotides was transfected into NSCLC cell lines A549 and LC319, which showed highest expression for *PKP3.* Two days after transfection, these antisense S-oligonucleotides were demonstrated to clearly suppress cell proliferation compared with control by the MTT assay (Fig.2). Thus, the suppression of *PKP3* was suggested to reduce growth, proliferation and/or survival of cells. The result was also confirmed by focus formation (staining by Giemsa)(data not shown).
NSC 956:*SIAHBP1*; two effective antisense S-oligonucleotides, AS1 (SEQ ID NO:485) and AS2 (SEQ ID NO:487), and reverse S-oligonucleotides(control), R1 (SEQ ID NO:486) and R2 (SEQ ID NO:488) corresponding to *SIAHBP1* were synthesized. Each of the S-oligonucleotides was transfected into NSCLC cell line A549, NCI-H226 and NCI-H522, which showed highest expression for *SIAHBP1*. Two days after transfection, these antisense S-oligonucleotides were shown to clearly suppress cell proliferation compared with control by the MTT assay (Fig.2), suggesting that suppression of *SIAHBP1* reduces growth, proliferation and/or survival of cells. The result was also confirmed by focus formation (staining by Giemsa)(data not shown).
NSC 994:*DKFZP434E2318*; four effective antisense S-oligonucleotides (AS1 (SEQ ID NO:489), AS3 (SEQ ID NO:491), AS4 (SEQ ID NO:493) and AS5 (SEQ ID NO:495)), and reverse S-oligonucleotides(control)(R1 (SEQ ID NO:490), R3 (SEQ ID NO:492), R4 (SEQ ID NO:494) and R5 (SEQ ID NO:496)), corresponding to *DKFZP434E2318* were synthesized. Each of the S-oligonucleatides was transfected into NSCLC cell lines A549, NCI-H226 and NCI-H522, which showed highest expression for *DKFZP434E2318.* Two days after transfection, these antisense S-oligonucleotides were shown to clearly suppress cell proliferation compared with control by the MTT assay (Fig.2). Thus, the suppression of *DkFZP434E2318* reduced growth, proliferation and/or survival of cells. The result was also confirmed by focus formation (staining by Giemsa)(data not shown).
NSC *1075:URLC4*; an effective antisense S-oligonucleotide AS5 (SEQ ID NO:497) and reverse S-oligonucleotide (control) R5 (SEQ ID NO:498), corresponding to *URLC4* was synthesized. Each of the s-oligonucleotides was transfected into NSCLC cell lines A549, NCI-H226 and NCI-H522, which showed highest expression of *URLC4.* Two days after transfection, the antisense S-oligonucleotides were shown to clearly suppress cell proliferation compared with control by the MTT assay (Fig.2). Thus, the suppression of *URLC4* was suggested to reduce growth, proliferation and/or survival of cells. The resuls was also confirmed by focus formation (staining by Giemsa)(data not shown).
NSC 1107:*URLC8*; two effective antisense S-oligonucleotides, AS1(SEQ ID NO:499) and AS4 (SEQ ID NO:501), and reverse S-oligonucleotides(control), R1(SEQ ID NO:500) and R4 (SEQ ID NO:502) corresponding to *URLC8* were synthesized and transfected respectively into NSCLC cell line A549, which showed highest expression for *URLC8.* Two days after transfection, these antisense S-oligonucleotides were shown to clearly suppress cell proliferation compared with control by the MTT assay (Fig.2). Thus, the suppression of *URLC8* was suggested to reduce growth, proliferation and/or survival of cells. The result was also confirmed by focus formation (staining by Giemsa)(data not shown).
NSC 1113: *URLC5*; two effective antisense S-oligonucleotides, AS1 (SEQ ID NO: 503) and AS2 (SEQ ID NO:505), and reverse S-oligonucleotides (control), R1 (SEQ ID NO: 504) and R2 (SEQ ID NO:506), corresponding to *URLC5* were synthesized. Each of the S-oligonucleotides was transfected into NSCLC cell lines A549, NCI-H226 and NCI-H522, which showed highest expression for *URLC5.* Two days after transfection, the antisense S-oligonucleotides were shown to clearly suppress cell proliferation compared with controls by focus formation (staining by Giemsa) (data not shown), suggesting that suppression of *URLC5* reduces growth, proliferation and/or survival of cells.
NSC 1131:*SYNJ2BP*; an effective antisense S-oligonucleotide AS1 (SEQ ID NO:507) and reverse S-oligonucleotide(control) R1 (SEQ ID NO:508) corresponding to *SYNJ2BP* were synthesized. Each of the S-oligonucleotides was transfected into NSCLC cell lines A549 and NCI-H226, which showed highest expression for *SYNJ2BP.* Two days after transfection, the antisense S-oligonucleotide was shown to clearly suppress cell proliferation compared with control by focus formation (stained by Giemsa)(data not shown), suggesting that suppression of *SYNJ2BP* reduces growth, proliferation and/or survival of cells.
NSC 1142:*NAPG*; an effective antisense S-oligonucleotide AS1 (SEQ ID NO:509) and reverse S-oligonucleotide(control) R1 (SEQ ID NO:510) corresponding to *NAPG* were synthesized. Each of the S-oligonucleotides was transfected into NSCLC cell lines A549, NCI-H226 and NCI-H522, which showed highest expression for *NAPG.* Two days after transfection, compared with control, the antisense S-oligonucleotide was shown to clearly suppress cell proliferation by focus formation (stained by Giemsa)(data not shown), suggesting that suppression of *NAPG* reduces growth, proliferation and/or survival of cells.
NSC 1183:*BYSL*; three effective antisense S-oligonucleotides, AS1(SEQ ID NO:511), AS2 (SEQ ID NO:513) and AS3 (SEQ ID NO:515), and reverse S-oligonucleotides (control), R1 (SEQ ID NO:512), R2(SEQ ID NO:514) and R3 (SEQ ID NO:516) corresponding to *BYSL* were synthesized. Each of the S-oligonucleotides was transfected into NSCLC cell lines A549, NCI-H226 and NCI-H522, which showed highest expression for *BYSL.* Two days after transfection, compared with control, these antisense S-oligonucleotides were shown to clearly suppress cell proliferation by focus formation (staining by Giemsa)(data not shown), suggesting that suppression of *BYSL* reduces growth, proliferation and/or survival of cells.
NSC 1185:*URLC6*; two effective antisense S-oligonucleotides, AS4 (SEQ ID NO:517) and AS6 (SEQ ID NO:519), and reverse S-oligonucleotides(control), R4 (SEQ ID NO:518) and R6 (SEQ ID NO:520) corresponding to *URLC6* were synthesized. Each of the S-oligonucleotides was transfected into NSCLC cell lines A549 and NCI-H226 which showed highest expression for *URLC6.* Two days after transfection, compared with control, these antisense S-oligonucleotides were shown to clearly suppress cell proliferation by focus formation (staining by Giemsa)(data not shown), suggesting that suppression of *URLC6* reduces growth, proliferation and/or survival of cells.
NSC 1191:*COX17*; three effective antisense S-oligonucleotides, AS2 (SEQ ID NO:521), AS4 (SEQ ID NO:523) and AS5 (SEQ ID NO:525), and reverse S-oligonucleotides (control), R2 (SEQ ID NO:522), R4 (SEQ ID NO:524) and R5 (SEQ ID NO:526) corresponding to *COX17* were synthesized. Each of the S-oligonucleotides was transfected into NSCLC cell lines A549, NCI-H226 and NCI-H522, which showed highest expression of *COX17.* Two days after transfection, these antisense S-oligonucleotides were shown to clearly suppress cell proliferation compared with control by the MTT assay (Fig.2), suggesting that suppression of *COX17* reduces growth, proliferation and/or survival of cells. The result was also confirmed by focus formation (staining by Giemsa)(data not shown).
NSC 1273:*FLJ32549*; two effective antisense S-oligonucleotides, AS1 (SEQ ID NO:527) and AS2 (SEQ ID NO: 529), and reverse S-oligonucleotides (control), R1 (SEQ ID NO:528) and R2 (SEQ ID NO: 530), corresponding to *FLJ32549* were synthesized. Each of the S-oligonucleotides was transfected into NSCLC cell lines A549, NCI-H226 and NCI-H522, which showed highest expression of *FLJ32549.* Two days after transfection, the antisense S-oligonucleotides were shown to clearly suppress cell proliferation compared with controls by focus formation (staining by Giemsa)(data not shown), suggesting that suppression of *FLJ32549* reduces growth, proliferation and/or survival of cells.
NSC 1389:*NMU*; an effective antisense S-oligonucleotide AS (SEQ ID NO:531) and reverse S-oligonucleotide (control) R (SEQ ID NO:532), corresponding to *NMU* were synthesized. Each of the S-oligonucleotides was transfected into NSCLC cell lines A549 and LC319, which showed highest expression of *NMU*. Two days after transfection, the antisense S-oligonucleotide clearly suppressed cell proliferation compared with control by the MTT assay (Fig.2), suggesting that suppression of *NMU* reduces growth, proliferation and/or survival of cells. The result was also confirmed by focus formation (staining by Giemsa)(data not shown).

**Table5; Sequences of the effective pairs of S-oligonucleotides**

| **NSC Assign ment** | **Symbol** | **No.** | **S-oligo AS** | **SEQ ID NO:** | **S-oligo REV(control)** | **SEQ ID NO:** |
|---|---|---|---|---|---|---|
| 810 | TTK | | 5'-TAAATCCTCGGATTC CAT-3' | 423 | 5'-TACCTTAGGCTCCTAA AT-3' | 424 |
| 811 | SDC1 | 1 | 5'-CGCCGCGCGCCTCA TGCT-3' | 425 | 5'-TCGTACTCCGCGCGC CGC-3' | 426 |
| | | 2 | 5'-CGGCCGCACTCACC GGCA-3' | 427 | 5'-ACGGCCACTCACGCC GGC-3' | 428 |
| | | 4 | 5'-ACGACTGCTTGAAA GAGG-3' | 429 | 5'-GGAGAAAGTTCGTCA[?] GCA-3' | 430 |
| 812 | NMB | 1 | 5'-AGTGCACTCGGATC TTGCT-3' | 431 | 5'-TCGTTCTAGGCTCAC GTGA-3' | 432 |
| | | 2 | 5'-GCCTCCTGTACTGGA TTT-3' | 433 | 5'-TTTAGGTCATGTCCTC CG-3' | 434 |
| 816 | PIR51 | 1 | 5'-TTGACTGGTTTCTTA TGT-3' | 435 | 5'-TGTATTCTTTGGTCAG TT-3' | 436 |
| 825 | ANLN | 1 | 5'-CTCCGTAAACGGAT CCAT-3' | 437 | 5'-TACCTAGGCAAATGC CTC-3' | 438 |
| | | 3 | 5'-CGGATCCATCGCCCC AGG-3' | 439 | 5'-GGACCCCGCTACCTA GGC-3' | 440 |
| | | 5 | 5'-ACCAAAGACGCATC ATCA-3' | 441 | 5'-ACTACTACGCAGAAA CCA-3' | 442 |
| 841 | URLC2 | 4 | 5'-CCCTCGATTCCTCCG AGT-3' | 443 | 5'-TGAGCCTCCTTAGCT CCC-3' | 444 |
| | | 5 | 5'-AACTGCCACACAGT AGTA-3' | 445 | 5'-ATGATGACACACCGT CAA-3' | 446 |
| 857 | TIGD5 | 3 | 5'-ATCCTCGCTGTCCAG GGC-3' | 447 | 5'-CGGGACCTGTCGCTC CTA-3' | 448 |
| | | 4 | 5'-CGTCCAGGTGCAGC CACT-3' | 449 | 5'-TCACCGACGTGGACC TGC-3' | 450 |
| 859 | URLC3 | 2 | 5'-GTTCCCATTCAAGA ACAT-3' | 451 | 5'-TACAAGAACTTACCC TTG-3' | 452 |
| | | 3 | 5'-CATGAGTGATGGTG GCTC-3' | 453 | 5'-CTCGGTGGTAGTGAG TAC-3' | 454 |
| | | 5 | 5'-CCTCTCCCATGGCTT CAA-3' | 455 | 5'-AACTTCGGTACCCTC TCC-3' | 456 |
| 885 | BAG5 | 1 | 5'-GGACAGGAACCAAT GTAC-3' | 457 | 5'-CATGTAACCAAGGAC AGG-3' | 458 |
| | | 2 | 5'-ACAATACAATGTGA CAAG-3' | 459 | 5'-GAACAGTGTAACATA ACA-3' | 460 |
| 893 | MPHOSPH1 | 1 | 5'-AGATTCCATTCTGCA AAC-3' | 461 | 5'-CAAACGTCTTACCTT AGA-3' | 462 |
| | | 2 | 5'-GATTAAAATTAGATT CCAT-3' | 463 | 5'-TACCTTAGATTAAAAT TAG-3' | 464 |
| 905 | URLC1 | 2 | 5'-CATCTTGAGATCCTA TTC-3' | 465 | 5'-CTTATCCTAGAGTTCT AC-3' | 466 |
| | | 3 | 5'-TGGGGGCTTTTTACT CAT-3' | 467 | 5'-TACTCATTTTTCGGGG GT-3' | 468 |
| | | 5 | 5'-AGGTACTTTAAACC ACTT-3' | 469 | 5'-TTCACCAAATTTCATG GA-3' | 470 |
| 909 | FLJ10468 | 1 | 5'-AGGAGCCATGGCGC TCGG-3' | 471 | 5'-GGCTCGCGGTACCGA GGA-3' | 472 |
| 920 | CHAF1A | 1 | 5'-GCAATCCATGGCTGT GGC-3' | 473 | 5'-CGGTGTCGGTACCTA ACG-3' | 474 |
| | | 4 | 5'-AATAATTACCTTGTA TTA-3' [?] | 475 | 5'-TACCTAACGTTTCTAT CT-3' | 476 |
| 947 | PKP3 | 1 | 5'-GAAGTTACCGTCCT GCAT-3' | 477 | 5'-TACGTCCTGCCATTGA AG-3' | 478 |
| | | 2 | 5'-GCAGGAAGTTACCG TCCT-3' | 479 | 5'-TCCTGCCATTGAAGG ACG-3' | 480 |
| | | 3 | 5'-GTTGTTGAGCACAG CTAT-3' | 481 | 5'-TATCGACACGAGTTG TTG-3' | 482 |
| | | 4 | 5'-GAAGTCCTCCTTCC GATA-3' | 483 | 5'-ATAGCCTTCCTCCTGA, AG-3' | 484 |
| 956 | SIAHBP1 | 1 | 5'-CCGTCGCCATCTTGC GTC-3' | 485 | 5'-CTGCGTTCTACCGCT GCC-3' | 486 |
| | | 2 | 5'-TATGGTCGCCGTCGC CAT-3' | 487 | 5'-TACCGCTGCCGCTGG TAT-3' | 488 |
| 994 | DKFZp434E 2318 | 1 | 5'-GGACTGCATGGTGG AGAT-3' | 489 | 5'-TAGAGGTGGTACGTC AGG-3' | 490 |
| | | 3 | 5'-CATGGTGGAGATGG CGAC-3' | 491 | 5'-CAGCGGTAGAGGTGG TAC-3' | 492 |
| | | 4 | 5'-AGCAGGGCTGCAGA ATGG-3' | 493 | 5'-GGTAAGACGTCGGGA CGA-3' | 494 |
| | | 5 | 5'-TGCTCTTGAAGTCG GGAC-3' | 495 | 5'-CAGGGATGAAGTTCT CGT-3' | 496 |
| 1075 | URLC4 | 5 | 5'-GCAGTTGAGATGATT ATT-3' | 497 | 5'-TTATTAGTAGAGTTGA CG-3' | 498 |
| 1107 | URLC8 | 1 | 5'-CAAAATCATTTCCTC CTC-3' | 499 | 5'-CTCCTCCTTTACTAAA AC-3' | 500 |
| | | 4 | 5'-CTTGTTCATGAACAT CTCT-3' | 501 | 5'-AAGGCACTACCACCG GGC-3' | 502 |
| 1113 | URLC5 | 1 | 5'-ACGATTCATTGCTGC CTT-3' | 503 | 5'-TTCCGTCGTTACTTAG CA-3' | 504 |
| | | 2 | 5'-ACACAAGACACGAT TCAT-3' | 505 | 5'-TACTTAGCACAGAAC ACA-3' | 506 |
| 1131 | SYNJ2BP | 1 | 5'-ATCCACTCTTCCGTT CAT-3' | 507 | 5'-TACTTGCCTTCTCACC TA-3' | 508 |
| 1142 | NAPG | 1 | 5'-AGCCGCCATCTCCA CAGT-3' | 509 | 5'-TGACACCTCTACCGC CGA-3' | 510 |
| 1183 | BYSL | 1 | 5'-CTTGTTCATGAACAT CTCT-3' | 511 | 5'-TCTCTACAAGTACTTG TTC-3' | 512 |
| | | 2 | 5'-TGGCAGGAGGGTTC TTGT-3' | 513 | 5'-TACTTGTTCTTGGGA GGA-3' | 514 |
| | | 3 | 5'-CAGGCCTACCTGGC AGGA-3' | 515 | 5'-AGGACGGTCCATCCG GAC-3' | 516 |
| 1185 | URLC6 | 4 | 5'-ACCGCTTACGGTTG GCTG-3' | 517 | 5'-GTCGGTTGGCATTCG CCA-3' | 518 |
| | | 6 | 5'-TCTGAAGAAAATAG ATCA-3' | 519 | 5'-ACTAGATAAAAGAAG TCT-3' | 520 |
| 1191 | COX17 | 2 | 5'-GTCAACCAGACCCG GCAT-3' | 521 | 5'-TACGGCCCAGACCAA CTG-3' | 522 |
| | | 4 | 5'-TCTCCTTTCTCGATC ATA-3' | 523 | 5'-ATAGTAGCTCTTTCCT CT-3' | 524 |
| | | 5 | 5'-ATTCCTTGTGGGCCT CAA-3' | 525 | 5'-AACTCCGGGTGTTCC TTA-3' | 526 |
| 1273 | FLJ32549 | 1 | 5'-CCCATGCGAGCTGC GCC-3' | 527 | 5'-CCGCGTCGAGCGTAC CC-3' | 528 |
| | | 2 | 5'-AGTGATAAACAGAA AGCG-3' | 529 | 5'-GCGAAAGACAAATAG TGA-3' | 530 |
| 1389 | NMU | | 5'-TATCCTCGACTTTGA CTT-3' | 531 | 5'-TTCAGTTTCAGCTCCT AT-3' | 532 |

### (4) RNA interference assay

A vector-based RNAi system, psiH1BX3.0, which directs the synthesis of small interfering RNAs (siRNAs) in mammalian cells was used for suppressing the expression of each of the endogenous genes in NSCLC cells. Five vectors were designed for directing the synthesis of five different 19-base pair double-stranded nucleotides against the target sequence of each of the genes. The vectors were transfected into four NSCLC cell lines using Lipofectamin 2000 reagent (Invitrogene, Carlsbad, CA, USA), which resulted in transfection with more than 60-90% transfection efficiency. Cells were cultured for 5-9 days in the presence of an appropriate concentration of geneticin (G418). The cell numbers or cell viability was determined upon Giemsa staining and/or MTT assay in triplicate.

### (5) Flow cytometry

Cells were plated at a density of X 10⁵ cells/100-mm dish and transfected with siRNA-expression vector as mentioned above. 24-48 hours after the infection, the cells were trypsinized, collected in PBS and fixed in 70% cold ethanol for 30 min. After the treatment with 100 µg/ml RNase (Sigma Chemical Co.-Aldrich, St. Louis, MO), the cells were stained with 50 µg/ml propidium iodide (Sigma-Aldrich) in PBS. Flow cytometry was performed on Becton Dickinson FACScan and analyzed by ModFit software (Verity Software House, Inc., Topsham, ME). The percentages of nuclei in G0/G1, S and G2/M phases of the cell cycle and sub-G1 population were determined from at least 20,000 ungated cells. Apoptosis was also detected by flow cytometry based on the binding to annexin V

### (6) RNAi

To identify and characterize new molecular targets that regulate growth, proliferation and survival of cancer cells, the RNA interferance technique was conducted using the psiH1BX3.0 vector to suppress the endogenous expression of respective candidate genes that were selected through the above process. Specific methods and results for each of the candidate genes was as follows:
NSC 807:KOC1: Three effective vectors directing the synthesis of 19-base pair double-stranded sequences targeting the gene were designed and transfected into NSCLC cell lines A549 and LC319. Five days after transfection, the introduction of the RNAi vector (No.1) against this gene clearly suppressed the number of colony compared with control (Fig.3A and 3B), suggesting that suppression of *KOC1* reduces growth, proliferation and/or survival of cells. The result was also confirmed by focus formation assay (Giemsa staining)(data not shown).
NSC 810:TTK: Three effective vectors directing the synthesis of 19-base pair double-stranded sequences targeting the gene were designed and transfected into NSCLC cell lines A549 and LC319. Five days after transfection, the introduction of the RNAi vector (TTK-1) against this gene was shown to clearly suppress cell proliferation compared with control by MTT assay (Fig.3A and 3B), suggesting that suppression of *TTK* reduces growth, proliferation and/or survival of cells. The result was also confirmed by focus formation assay (Giemsa staining)(data not shown). The LC319 cells transfected with effective TTK RNAi was further examined under microscopy and images were captured every 24 or 48 days. The TTK RNAi transfected LC319 cells showed multi-nucleated cell phenotype and underwent complete cell death, whereas cells transfected with EGFP RNAi showed mono-nucleated cell phenotype (Fig 3C). By Western blot analysis using anti-TTK monoclonal antibodies, the expression of native TTK protein and its suppression by TTK RNAi in LC319 cells were detected (Fig 3E).
NSC 825:ANLN: Two effective vectors directing the synthesis of 19-base pair double-stranded sequences targeting the gene were designed and transfected into NSCLC cell lines A549 and LC319. Five days after transfection, the introduction of these RNAi vectors against this gene was shown to clearly suppress cell proliferation compared with control by MTT assay (Fig.3A and 3B), suggesting that suppression of *ANLN* reduces growth, proliferation and/or survival of cells. The result was also confirmed by focus formation assay (Giemsa staining)(data not shown). The LC319 cells transfected with any of the two effective ANLN RNAi had multi-nucleated cell phenotype and underwent complete cell death, whereas cells transfected with EGFP RNAi showed mono-nucleated cell phenotype (Fig 3C). The cell cycle profile of ANLN RNAi transfected cells determined by flow cytometry showed abnormal cell cycle and polyploidy (>4N DNA content)(Fig 3D).
NSC 841:URLC2: Two effective vectors directing the synthesis of 19-base pair double-stranded sequences targeting the gene were designed and transfected into NSCLC cell lines A549 and LC319. Five days after transfection, the introduction of these RNAi vectors against this gene was shown to clearly suppress cell proliferation compared with control by MTT assay (Fig.3A and 3B), suggesting that suppression of *URLC2* reduces growth, proliferation and/or survival of cells. The result was also confirmed by focus formation assay (Giemsa staining)(data not shown). Flow cytometry was conducted 24 hours after the transfection of the siRNA. As a result, 28 % increase of the sub-G1 populations of LC319 cells was detected. The URLC2-siRNA markedly induced apoptosis which was also assessed by flowcytometric analysis of annexin V binding (Fig 3D).
NSC 903:LTRLC9: Two effective vectors directing the synthesis of 19-base pair double-stranded sequences targeting the gene were designed and transfected into NSCLC cell lines A549 and LC319. Five days after transfection, the introduction of these RNAi vectors against this gene clearly suppressed the number of colony compared with control (Fig.3A and 3B), suggesting that suppression of *URLC9* reduces growth, proliferation and/or survival of cells. The result was also confirmed by focus formation assay (Giemsa staining)(data not shown).
NSC 956:SIAHBP1: Two effective vectors directing the synthesis of 19-base pair double-stranded sequences targeting the gene were designed and transfected into NSCLC cell lines A549 and LC319. Five days after transfection, the introduction of these RNAi vectors against this gene was shown to clearly suppress cell proliferation compared with control by MTT assay (Fig.3A and 3B), suggesting that suppression of *SIAHBP1* reduces growth, proliferation and/or survival of cells. The result was also confirmed by focus formation assay (Giemsa staining)(data not shown).
NSC 994:DKFZP434E2318: Two effective vectors directing the synthesis of 19-base pair double-stranded sequences targeting the gene were designed and transfected into NSCLC cell lines A549 and LC319. Five days after transfection, the introduction of the RNAi vector (No.1) against this gene was shown to clearly suppress cell proliferation compared with control by MTT assay (Fig.3A and 3B), suggesting that suppression of *DKFZP434E2318* reduces growth, proliferation and/or survival of cells. The result was also confirmed by focus formation assay (Giemsa staining)(data not shown).
NSC 1107:URLC8: Five effective vectors directing the synthesis of 19-base pair double-stranded sequences targeting the gene were designed and transfected into NSCLC cell lines A549 and LC319. Five days after transfection, the introduction of these RNAi vectors against this gene clearly suppressed the number of colony compared with control (Fig.3A and 3B), suggesting that suppression of *URLC8* reduces growth, proliferation and/or survival of cells. The result was also confirmed by focus formation assay (Giemsa staining)(data not shown).
NSC 1191:COX17: Two effective vectors directing the synthesis of 19-base pair double-stranded sequences targeting the gene were designed and transfected into NSCLC cell lines A549 and LC319. Five days after transfection, the introduction of the RNAi vector (No.2) against this gene was shown to clearly suppress cell proliferation compared with control by MTT assay (Fig.3A and 3B), suggesting that suppression of *COX17* reduces growth, proliferation and/or survival of cells. The result was also confirmed by focus formation assay (Giemsa staining)(data not shown).
NSC 1246:SUPT3H: Three effective vectors directing the synthesis of 19-base pair double-stranded sequences targeting the gene were designed and transfected into NSCLC cell line A549. Five days after transfection, the introduction of the RNAi vector (No.2) against this gene was shown to clearly suppress cell proliferation compared with control by MTT assay (Fig.3A), suggesting that suppression of *SUPT3H* reduces growth, proliferation and/or survival of cells. The result was also confirmed by focus formation assay (Giemsa staining)(data not shown).
NSC 1389:NMU: Two effective vectors directing the synthesis of 19-base pair double-stranded sequences targeting the gene were designed and transfected into NSCLC cell lines A549 and LC319. Five days after transfection, the introduction of the RNAi vector (No.2) against this gene was shown to clearly suppress cell proliferation compared with control by MTT assay (Fig.3A and 3B), suggesting that suppression of *NMU* reduces growth, proliferation and/or survival of cells. The result was also confirmed by focus formation assay (Giemsa staining)(data not shown). Then, flow cytometry was performed 24 hours after the transfection of siRNA. As a result, 34.5 % increase in the sub-G1 populations of LC319 cells was detected (Fig.3D).
NSC 1395:FBN2: An effective vectors directing the synthesis of 19-base pair double-stranded sequences targeting the gene were designed and transfected into NSCLC cell lines A549 and LC319. Five days after transfection, the introduction of the RNAi vector (No.2) against this gene was shown to clearly suppress cell proliferation compared with control by focus formation assay (Giemsa staining)(data not shown)

### (7) Cytochrome c oxidase activity

Cytochrome c oxidase (CCO) activity and its inhibition by COX17 RNAi in A549 cells were examined. Schematic illustration explaining the method of measuring the CCO activity is shown in Fig.3F. Specifically, the cells were separated into mitochondria and other fractions using digitonin (Wako, Osaka, Japan). Cytochrome c (63mM) in buffer (10mM Tris, 0.2mM EDTA, 0.05% *n*-dodecyl-b-D-maltoside, pH7.6) was incubated with 12.5mM L(+)-ascorbic acid for 30min at room temperature (18 °C), to convert ferric cytochrome c to ferrous cytochrome c. Twenty micro-liters of 1mg/ml mitochondrial protein solution was then added to 2ml of the mix-ture at 37 °C. The reaction for CCO activity was measured at 550nm.

To clarify whether the native COX17 protein has cytochrome c oxidase (CCO) activity in human NSCLC cells, effective COX17 RNAi vectors were transfected into A549 cell line to detect the CCO activity. 2 or 5 days after transfection, the COX activity was reduced due to the suppression of the endogenous COX17 gene. The result confirmed the importance of the CCO activity exerted by COX17 in human NSCLC (Fig.3F, G and H).

Sequences of the synthetic oligonucleotides that are effective as an RNAi are shown in Table 6. 30 genes were identified which inhibition by transfection of an antisense S-oligonucleotide and/or RNAi results in the suppression of growth, proliferation and survival of cancer cells.

**Table6; Sequences of the effective synthetic oligonucleotides for RNAi**

| **NSC Assignment** | **Symbol** | **NO.** | **RNAi** | **SEQ ID No:** |
|---|---|---|---|---|
| 807 | KOC1 | 1 | 5'-GGACCAAGCTAGACAAGCA-3' | 533 |
| 810 | TTK | 1 | 5'-ACAGTGTTCCGCTAAGTGA-3' | 534 |
| 825 | ANLN | 1 | 5'-CCAGTTGAGTCGACATCTG-3' | 535 |
| | | 2 | 5'-GCAGCAGATACCATCAGTG-3' | 536 |
| 841 | URLC2 | 1 | 5'-CGCAGCTGCGAAGTGTTGTA-3' | 537 |
| | | 2 | 5'-GATACGAAAGCAGCTGCGA-3' | 538 |
| 903 | URLC9 | 1 | 5'-GAGCGATTCATCTTCATCA-3' | 539 |
| | | 2 | 5'-CTGCAATTGAGGCTCCTTC-3' | 540 |
| 956 | SIAHBP1 | 1 | 5'-GAGTGTGCTGGTGAAGCAG-3' | 541 |
| | | 2 | 5'-GATCAAGTCCTGCACACTG-3' | 542 |
| 994 | DKFZp434E2318 | 1 | 5'-CGTGCTAGCAGCTGCGTGT-3' | 543 |
| 1107 | URLC8 | 1 | 5'-TGAGGTGCTCAGCACAGTG-3' | 544 |
| | | 2 | 5'-CGGAGGATCTCATGACCAC-3' | 545 |
| | | 3 | 5'-GATTCGCATCCTGCCATCG-3' | 546 |
| | | 4 | 5'-CAGTATTCGGACATAGAGG-3' | 547 |
| | | 5 | 5'-CACCAAGTACTGCTTGTGC-3' | 548 |
| 1191 | COX17 | 2 | 5'-GGAGAAGAACACTGTGGAC-3' | 549 |
| 1246 | SUPT3H | 2 | 5'-GACAAATTGAGTGGCAGCA-3' | 550 |
| 1389 | NMU | 2 | 5'-GAGATTCAGAGTGGACGAA-3' | 551 |
| 1395 | FBN2 | 2 | 5'-GAGAGCAATGAGGATGACT-3' | 552 |

### (8) Immunocytochemical analysis

To prepare c-myc-His tagged proteins, vectors containing genes encoding the c-myc-His epitope sequence (LDEESILKQE-HHHHHH) at the C-terminus of each protein were constructed and transfected into COS-7 cells. The transiently transfected COS-7 cells replated on chamber slides were fixed with PBS containing 4% paraformaldehyde, then rendered permeable with PBS containing 0.1% Triton X-100 for 3 min at 4°C. The cells were covered with blocking solution (2% BSA in PBS) for 30 min at room temperature to block nonspecific antibody-binding sites. Then the cells were incubated with mouse anti-c-myc antibody (diluted 1: 800 in blocking solution). The antibody was stained with goat anti-mouse secondary antibody conjugated with FTTC to observe them under ECLIPSE E800 microscope (Nikon). To confirm the expression of the c-myc-tagged proteins in transfected cells, Western-blotting was conducted as described previously (Shiratsuchi et al., Biochem Biophys Res Commun 247: 597-604 (1998)).

### (9) Localization of product of potential target genes in mammalian cells

To investigate cellular localization of proteins encoded by these candidate genes in mammalian cells, COS-7 cells were transfected with pcDNA3.1 (+)/c-myc-His, a plasmid containing a gene encoding the c-myc-His-epitope sequence (LDEESILKQE-HHHHHH) at the C-terminus of each of the proteins. Using anti-c-myc antibodies, 24 proteins were detected in individual subcellular locations. The expression of some of the proteins in transfected cells were confirmed by immunoblotting (Fig 5A).

### (10) Selection of transmembrane/secretary proteins as targets for anti-cancer therapy and diagnosis

14 transmembrane/secretary proteins that may be overexpressed on the surface of tumor cells were screened. These proteins are expected to be a good target for receptor-targeted/antibody-based therapeutics and diagnostics for cancer. The expression and cellular localization of some of the proteins in transfected COS-7 cells were confirmed by immunocytochemical analysis.

To determine the subcellular localization of the protein encoded by each of the genes, COS-7 cells were transfected with plasmids expressing the proteins tagged with c-myc-His or Flag. Result of the immunocytochemical analysis on each of the genes are given below:
NSC 807:KOC1: ROC1/c-myc-His protein was mainly detected in the cytoplasm (data not shown).
NSC 810:TTK: TTK/c-myc-His protein was mainly detected in the nucleus (data not shown).
NSC 825:ANLN: ANLN /c-myc-His protein was mainly detected in the nucleus and cytoplasm (data not shown).
NSC 841:URLC2: URLC2/c-myc-His protein was mainly detected in the nucleus and cytoplasm (data not shown).
NSC 849:GJB5: GJB5/c-myc-His protein was mainly detected in the cytoplasmic membrane (Fig.5A).
NSC 855:LNIR: LNIR/c-myc-His protein was mainly detected in the cytoplasmic membrane (Fig.5A).
NSC 895:FAM3D: FAM3D/c-myc-His protein was mainly detected in the cytoplasmic granules, golgi and cytoplasmic membrane (Fig.5A). Secretion of FAM3D in culture medium was detected by Western blotting (Fig.5B). Thus, FAM3D was supposed to be a secretory protein.
NSC 903:URLC9: URLC9/c-myc-His protein was mainly detected in the nucleus (data not shown).
NSC 915:URLC10: URLC10/c-myc-His protein was mainly detected in the cytoplasmic granule and golgi, and also as dots on the surface of the cytoplasmic membrane (Fig.5A).
NSC 948:TASK-2: TASK-2/c-myc-His protein was mainly detected in the cytoplasmic membrane (Fig.5A).
NSC 956:SIAFMP1: SLAHBP1/c-myc-His protein was mainly detected in the cytoplasm (data not shown).
NSC 994:DKFZp434E2318: DKFZp434E2318/c-myc-His protein was mainly detected in the cytoplasm (data not shown).
NSC 1000:PSK-1: PSK-1/c-myc-His protein was mainly detected in the cytoplasmic membrane (Fig.5A).
NSC 1103:KCNK1: KCNK1/c-myc-His protein was mainly detected in the cytoplasmic membrane (Fig.5A).
NSC 1107:URLC8: URLC8/c-myc-His protein was mainly detected in the nucleus (data not shown).
NSC 1164:NPTX1: NPTX1/c-myc-His protein was mainly detected in the cytoplasmic granules (Fig.5A). Secretion of NPTX1 in culture medium was detected by Western blotting (Fig.5B). Thus, NPTX1 was supposed to be a secretory protein.
NSC 1191:COX17: COX17/c-myc-His protein was mainly detected in the mitochondoria (data not shown).
NSC 1201:SLC7A1: SLC7A1/c-myc-His protein was mainly detected in the cytoplasmic membrane and golgi (Fig.5A).
NSC 1246:SUPT3H: SUPT3H/c-myc-His protein was mainly detected in the nucleus and cytoplasm (data not shown).
NSC 1288:PTGFRN: PTGFRN/c-myc-His protein was mainly detected in the cytoplasmic membrane and golgi (Fig.5A).
NSC 1295:ADAM8: ADAM8/c-myc-His protein was mainly detected in the cytoplasmic membrane (Fig.5A). Secretion of three cleavaged forms of ADAM8 in culture medium was detected by Western blotting (Fig.5B). Thus, ADAM8 was supposed to be a secretory protein.
NSC 1389:NMU: NMU/c-myo-His protein was mainly detected in the golgi body and as a secreted protein (Fig.5A).
NSC 1420:CHDOL: CHDOLJc-myc-His protein was mainly detected in the cytoplasmic membrane and golgi (Fig.5A).
NSC 1441:HSCOV: HSNOV/c-myc-His protein was mainly detected in the cytoplasmic membrane and golgi (Fig.5A).

### (12) Cell growth assay and colonv formation assay

Stable transfectants were established according to a standard protocol. Specifically, after the transfection of plasmids expressing the target gene (pcDNA3.1/myc-His) or a complementary strand of the gene (pcDNA3.1-antisense), or mock plasmids (pcDNA3.1) into COS-7 cells, the cells were cultured with geneticin (G418) for 14 days. Then, colonies were selected and the expression of the gene was detected by Western blotting. Established stable transfectants were confirmed to be monoclonal by immunostaining with anti-c-myc antibody (data not shown). The stable transfectants of COS-7 cells were seeded on a 6-well microtiterplate (5 X 10⁴ cells/well), and maintained in media containing 10% FBS supplemented antibiotics for 24, 48, 72, 96, 120 and 144 hours. At each of the time points, the cell proliferation activity was evaluated using Cell Counting kit (WAKO) or by MTT assay.

### (13) Cell growth assay of stable transformant and autocrine assay

NSC 810:TTK; to determine the effect of TTK on mammalian cell growth, COS-7 cells expressing exogenous TTK (COS-7-TTK1 and 2) were established and their growth was compared with that of control cells transfected with mock vector (TTK-mock). As shown in Fig 6, the growth of the COS-7-TTK cells were markedly promoted compared with that of the control cells in accordance with the expression level of the pcDNA3.1-TTK-c-myc-His protein. The result was confirmed by three independent experiments. The COS-7-TTK cells also exhibited a remarkable tendency to form larger colonies compared with the control cells (data not shown).
NSC 841:URLC2; to determine the effect of URLC2 on mammalian cell growth, NIH3T3 cells expressing exogenous URLC2 (NTH3T3-URLC2-3 and 5) were established and their growth was compared with that of control cells transfected with mock vector (NIH3T3-mock). As shown in Fig 6, the growth of the NIH3T3-URLC2 cells were markedly promoted compared with that of the control cells in accordance with the expression level of the pcDNA3.1-URLC2-myc-His protein. The result was confirmed by three independent experiments. The NIH3T3-URLC2 cells also showed a remarkable tendency to form larger colonies compared with the control cells (data not shown).
NSC 1389:NMU; to determine the effect ofNMU on mammalian cell growth, COS-7 cells expressing exogenous NMU (COS-7-NMU-2, 3 and 5) were established and their growth was compared with that of control cells transfected with antisense strand or mock vector (COS-7-AS-1 and 2; COS-7-mock). As shown in Fig 6, the growth of the COS-7-NMU cells were markedly promoted compared with that of the control cells in accordance with the expression level of the pcDNA3.1-NMU-c-myc/His protein. The result was confirmed by four independent experiments. The COS -7-NMU cells also showed a remarkable tendency to form larger colonies compared with the control cells. The result suggested that overexpressed NMU have transforming effect on the mammalian cells.

### (14) Autocrine assay

To confirm the autocrine function ofNMU in cell growth, COS-7 cells were cultured in medium containing the active form of the 25 amino acid polypeptide of NMU (NMU-25) (Alpha diagnostic international: ADI) at a final concentration of 1 µg∼50 µg (3 µM∼15 µM/ml). Medium containing bovine serum albumin (BSA) at the same concentration served as a control. The polypeptides or BSA was added at every 48 hours for 7days. At the time point of 24, 48, 72, 96, 120 and 144 hours, the cell viability was measured by MTT assay. To confirm the growth promoting effect of NMU protein on COS-7 cells, anti-NMU antibody was added at a final concentrations of 0.5 µM∼7.5 µM/ml into the culture media containing 3 µM/ml of NMU-25.

As a result, the COS-7 cells incubated with NMU-25 showed larger and faster cell growth compared to those with BSA in a dose dependent manner (Fig 7A).

Next, anti-NMU antibody was added at a final concentrations of 0.5 µM∼7.5 µM/ml into the culture media of COS-7 containing 3 µM/ml of NMU-25. According to the MTT assay, the COS-7 cells incubated with NMU-25 and anti-NMU antibody were shown to exhibit a slower cell growth compared to those with controls in a dose dependent manner (Fig 7B).

Furthermore, anti-NMU antibody was added at the same concentration in the culture media for LC319 cells, which overexpress endogenous NMU. By the MTT assay, the LC319 cells incubated with anti-NMU antibody were demonstrated to show slower cell growth compared to those with controls in a dose dependent manner (Fig 7C).

### (15) Immunohistochemical analysis

To examine the expression of the proteins in clinical tissue samples including normal lung and NSCLCs, sections were stained using ENVISION+ Kit/HRP (DAKO). Specifically, following the endogenous peroxidase and protein blocking reactions, anti-human antibody was added as the primary antibody and then the tissue samples were treated with HRP labeled anti-rabbit IgG as the secondary antibody. Then, chromogen was added as the substrate to counterstain the tissue specimens with hematoxylin.

To confirm overexpression of the TTK protein in NSCLC, the protein in NSCLC cell lines, A549, LC319 and NCI-H522, was first identified by Western blotting analysis (Fig 8). Then, immunohistochemical staining was conducted for each of the genes as follows:
NSC 947:PKP3; immunohistochemical staining was carried out with anti-PFCP3 antibody on surgically obtained NSCLC (squamous cell carcinoma) samples, which had been frozen and embedded in OCT medium. Cytoplasm of all tumor tissue samples were mainly stained with the anti-PKP3 antibody, whereas normal lung tissues were not stained (Fig 9).
NSC 1164:NPTX1; immunohistochemical staining was carried out with anti-NPTX1 antibody on surgically obtained NSCLC samples, which had been frozen and embedded in OCT medium. Cytoplasm of all tumor tissue samples was mainly stained with the anti-NPTX1 antibody, whereas normal lung tissues were not stained(Fig 9).
NSC 1295:ADAM8; immunohistochenical staining was carried out with anti-ADAM8 antibody on surgically obtained NSCLC samples, which had been frozen and embedded in OCT medium. All tumor tissue samples were strongly stained with the anti-ADAM8 antibody, whereas normal lung tissues were weakly stained (Fig 9).
NSC 1389:NMU; immunohistochemical staining was carried out with anti-NMU antibody on surgically obtained NSCLC samples, which had been frozen and embedded in OCT medium. Cytoplasm of all tumor tissue samples were mainly stained with the anti NMU antibody, whereas normal lung tissues were not stained. In adenocarcinoma samples, NMU was detected in duct cells and in squamous cell carcinomas around the nucleus, especially at the cytoplasmic granules (Fig 9).

### (16) Full-length sequencing, Northern blotting and semiquantitative RT-PCR analyses of target genes

By combining the list of over-expressed genes that showed 5-fold elevation in their expression in more than 50% of NSCLCs compared to those in 34 normal tissues, 642 candidate genes were selected as tumor markers or therapeutic targets, which are specifically expressed in NSCLCs but not in normal tissues, except reproductive tissues or fetal organs which are not critical for the survival or can be replaced. The full-length sequences of the target genes were determined by EST screening, and their gene expression patterns were confirmed in tumor and normal tissues using semiquantitative RT-PCR.

A novel genes URLC1 were found. It's nucleotide sequence and the amino acid sequence encoded thereby are shown with following SEQ ID NOs in the sequence listing:

| | Nucleic sequence | Amino acid sequence |
|---|---|---|
| URLC1 | SEQ ID NO:1 | SEQ ID NO:2 |

The results obtained above are summarized below for each of the genes of the present invention:
NSC 807: KOC1; this gene encodes an hnRNA K-homology (KH) domain and an RNA recognition motif (RRM) domain. The function of the KH domain to bind to the 5'UTR of the IGF-II (IGF2) leader 3' mRNA, may repress the translation of IGF-II during late development. The lower expression of this gene in normal tissues, high expression in NSCLCs, and reduced growth, proliferation and/or survival of the transfected cells by the suppression of this gene suggest usefulness of this gene as a novel diagnostic marker and target for new drugs and immunotherapy.
NSC 810: TTK; this gene encodes an S TKc domain. The protein encoded by the gene phosphorylates proteins on serine, threonine and tyrosine, which phosphorylation probably is associated with cell proliferation. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy. According to the present invention, the TTK protein expressed in stable transfectant promoted the growth of COS-7 cells in a dose dependent manner. This result suggested that overexpression of TTK exerts a transforming effect on mammalian cells. These data reveal that TTK might be a novel oncogene for NSCLC and suggest that a promising therapeutic strategy for treating lung cancers may be established by targeting TTK.
NSC 811: SDC1; this gene encodes a putative band 4.1 homologues' binding motif (4.1m) domain. The protein encoded by the gene is a cell surface proteoglycan, syndecan, which is an integral membrane protein acting as a receptor for extracellular matrix. It belongs to the group of transmembrane heparan sulfate proteoglycans. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 812:NMB; this gene encodes a signal peptide and a transmembrane domain. The protein encoded by the gene functions as a neuromedin B, a member of the bombesin family, which is an autocrine growth factors for lung carcinomas. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 816: PIR51; the protein encoded by the gene is localized in the nucleus and no domain was found in the protein. The protein functions as a DNA- and RNA-binding protein; interacts with the RAND51 recombinase protein involved in DNA recombination and repair. The lower expression of this gene in normal tissues, high expression in NSCLCs, and reduced growth, proliferation and/or survival of the transfected cells by the suppression of this gene suggest that this gene might be useful as novel diagnostic marker and target for new drugs and immunotherapy.
NSC 825: ANLN; this gene encodes a PH domain, several putative functions of which have been suggested: (1) binding to the beta/gamma subunit of heterotrimeric G proteins; (2) binding to lipids, e.g., phosphatidylinositol-4,5-bisphosphate; (3) binding to phosphorylated Ser/Thr residues; and (4) attachment to membranes by an unknown mechanism The gene encodes an actin binding protein that interacts with cleavage furrow proteins, such as septins, and may play a role in cytokinesis. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 841: URLC2; this gene encodes a Jmjc domain (a domain family that is a part of the cupin metalloenzyme). The protein encoded by the gene probably is an enzyme with unknown functions that regulates the chromatin reorganization processes. The lower expression of this gene in normal tissues, high expression in NSCLCs, and reduced growth, proliferation and/or survival of the transfected cells by the suppression of this gene suggest that this gene might be useful as novel diagnostic marker and target for new drugs and immunotherapy. According to the present study, the suppression of URLC2 induced apoptosis of LC319 cells. Moreover, URLC2 protein expressed in stable transfectant promoted the growth of NIH3T3 cells in a dose dependent manner. This result suggests that overexpressed URLC2 have a transforming effect on mammalian cells. These data reveal that URLC2 might be a novel oncogene for NSCLC and suggest that a promising therapeutic strategy for treating lung cancers can be established by focusing on the URLC2.
NSC 849:GJB5; this gene encodes a gap junction protein, beta 5 (connexin 31.1). GJB5 is a member of the connexin family (beta-type (group i) subfamily). It is reported that one gap junction consists of a cluster of closely packed pairs of transmembrane channels, connexons, through which materials of low molecular weight diffuse from one cell to a neighboring cell. A connexon is composed of a hexamer of connexins. The protein encoded by the gene was mainly detected in the cytoplasmic membrane by the immunocytochemical analysis. The lower expression of this gene in normal tissues and high expression in NSCLCs suggest that this gene might be useful as a diagnostic marker (i.e., in diagnosis using serum or sputum) and therapeutic target for NSCLCs.
NSC 855: LNIR; this gene encodes a signal peptide, immunoglobulin, immunoglobulin C2 domain and one transmembrane domain. The transmembrane protein encoded by the gene is supposed to be overexpressed on the surface of tumor cells but not on normal cells. Thus, the gene may be a good target for receptor-targeted therapy or diagnosis.
NSC 857: TIGD5; this gene encodes a Centromere Protein B (CENP-B). CENP-B is a DNA-binding protein localized to the centromere. Within the N-terminal 125 residues of the protein, there is a DNA-binding domain, which binds to a corresponding 17bp CENP-B box sequence. In the C-terminal 59 residues, CENP-B has a dimerization domain. CENP-B dimers binds either a two separate DNA molecule or two CENP-B boxes on one DNA molecule, with the intervening stretch of DNA forming a loop structure. This gene belongs to the tigger subfamily of the pogo superfamily of DNA-mediated transposons in humans. The proteins belonging to this subfamily are related to DNA transposons found in fungi and nematodes, and more distantly to the Tc1 and mariner transposases. The protein encoded by the gene is also very similar to the major mammalian centromere protein B. The exact function of this gene is unknown. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 859: URLC3; this gene does not encode any known domain, and the protein encoded by the gene has 70% similarity over 56 amino acids to an eukaryotic translation initiation factor 3 subunit (Homo sapiens). The subunit binds to the 40s ribosome and promotes the binding of methionyl-tRNAi and mRNA (by similarity). The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 885: BAG5; this gene encodes a BAG domain. Thus, the protein encoded by this gene is a member of the BAG1-related protein family. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 893: MPHOSP1; this gene encodes a KISc domain and microtubule-dependent molecular motors that play important roles in intracellular transport of organelles and in cell division. The protein encoded by the gene belongs to the kinesin-like protein family and interacts with guanosine triphosphate (gtp)-bound forms of rab6a. and rab6b. The protein may act as a motor required for the retrograde rab6 regulated transport of golgi membranes and associated vesicles along microtubles. The protein has a microtubule plus end-directed motility, and is phosphorylated during the M-phase of the cell cycle. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 895: FAM3D; this gene encodes a protein having a signal peptide domain at the N-terminal end which is supposed to be a secretory protein, although its function remains to be elucidated. This protein was mainly detected in the cytoplasmic granules and golgi in the immunocytochemical analysis, suggesting that this protein might be secreted. The lower expression of this gene in normal tissues and high expression in NSCLCs suggest that this gene might be useful as a diagnostic marker (i.e., in the diagnosis using serum or sputum) for NSCLCs.
NSC 898: URLC7; the protein encoded by this gene is localized in the nucleus and no known domain existed in the protein. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 903: URLC9; the protein encoded by this gene was localized in the nucleus and no known domain existed in the protein. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 905: URLC 1; this gene encodes a TUDOR domain, for which several putative functions have been suggested: (1) RNA-binding; and (2) nucleic acid binding. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 909: FLJ10468; the protein encoded by this gene was localized in the nucleus and no known domain existed in the protein. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 915: URLC10; this gene encodes 2 transmembrane domains. The protein encoded by the gene has a region with low similarity to GML. The protein was mainly detected in the cytoplasmic granule and golgi, and as dots on the surface of the cytoplasmic membrane by immunocytochemical analysis. This transmembrane protein is supposed to be overexpressed on the surface of tumor cells but not on normal cells. Thus, the protein may serve as a good target for receptor-targeted therapy or diagnosis.
NSC 920: CHAF1A; no known domain has been detected to be encoded by the gene. The protein encoded by the gene has a 150 kDa subunit of chromatin assembly factor 1, which helps deposit of histones H3 acetylated H4 onto replicating DNA. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 947: PKP3; the gene encodes an armadillo/beta-catenin-like repeats domain (ARM). The armadillo repeat is an approximately 40 amino acid long tandemly repeated sequence motif first identified in the Drosphia segment polarity gene armadillo. Similar repeats were later found in the mammalian armadillo homolog beta-catenin, the junctional plaque protein plakoglobin, the adenomatous polyposis coli (APC) tumor suppressor protein and a number of other proteins. The protein encoded by the gene function as a plakophillin 3, which mediates protein-protein interactions and is a member of the armadilloprotein family. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy. The immunohistochemical staining demonstrated that PKP3 is strongly stained in the cytoplasm of squamous cell carcinoma cells. These data suggest that PKP3 may be a promising therapeutic and diagnostic target for treating lung cancers.
NSC 948: TASK-2; this gene encodes an ion transporter domain, signal peptide (SOSUI). This gene encodes a protein belonging to the superfamily of potassium channel proteins containing two pore-forming P domains. mRNA of this gene is mainly expressed in the cortical distal tubules and collecting ducts of the kidney. The protein encoded by the gene is highly sensitive to eternal pH and this, in combination with its expression pattern, suggests that it may play an important role in renal potassium transport. This transmembrane protein is supposed to be overexpressed on the surface of tumor cells but not on normal cells. Thus, the protein may serve as a good target for receptor-targeted therapy or diagnosis.
NSC 956: SIAHBP1; this gene encodes an RNA recognition motif (RRM) domain, which is known as a nucleic acid binding domain. The protein encoded by this gene is a Ro RNS-binding protein. It interacts with Ro RNPs to activate the function of Ro RNPs. The protein also forms a ternary complex with a far upstream element (FUSE) and FUSE-binding protein. It can repress a c-myc reporter via the binding with FUSE. The transcription factor IIH is also known as the target of the protein and the protein inhibits activated transcription. This gene is implicated in the xeroderma pigmentosum disorder. Two alternatively spliced transcript variants exist for this gene that encode different isofonns. Multiple polyadenyllation sites seem to exist on this gene. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 991: DOLPP1; this gene encodes a transmembrane domain and an acid phosphatase homologues domain. The transmembrane protein encoded by the gene is supposed to be overexpressed on the surface of tumor cells but not on normal cells. Thus, the protein may serve as a good target for receptor-targeted therapy or diagnosis.
NSC 994: DKFZP434E2318; this gene encodes a BTB/POZ domain and a Kelch domain. The BTB/POZ domain is known to be a protein-protein interaction motif. The BTB/POZ domain mediates homomeric dimerization and also, in some instances, heteromeric dimerization. The POZ domain of several zinc finger proteins have been shown to mediate transcriptional repression and to interact components of histone deacetylase co-repressor complexes including N-CoR and SMART. The Kelch domain is a beta propeller domain involved in protein-protein interactions and have some enzymatic activities like gycolate oxidase. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 1000: PSK-1; this gene encodes a signal peptide, a CUB domain, a Sushi domain (SCR repeat) and one transmembrane domain. The protein encoded by the gene is highly similar to murin Sez6, an adhesion protein which contains five sushi (SCR) domains and an extracellular CUB domain. This transmembrane protein is supposed to be overexpressed on the surface of tumor cells but not on normal cells. Thus, the protein may serve as a good target for receptor-targeted therapy or diagnosis.
NSC 1066: MCM8; this gene encodes an ATPase associated with a variety of cellular activities (AAA) domain and a minichromosome maintenance protein (MCM) domain. The lower expression of this gene in normal tissues and high expression in NSCLCs suggest that this gene might be useful as a novel diagnostic marker and target for new drugs and immunotherapy.
NSC 1075: URLC4; no known domain was detected to be encoded by the gene. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 1103: KCNK1; this gene encodes a protein belonging to the superfamily of potassium channel proteins containing two pore-forming P domain. The product of this gene has not been shown to be a functional channel. Other non-pore-forming proteins may be necessary for the activity as the functional channel. This transmembrane protein is supposed to be overexpressed on the surface of tumor cells but not on normal cells. Thus, the protein may serve as a good target for receptor-targeted therapy or diagnosis.
NSC 1107: URLC8; this gene encodes a double-strand RNA binding motif (DSRM) domain. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 1113: URLC5; no known domain was detected to be encoded by the gene. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 1131:SYNJ2BP; this gene encodes a PDZ transmembrane domain. The protein encoded by the gene may be a membrane-targeted signaling protein, containing a PDZ domain. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 1141: URLC11; this gene encodes 9 transmembrane domains. The transmembrane protein encoded by the gene is supposed to be overexpressed on the surface of tumor cells but not on normal cells. Thus, the protein may serve as a good target for receptor-targeted therapy or diagnosis.
NSC 1142: NAPG; no known domain was detected to be encoded by the gene. The sequence of the predicted 312-amino acid human protein encoded by *NAPG* is 95% identical to bovine gamma-SNAP. The NAPG protein mediates platelet exocytosis and controls the membrane fusion event of this process. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy. NSC 1164:NPTX1; this gene encodes a Pentaxin/C-reactive protein. *NPTX1* is a member of the neuronal pentraxin gene family. Neuronal pentraxin 1 is similar to the rat NP1 gene which encodes a binding protein for the snake venom toxin taipoxin. The protein encoded by the gene was mainly detected in the cytoplasmic granules by the immunocytochemical analysis. The lower expression of this gene in normal tissues and high expression in NSCLCs suggest that this gene might be useful as a diagnostic marker (i.e., for diagnosis using serum or sputum) and therapeutic target for NSCLCs. The immunohistochemical staining demonstrated that NPTX1 is strongly stained in the cytoplasm of adenocarcinoma cells. These data suggested that NPTX1 might be a promising therapeutic and diagnostic target for treating lung cancers.
NSC 1183: BYSL; no known domain was detected to be encoded by the gene. The protein encoded by the gene has a function of bystin, which forms a cell adhesion molecule complex with trophinin (TRO) and TASTIN that may be important for the embryo implantation. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 1185: URLC6; this gene encodes a zinc finger RNA recognition motif domain. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 1191: COX17; the protein encoded by this gene localizes in the mitochondrial intermembrane space (by similarity) and may function to transport copper to the mitochondria. Further, the protein may be required for the expression of cytochrome oxidase. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 1201: SLC7A1; this gene encodes 14 transmembrane domains. The protein encoded by this gene has strong similarity to the murine Rec-1 (Atrc 1) that functions as a cationic amino acid transporter (ecotropic retroviral receptor) which transports arginine, lysine and ornithine across the plasma membrane. This protein was mainly detected in the cytoplasmic membrane and golgi by the immunocytochemical analysis. This transmembrane protein is supposed to be overexpressed on the surface of tumor cells but not on normal cells. Thus, the protein may serve as a good target for receptor-targeted therapy or diagnosis.
NSC 1240: FLJ00159; this gene encodes 4 transmembrane domains. The transmembrane protein encoded by this gene is supposed to be overexpressed on the surface of tumor cells but not on normal cells. Thus, the protein may serve as a good target for receptor-targeted therapy or diagnosis.
NSC 1246: SUPT3H; this gene encodes a transcription initiation factor αD, a 18kD subunit The family including the protein encoded by the gene includes the Spt3 yeast transcription factors and the 18kD subunit of the human transcription initiation factor αD (TFαD-18). The determination of the crystal structure revealed an atypical histone fold. The lower expression of this gene in normal tissues and high expression in NSCLCs suggest that this gene might be useful as a novel diagnostic marker and target for new drugs and immunotherapy.
NSC 1254: FLJ10815; this gene encodes a transmebmrane amino acid transporter protein. This transmembrane region is found in many amino acid transporters including UNC-47 and MTR. UNC-47 encodes a vesicular amino butyric acid (GABA) transporter, (VGAT). The protein encode by the gene has a function a little similar to the membrane transporters of the amino acid/auxin permease (AAAP) family. This transmembrane protein is supposed to be overexpressed on the surface of tumor cells but not on normal cells. Thus, the protein may serve as a good target for receptor-targeted therapy or diagnosis.
(NSC 1265: SLC28A2; this gene encodes an Na+ dependent nucleoside transporter. The protein encoded by this gene functions as a sodium-coupled nucleoside transporter 2, which transports purine nucleosides and uridine. This transmembrane protein is supposed to be overexpressed on the surface of tumor cells but not on normal cells. Thus, the protein may serve as a good target for receptor-targeted therapy or diagnosis.
NSC 1273: FLJ32549; no known domain was detected to be encoded by the gene. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 1288: PTGFRN; this gene encodes a signal peptide, six immunoglobulin domains and one-transmembrane domain. The protein encoded by this gene inhibits the binding of prostaglandin f2-alpha (pgf2-alpha) to its specific fp receptor by decreasing the receptor number rather than the affinity constant The protein seems to functionally couple with the prostaglandin f2-alpha receptor. This protein was mainly detected in the cytoplasmic membrane and golgi by the immunocytochemical analysis. This transmembrane protein is supposed to be overexpressed on the surface of tumor cells but not on normal cells. Thus, the protein may server as a good target for receptor-targeted therapy or diagnosis
NSC 1292: C17orf26; this gene encodes 3 transmembrane domains, a Zinc transporter domain and a signal peptide (SOSUI). The transmembrane protein encoded by the gene is supposed to be overexpressed on the surface of tumor cells but not on normal cells. Thus, the protein may serve as a good target for receptor-targeted therapy or diagnosis.
NSC 1295:ADAM8; this gene encodes a protein homologous to snake disintegrins, Reprolysin family propeptide and Reprolysin (M12B) family zinc metalloprotease. Members of the ADAM family are cell surface proteins with a unique structure possessing both potential adhesion and protease domains. The extracellular region of ADAM8 shows significant amino acid sequence homology to the hemorrhagic snake venom proteins, including the metalloprotease and disintegrin domains. The lower expression of this gene in normal tissues and high expression in NSCLCs suggest that this gene might be useful as a diagnostic marker (i.e., for diagnosis using serum or sputum) and therapeutic target for NSCLCs. The immunohistochemical staining demonstrated that ADAM8 is strongly stained in adenocarcinoma cells. These data suggested that ADAM8 might be a promising therapeutic and diagnostic target for treating lung cancers.
NSC 1306:ABCA4; this gene encodes a signal peptide and an AAA domain. The membrane-associated protein encoded by this gene is a member of the superfamily of ATP-binding cassette (ABC) transporters. The ABC proteins transport various molecules across extra-and intracellular membranes. The ABC genes are divided into seven distinct subfamilies (ABC1, MDR/IAP, MRP, ALD, OABP, GCN20 and White). The protein encoded by this gene is a member of the ABC1 subfamily. Members of the ABC1 subfamily comprise the only major ABC subfamily found exclusively in multicellular eukaryotes. This protein is a retina-specific ABC transporter which uses N-retinylidene-PE as a substrate. The protein is exclusively expressed in retina photoreceptor cell, indicating the gene product mediates transport of an essential molecule across the photoreceptor cell membrane. Mutations in this gene are found in patients who are diagnosed as having Stargardt disease and are associated with retinitis pigmentosa-19 and macular degeneration age-related 2. This transmembrane protein is supposed to be overexpressed on the surface of tumor cells but not on normal cells. Thus, the protien may serve as a good target for receptor-targeted therapy or diagnosis.
NSC 1343: GPR49; this gene encodes a signal peptide, a Leucine rich repeat N-terminal domain and a 7 transmembrane receptor (rhodopsin family). The protein encoded by this gene belongs to the G protein-coupled receptor family, which members have a large extracellular region containing leucine-rich receptor. This transmembrane protein is supposed to be overexpressed on the surface of tumor cells but not on normal cells. Thus, the protein may serve as a good target for receptor-targeted therapy or diagnosis.
NSC 1362: SCAMP5; this gene encodes 4 transmembrane domains. The transmembrane protein encoded by the gene is supposed to be overexpressed on the surface of tumor cells but not on normal cells. Thus, the protein may serve as a good target for receptor-targeted therapy or diagnosis.
NSC 1389: NMU; this gene encodes an NMU domain. Like most active peptides, the protein encoded by this gene is proteolytically processed from a larger precursor protein. The mature peptides of the protein are 8 to 25 residues long and its C-terminus is amidated. The protein stimutates muscle contractions, specifically, that of the gastrointestinal tract and inhibit feeding. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy. According to the present study, the NMU protein secreted into the culture medium by stable transfectant, or the active form of the NMU peptides added into the medium promoted the growth of COS-7 cells in a dose dependent manner. According to the immunohistochemical staining, the NMU protein was strongly stained in the cytoplasm of both adenocarcinoma and squamous cell carcinoma cells. These data revealed that NMU might be an important autocrine growth factor for NSCLC and suggested that a promising therapeutic and diagnostic strategy for treating lung cancers may be developed by focusing on the NMU ligand-receptor system. Furthermore, the suppression of NMU induced apoptosis in LC319 cells. Moreover, the suppression of NMU protein by anti-NMU antibody induced growth suppression compared with controls in LC319 cells. These results suggest that lung cancer may be treated using antibody or siRNA targeting NMU.
NSC 1395: FBN2; this gene encodes a Calcium-binding EGF-like (EGF CA) domain and an EGF like (unclassified subfamily) domain. The protein encoded by this gene functions as a fibrillin 2, which is an extracellular matrix protein that may regulate the formation and maintenance of extracellular microfibrils. Mutations in FBN2 may cause congenital contractual arachnodactyly. The lower expression of this gene in normal tissues, high expression in NSCLCs, and reduced growth, proliferation and/or survival of the transfected cells by the suppression of this gene suggest that this gene might be useful as a novel diagnostic marker and target for new drugs and immunotherapy.
NSC 1420: CHDOL; this gene encodes a type I membrane protein with a carbohydrate recognition domain that is characteristic of C-type lectins in its extracellular portion. In other proteins, this domain is involved in endocytosis of glycoproteins and exogenous sugar-bearing pathogens. The protein encoded by this gene predominantly localizes to the perinuclear region. This protein was mainly detected in the cytoplasmic membrane and golgi by the immunocytochemical analysis. This transmembrane protein is supposed to be overexpressed on the surface of tumor cells but not on normal cells. Thus, the protein may serve as a good target for receptor-targeted therapy or diagnosis.
NSC 1441: HSNOV1; this gene encodes an intergral membrane protein DFU6 domain. The protein encoded by this gene was mainly detected in the cytoplasmic membrane and golgi by the immunocytochemical analysis. This transmembrane protein is supposed to be overexpressed on the surface of tumor cells but not on normal cells. Thus, the protein may serve as a good target for receptor-targeted therapy or diagnosis.

### Industrial Applicability

The gene-expression analysis on non-small cell lung cancer described herein, obtained through a combination of laser-capture dissection and genome-wide cDNA microarray, has identified specific genes as targets for prevention and therapy of non-small cell lung cancer. Based on the expression of a subset of these differentially expressed genes, the present invention provides molecular diagnostic markers for identifying or detecting non-small cell lung cancer.

The methods described herein are also useful in the identification of additional molecular targets for prevention, diagnosis and treatment of non-small cell lung cancer. The data reported herein add to a comprehensive understanding of non-small cell lung cancer, facilitate development of novel diagnostic strategies and provide clues for identification of molecular targets for therapeutic drugs and preventative agents. Such information contributes to a more profound understanding of carcinogenesis, and provides indicators for developing novel strategies for diagnosis, treatment and ultimately prevention of non-small cell lung cancer.

While the invention has been described in detail and with reference to specific embodiments thereof, it will be apparent to one skilled in the art that various changes and modifications can be made therein without departing from the spirit and scope of the invention.

Furthermore, the present invention relates to the following items:
1. A method of diagnosing non-small cell lung cancer or a predisposition to developing non-small cell lung cancer in a subject, comprising determining an expression level of a non-small cell lung cancer-associated gene in a biological sample derived from the subject, wherein an increase or decrease of said level compared to a normal control level of said gene indicates that said subject suffers from or is at risk of developing non-small cell lung cancer.
2. The method of item 1, wherein said non-small cell lung cancer-associated gene is selected from the group consisting of NSC 807-1448, wherein an increase in said level compared to a normal control level indicates said subject suffers from or is at risk of developing non-small cell lung cancer.
3. The method of item 2, wherein said increase is at least 10% greater than said normal control level.
4. The method of item 1, wherein said non-small cell lung cancer-associated gene is selected from the group consisting of NSC 1-806, wherein a decrease in said level compared to a normal control level indicates said subject suffers from or is at risk of developing non-small cell lung cancer.
5. The method of item 4, wherein said decrease is at least 10% lower than said normal control level.
6. The method of item 1, wherein said method further comprises determining said level of a plurality of non-small cell lung cancer-associated genes.
7. The method of item 1, wherein said level is determined by any one method select from the group consisting of:
   (1) detecting the mRNA of the non-small cell lung cancer-associated genes;
   (2) detecting the protein encoded by the non-small cell lung cancer-associated genes; and
   (3) detecting the biological activity of the protein encoded by the non-small cell lung cancer-associated genes.
8. The method of item 1, wherein said level is determined by detecting hybridization of a non-small cell lung cancer-associated gene probe to a gene transcript of said patient-derived biological sample.
9. The method of item 8, wherein said hybridization step is carried out on a DNA array.
10. The method of item 1, wherein said biological sample comprises sputum or blood.
11. A non-small cell lung cancer reference expression profile, comprising a pattern of gene expression of two or more genes selected from the group consisting of NSC 1-1448.
12. A method of identifying a compound that inhibits the expression or activity of a non-small cell lung cancer-associated gene, comprising the steps of:
   (1) contacting a test cell expressing said non-small cell lung cancer-associated gene with a test compound;
   (2) detecting the expression level of said non-small cell lung cancer-associated gene; and
   (3) determining the compound that suppresses said expression level compared to a normal control level of said gene as an inhibitor of said non-small cell lung cancer-associated gene.
13. The method of item 12, wherein said test cell is NSCLC cell.
14. A method of identifying a compound that enhances the expression or activity of a non-small cell lung cancer-associated gene, comprising the steps of:
   (1) contacting a test cell expressing said non-small cell lung cancer-associated gene with a test compound;
   (2) detecting the expression level of said non-small cell lung cancer-associated gene; and
   (3) determining the compound that increases said expression level compared to a normal control level of said gene as an enhancer of said non-small cell lung cancer-associated gene.
15. The method of item 14, wherein said test cell is NSCLC cell.
16. A method of screening for a compound for treating or preventing non-small cell lung cancer, said method comprising the steps of:
   (1) contacting a test compound with a polypeptide encoded by a polynucleotide selected from the group consisting of NSC 1-1448;
   (2) detecting the binding activity between the polypeptide and the test compound; and
   (3) selecting a compound that binds to the polypeptide.
17. A method of screening for a compound for treating or preventing non-small cell lung cancer, said method comprising the steps of:
   (a) contacting a test compound with a polypeptide encoded by a polynucleotide selected from the group consisting of NSC 1-1448;
   (b) detecting the biological activity of the polypeptide of step (a); and
   (c) selecting a compound that suppresses the biological activity of the polypeptide encoded by the polynucleotide selected from the group consisting of NSC 807-1448 in comparison with the biological activity detected in the absence of the test compound, or enhances the biological activity of the polypeptide encoded by the polynucleotide selected from the group consisting of NSC 1-806 in comparison with the biological activity detected in the absence of the test compound.
18. The method of item 17, wherein said biological activity is cell proliferative activity.
19. A method of screening for a compound for treating or preventing non-small cell lung cancer, said method comprising the steps of:
   (1) contacting a test compound with a cell expressing one or more marker genes, wherein the one or more marker genes is selected from the group consisting of NSC 1-1448; and
   (2) selecting a compound that reduces the expression level of one or more marker genes selected from the group consisting of NSC 807-1448, or elevates the expression level of one or more marker genes selected from the group consisting of NSC 1-806.
20. The method of item 19, wherein said cell is NSCLC cell.
21. A method of screening for compound for treating or preventing non-small cell lung cancer, said method comprising the steps of:
   (1) contacting a test compound with a cell into which a vector comprising the transcriptional regulatory region of one or more marker genes and a reporter gene that is expressed under the control of the transcriptional regulatory region has been introduced, wherein the one or more marker genes are selected from the group consisting of NSC 1-1448;
   (2) measuring the activity of said reporter gene; and
   (3) selecting a compound that reduces the expression level of said reporter gene when said marker gene is an up-regulated marker gene selected from the group consisting of NSC 807-1448 or that enhances the expression level of said reporter gene when said marker gene is a down-regulated marker gene selected from the group consisting of NSC 1-806, as compared to a control.
22. A kit comprising two or more detection reagents which binds to one or more genes selected from the group consisting of NSC 1-1448 or polypeptides encoded thereby.
23. An array comprising two or more polynucleotides which bind to one or more genes selected from the group consisting of NSC 1-1448.
24. A method of treating or preventing non-small cell flung cancer in a subject comprising administering to said subject an antisense composition, said composition comprising a nucleotide sequence complementary to a coding sequence of a gene selected from the group consisting of NSC 807-1448.
25. A method of treating or preventing non-small cell lung cancer in a subject comprising administering to said subject an siRNA composition, wherein said composition reduces the expression of a gene selected from the group consisting of NSC 807-1448.
26. A method for treating or preventing non-small cell lung cancer in a subject comprising the step of administering to said subject a pharmaceutically effective amount of an antibody or fragment thereof that binds to a polypeptide encoded by a gene selected from the group consisting of NSC 807-1448.
27. A method of treating or preventing non-small cell lung cancer in a subject comprising administering to said subject a vaccine comprising a polypeptide encoded by a gene selected from the group consisting of NSC 807-1448 or an immunologically active fragment of said polypeptide, or a polynucleotide encoding the polypeptide.
28. A method of treating or preventing non-small cell lung cancer in a subject comprising administering to said subject a compound that increases the expression or activity of a gene selected from the group consisting of NSC 1-806.
29. A method for treating or preventing non-small cell lung cancer in a subject, said method comprising the step of administering a compound that is obtained by the method according to any one of items 12-21.
30. A method of treating or preventing non-small cell lung cancer in a subject comprising administering to said subject a pharmaceutically effective amount of a polynucleotide select from the group consisting of NSC 1-806, or polypeptide encoded thereby
31. A composition for treating or preventing non-small cell lung cancer, said composition comprising a pharmaceutically effective amount of an antisense polynucleotide or siRNA against a gene selected from the group consisting of NSC 807-1448.
32. A composition for treating or preventing non-small cell lung cancer, said composition comprising a pharmaceutically effective amount of an antibody or fragment thereof that binds to a polypeptide encoded by a gene selected from the group consisting of NSC 807-1448.
33. A composition for treating or preventing non-small cell lung cancer, said composition comprising a pharmaceutically effective amount of the compound selected by the method of any one of items 12-21 as an active ingredient, and a pharmaceutically acceptable carrier.
34. An substantially pure polypeptide selected from the group consisting of:
   (a) a polypeptide comprising an amino acid sequence of SEQ ID NO: 2;
   (b) a polypeptide that comprises the amino acid sequence of SEQ ID NO: 2 in which up to 5% of the amino acids are substituted, deleted, inserted and/or added and that has a biological activity equivalent to a protein consisting of the amino acid sequence of SEQ ID NO: 2; and
   (c) a polypeptide encoded by a polynucleotide that hybridizes under stringent conditions to a gene consisting of the nucleotide sequence of SEQ ID NO: 1 wherein the polypeptide has a biological activity equivalent to a protein consisting of the amino acid sequence of SEQ ID NO: 2.
35. An isolated polynucleotide encoding the polypeptide of item 34.
36. The isolated polynucleotide of item 35 comprising the nucleotide sequence of SEQ ID NO: 1.
37. A vector comprising the polynucleotide of item 35.
38. A host cell harboring the polynucleotide of item 35 or a vector comprising the polynucleotide.
39. A method for producing the polypeptide of item 34, said method-comprising the steps of:
   (1) culturing the host cell harboring a polynucleotide encoding the polypeptide of item 34 or a vector comprising the polynucleotide;
   (2) allowing the host cell to express the polypeptide; and
   (3) collecting the expressed polypeptide.
40. An antibody binding to the polypeptide of item 34.
41. A polynucleotide that is complementary to the polynucleotide of item 35 or to the complementary strand thereof and that comprises at least 15 nucleotides.
42. An antisense polynucleotide or siRNA against the polynucleotide of item 35.
43. An antisense polynucleotide selected from the group consisting of polynucleotides comprising the nucleotide sequence of SEQ ID NO: 423, 425, 427, 429, 431, 433, 435, 437, 439, 441, 443, 445, 447, 449, 451, 453, 455, 457, 459, 461, 463, 465, 467, 469, 471, 473, 475, 477, 479, 481, 483, 485, 487, 489, 491, 493, 495, 497, 499, 501, 503, 505, 507, 509, 511, 513, 515, 517, 519, 521, 523, 525, 527, 529, or 531.
44. An siRNA selected from the group consisting of the polynucleotides comprising the nucleotide sequence of SEQ ID NO: 533, 534, 535, 536, 537, 538, 539, 540, 541, 542, 543, 544, 545, 546, 547, 548, 549, 550, 551, and 552 as the target sequence.
45. A composition for treating or preventing non-small cell lung cancer, said composition comprising a pharmaceutically effective amount of the antisense polynucleotide of item 43.
46. A composition for treating or preventing non-small cell lung cancer, said composition comprising a pharmaceutically effective amount of the siRNA of item 44.
47. A method for treating or preventing non-small cell lung cancer in a subject comprising administering to said subject the antisense composition of item 45.
48. A method for treating or preventing non-small cell lung cancer in a subject comprising administering to said subject the siRNA composition of item 46.
49. A pharmaceutical composition for treating or preventing a non-small cell lung cancer, said composition comprising a pharmaceutically effective amount of the polypeptide of claim 34, or a polynucleotide encoding the polypeptide.
50. The pharmaceutical composition of item 49, wherein the polynucleotide is incorporated in an expression vector.
51. A method for inducing anti tumor immunity, said method comprising the Step of administering a polypeptids encoded by a gene selected from the group consisting of NSC 807-1448 or an immunologically active fragment of said polypeptide, or a polynucleotide encoding the polypeptide or fragment.
52. The method for inducing anti tumor immunity of item 51, wherein the method further comprising the step of administering the antigen presenting cells to a subject.

## Claims

1. A method of diagnosing non-small cell lung cancer or a predisposition to developing non-small cell lung cancer in a subject, comprising determining an expression level of a non-small cell lung cancer-associated gene in a biological sample derived from the subject, wherein an increase or decrease of said level compared to a normal control level of said gene indicates that said subject suffers from or is at risk of developing non-small cell lung cancer, wherein said non-small cell lung cancer-associated gene is NSC 1107 (URLC8), wherein an increase in said level compared to a normal control level indicates said subject suffers from or is at risk of developing non-small cell lung cancer.

2. The method of claim 1, wherein said increase is at least 10% greater than said normal control level.

3. The method of claim 1, wherein said method further comprises determining said level of a plurality of non-small cell lung cancer-associated genes.

4. The method of claim 1, wherein said level is determined by any one method select from the group consisting of:
(1) detecting the mRNA of the non-small cell lung cancer-associated gene;
(2) detecting the protein encoded by the non-small cell lung cancer-associated gene;
and
(3) detecting the biological activity of the protein encoded by the non-small cell lung cancer-associated gene.

5. The method of claim 1, wherein said level is determined by detecting hybridization of a non-small cell lung cancer-associated gene probe to a gene transcript of said patient-derived biological sample.

6. The method of claim 5, wherein said hybridization step is carried out on a DNA array.

7. The method of claim 1, wherein said biological sample comprises lung tissue.

8. A kit for diagnosing non-small cell lung cancer comprises an oligonucleotide that is complementary to NSC 1107 (URLC8) polynucleotide or antibody which binds to polypeptide encoded by NSC 1107 (URLC8) polynucleotide, and positive control reagent for non-small cell lung cancer.
